# EUROPEAN PATENT APPLICATION

(11) **EP 2 465 855 A1**
(43) Date of publication of application: **20.06.2012**
(21) Application number: 12157719.1
(22) Date of filing: 29.08.2007
(51) Int. Cl.: C07D 277/24, C07D 417/12, A61K 31/427, A61K 31/426, A61P 43/00

(54) **Cytochrome P450 oxidase inhibitors and uses thereof**

(30) Priority: 31.08.2006 US 841397 P
(62) Divisional of application: 07814515.8
(71) Applicant: Abbott Laboratories, Abbott Park, Illinois 60064-6008 (US)
(72) Inventor: Klein, Larry, L., Lake Forest, IL Illinois 60045 (US); Chen, Hui-Ju, Grayslake, IL Illinois 60030 (US); Yeung, Ming, C., Grayslake, IL Illinois 60030 (US); Flentge, Charles, A., Salem, WI Wisconsin 53168 (US); Randolph, John, T., Libertyville, IL Illinois 60048 (US); Huang, Peggy, P., Lake Bluff, IL Illinois 60044 (US); Hutchinson, Douglas, K., Antioch, IL Illinois 60002 (US); Kempf, Dale, J., Libertyville, IL Illinois 60048 (US)
(74) Representative: Modiano, Micaela Nadia

(57) **Abstract**

The present invention features compounds of formula (I) or pharmaceutically acceptable salts, solvates thereof, and compounds of formula (I) for use in inhibiting the metabolizing activities of CYP enzymes. The present invention also features compounds of formula (I) for use in improving the pharmacokinetics of drugs that are metabolized by CYP enzymes.

## Description

### CROSS-REFERENCE TO RELATED PATENT APPLICATIONS

This patent application claims priority to U.S. Provisional Patent Application No. 60/841,397 (filed August 31, 2006). The entire text of that application is incorporated by reference into this application.

### TECHNICAL FIELD

The present invention relates to cytochrome P450 oxidase inhibitors and methods of using the same to improve the pharmacokinetics of drugs.

### BACKGROUND OF THE INVENTION

Cytochrome P450 oxidases (CYPs) include a large number of related but distinct oxidative enzymes. These enzymes are often membrane-bound, either in the inner membrane of mitochondria or in the endoplasmic reticulum of cells, where they metabolize a variety of endogenous and exogenous molecules. CYP enzymes are also involved in the synthesis of cholesterol, steroids, and other lipids. At least 18 CYP gene families have been identified in human. A typical human Cytochrome P450 oxidase has about 500 amino acid residues and a heme group at the active site.

Many drugs are metabolized by CYPs. This often leads to unfavorable pharmacokinetics of the drugs and the need for higher and more frequent doses than are most desirable. Therefore, there is a need for new compounds that can inhibit the enzymatic activities of CYPs, thereby improving the pharmacokinetics of drugs.

### BRIEF SUMMARY OF THE INVENTION

The present invention features compounds of formula I, 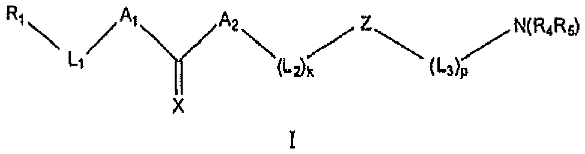 or pharmaceutically acceptable salts, solvates or prodrugs thereof, wherein
R₁ is a 3-, 4-, 5-, 6-, 7-, 8-, 9-, or 10-membered heterocyclyl comprising at least one nitrogen ring atom;
L₁ is a bond, C₁-C₁₀alkylene, C₂-C₁₀alkenylene or C₂-C₁₀alkynylene;
A₁ is a bond or selected from the group consisting of -O-L_{A1}-, -S-L_{A1}- and -N(R_{A1})-L_{A1}-, wherein L_{A1} is a bond, C₁-C₁₀alkylene, C₂-C₁₀alkenylene or C₂-C₁₀alkynylene, and R_{A1} is hydrogen, C₁-C₆alkyl, C₂-C₆alkenyl or C₂-C₆alkynyl;
X is O or S;
A₂ is a bond or selected from the group consisting of -L_{A2}-O-, -L_{A2}-S- and -L_{A2}-N(R_{A2})-, wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, carbocyclyl, carbocyclylC₁-C₆alkyl, heterocyclyl, heterocycloC₁C₆alkyl, -L_{E}-carbocyclyl-L₄-Y-L_{4'}-R_{E} and -L_{E}-heterocyclyl-L₄-Y-L_{4'}-R_{E}, wherein L_{D}, L_{E}, L₄ and L_{4'} are each independently selected at each occurrence from a bond, C₁-C₁₀ alkylene, C₂-C₁₀alkenylene or C₁-C₁₀alkynylene, wherein R_{D}, R_{D'} and R_{D''} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₁₀alkylene, C₂-C₁₀alkenylene, C₂-C₁₀alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆alkyl;
k is 0 or 1, and at each occurrence L₂ independently represents -L₉-V-L₉-, wherein L₉ and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, -S-, -O-, -C(O)-, -N(R_{V})C(O)-, -C(O)N(R_{V})-, -C(O)O- and -OC(O)-, wherein R_{V} is independently selected at each occurrence from hydrogen, C₁-C₆ alkyl, C₂-C₆alkenyl or C₂-C₆alkynyl;
Z is -C(R₂R₃₎-, =C(R₂)- or -C(R₂)=, wherein R₂ is selected from the group consisting of carbocyclyl, carbocyclylC₁-C₆alkyl, heterocyclyl, heterocycloC₁-C₆alkyl, -L_{D}-O-R_{E}, -L_{D}-S-R_{E}, -L_{D}-C(O)R_{E}, -L_{D}-OC(O)R_{E}, -L_{D}-C(O)OR_{E}, -L_{D}-N_{D}R_{E}R_{D}, -L_{D}-S(O)R_{E}, -L_{D}-SO₂R_{E}, -L_{D}-C(O)-NR_{D}R_{E}, - L_{D}-N(R_{D})C(O)R_{E}, -L_{D}-N(R_{D})SO₂R_{E}, -L_{D}-N(R_{D})SO₂NR_{D'}R_{E}, -L_{E}-carbocyclyl-L₄-Y-L_{4'}-R_{E} and -L_{E}-heterocyclyl-L₄-Y-L₄-R_{E}, wherein R₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L₄, L_{4'}, Y, L_{D}, L_{E}, R_{E}, R_{D}, R_{D'} and R_{D''} are as defined immediately above;
or Z is selected from the group consisting of 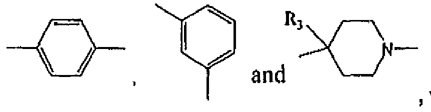 wherein R₃ is as defined immediately above;
or Z, taken together with (L₃)_{P} and N(R₄R₅), forms 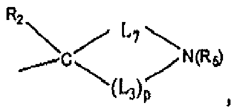 wherein L₇ is C₁-C₁₀alkylene, C₂-C₁₀alkenylene or C₂-C₁₀alkynylene, R₂ is as defined immediately above, and L₃, p and R₅ are as defined immediately below, and wherein 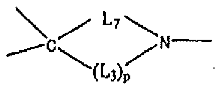 comprises from 3 to 10 ring atoms;
or Z is a bond;
p is an integer selected from 0, 1, 2, or 3, and at each occurrence L₃ independently represents -L₅-W-L_{5'}-, wherein W is independently selected at each occurrence from the group consisting of a bond, C₁-C₁₀alkylene, C₂-C₁₀alkenylene, C₂-C₁₀alkynylene, -S-, -O-, -C(O)-, -N(R_{w})CO-, -C(O)N(R_{w})-, -C(O)O- and -OC(O)-, and R_{w} is independently selected at each occurrence from hydrogen, C₁-C₆alkyl, C₂-C₆alkenyl or C₂-C₆alkynyl, wherein L₅ and L_{5'} are each independently selected at each occurrence from a bond, C₁-C₁₀alkylene, C₂-C₁₀alkenylene or C₂-C₁₀alkynylene, and are each independently optionally substituted at each occurrence with 1, 2, 3 or more substituents each of which is independently selected at each occurrence from the group consisting of halogen, oxo, thioxo, hydroxy, nitro, cyano, amino, formyl, carbocyclyl, heterocyclyl, -O-R_{D}, -S-R_{D}, -C(O)R_{D}, -OC(O)R_{D}, -C(O)OR_{D}, -NR_{D}R_{D'}, -S(O)R_{D}, -SO₂R_{D}, -C(O)NR_{D}R_{D'}, -N(R_{D})C(O)R_{D'}, -N(R_{D})C(O)OR_{D'}, -N(R_{D})SO₂R_{D'}, -N(R_{D})SO₂NR_{D'}R_{D''}, -carbocyclyl-L₄-Y-L_{4'}-R_{E} and -heterocyclyl-L₄-Y-L_{4'}-R_{E}, and wherein R_{D}, R_{D'}, R_{D''}, R_{E}, L₄, L_{4'} and Y are as defined immediately above;
or p is 1, L₃ is -L₅-C(R₆R₇)-L_{5'}-, A₂ is -L_{A2}-NR_{A2}-, and R_{A2} and R₇ are bonded together to form -C(O)O-, wherein R₆ is selected from the group consisting of hydrogen, C₁-C₆alkyl, C₂-C₆alkenyl, C₂-C₆ alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L_{A2}, L_{D}, L_{E}, L₄, L_{4'} Y, R_{E}, R_{D}, R_{D'}, R_{D''}, L₅ and L₅ are as defined immediately above;
or p is 1, L₃ is -L₅-C(R₆R₇)-L_{5'}-, and R₄ and R₇ are bonded together to form -OC(O)-, wherein R₆, L₅ and L₅ are as defined immediately above;
R₄ and R₅, unless otherwise provided, are each independently selected from the group consisting of N-protecting group, hydrogen, C₁-C₆alkyl, C₂-C₆alkenyl, C₂-C₆alkynyl, carbocyclyl, carbocyclylC₁-C₆ alkyl, heterocyclyl, heterocycloC₁-C₆alkyl, -L_{E}-carbocyclyl-L₄-Y-L_{4'}-R_{E}, -L_{E}-heterocyclyl-L₄-Y-L₄-R_{E}, -L₆-O-R₈, -L₆-C(O)OR₈, -L₆-OC(O)R₈, -L₆-C(O)NR₈R₉, -N(R₉)C(O)OR₈, -L₆-C(O) -L₆-O-R₈, -L₆-C(O)-L_{6'}-NR₈R₉, -L₆-C(O)-L₆-N(R₉)C(O)OR₈, -L₆-C(O)-L₆-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₂-C₁₀ alkynylene, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -LE-carbocyclyl-L₄-Y-L_{4'}-R_{E} and -L_{E}-heterocyclyl-L₄-Y-L_{4'}-R_{E}, and wherein L_{D}, R_{D}, R_{D'}, R_{D''}, L_{E}, L₄, L_{4'}, Y and R_{E} are as defined immediately above;
or R₄ and R₅, together with the N attached thereto, form a heterocyclyl;
wherein at each occurrence L₁, L_{A1}, R_{A1}, Y, V, W, R_{Y}, R_{V}, R_{W}, L_{A2}, R_{A2}, L_{D}, L_{E}, L₄, L_{4'}, L₆, L_{6'}, L₇, L₉, L_{9'}, R₂, R₃, R₄, R₅, R₆, R₈, R₉, R₁₀, R_{E}, R_{D}, R_{D'} and R_{D''} are each independently optionally substituted with at least one substituent selected from the group consisting of halogen, oxo, thioxo, hydroxy, nitro, cyano, amino, formyl, carbocyclyl, heterocyclyl, -O-R_{L}, -S-R_{L}, -C(O)R_{L}, -OC(O)R_{L}, -C(O)OR_{L}, -NR_{L}R_{L'}, -S(O)R_{L}, -SO₂R_{L}, -C(O)NR_{L}R_{L'}, -N(R_{L})C(O)R_{L'}, -N(R_{L})SO₂R_{L'}, and -N(R_{L})SO₂NR_{L'}R_{L''}, and wherein R_{L}, R_{L'} and R_{L''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆ alkylaminoC₁-C₆alkyl, carbocyclyl, carbocyclylC₁-C₆alkyl, heterocyclyl and heterocycloC₁-C₆alkyl;
wherein each carbocyclyl moiety (including any optional substitution carbocyclyl) in L₁, A₁, A₂, (L₂)ₖ, Z, (L₃)_{P} and N(R₄R₅) is independently selected at each occurrence from 3-, 4-, 5-, 6-, 7-, 8-, 9- or 10-membered carbocyclyls (e.g., C₃-C₁₀cycloalkyl, C₃-C₁₀cycloalkenyl or C₆-C₁₀aryl), and each heterocyclyl moiety (including any optional substitution heterocyclyl) in L₁, A₁ A₂, (L₂)ₖ, Z, (L₃)ₚ and N(R₄R₅) is independently selected at each occurrence from 3-, 4-, 5-, 6-, 7-, 8-, 9- or 10-membered heterocyclyls (e.g., H₅-H₁₀heteroaryl, H₃-H₁₀heterocycloalkyl or H₃-H₁₀heterocycloalkenyl); and
wherein each carbocyclyl and heterocyclyl moiety in the compound (e.g., in R₁, L₁, A₁, A₂, (L₂)ₖ, Z, (L₃)ₚ or N(R₄R₅), including any optional substitution carbocyclyl or heterocyclyl) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{S}-O-R_{S} -L_{S}-S-R_{S}, -L_{S}-C(O)R_{S}, -L_{S}-OC(O)R_{S}, -L_{S}-C(O)OR_{S}, -L_{S}-NR_{S}R_{S'}, -L_{S}-S(O)R_{S}, -L_{S}-SO₂R_{S}, -LS-C(O)NR_{S}R_{S'}, -L_{S}-N(R_{S})C(O)R_{S'}, -L_{S}-N(R_{S})SO₂R_{S'}, -L_{S}-N(R_{S})SO₂NR_{S'}R_{S''}, carbocyclyl, carbocyclylC₁-C₆alkyl, heterocyclyl and heterocycloC₁-C₆alkyl, wherein L_{S} is independently selected at each occurrence from the group consisting of a bond, C₁-C₁₀alkylene, C₂-C₁₀alkenylene and C₂-C₁₀ alkynylene, and R_{S}, R_{S'} and R_{S''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl, C₁-C₆alkylamino C₁-C₆alkyl, C₃-C₁₀carbocyclyl, C₃-C₁₀carbocyclylC₁-C₆alkyl, H₃-H₁₀heterocyclyl and H₃-H₁₀heterocyclo C₁-C₆alkyl;
with the proviso that if Z is a bond, then A2 is -L_{A2}-NR_{A2}, p is an integer selected from 1, 2 or 3, L₃ at each occurrence independently represents -L₅-W-L_{5'}-, and R_{A2} and R₅ are each independently selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, carbocyclyl, carbocyclylC₁-C₆alkyl, heterocyclyl, heterocycloC₁-C₆alkyl, -L_{E}-carbocyclyl-L₄-Y-L_{4'}-R_{E} and -L_{E}-heterocyclyl-L₄-Y-L₄-R_{E}, wherein L_{A2}, L_{D}, L_{E}, L₄, L_{4'}, Y, R_{E}, R_{D}, R_{D'}, R_{D''}, L₅, W and L_{5'} are as defined immediately above;
with the further proviso that said compound is not ritonavir (i.e., (2S,3S,5S)-5-(N-(N-((N-Methyl-N-((2-isopropyl-4-thiazolyl)methyl)amino)carbonyl)-L-valinyl)amino)-2-(N-((5-thiazolyl)methoxy carbonyl)amino)-1,6-diphenyl-3-hydroxyhexane),

The present invention also features compounds of formula II, 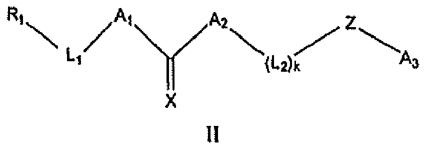 or pharmaceutically acceptable salts, solvates or prodrugs thereof, wherein
R₁ is a 3-, 4-, 5-, 6-, 7-, 8-, 9-, or 10-membered heterocyclyl comprising at least one nitrogen ring atom;
L₁ is a bond, C₁-C₁₀alkylene, C₂-C₁₀alkenylene or C₂-C₁₀alkynylene;
A₁ is a bond or selected from the group consisting of -O-L_{A1}-, -S-L_{A1}-, and -N(R_{A1})-L_{A1}-, wherein L_{A1} is a bond, C₁-C₁₀alkylene, C₂-C₁₀alkenylene or C₂-C₁₀alkynylene, and R_{A1} is hydrogen, C₁-C₆alkyl, C₂-C₆alkenyl or C₂-C₆alkynyl;
X is O or S;
A₂ is a bond or selected from the group consisting of -L_{A2}-O-, -L_{A2}-S- and -L_{A2}-N(R_{A2})-, wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, carbocyclyl, carbocyclylC₁-C₆alkyl, heterocyclyl, heterocycloC₁-C₆alkyl, -L_{E}-carbocyclyl-L₄-Y-L_{4'}-R_{E} and -L_{E}-heterocyclyl-L₄-Y-L₄-R_{E}, wherein L_{D}, L_{E}, L₄ and L_{4'} are each independently selected at each occurrence from a bond, C₁-C₁₀ alkylene, C₂-C₁₀alkenylene or C₂-C₁₀alkynylene, wherein R_{D}, R_{D}, and R_{D''} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₁₀alkylene, C₂-C₁₀alkenylene, C₂-C₁₀alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆ alkyl;
k is 0 or 1, and at each occurrence L₂ independently represents -L₉-V-L₉-, wherein L₉ and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, -S-, -O-, -C(O)-, -N(R_{V})C(O)-, -C(O)N(R_{V})-, -C(O)O- and -OC(O)-, wherein R_{V} is independently selected at each occurrence from hydrogen, C₁-C₆ alkyl, C₂-C₆alkenyl or C₂-C₆alkynyl;
Z is -C(R₂R₃)-, =C(R₂)- or -C(R₂)=, wherein R₂ is selected from the group consisting of carbocyclyl, carbocyclylC₁-C₆alkyl, heterocyclyl, heterocycloC₁-C₆alkyl, -L_{D}-O-R_{E}, -L_{D}-S-R_{E}, -L_{D}-C(O)R_{E}, -L_{D}-OC(O)R_{E}, -L_{D}-C(O)OR_{E}, -L_{D}-N_{D}R_{E}R_{D}, -L_{D}-S(O)R_{E}, -L_{D}-SO₂R_{E}, -L_{D}-C(O)NR_{D}R_{E}, -L_{D}-N(R_{D})C(O)R_{E}, -L_{D}-N(R_{D})SO₂R_{E}, -L_{D}-N(R_{D})SO₂NR_{D'}R_{E}, -L_{E}-carbocyclyl-L₄-Y-L₄-R_{E} and -L_{E}-heterocyclyl-L₄-Y-L_{4'}-R_{E}, wherein R₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heteracycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L₄, L_{4'}, Y, L_{D}, L_{E}, R_{E}, R_{D}, R_{D'} and R_{D''} are as defined immediately above in this aspect;
or Z is selected from the group consisting of 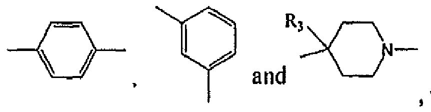 wherein R₃ is as defined immediately above in this aspect; A₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{E}-carbocyclyl-L₄-Y-L_{4'}-R_{E}, -L_{E}-heterocyclyl-L₄-Y-L_{4'}-R_{E}, -L₆-O-R₈, -L₆-C(O)R₈, -L₆-C(O)OR₈, -L₆-OC(O)R₈, -L₆-C(O)NR₈R₉, -L₆-N(R₈)-C(O)R₉, -L₆-N(R₉)C(O)OR₈, -L₆-NR₈R₉, -L₆-C(O)-L_{6'}-NR₈R₉, -L₆-C(O)-L_{6'}-N(R₉)C(O)OR₈, -L₆-C(O)-L_{6'},-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₁-C₁₀alkynylene, wherein R₈, R₉ and R₁₀ are each independently selected at each occurrence from the group consisting of hydrogen, C₁-C₆alkyl, C₂-C₆ alkenyl, C₂-C₆alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L₄-R_{E} and -L_{E}-heterocyclyl-L₄-Y-L_{4'}-R_{E}, and wherein L_{D}, R_{D}, R_{D'}, R_{D''}, L_{E}, L₄, L_{4'}, Y and R_{E} are as defined immediately above in this aspect;
wherein at each occurrence L₁, L_{A1}, R_{A1}, Y, V, R_{Y}, R_{V}, L_{A2}, R_{A2}, L_{D}, L_{E}, L₄, L_{4'}, L₆, L_{6'}, L₉, L_{9'}, R₂, R₃, R₈, R₉, R₁₀, R_{E}, R_{D}, R_{D'}, R_{D''} and A₃ are each independently optionally substituted with at least one substituent selected from the group consisting of halogen, oxo, thioxo, hydroxy, nitro, cyano, amino, formyl, carbocyclyl, heterocyclyl, -O-R_{L}, -S-R_{L}, -C(O)R_{L}, -OC(O)R_{L}, -C(O)OR_{L}, -NR_{L}R_{L'}, -S(O)R_{L}, -SO₂R_{L}, -C(O)NR_{L}R_{L'}, -N(R_{L})C(O)R_{L'}, -N(R_{L})SO₂R_{L'} and -N(R_{L})SO₂NR_{L'}R_{L''}, and wherein R_{L}, R_{L'} and R_{L''} are each independently selected at each occurrence from the group consisting of hydrogen, C₁-C₆ alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkyl carbonyl, C₁-C₆alkylcarbonylC₁-C₆alkyl, C₁-C₆alkoxycarbonyl, C₁-C₆alkoxycarbonylC₁-C₆alkyl, C₁-C₆ alkylcarbonyloxy, C₁-C₆alkylcarbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl;
wherein each carbocyclyl moiety in L₁, A₁, A₂, (L₂)ₖ, Z and A₃ is independently selected at each occurrence from 3-, 4-, 5-, 6-, 7-, 8-, 9- or 10-membered carbocyclyls, and each heterocyclyl moiety in L₁, A₁, A₂, (L₂)ₖ, Z and A₃ is independently selected at each occurrence from 3-, 4-, 5-, 6-, 7-, 8-, 9- or 10-membered heterocyclyls; and
wherein each carbocyclyl and heterocyclyl moiety in the compound (e.g., in L₁, A₁, A₂, (L₂)ₖ, Z and A₃, including optional substitution carbocyclyl or heterocyclyl) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{S}-O-R_{S}, - L_{S}-S-R_{S}, -L_{S}-C(O)R_{S}, -L_{S}-OC(O)R_{S}, -L_{S}-C(O)OR_{S}, -L_{S}-NR_{S}R_{S'}, -L_{S}-S(O)R_{S}, -L_{S}-SO₂R_{S}, -L_{S}-C(O)NR_{S}R_{S'}, -L_{S}-N(R_{S})C(O)R_{S'}, -L_{S}-N(R_{S})SO₂R_{S'}, -L_{S}-N(R_{S})SO₂NR_{S'}R_{s''}, carbocyclyl, carbocyclylC₁-C₆alkyl, heterocyclyl and heterocycloC₁-C₆alkyl, wherein at each occurrence L_{S} is independently selected from a bond, C₁-C₁₀alkylene, C₂-C₁₀alkenylene or C₂-C₁₀alkynylene, and R_{S}, R_{S'}, and R_{S''} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆alkylaminoC₁-C₆alkyl, C₃-C₁₀carbocyclyl, C₃-C₁₀carbocyclylC₁-C₆alkyl, H₃-H₁₀heterocyclyl and H₃-H₁₀heterocycloC₁₋C₆alkyl;
with the proviso that said compound is not ritonavir.

The present invention also features pharmaceutical compositions comprising the above described compounds, salts, solvates, and prodrugs.

The present invention further features methods of use of the above described compounds, salts, solvates, and prodrugs to, for example, inhibit a metabolizing activity of a CYP enzyme, improve pharmacokinetics of a drug that is metabolizable by a CYP enzyme, or increase blood or liver level of a drug that is metabolizable by a CYP enzyme.

### DETAILED DESCRIPTION OF THE INVENTION

The present invention features compounds capable of inhibiting cytochrome P450 oxidases, such as CYP3A4, CYP2D6 and CYP2C9. The present invention also features methods of using these compounds to improve the pharmacokinetics of drugs that are metabolizable by CYP enzymes.

In one aspect, the present invention features compounds of formula I, 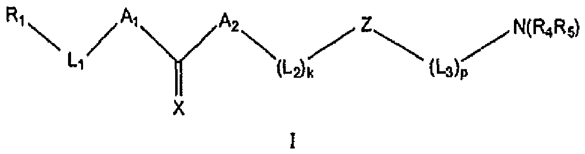 or pharmaceutically acceptable salts, solvates or prodrugs thereof, wherein
R₁ is a 3-, 4-, 5-, 6-, 7-, 8-, 9-, or 10-membered heterocyclyl comprising at least one nitrogen ring atom;
L₁ is a bond, C₁-C₁₀alkylene, C₁-C₁₀alkenylene or C₂-C₁₀alkynylene;
A₁ is a bond or selected from the group consisting of -O-L_{A1}-, -S-L_{A1}- and -N(R_{A1})-L_{A1}-, wherein L_{A1} is a bond, C₁-C₁₀alkylene, C₂-C₁₀alkenylene or C₂-C₁₀alkynylene, and R_{A1} is hydrogen, C₁-C₆alkyl, C₂-C₆alkenyl or C₂-C₆alkynyl;
X is O or S;
A₂ is a bond or selected from the group consisting of -L_{A2}-O-, -L_{A2}-S- and -L_{A2}-N(R_{A2})-, wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, carbocyclyl, carbocyclylC₁-C₆alkyl, heterocyclyl, hoterocycloC₁-C₆alkyl, -L_{E}-carbocyclyl-L₄-Y-L_{4'}-R_{E} and -L_{E}-heterocyclyl-L₄-Y-L_{4'}-R_{E}, wherein L_{D}, L_{E}, L₄ and L_{4'} are each independently selected at each occurrence from a bond, C₁-C₁₀ alkylene, C₂-C₁₀alkenylene or C₂-C₁₀alkynylene, wherein R_{D}, R_{D'} and R_{D''} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₁₀alkylene, C₂-C₁₀alkenylene, C₂-C₁₀alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆alkyl;
k is 0 or 1, and at each occurrence L₂ independently represents -L₉-V-L_{9'}-, wherein L₉ and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, -S-, -O-, -C(O)-, -N(R_{V})C(O)-, -C(O)N(R_{V})-, -C(O)O- and -OC(O)-, wherein R_{V} is independently selected at each occurrence from hydrogen, C₁-C₆ alkyl, C₂-C₆alkenyl or C₂-C₆alkynyl;
Z is -C(R₂R₃)-, =C(R₂)- or -C(R₂)=, wherein R₂ is selected from the group consisting of carbocyclyl, carbocyclylC₁-C₆alkyl, heterocyclyl, heterocycloC₁-C₆alkyl, -L_{D}-O-R_{E}, -L_{D}-S-R_{E}, -L_{D}-C(O)R_{E}, -L_{D}-OC(O)R_{E}, -L_{D}-C(O)OR_{E}, -L_{D}-N_{D}R_{E}R_{D}, -L_{D}-S(O)R_{E}, -L_{D}-SO₂R_{E}, -L_{D}-C(O)NR_{D}R_{E}, -L_{D}-N(R_{D})C(O)R_{E}, -L_{D}-N(R_{D})SO₂R_{E}, -L_{D}-N(R_{D)}SO₂NR_{D'}R_{E}, -L_{E}-carbocyclyl-L₄-Y-L_{4'}R_{E} and -L_{E}-heterocyclyl-L₄-Y-L₄-R_{E}, wherein R₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{D}- O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}. -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L₄, L_{4'}, Y, L_{D}, L_{E}, R_{E}, R_{D}, R_{D'} and R_{D''} are as defined immediately above;
or Z is selected from the group consisting of 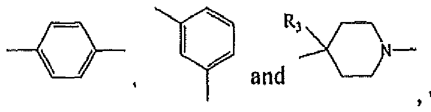 wherein R₃ is as defined immediately above;
or Z, taken together with (L₃)ₚ and N(R₄R₅), forms 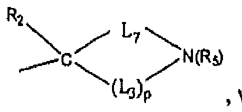 wherein L₇ is C₁-C₁₀alkylene, C₂-C₁₀alkenylene or C₂-C₁₀alkynylene, R₂ is as defined immediately above, and L₃, p and R₅ are as defined immediately below, and wherein 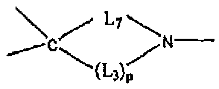 comprises from 3 to 10 ring atoms;
or Z is a bond;
p is an integer selected from 0, 1, 2, or 3, and at each occurrence L₃ independently represents -L₅-W-L_{5'}-, wherein W is independently selected at each occurrence from the group consisting of a bond, C₁-C₁₀alkylene, C₂-C₁₀alkenylene, C₂-C₁₀alkynylene, -S-, -O-, -C(O)-, -N(R_{W})CO-, -C(O)N(R_{W})-, -C(O)O- and -OC(O)-, and R_{W} is independently selected at each occurrence from hydrogen, C₁-C₆alkyl, C₂-C₆alkenyl or C₁-C₆alkynyl, wherein L₅ and L_{5'} are each independently selected at each occurrence from a bond, C₁-C₁₀alkylene, C₂-C₁₀alkenylene or C₂-C₁₀alkynylene, and are each independently optionally substituted at each occurrence with 1, 2, 3 or more substituents each of which is independently selected at each occurrence from the group consisting of halogen, oxo, thioxo, hydroxy, nitro, cyano, amino, formyl, carbocyclyl, heterocyclyl, -O-R_{D}, -S-R_{D}, -C(O)R_{D}, -OC(O)R_{D}, -C(O)OR_{D}, -NR_{D}R_{D'}, -S(O)R_{D}, -SO₂R_{D}, -C(O)NR_{D}R_{D'}, -N(R_{D})C(O)R_{D'}, -N(R_{D})C(O)OR_{D'}, -N(R_{D})SO₂R_{D'}, -N(R_{D})SO₂NR_{D'}R_{D''}, -carbocyclyl-L₄-Y-L_{4'}-R_{E} and -heterocyclyl-L₄-Y-L_{4'}-R_{E}, and wherein R_{D}, R_{D'}, R_{D''}, R_{E}, L₄, L_{4'} and Y are as defined immediately above;
or p is 1, L₃ is -L₅-C(R₆R₇)-L_{5'}-, A₂ is -L_{A2}-NR_{A2}-, and R_{A2} and R₇ are bonded together to form -C(O)O-, wherein R₆ is selected from the group consisting of hydrogen, C₁-C₆alkyl, C₂-C₆alkenyl, C₂-C₆ alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L_{A2}, L_{D}, L_{E}, L₄, L_{4'} Y, R_{E}, R_{D}, R_{D'}, R_{D''}, L₅ and L_{5'} are as defined immediately above;
or p is 1, L₃ is -L₅-C(R₆R₇)-L_{5'}-, and R₄ and R₇ are bonded together to form -OC(O)-, wherein R₆, L₅ and L_{5'} are as defined immediately above;
R₄ and R₅, unless otherwise provided, are each independently selected from the group consisting ofN-protecting group, hydrogen, C₁-C₆alkyl, C₂-C₆alkenyl, C₂-C₆alkynyl, carbocyclyl, carbocyclylC₁-C₆ alkyl, heterocyclyl, heterocycloC₁-C₆alkyl, -L_{E}-carbocyclyl-L₄-Y-L₄-R_{E}, -L_{E}-heterocyclyl-L₄-Y-L₄-R_{E}, -L₆-O-R₈, -L₆-C(O)R₈, -L₆-C(O)OR₈, -L₆-OC(O)R₈, -L₆-C(O)NR₈R₉, -N(R₉)C(O)OR₈, -L₆-C(O) -L_{6'}-O-R₈, -L₆-C(O)-L_{6'}-NR₈R_{9,} -L₆-C(O)-L_{6'}-N(R₉)C(O)OR₈, -L₆-C(O)-L₆-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₂-C₁₀ alkynylene, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D''}, -L_{E}-carbocyclyl-L₄-Y-L_{4'}-R_{E} and -L_{E}-heterocyclyl-L₄-Y-L_{4'}-R_{E}, and wherein L_{D}, R_{D}, R_{D'}, R_{D''}, L_{E}, L₄, L_{4'}, Y and R_{E} are as defined immediately above;
or R₄ and R₅, together with the N attached thereto, form a heterocyclyl;
wherein at each occurrence L₁, L_{A1}, R_{A1}, Y, V, W, R_{Y}, R_{V}, R_{W}, L_{A2}, R_{A2}, L_{D}, L_{E}, L₄, L_{4'}, L₆, L_{6'}, L₇, L₉, L_{9'}, R₂, R₃, R₄, R₅, R₆, R₈, R₉, R₁₀, R_{E,} R_{D,} R_{D'} and R_{D''} are each independently optionally substituted with at least one substituent selected from the group consisting of halogen, oxo, thioxo, hydroxy, nitro, cyano, amino, formyl, carbocyclyl, heterocyclyl, -O-R_{L}, -S-R_{L}, -C(O)R_{L}, -OC(O)R_{L}, -C(O)OR_{L}, -NR_{L}R_{L'}, -S(O)R_{L}, -SO₂R_{L}, -C(O)NR_{L}R_{L'}, -N(R_{L})C(O)R_{L'}, -N(R_{L})SO₂R_{L'} and -N(R_{L})SO₂NR_{L'}R_{L''}, and wherein R_{L}, R_{L'} and R_{L''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆ alkylaminoC₁-C₆alkyl, carbocyclyl, carbocyclylC₁-C₆alkyl, heterocyclyl and heterocycloC₁-C₆alkyl;
wherein each carbocyclyl moiety (including any optional substitution carbocyclyl) in L₁, A₁, A₂, (L₂)ₖ, Z, (L₃)ₚ and N(R₄R₅) is independently selected at each occurrence from 3-, 4-, 5-, 6-, 7-, 8-, 9- or 10-membered carbocyclyls (e.g., C₃-C₁₀cycloalkyl, C₃-C₁₀cycloalkenyl or C₆-C₁₀aryl), and each heterocyclyl moiety (including any optional substitution heterocyclyl) in L₁, A₁, A₂, (L₂)ₖ, Z, (L₃)ₚ and N(R₄R₅) is independently selected at each occurrence from 3-, 4-, 5-, 6-, 7-, 8-, 9- or 10-membered heterocyclyls (e.g., H₅-H₁₀heteroaryl, H₃-H₁₀heterocycloalkyl or H₃-H₁₀heterocycloalkenyl); and
wherein each carbocyclyl and heterocyclyl moiety in the compound (e.g., in R₁, L₁, A₁, A₂, (L₂)ₖ, Z, (L₃)ₚ or N(R₄R₅), including any optional substitution carbocyclyl or heterocyclyl) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{S}-O-R_{S}, -L_{S}-S-R_{S}, -L_{S}-C(O)R_{S}, -L_{S}-OC(O)R_{S}, -L_{S}-C(O)OR_{S}, -L_{S}-NR_{S}R_{S'}, -L_{S}-S(O)R_{S}, -Ls-SO₂R_{S}, -L_{S}-C(O)NR_{S}R_{S'}, -L_{S}-N(R_{S})C(O)R_{S'}, -L_{S}-N(R_{S})SO₂R_{S'}, -L_{S}-N(R_{S})SO₂NR_{S'}R_{S''}, carbocyclyl, carbocyclylC₁-C₆alkyl, heterocyclyl and heterocycloC₁-C₆alkyl, wherein L_{S} is independently selected at each occurrence from the group consisting of a bond, C₁-C₁₀alkylene, C₂-C₁₀alkenylene and C₂-C₁₀ alkynylene, and R_{S}, R_{S'} and R_{S''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl, C₁-C₆alkylamino C₁-C₆alkyl, C₃-C₁₀carbocyclyl, C₃-C₁₀carbocyclylC₁-C₆alkyl, H₃-H₁₀heterocyclyl and H₃-H₁₀heterocyclo C1-C₆alkyl;
with the proviso that if Z is a bond, then A2 is -L_{A2}-NR_{A2}, p is an integer selected from 1, 2 or 3, L₃ at each occurrence independently represents -L₅-W-L_{5'}-, and R_{A2} and R₅ are each independently selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NP_{D}R_{D'}, -L_{D}-S(O)R_{D}, -L_{D}-SO₂R_{D}, -L_{D}-C(O)NR_{D}R_{D'}, -L_{D}-(R_{D})C(O)R_{D'}, -L_{D}-N(R_{D})SO₂R_{D'}, -L_{D}-N(R_{D})SO₂NR_{D'}R_{D''}, carbocyclyl, carbocyclylC₁-C₆alkyl, heterocyclyl, heterocycloC₁-C₆alkyl, -L_{E}-carbocyclyl-L₄-Y-L_{4'}-R_{E} and -L_{E}-heterocyclyl-L₄-Y-L_{4'}-R_{E}, wherein L_{A2}, L_{D}, L_{E}, L₄, L_{4'}, Y, R_{E}, R_{D}, R_{D'} R_{D''}, L₅, W and L_{5'}, are as defined immediately above;
with the further proviso that said compound is not ritonavir (i.e., (2S,3S,5S)-5-(N-(N-((N-Methyl-N-((2-isopropyl-4-thiazolyl)methyl)amino)carbonyl)-L-valinyl)amino)-2-(N-((5-thiazolyl)methoxy carbonyl)amino)-1,6-diphenyl-3-hydroxyhexane).

In one embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, -L_{S}-O-R_{S}, -L_{S}-S-R_{S}, -L_{S}-C(O)R_{S}, -L_{S}-OC(O)R_{S}, -L_{S}-C(O)OR_{S}, -L_{S}-NR_{S}R_{S'}, -L_{S}-S(O)R_{S}, -L_{S}-SO₂R_{S}, -L_{S}-C(O)NR_{S}R_{S'}, -L_{S}-N(R_{S})C(O)R_{S'}, -L_{S}-NR_{S}SO₂R_{S'} and -L_{S}-NR_{S}SO₂NR_{S'}R_{S''}, wherein L_{S} is independently selected at each occurrence from a bond, C₁-C₁₀ alkylene, C₂-C₁₀alkenylene or C₂-C₁₀alkynylene, and R_{S}, R_{S'} and R_{S''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is -O-L_{A1}-, wherein L_{A1} is a bond;
X is O;
A₂ is -L_{A2}-N(R_{A2})-, wherein L_{A2} is a bond, and R_{A2} is hydrogen, C₁-C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(R_{V})-, and wherein R_{V} is selected from hydrogen, C₁-C₆alkyl, C₂-C₆alkenyl or C₂-C₆alkynyl;
Z is -C(R₂R₃)-, wherein R₂ is carbocyclylC₁-C₆alkyl (e.g., phenylC₁-C₆alkyl, such as benzyl), heterocycloC₁-C₆alkyl, R_{E}-carbocyclylC₁-C₆alkyl- or R_{E}-heterocyclylC₁-C₆alkyl-, and R₃ is hydrogen, C₁-C₆alkyl, C₂-C₆alkenyl or C₂-C₆alkynyl, wherein R_{E} is independently selected at each occurrence from carbocyclyl, heterocyclyl, carbocyclylC₁-C₆alkyl or heterocycloC₁-C₆alkyl;
p is 1;
L₃ represents:
- L₅-W-L_{5'}-, wherein W is a bond; or
- L₅-C(R₆R₇)-L_{5'}-, and R_{A2} and R₇ are bonded together to form -C(O)O-; or
- L₅-C(R₆R₇)-L_{5'}-,wherein R₄ and R₇ are bonded together to form -OC(O)-;
wherein L₅ is a bond, C₁-C₆alkylene, C₁-C₆alkenylene or C₂-C₆alkynylene;
L_{5'} is C₁-C₆alkylene, C₂-C₆alkenylene or C₂-C₆alkynylene and is substituted with at least one moiety selected from the group consisting of carbocyclyl (e.g., phenyl), carbocyclylC₁-C₆alkyl (e.g., phenylC₁-C₆alkyl, such as benzyl), heterocyclyl, heterocycloC₁-C₆alkyl, -carbocyclyl-R_{E} and -heterocyclyl-R_{E}, wherein R_{E} is as defined immediately above in this embodiment;
L₅ and L_{5'} are each independently optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D'} and -C(O)NR_{D}R_{D'}, wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkyl carbonyloxy, C₁-C₆alkylcarbonyloxyC₁-C₆alkyl and C₁-C₆alkylaminoC₁-C₆alkyl;
and
R₆ is hydrogen, C₁-C₆alkyl, C₂-C₆alkenyl or C₂-C₆alkynyl;
R₄ and R₅, unless otherwise provided, are each independently selected from the group consisting of hydrogen, C₁-C₆alkyl, C₂-C₆alkenyl, C₁-C₆alkynyl, carbocyclyl, carbocyclylC₁-C₆alkyl, heterocyclyl, heterocycloC₁-C₆alkyl, -L₆-O-R₈, -L₆-C(O)R₈, -C(O)OR₈, -OC(O)R₈, -C(O)NR₈R₉, -N(R₉)C(O)OR₈, -C(O)-L_{6'}-O-R₈, -C(O)-L_{6'}-NR₈R₉, -C(O)-L_{6'}-N(R₉)C(O)OR₈, -C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆ alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆ alkynylene, and wherein R_{D} and R_{D'} are as defined immediately above in this embodiment;
or R₄ and R₅, together with the N attached thereto, form a heterocyclyl (e.g., 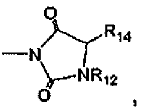 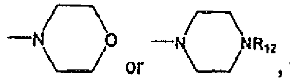 wherein R₁₂ is selected from the group consisting of N-protecting group, hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆-O-R₈, -L₆-C(O)R₈, -L₆-C(O)OR₈, -L₆-OC(O)R₈, -L₆-C(O)NR₈R₉, -N(R₉)C(O)OR₈, -L₆-C(O)-L_{6'}-NR₈R₉, -L₆-C(O)-L_{6'}-N(R₉)C(O)OR₈, -L₆-C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, and j, R₈, R₉, R₁₀, L₆ and L_{6'} are as defined immediately above in this embodiment, and wherein R₁₄ is hydrogen, C₁-C₆alkyl, C₂-C₆alkenyl or C₂-C₆alkynyl);
wherein at each occurrence R₄ and R₅ (or L₁, R_{V}, L_{D}, L₆, L_{6'}, L₉, R₂, R₃, R₄, R₅, R₆, R₈, R₉, R₁₀, R₁₂, R₁₄, R_{A2}, R_{E}, R_{D} and R_{D'}) are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
wherein each carbocyclyl moiety in A₂, Z, (L₃)ₚ and N(R₄R₅) is independently selected at each occurrence from 5-, 6- or 7-membered carbocyclyls (e.g., cyclopentyl, cyclopentenyl, cyclohexyl or phenyl), and each heterocyclyl moiety in A₂, Z, (L₃)ₚ and N(R₄R₅) is independently selected at each occurrence from 5-, 6- or 7-membered heterocyclyls (e.g., dioxanyl, dithianyl, dihydrofuranyl, tetrahydrofuranyl, furanyl, furazanyl, thiazolyl, imidazolyl, isoxazolyl, isoxazolinyl, isoxazolidinyl, isothiazolyl, morpholinyl, 3-oxo-morpholinyl, oxazolyl, oxazolinyl, oxadiazolyl, oxazolidinyl, piperidinyl, piperazinyl, piperidyl, pyrimidinyl, pyrazinyl pyrazolyl, pyridyl, pyridazinyl, pyrrolidinyl, pyridinyl, pyrrolyl, tetrazolyl, tetrahydropyranyl, thiadiazolyl, thiadiazolidinyl, thiazolinyl, thiazolidinyl, thienyl, triazinyl, or triazolyl); and
wherein each carbocyclyl and heterocyclyl moiety in N(R₄R₅) (or in A₂, Z, (L₃)ₚ, and N(R₄R₅)) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K''}, wherein L_{H} is independently selected at each occurrence from a bond, C₁-C₆alkylene, C₂-C₆alkenylene or C₂-C₆alkynylene, and R_{K}, R_{K'} and R_{K''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl.

In a non-limiting example, R₈ is selected from the group consisting of H₅-H₆heterocyclyl, H₅-H₆heterocycloC₁-C₆alkyl, (H₅-H₆heterocyclo)oxyC₁-C₆alkyl and (H₅-H₆heterocyclo)C₁-C₆alkoxyC₁-C₆ alkyl, wherein each H₅-H₆heterocyclyl or H₅-H₆heterocyclo moiety comprises at least one nitrogen ring atom.

In another non-limiting example, L₃ represents -L₅-W-L_{5'}-, wherein L₅ and W are bonds, and L_{5'} is -(CH₂)₂-CH(R₁₃)-, wherein R₁₃ is heterocyclocarbocyclyl (e.g., pyridylphenyl) or heterocyclocarbocyclylC₁-C₆alkyl (e.g., pyridylbenzyl), and wherein L_{5'} is optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D'} and -C(O)NR_{D}R_{D'}, and R_{D} and R_{D'} are as defined immediately above in this embodiment.

In still another non-limiting example, R₁ is thiazolyl, L₁ is -CH₂-, R_{A2} is hydrogen, R₂ is benzyl, L₃ is ―L₅―W―L₅―, L₅ and W are bonds, L₅, is ―(CH₂)₂-CH(R₁₃)―, and R₁₃ is pyridylbenzyl, wherein L_{5'} is optionally substituted with at least one moiety selected from halogen, oxo, thioxo, hydroxy, nitro, cyano, amino, ―O―R_{D}, ―S―R_{P}, ―C(O)R_{D}, ―OC(O)R_{D}, ―C(O)OR_{D}, ―NR_{D}R_{D'} and ―C(O)NR_{D}R_{D'}, and R_{D} and R_{D'} are as defined immediately above in this embodiment.

In still yet another non-limiting example, R₁ is thiazolyl, L₁ is ―CH₂―, R_{A2} is hydrogen, R₂ is pyridylbenzyl, L₃ is ―L₅―W―L_{5'}―, L₅ and W are bonds, L₅, is ―(CH₂)₂CH(R₁₃)―, and R₁₃ is benzyl, wherein L_{5'} is optionally substituted with at least one moiety selected from halogen, oxo, thioxo, hydroxy, nitro, cyano, amino, ―O―R_{D}, ―S―R_{D}, -C(O)R_{D}, ―OC(O)R_{D}, ―C(O)OR_{D}, ―NR_{D}R_{D'} and ―C(O)NR_{D}R_{D'}, and R_{D} and R_{D'} are as defined immediately above in this embodiment.

In a further non-limiting example, R₁ is thiazolyl, L₁ is ―CH₂―, R_{A2} is hydrogen, R₂ is benzyl, L₃ is ―L₅-W-L_{5'}―, L₅ and W are bonds, L_{5'} is ―(CH₂)₂―CH(R₁₃)―, and R₁₃ is benzyl, wherein L_{5'} is optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'}, and R_{D} and R_{D'} are as defined immediately above in this embodiment.

In still another non-limiting example, R₁ is thiazolyl, L₁ is ―CH₂―, R_{A2} is hydrogen, R₂ is benzyl, L₃ is ―L₅―C(R₆R₇)―L₅―, L₅ is a bond, L_{5'} is ―(CH₂)―CH(R₁₃)―, and R₄ and R₇ are bonded together to form ―OC(O)―, wherein R₁₃ is benzyl.

In yet another non-limiting example, R₁ is thiazolyl, L₁ is ―CH₂―, k is 0, R₂ is benzyl, L₃ is ―L₅―C(R₆R₇)―L_{5'}―, L₅ is a bond, L_{5'} is ―(CH₂)CH(R₁₃)-, and R_{A2} and R₇ are bonded together to form ―OC(O)―, wherein R₁₃ is benzyl.

In any of the above examples, R₅ or R₄ can be, without limitation, (H₅-H₆heterocyclo)C₁-C₆ alkoxycarbonyl, wherein the H₅-H₆heterocyclyl moiety comprises at least one nitrogen ring atom. For instance, R₅ or R₄ can be thiazolylC₁-C₆alkoycarbonyl, such as thiazolylmethoxycarbonyl.

Non-limiting examples of the compounds of this embodiment include:
1,3-thiazol-5-ylmethyl 3-hydroxy4-{[*N*-(methoxycarbonyl)-3-mothylvalyl]amino}-5-phenyl-1-(4-pyridin-2-ylbenzyl)pentylcarbamate;
methyl 1-({[3-hydroxy-4-({3-methyl-*N*-[(1,3-thiazol-5-ylmethoxy)carbonyl)valyl}amino)-5-phenyl-1-(4-pyridin-2-ylbenzyl)pentyl]amino}carbonyl)-2,2-dimethylpropylcarbamate;
methyl 1-({[3-hydroxy-4-({3-methyl-*N*-[(1,3-thiazol-5-ylmethoxy)carbonyl]-valyl}amino)-5-phenyl-1-(4-pyridin-2-ylbenzyl)pentyl]amino}carbonyl)-2,2-dimethylpropylcarbamate;
*tert*-butyl 3-hydroxy-5-phenyl-1-(4-pyridin-2-ylbenzyl)-4-{[(1,3-thiazol-5-ylmethoxy)carbonyl] amino}pentylcarbamate;
*tert*-butyl 3-hydroxy-4-({3-methyl-*N*-[(1,3-thiazol-5-ylmethoxy)carbonyl]valyl}amino)-5-phenyl-1-(4-pyridin-2-ylbenzyl)pentylcarbamate;
*tert*-butyl 3-hydroxy-5-phenyl-1-(4-pyridin-2-ylbenzyl)-4-({*N*-[(1,3-thiazol-5-ylmethoxy) carbonyl]alanyl}amino)pentylcarbamate;
methyl 3-hydroxy-4-({3-methyl-*N*-[(1,3-thiazol-5-ylmethoxy)carbonyl]-valyl}amino)-5-phenyl-1-(4-pyridin-2-ylbenzyl)pentylcarbamate;
methyl 3-hydroxy-5-phenyl-1-(4-pyridin-2-ylbenzyl)-4-({*N*-[(1,3-thiazol-5-ylmethoxy)carbonyl]-alanyl}amino)pentylcarbamate;
*N*'-(3-hydroxy-5-phenyl-1-(4-pyridin-2-ylbenzyl)-4-{[(1,3-thiazol-5-ylmethoxy)carbonyl]amino} pentyl)-*N*²-(methoxycarbonyl)-3-methylvalinamide;
1,3-thiazol-5-ylmethyl 1-benzyl-3-hydroxy-5-phenyl-4-{[(1,3-thiazol-5-ylmethoxy)carbonyl] amino}pentylcarbamate;
N¹-{1-benzyl-4-[(tert-butoxycarbonyl)amino]-2-hydroxy-5-phenylpentyl}-3-methyl-N²-[(1,3-thiazol-5-ylmethoxy)carbonyl]valinamide;
N¹-(1-benzyl-2-hydroxy-5-phenyl-4-{[(1,3-thiazol-5-ylmethoxy)carbonyl]amino}pentyl)-N²-(methoxycarbonyl)-3-methylvalinamide;
1,3-thiazol-5-ylmethyl 4-(acetylamino)-1-benzyl-2-hydroxy-5-phenylpentylcarbamate;
1,3-thiazol-5-ylmethyl 4-amino-1-benzyl-2-hydroxy-5-phenylpentylcarbamate;
3-(acetylamino)-4-phenyl-1-((1S)-2-phenyl-)-1-{[(1,3-thiazol-5-ylmethoxy)carbonyl]amino} ethyl)butyl acetate;
1,3-thiazol-5-ylmethyl 1-benzyl-2-hydroxy-4-[(methylsulfonyl)amino]-5-phenylpentylcarbamate;
1,3-thiazol-5-ylmethyl 1-benzyl-4-{[(dimethylamino)carbonyl]amino}-2-hydroxy-5-phenyl pentylcarbamate;
methyl 1-benzyl-3-hydroxy-5-phenyl-4-{[(1,3-thiazol-5-ylmethoxy)carbonyl]amino}pentyl carbamate;
1,3-thiazol-5-ylmethyl 1-benzyl-4-{[(dimethylamino)sulfonyl]amino}-2-hydroxy-5-phenyl pentylcarbamate;
*tert*-butyl 1-benzyl-3-hydroxy-5-phenyl-4-{[(1,3-thiazol-5-ylmethoxy)carbonyl]amino}pentyl carbamate;
1,3-thiazol-5-ylmethyl 1-benzyl-2-hydroxy-4-{[(2S)-3-methyl-2-(2-oxotetrahydropyrimidin-1(2H)-yl)butanoyl]amino}-5-phenylpentylcarbamate;
isobutyl 1-benzyl-3-hydroxy-5-phenyl-4-{[(1,3-thiazol-5-ylmethoxy)carbonyl]amino}pentyl carbamate;
isopropyl 1-benzyl-3-hydroxy-5-phenyl-4-{[(1,3-thiazol-5-ylmethoxy)carbonyl]amino}pentyl carbamate;
*tert*-butyl 1-benzyl-3-hydroxy-5-phenyl-4-{[(1,3-thiazol-5-ylmethoxy)carbonyl]amino}pentyl carbamate;
1,3-thiazol-5-ylmethyl 4-benzyl-5-{2-[4-isopropyl-2,5-dioxoimidazolidin-1-yl]-3-phenylpropyl}-2-oxo-1,3-oxazolidine-3-carboxylate;
N¹-(1-benzyl-3-hydroxy-5-phenyl-4-{[(1,3-thiazol-5-ylmethoxy)carbonyl]amino}pentyl) valinamide;
N¹-(1-benzyl-3-hydroxy-5-phenyl-4-{[(1,3-thiazol-5-ylmethoxy)carbonyl] amino}pentyl)-N²-[(dimethylamino)carbonyl]valinamide;
N¹-(1-benzyl-3-hydroxy-5-phenyl-4-{[(1,3-thiazol-5-ylmethoxy)carbonyl] amino}pentyl)-N²-[(methylamino)carbonyl]valinamide;
N¹-(1-benzyl-3-hydroxy-5-phenyl-4-{[(1,3-thiazol-5-ylmethoxy)carbonyl]amino}pentyl)-N²-[(2-hydroxypropoxy)carbonyl]valinamide;
1,3-thiazol-5-ylmethyl 1-[4-benzyl-2-oxo-1,3-oxazinan-6-yl]-2-phenylethylcarbamate;
N¹-(1-benzyl-3-hydroxy-5-phenyl-4-{[(1,3-thiazol-5-ylmethoxy)carbonyl]amino}pentyl)-N²-[(2,3-dihydroxypropoxy)carbonyl]valinamide;
1,3-thiazol-5-ylmethyl 1-benzyl-2-hydroxy-5-phenyl-4-{[(4-phonylpiperazin-1-yl)carbonyl] amino}pentylcarbamate;
N¹-(1-benzyl-3-hydroxy-5-phenyl-4-{[(3H-1 lambda~4~,3-thiazol-4-ylmethoxy)carbonyl] amino}pentyl)-N²-{[[(2-isopropyl-1,3-thiazol-4-yl)methyl](methyl)amino]carbonyl}valinamide;
1,3-thiazol-4-ylmethyl 1-benzyl-3-hydroxy-5-phenyl-4-{[(1,3-thiazol-4-ylmethoxy)carbonyl] amino}pentylcarbamate;
1,3-thiazol-5-ylmethyl 1-benzyl-2-hydroxy-4-[4-isopropyl-2,5-dioxoimidazolidin-1-yl]-5-phenylpentylcarbamate;
1,3-thiazol-5-ylmethyl 1-benzyl-2-hydroxy-4-{[(2-isopropyl-1,3-thiazol-4-yl)acetyl]amino}-5-phenylpentylcarbamate;
1,3-thiazol-5-ylmethyl 1-benzyl-4-{[(2,6-dimethylphenoxy)acetyl]amino}-2-hydroxy-5-phenyl pentylcarbamate;
1,3-thiazol-5-ylmethyl 1-benzyl-4-{[(tert-butylamino)carbonyl]amino}-2-hydroxy-5-phenyl pentylcarbamate;
N¹-(1-benzyl-3-hydroxy-5-phenyl-4-{[(1,3-thiazol-5-ylmethoxy)carbonyl]amino} pentyl)-N²-(methoxycarbonyl)-3-methylvalinamide; and
1,3-thiazol-5-ylmethyl 1-benzyl-4-{[(2,6-dimethylphenoxy)acetyl]amino}-3-hydroxy-5-phenyl pentylcarbamate.

Preferred exemplary compounds of this embodiment include, but are not limited to:
1,3-thiazol-5-ylmethyl (1*S*,3*S*,4*S*)-3-hydroxy-4-{[*N*-(methoxycarbonyl)-3-methyl-L-valyl]amino} -5-phenyl-1-(4-pyridin-2-ylbenzyl)pentylcarbamate;
methyl (1*S*)-1-({[(1*R*,3*S*,4,*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl)pentyl]amino} carbonyl)-2,2-dimethylpropylcarbamate;
methyl (1*S*)-1-({[(1*S*,3*S*,4*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl)pentyl]amino}carbonyl)-2,2-dimethylpropylcarbamate;
*tert*-butyl (1*S*,3*S*,4*S*)-3-hydroxy-5-phenyl-1-(4-pyridin-2-ylbenzyl)-4-{[(1,3-thiazol-5-yl methoxy)carbonyl]amino}pentylcarbamate;
*tert*-butyl (1*S*,3*S*,4*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl)pentylcarbamate;
*tert*-butyl (1*S*,3*S*,4*S*)-3-hydroxy-5-phenyl-1-(4-pyridin-2-ylbenzyl)-4-({*N*-[(1,3-thiazol-5-ylmethoxy)carbonyl]-L-alanyl}amino)pentylcarbamate;
methyl(1*S*,3*S*,4*S*)-3-hydroxy-4-({3-methyl-*N-*[(1,3-thiazol-5-ylmethoxy)carbonyl]-L-valyl} amino)-5-phenyl-1-(4-pyridin-2-ylbenzyl)pentylcarbamate;
methyl(1*S*,3*S*,4*S*)-3-hydroxy-5-phenyl-1-(4-pyridin-2-ylbenzyl)-4-({*N*-[(1,3-thiazol-5-yl methoxy)carbonyl]L-alanyl}amino)pentylcarbamate;
*N*¹-((1*S*,3*S*,4*S*)-3-hydroxy-5-phenyl-1-(4-pyridin-2-ylbenzyl)-4-{[(1,3-thiazol-5-ylmethoxy) carbonyl]amino}pentyl)-*N*²-(methoxycarbonyl)-3-methyl-L-valinamide;
1,3-thiazol-5-ylmethyl(1*S*,3*S*,4*S*)-1-benzyl-3-hydroxy-5-phenyl-4-{[(1,3-thiazol-5-ylmethoxy) carbonyl]amino}pentylcarbamate;
N¹-{(1S,2S,4S)-1-benzyl-4-[(tert-butoxycarbonyl)amino]-2-hydroxy-5-phenylpentyl}-3-methyl-N²-[(1,3-thiazol-5-ylmethoxy)carbonyl]-L-valinamide;
N¹-((1S,2S,4S)-1-benzyl-2-hydroxy-5-phenyl-4-{[(1,3-thiazol-5-ylmethoxy)carbonyl]amino} pentyl)-N²-(methoxycarbonyl)-3-methyl-L-valinamide;
1,3-thiazol-5-ylmethyl(1S,2S,4S)-4-(acetylamino)-1-benzyl-2-hydroxy-5-phenylpentylcarbamate;
1,3-thiazol-5-ylmethyl(1S,2S,4S)-4-amino-1-benzyl-2-hydroxy-5-phenylpentylcarbamate;
(1S,3S)-3-(acetylamino)-4-phenyl-1-((1S)-2-phenyl-1-{[(1,3-thiazol-5-ylmethoxy)carbonyl] amino}ethyl)butyl acetate;
1,3-thiazol-5-ylmethyl(1S,2S,4S)-1-benzyl-2-hydroxy-4-[(methylsulfonyl)amino]-5-phenyl pentylcarbamate;
1,3-thiazol-5-ylmethyl(1S,2S,4S)-1-benzyl-4-{[(dimethylamino)carbonyl]amino}-2-hydroxy-5-phenylpentylcarbamate;
methyl(1*S*,3*S*,4*S*)-1-benzyl-3-hydroxy-5-phenyl-4-{[(1,3-thiazol-5-ylmethoxy)carbonyl]amino} pentylcarbamate;
1,3-thiazol-5-ylmethyl(1S,2S,4S)-1-benzyl-4-{[(dimethylamino)sulfonyl]amino}-2-hydroxy-5-phenylpentylcarbamate;
*tert*-butyl(1*S*,3*S*,4*S*)-1-benzyl-3-hydroxy-5-phenyl-4-{[(1,3-thiazol-5-ylmethoxy)carbonyl] amino}pentylcarbamate;
1,3-thiazol-5-ylmethyl(1S,2S,4S)-1-benzyl-2-hydroxy-4-{[(2S)-3-methyl-2-(2-oxotetrahydro pyrimidin-1(2H)-yl)butanoyl]amino}-5-phenylpentylcarbamate;
isobutyl(1*S*,3*S*,4*S*)-1-benzyl-3-hydroxy-5-phenyl-4-{[(1,3-thiazol-5-ylmethoxy)carbonyl]amino} pentylcarbamate;
isopropyl(1*S*,3*S*,4*S*)-1-benzyl-3-hydroxy-5-phenyl-4-{[(1,3-thiazol-5-ylmethoxy)carbonyl] amino}pentylcarbamate;
*tert*-butyl(1*S*,3*R*,4*S*)-1-benzyl-3-hydroxy-5-phenyl-4-{[(1,3-thiazol-5-ylmethoxy)carbonyl] amino} pentylcarbamate;
1,3-thiazol-5-ylmethyl(4S,5S)-4-benzyl-5-{(2S)-2-[(4S)-4-isopropyl-2,5-dioxoimidazolidin-1-yl]-3-phenylpropyl}-2-oxo-1,3-oxazolidine-3-carboxylate;
N¹-((1S,3S,4S)-1-benzyl-3-hydroxy-5-phenyl-4-{[(1,3-thiazol-5-ylmethoxy)carbonyl] amino}pentyl)-L-valinamide;
N¹-((1S,3S,4S)-1-benzyl-3-hydroxy-5-phenyl-4-{[(1,3-thiazol-5-ylmethoxy)carbonyl] amino}pentyl)-N²-[(dimethylamino)carbonyl]-L-valinamide;
N¹-((1S,3S,4S)-1-benzyl-3-hydroxy-5-phenyl-4-{[(1,3-thiazol-5-ylmethoxy)carbonyl]amino} pentyl)-N²-(methylamino)carbonyl]valinamide;
H¹-((1S,3S,4S)-1-benzyl-3-hydroxy-5-phenyl-4-{[(1,3-thiazol-5-ylmethoxy)carbonyl]amino} pnetyl)-N²-[(2-hydroxypropoxy)carbonyl]-L-valinamide;
1,3-thiazol-5-ylmethyl(1S)-1-[(4S,6S)-4-benzyl-2-oxo-1,3-oxazinan-6-yl]-2-phenylethyl carbamate;
N¹-((1S,3S,4S)-1-benzyl-3-hydroxy-5-phenyl-4-{[(1,3-thiazol-5-ylmethoxy)carbonyl]amino} pentyl)-N²-[(2,3-dihydroxypropoxy)carbonyl]-L-valinamide;
1,3-thiazol-5-ylmethyl(1S,2S,4S)-1-benzyl-2-hydroxy-5-phenyl-4-{[(4-phenylpiperazin-1-yl) carbonyl]amino}pentylcarbamate;
*N*¹-((1*S*,3*S*,4*S*)-1-benzyl-3-hydroxy-5-phenyl-4-{[(1,3-thiazol-4-ylmethoxy)carbonyl]amino} pentyl)-*N*²-{[[(2-isopropyl-1,3-thiazol-4-yl)methyl](methyl)amino]carbonyl}-L-valinamide;
1,3-thiazol-4-ylmethyl(1*S*,3*S*,4*S*)-1-benzyl-3-hydroxy-5-phenyl-4-{[(1,3-thiazol-4-ylmethoxy) carbonyl]amino}pentylcarbamate;
1,3-thiazol-5-ylmethyl(1S,2S,4S)-1-benzyl-2-hydroxy-4-[(4S)-4-isopropyl-2,5-dioxo imidazolidin-1-yl]-5-phenylpentylcarbamate;
1,3-thiazol-5-ylmethyl(1S,2S,4S)-1-benzyl-2-hydroxy-4-{[(2-isopropyl-1,3-thiazol-4-yl) acetyl]amino}-5-phenylpentylcarbamate;
1,3-thiazol-5-ylmethyl(1S,2S,4S)-1-benzyl-4-{[(2,6-dimethylphenoxy)acetyl]amino}-2-hydroxy-5-phenylpentylcarbamate;
1,3-thiazol-5-ylmethyl (1S,2S,4S)-1-benzyl-4-{[(tert-butylamino)carbonyl]amino}-2-hydroxy-5-phenylpentylcarbamate;
N¹-((1S,3S,4S)-1-benzyl-3-hydroxy-5-phenyl-4-{[(1,3-thiazol-5-ylmethoxy)carbonyl]amino}pentyl)-N²-(methoxycarbonyl)-3-methyl-L-valinamide; and
1,3-thiazol-5-ylmethyl (1S,3S,4S)-1-benzyl-4-{[(2,6-dimethylphenoxy)acetyl]amino}-3-hydroxy-5-phenylpentylcarbamate.

In another embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, ―Lₛ―O―Rₛ, ―Lₛ―S―Rₛ, ―Lₛ-C(O)Rₛ, ―Lₛ―OC(O)Rₛ, ―Lₛ―C(O)Rₛ, ―Lₛ―NRₛR_{s'}, ―Lₛ―S(O)Rₛ, ―Lₛ―SO₂Rₛ, ―Lₛ-C(O)NRₛR_{s'}, ―Lₛ―NRₛSO₂R_{s'} and ―Lₛ―NRₛSO₂NR_{s'}R_{s''}, wherein Lₛ is independently selected at each occurrence from a bond, C₁-C₁₀ alkylene, C₂-C₁₀alkenylene or C₂-C₁₀alkynylene, and Rₛ, R_{s'} and R_{s''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonyl C₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is ―O―L_{A1}―, wherein L_{A1} is a bond;
X is O;
A₂ is ―L_{A2}―N(R_{A2})―, wherein L_{A2} is a bond, and R_{A2} is hydrogen, C₁-C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents ―L₉―V―L_{9'}―, wherein L₉ is independently selected from a bond, C₁-C₆alkylone, C₂-C₆alkenylene or C₁-C₆alkynylene, L_{9'} is a bond, and V is selected from the group consisting of a bond or ―C(O)N(Rᵥ)―, and wherein Rᵥ is selected from hydrogen, C₁-C₆alkyl, C₂-C₆alkenyl or C₂-C₆alkynyl;
Z is ―C(R₂R₃)―, wherein R₂ is carbocyclylC₁-C₆alkyl (e.g., phenylC₁-C₆alkyl, such as benzyl), heterocycloC₁-C₆alkyl, R_{E}―carbocyclylC₁-C₆alkyl― or R_{E}―heterocyclylC₁-C₆alkyl―, and R₃ is hydrogen, C₁-C₆alkyl, C₂-C₆alkenyl or C₂-C₆alkynyl, wherein R_{E} is independently selected at each occurrence from carbocyclyl, heterocyclyl, carbocyclylC₁-C₆alkyl or heterocycloC₁-C₆alkyl;
p is 0 or 1;
L₃ represents:
- L₅-W-L_{5'}―, wherein W is a bond or ―C(O)―; or
- L₅―C(R₆R₇)―L_{5'}―, and R_{A2} and R₇ are bonded together to form ―C(O)O―; or
- L₅―C(R₆R₇)―L_{5'}―,wherein R₄ and R₇ are bonded together to form ―OC(O)―;
   wherein L₅ and L_{5'} are each independently selected from a bond, C₁-C₆alkylene, C₂-C₆ alkenylene or C₂-C₆alkynylene, and are each independently optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'};
   wherein R_{D} and R_{D'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆ alkyl, C₁-C₆alkylcarbonyloxy, C₁-C₆alkylcarbonyloxyC₁-C₆alkyl and C₁-C₆ alkylaminoC₁-C₆alkyl; and
   wherein R₆ is hydrogen, C₁-C₆alkyl, C₂-C₆alkenyl or C₂-C₆alkynyl;
R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆― O―R₈, ―L₆―C(O)R₈, ―C(O)OR₈, ―OC(O)R₈, ―C(O)NR₈R₉, ―N(R₉)C(O)OR₈, ―C(O)―L₆―O―R₈, ―C(O)― L_{6'}―NR₈R₉, ―C(O)―L_{6'}―N(R₉)C(O)OR₈, ―C(O)―L_{6'}―N(R₉)C(O)NR₈R₁₀, ―L₆-S(O)ⱼR₈, ―L₆―N(R₉)S(O)ⱼR₈, ―L₆―S(O)ⱼNR₈R₉ and ―L₆―N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'} and ―L_{D}―C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene, and wherein R_{D} and R_{D'} are as defined immediately above in this embodiment;
or R₄ and R₅, together with the N attached thereto, form a heterocyclyl (e.g., 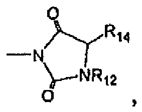 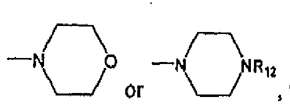 wherein R₁₂ is selected from the group consisting of N―protecting group, hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆―O―R₈, -L₆-C(O)R₈, ―L₆―C(O)OR₈, ―L₆―OC(O)R₈, ―L₆―C(O)NR₈R₉, ―N(R₉)C(O)OR₈, ―L6―C(O)―L_{6'}―NR₈R₉, ―L₆―C(O)―L₆―N(R₉)C(O)OR₈, ―L₆―C(O)―L_{6'}―N(R₉)C(O) NR₈R₁₀, ―L₆―S(O)ⱼR₈, ―L₆―N(R₉S(O)ⱼR₈, ―L₆-S(O)ⱼNR₈R₉ and ―L₆―N(R₉)S(O)₂NR₈R₁₀, and j, R₈, R₉, R₁₀, L₆ and L_{6'} are as defined immediately above in this embodiment, and wherein R₁₄ is hydrogen, C₁-C₆ alkyl, C₂-C₆alkenyl or C₂-C₆alkynyl);
wherein at each occurrence R₄ and R₅ (or L₁, R_{V}, L_{D}, L₆, L_{6'}, L₉, R₂, R₃, R₄, R₅, R₆, R₈, R₉, R₁₀, R₁₂, R₁₄, R_{A2}, R_{E}, R_{D} and R_{D'}) are each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
wherein each carbocyclyl moiety in A₂, Z, and N(R₄R₅) is independently selected at each occurrence from 5-, 6- or 7-membered carbocyclyls, and each heterocyclyl moiety in A₂, Z, and N(R₄R₅) is independently selected at each occurrence from 5-, 6- or 7-membered heterocyclyls; and
wherein each carbocyclyl and heterocyclyl moiety in N(R₄R₅) (or in A₂, Z, and N(R₄R₅)) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{H}―O―R_{K}, ―L_{H}―S―R_{K}, ―L_{H}―C(O)R_{K}, ―L_{H}―OC(O)R_{K}, ―L_{H}―C(O)OR_{K}, ―L_{H}―NR_{K}R_{K'}, ―L_{H}― S(O)Rₖ, ―L_{H}―SO₂R_{K}, ―L_{H}―C(O)NR_{K}R_{K'}, ―L_{H}―N(R_{K})C(O)R_{K'}, ―L_{H}-NR_{K}SO₂R_{K'} and ―L_{H}-NR_{K}SO₂NR_{K'}R_{K''}, wherein L_{H} is independently selected at each occurrence from a bond, C₁-C₆ alkylene, C₂-C₆alkenylene or C₂-C₆alkynylene, and R_{K}, R_{K'} and R_{K''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl.

In a non-limiting example, R_{A2} is C₁-C₆alkyl, carbocyclylC₁-C₆alkyl (e.g., benzyl) or heterocycloC₁-C₆alkyl, k is 1, L₂ is ―L₉―V―L_{9'}―, and p is 0, wherein L_{9'} and V are bonds, and L₉ is C₁-C₃ alkylene optionally substituted with at least one substituent selected from the group consisting of halogen, oxo, thioxo, hydroxy, nitro, cyano and amino.

In another non-limiting example, k is 0, p is 1, and L₃ is ―L₅―W―L_{5'}―, wherein W is ―C(O)―, and L₅ and L_{5'} are bonds,

In yet another non-limiting example, R₁ is thiazolyl, L₁ is ―CH₂―, R_{A2} is hydrogen, k is 0, R₂ is benzyl, p is 1, and L₃ is ―L₅-W-L_{5'}―, wherein W is ―C(O)―, and L₅ and L_{5'} are bonds.

In still another non-limiting example, R₁ is thiazolyl, L₁ is ―CH₂―, R_{A2} is C₁-C₆alkyl or benzyl, k is 1, L₂ is ―L₉―V―L_{9'}―, R₂ is benzyl, and p is 0, wherein L_{9'} and V are bonds, and L₉ is C₁-C₃ alkylene optionally substituted with at least one substituent selected from the group consisting of halogen, oxo, thioxo, hydroxy, nitro, cyano and amino.

In any of the above examples, R₅ or R₄ can be, without limitation, (H₅-H₆heterocyclo)C₁-C₆alkoxycarbonyl, wherein the H₅-H₆heterocyclyl moiety comprises at least one nitrogen ring atom. For instance, R₅ or R₄ can be thiazolylC₁-C₆alkoycarbonyl, such as thiazolylmethoxycarbonyl.

In many cases, R₄, R₅ or R_{A2} in this embodiment comprises at least one carbocyclyl or heterocyclyl moiety, such as phenyl or thiazolyl.

Non-limiting examples of the compounds of this embodiment include:
*tert*-butyll-benzyl-2-hydroxy-3-{isobutyl[(1,3-thiazol-5-ylmethoxy)carbonyl]amino} propyl carbamate;
N,N-dimethyl-N-[(1,3-thiazol-5-ylmethoxy)carbonyl]phenylalaninamide;
*N*-[1-benzyl-2-morpholin-4-yl-2-oxoethyl]-*N*-[(1,3-thiazol-5-ylmethoxy) carbonyl]amine;
N,N-diisobutyl-N-[(1,3-thiazol-5-ylmethoxy)carbonyl]phenylalaninamide;
N-isobutyl-N-[(1,3-thiazol-5-ylmethoxy)carbonyl]phenylalaninamide;
N,N-dibenzyl-N-[(1,3-thiazol-5-ylmethoxy)carbonyl]phenylalaninamide;
N-benzyl-N-(2-phenylethyl)-N-[(1,3-thiazol-5-ylmethoxy)carbonyl] phenylalaninamide;
*tert*-butyl 1-benzyl-3-{benzyl[(1,3-thiazol-5-ylmethoxy)carbonyl]amino}-2-hydroxypropyl carbamate;
*tert*-butyl (1-benzyl-3-{benzyl[(1,3-thiazol-5-ylmethoxy)carbonyl]amino}-2-hydroxypropyl carbamate; and
*tert*-butyl benzyl(2-hydroxy-4-phenyl-3-{[(1,3-thiazol-5-ylmethoxy)carbonyl] amino}butyl) carbamate.

Preferred exemplary compounds of this embodiment include, but are not limited to:*tert*-butyl (1*S*,2*R*)-1-benzyl-2-hydroxy-3-{isobutyl[(1,3-thiazol-5-ylmethoxy)carbonyl] amino} propylcarbamate;
N,N-dimethyl-N-[(1,3-thiazol-5-ylmethoxy)carbonyl]-L-phenylalaninamide;
*N*-[(1*S*)-1-benzyl-2-morpholin-4-yl-2-oxoethyl]-*N*-[(1,3-thiazol-5-ylmethoxy)carbonyl] amine;
N,N-diisobutyl-N-[(1,3-thiazol-5-ylmethoxy)carbonyl]-L-phenylalaninamide;
N-isobutyl-N-[(1,3-thiazol-5-ylmethoxy)carbonyl]-L-phenylalaninamide;
N,N-dibenzyl-N-[(1,3-thiazol-5-ylmethoxy)carbonyl]-L-phenylalaninamide;
N-benzyl-N-(2-phenylethyl)-N-[(1,3-thiazol-5-ylmethoxy)carbonyl]-L-phenylalaninamide;
*tert*-butyl (1*S*,2*S*)-1-benzyl-3-{benzyl[(1,3-thiazol-5-ylmethoxy)carbonyl]amino}-2-hydroxy propylcarbamate;
*tert*-butyl (1*S*,2*R*)-1-benzyl-3-{benzyl[(1,3-thiazol-5-ylmethoxy)carbonyl]amino}-2-hydroxy propylcarbamate; and
*tert*-butyl benzyl((2*R*,3*S*)-2-hydroxy-4-phenyl-3-{[(1,3-thiazol-5-ylmethoxy)carbonyl]amino} butyl)carbamate.

In a further embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, ―Lₛ―O―Rₛ, ―Lₛ―S―Rₛ, ―Lₛ―C(O)Rₛ, ―Lₛ―OC(O)Rₛ, ―Lₛ―C(O)ORₛ, ―Lₛ―NRₛR_{s'}, ―Lₛ―S(O)Rₛ, ―Lₛ―SO₂Rₛ, ―Lₛ―C(O)NRₛR_{s'}, ―Lₛ―N(Rₛ)C(O)R_{s'}, ―Lₛ―NRₛSO₂R_{s'} and ―Lₛ―NRₛSO₂NR_{s'}R_{s''}, wherein Lₛ is independently selected at each occurrence from a bond, C₁-C₁₀alkylene, C₂-C₁₀alkenylene or C₂-C₁₀alkynylene, and Rₛ, R_{s'} and R_{s''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is ―O―L_{A1}―, wherein L_{A1} is a bond;
X is O;
A₂ is ―L_{A2}―N(R_{A2})―, wherein L_{A2} is a bond, and R_{A2} is hydrogen, C₁-C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents ―L₉―V―L₉―, wherein L₉ is independently selected from a bond, C₁-C₆alkylene, C₂-C₆alkenylene or C₂-C₆alkynylene, L_{9'} is a bond, and V is selected from the group consisting of a bond or ―C(O)N(Rᵥ)―, and wherein Rᵥ is selected from hydrogen, C₁-C₆alkyl, C₂-C₆alkenyl or C₂-C₆alkynyl;
Z is selected from the group consisting of 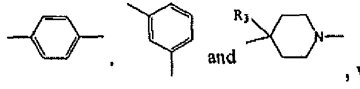 wherein R₃ is hydrogen, C₁-C₆alkyl, C₂-C₆alkenyl, C₂-C₆alkynyl, carbocyclylC₁-C₆alkyl (e.g., benzyl) or heterocycloC₁-C₆alkyl;
p is 0 or 1;
L₃ represents:
- L₅―W―L_{5'}―, wherein W is a bond or ―N(R_{w})CO―, and R_{w} is hydrogen, C₁-C₆alkyl, C2-C₆alkenyl or C₂-C₆alkynyl; or
- L₅―(R₆R₇)―L_{5'}―,wherein R₄ and R₇ are bonded together to form ―OC(O)―;
   wherein L₅ and L₅. are each independently selected from a bond, C₁-C₆alkylene, C₂-C₆ alkenylene or C₂-C₆alkynylene, and are each independently optionally substituted with at least one moiety selected from halogen, oxo, thioxo, hydroxy, nitro, cyano, amino, carbocyclyl (e.g., phenyl), carbocyclylC₁-C₆alkyl (e.g., benzyl), heterocyclyl, heterocycloC₁-C₆alkyl, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'};
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkyl carbonyloxy, C₁-C₆alkylcarbonyloxyC₁-C₆alkyl and C₁-C₆alkylaminoC₁-C₆alkyl; and
   wherein R₆ is hydrogen, C₁-C₆alkyl, C₂-C₆alkenyl or C₂-C₆alkynyl;
R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆― O―R₈, ―L₆―C(O)R₈, ―C(O)OR₈, ―OC(O)R₈, ―C(O)NR₈R₉, ―N(R₉)C(O)OR₈, ―C(O)―L₆―O―R₈, ―C(O)― L_{6'}―NR₈R₉, ―C(O)―L₆―N(R₉)C(O)OR₈, ―C(O)―L_{6'}―N(R₉)C(O)NR₈R₁₀, ―L₆―S(O)ⱼR₈, ―L₆―N(R₉)S(O)ⱼR₈, ―L₆-S(O)ⱼNR₈R₉ and ―L₆―(R₉)S(O)₂NR₈R₁₀, wherein R₈, R₉ and R₁₀ are each independently selected at each occurrence from the group consisting of hydrogen, C₁-C₆alkyl, C₁-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}-C(O)R_{D}, ―L_{D}-OC(O)R_{D}, ―L_{D}―C(O)OR_{D}, ―L_{D}―NR_{D}R_{D'} and ―L_{D}―C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene, and wherein R_{D} and R_{D'} are as defined immediately above in this embodiment;
or R₄ and R₅, together with the N attached thereto, form a heterocyclyl (e.g., 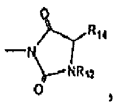 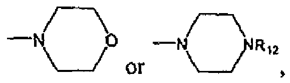 wherein R₁₂ is selected from the group consisting of N-protecting group,
hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆―O―R₈, ―L₆―C(O)R₈, ―L₆―C(O)OR₈, ―L₆―OC(O)R₈, ―L₆―C(O)NR₈R₉, ―N(R₉)C(O)OR₈, ―L₆―C(O)―L_{6'}―NR₈R₉, -L₆―C(O)―L_{6'}―N(R₉)C(O)OR₈, ―L₆―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, and j, R₈, R₉, R₁₀, L₆ and L_{6'} are as defined immediately above in this embodiment, and wherein R₁₄ is hydrogen, C₁-C₆alkyl, C₂-C₆alkenyl or C₂-C₆alkynyl);
wherein at each occurrence R₄ and R₅ (or L₁, Rᵥ, L_{D}, L₆, L_{6'}, L₉, R₃, R₄, R₅, R₆, R₈, R₉, R₁₀, R₁₂, R₁₄ R_{A2}, R_{w}, R_{D} and R_{D'}) are each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
wherein each carbocyclyl moiety in A₂, Z, (L₃)ₚ and N(R₄R₅) is independently selected at each occurrence from 5-, 6- or 7-membered carbocyclyls (e.g., cyclopentyl, cyclopentenyl, cyclohexyl or phenyl), and each heterocyclyl moiety in A₂, Z, (L₃)ₚ and N(R₄R₅) is independently selected at each occurrence from 5-, 6- or 7-membered heterocyclyls (e.g., dioxanyl, dithianyl, dihydrofuranyl, tetrahydrofuranyl, furanyl, furazanyl, thiazolyl, imidazolyl, isoxazolyl, isoxazolinyl, isoxazolidinyl, isothiazolyl, morpholinyl, 3-oxo-morpholinyl, oxazolyl, oxazolinyl, oxadiazolyl, oxazolidinyl, piperidinyl, piperazinyl, piperidyl, pyrimidinyl, pyrazinyl pyrazolyl, pyridyl, pyridazinyl, pyrrolidinyl, pyridinyl, pyrrolyl, tetrazolyl, tetrahydropyranyl, thiadiazolyl, thiadiazolidinyl, thiazolinyl, thiazolidinyl, thienyl, triazinyl, or triazolyl); and
wherein each carbocyclyl and heterocyclyl moiety in N(R₄R₅) (or in A₂, Z, (L₃)ₚ, and N(R₄R₅)) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{H}―O―R_{K}, ―L_{H}―S―R_{K}, ―L_{H}―C(O)R_{K}, ―L_{H}―OC(O)R_{K}, ―L_{H}―C(O)OR_{K}, ―L_{H}―NR_{K}R_{K'}, ―L_{H}― S(O)R_{K}, ―L_{H}―SO₂R_{K}, ―L_{H}―C(O)NR_{K}R_{K'}, ―L_{H}―N(R_{K})C(O)R_{K'}, ―L_{H}―NR_{K}SO₂R_{K'} and ―L_{H}―NR_{K}SO₂NR_{K'}R_{K''}, wherein L_{H} is independently selected at each occurrence from a bond, C₁-C₆ alkylene, C₂-C₆alkenylene or C₂-C₆alkynylene, and R_{K}, R_{K'} and R_{K''} are each independently selected at each occurrence from the group consisting of hydrogen, C₁₋C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁₋C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl.

In a non-limiting example, k is 0, p is 0, and at least one of R₄ or R₅ is selected from the group consisting of carbocyclylC₁-C₆alkyl (e.g., benzyl), heterocycloC₁-C₆alkyl, ―L₆―C(O)R_{8A}, ―C(O)OR_{8A}, ―OC(O)R_{8A}, ―C(O)NR_{8A}R₉, ―C(O)―L_{6'}―NR_{8A}R₉, ―C(O)―L_{6'}―N(R₉)C(O)OR_{8A}, ―C(O)―L_{6'}-N(R₉)C(O)NR_{8A}R₁₀, ―L₆―S(O)ⱼR_{8A}, ―L₆―N(R₉)S(O)ⱼR_{8A}, ―L₆―S(O)ⱼNR_{8A}R₉ and ―L₆―N(R₉)S(O)₂NR_{8A}R₁₀, wherein R_{8A} is C₁-C₆alkyl, C₂-C₆alkenyl, C₂-C₆alkynyl, carbocyclyl, carbocyclylC₁-C₆alkyl, heterocyclyl or heterocycloC₁-C₆alkyl.

In another non-limiting example, k is 0, p is 1, and L₃ is ―L₅―W―L_{5'}―, wherein L₅ is a bond, W is ―N(R_{w})CO―, and R_{w} is hydrogen, C₁-C₆alkyl, C₂-C₆alkenyl or C₂-C₆alkynyl, and wherein L_{5'} is C₁-C₆alkylene optionally substituted with at least one moiety selected from carbocyclyl (e.g., phenyl or cyclohexyl), carbocyclylC₁-C₆alkyl (e.g., benzyl), heterocyclyl or heterocycloC₁-C₆alkyl.

In yet another non-limiting example, k is 0, Z is 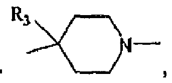 p is 1, L3 is ―L₅―W―L_{5'}―, wherein L₅ and W are bonds, L_{5'}, is C₁-C₆alkylene, and R₃ is carbocyclylC₁-C₆alkyl (e.g., benzyl) or heterocycloC₁-C₆alkyl.

In still another non-limiting example, R₁ is thiazolyl, L₁ is ―CH₂―, R_{A2} is H, k and p are 0, and Zis 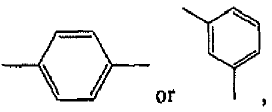 wherein at least one of R₄ or R₅ is selected from the group consisting of carbocyclylC₁-C₆alkyl (e.g., benzyl), heterocycloC₁-C₆alkyl, ―C(O)R_{8A}, ―C(O)OR_{8A}, ―OC(O)R_{8A}, ―C(O)NR_{8A}R₉, ―C(O)―C₁-C₆alkylene―NR_{8A}R₉, ―C(O)C₁―C₆alkylene―N(R₉)C(O)OR_{8A}, ―C(O)―C₁-C₆ alkylene―N(R₉)C(O)NR_{8A}R₁₀, ―S(O)ⱼR_{8A} and ―S(O)ⱼNR_{8A}R₉, and wherein R_{8A} is C₁-C₆alkyl, C₂-C₆ alkenyl, C₂-C₆alkynyl, carbocyclyl, carbocyclylC₁-C₆alkyl, heterocyclyl or heterocycloC₁-C₆alkyl. For instance, R_{8A} can be benzyl or pyridylmethyl, wherein the phenyl or pyridyl moiety comprised therein can be optionally substituted with at least one moiety selected from halogen, oxo, thioxo, hydroxy, nitro, cyano, amino, C₁-C₆alkyl, C₂-C₆alkenyl or C₂-C₆alkynyl.

In still yet another non-limiting example, R₁ is thiazolyl, L₁ is ―CH₂―, R_{A2} is H, k is 0, Z is 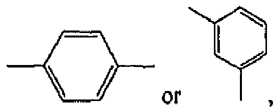 and p is I, wherein L₃ is ―L₅―W―L_{5'}―, L₅ is a bond, W is -N(H)CO-, and L_{5'} is C₁-C₃alkylene (e.g., -CH₂-) substituted with carbocyclyl (e.g., phenyl or cyclohexyl), carbocyclylC₁-C₆alkyl (e.g., benzyl or cyclohexylmethyl), heterocyclyl or heterocycloC₁-C₆alkyl, wherein at least one of R₄ or R₅ is selected from the group consisting of carbocyclylC₁-C₆alkyl (e.g., benzyl), heterocycloC₁-C₆alkyl, ―C(O)R_{8A}, ―C(O)OR_{8A}, ―OC(O)R_{8A}, ―C(O)NR_{8A}R₉, ―C(O)―C₁-C₆alkylene―NR_{8A}R₉, ―C(O)―C₁-C₆alkylene―N(R₉)C(O)OR_{8A}, ―C(O)―C₁-C₆alkylene―N(R₉)C(O)NR_{8A}R₁₀, ―S(O)ⱼR_{8A} and ―S(O)ⱼNR_{8A}R₉, and wherein R_{8A} is selected from C₁-C₆alkyl, C₂-C₆alkenyl, C₂-C₆alkynyl, carbocyclyl, carbocyclylC₁-C₆alkyl, heterocyclyl or heterocycloC₁-C₆alkyl.

In yet another non-limiting example, R₁ is thiazolyl, L₁ is ―CH₂―, R_{A2} is H, k is 0, Z is 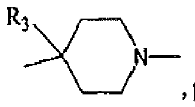 p is 1, and L3 is ―L₅―W―L_{5'}―, wherein L₅ and W are bonds, L_{5'} is C₁-C₃alkylene, and R₃ is benzyl.

In any of the above examples, R₅ or R₄ can be, without limitation, (H₅-H₆heterocyclo)C₁-C₆alkoxycarbonyl, wherein the H₅-H₆heterocyclyl moiety comprises at least one nitrogen ring atom. For instance, R₅ or R₄ can be thiazolylC₁-C₆alkoycarbonyl, such as thiazolylmethoxycarbonyl.

Non-limiting examples of the compounds of this embodiment include:
[3-(2-tert-Butoxycarbonylamino-3-phenyl-propionylamino)-phenyl]-carbamic acid thiazol-5-ylmethyl ester;
1,3-thiazol-5-ylmethyl 3-({2-[(tert-butoxycarbonyl)amino]-2-phenylethanoyl}amino)phenyl carbamate;
N²-(tert-butoxycarbonyl)-N¹-(3-{[(1,3-thiazol-5-ylmethoxy)carbonyl]amino}phenyl) isoleucinamide;
N²-(tert-butoxycarbonyl)-N¹-(3-{[(1,3-thiazol-5-ylmethoxy)carbonyl]amino}phenyl)valinamide;
N²-(tert-butoxycarbonyl)-3-methyt-N¹-(3-{[(1,3-thiazol-5-ylmethoxy)carbonyl]amino}phenyl) valinamide; and
1,3-thiazol-5-ylmethyl 3-({2-[(tert-butoxycarbonyl)amino]-2-cyclohexylethanoyl}amino)phenyl carbamate.

Preferred exemplary compounds of this embodiment include, but are not limited to:
1,3-thiazol-5-ylmethyl 4-[benzyl(pyridin-4-ylacetyl)amino]phenylcarbamate;
benzyl benzyl(4-{[(1,3-thiazol-5-ylmethoxy)carbonyl]amino}phenyl)carbamate;
*tert*-butyl 3-{[(1,3-thiazol-5-ylmethoxy)carbonyl]amino}phenylcarbamate;
1,3-thiazol-5-ylmethyl 4-{benzyl[(3-chlorophenyl)acetyl]amino}phenylcarbamate;
1,3-thiazol-5-ylmethyl 3-{[(3-chlorophenyl)acetyl]amino}phenylcarbamate;
1,3-thiazol-5-ylmethyl 3-[(pyridin-4-ylacetyl)amino]phenylcarbamate;
N²-(tert-butoxycarbonyl)-N²-methyl-N¹-(3-{[(1,3-thiazol-5-ylmethoxy)carbonyl amino}phenyl)-L-valinamide;
1,3-thiazol-5-ylmethyl 3-({[(3-methylphenyl)amino]carbonyl}amino)phenylcarbamate;
[3-((2S)-2-tert-Butoxycarbonylamino-3-phenyl-propionylamino)-phenyl]-carbamic acid thiazol-5-ylmethyl ester;
1,3-thiazol-5-ylmethyl 3-({(2S)-2-[(tert-butoxycarbonyl)amino]-2-phenylethanoyl}amino)phenyl carbamate;
1,3-thiazol-5-ylmethyl 3-[(3,3-dimethylbutanoyl)amino]phenylcarbamate;
N²-(tert-butoxycarbonyl)-N¹-(3-{[(1,3-thiazol-5-ylmethoxy)carbonyl]amino}phenyl)-L-isoleucinamide;
N²-(tert-butoxycarbonyl)-N¹-(3-{[(1,3-thiazol-5-ylmethoxy)carbonyl]amino}phenyl)-L-valinamide;
N²-(tert-butoxycarbonyl)-3-methyl-N¹-(3-{[(1,3-thiazol-5-ylmethoxy)carbonyl]amino}phenyl)-L-valinamide;
1,3-thiazol-5-ylmethyl 3-({(2S)-2-[(tert-butoxycarbonyl)amino]-2-cyclohexylethanoyl}amino) phenylcarbamate;
dibenzyl 2-(4-benzyl-4-{[(1,3-thiazol-5-ylmethoxy)carbonyl]amino}piperidin-1-yl)ethylimido dicarbonate;
1,3-thiazol-5-ylmethyl 4-benzyl-1-(2-{[(4-methylphenyl)sulfonyl]amino}ethyl)piperidin-4-yl carbamate;
1,3-thiazol-5-ylmethy 4-benzyl-1-(2-{[(1,3-thiazol-5-ylmethoxy)carbonyl]amino}ethyl) piperidin-4-ylcarbamate;
1,3-thiazol-5-ylmethyl 4-benzyl-1-[2-(dibenzylamino)ethyl]piperidin-4-ylcarbamate;
1,3-thiazol-5-ylmethyl 1-(2-aminoethyl)-4-benzylpiperidin-4-ylcarbamate.

In still yet another embodiment, the present invention features compounds af formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, ―Lₛ―O―Rₛ, ―Lₛ―S―Rₛ, ―Lₛ―C(O)Rₛ, ―Lₛ―OC(O)Rₛ, ―Lₛ―C(O)OR₅, ―Lₛ―NRₛR_{s'}, ― Lₛ―S(O)Rₛ, ―Lₛ―SO₂Rₛ, ―Lₛ―C(O)NRₛR_{s'}, ―Lₛ―N(Rₛ)C(O)R_{s'}, ―Lₛ―NRₛSO₂R_{s'} and ―Lₛ―NRₛSO₂NR_{s'}R_{s''}, wherein Lₛ is independently selected at each occurrence from a bond, C₁-C₁₀alkylene, C₂-C₁₀alkenylene or C₂-C₁₀alkynylene, and Rₛ, R_{s'} and R_{s''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is ―O―L_{A1}―, wherein L_{A1} is a bond;
X is O;
A₂ is ―L_{A2}―N(R_{A2})―, wherein L_{A2} is a bond, and R_{A2} is hydrogen, C₁-C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents ―L₉―V―L_{9'}―, wherein L₉ is independently selected from a bond, C₁-C₆alkylene, C₂-C₆alkenylene or C₂-C₆alkynylene, L_{9'} is a bond, and V is selected from the group consisting of a bond or ―C(O)N(Rᵥ)―, and wherein Rᵥ is selected from hydrogen, C₁-C₆alkyl, C₂-C₆alkenyl or C₂-C₆alkynyl;
Z is ―C(R₂R₃)―, wherein R₂ and R₃ are independently selected from carbocycloC₁-C₆alkyl (e.g., C₆-C₁₀arylC₁-C₆alkyl, such as benzyl) or heterocyclylC₁-C₆alkyl,
p is 0 or 1;
L₃ represents:
- L₅―W―L_{5'}―, wherein W is a bond; or
- L₅―C(R₆R₇)―L_{5'}―, and R_{A2} and R₇ are bonded together to form ―C(O)O―; or
- L₅―C(R₆R₇)―L_{5'}―,wherein R₄ and R₇ are bonded together to form ―OC(O)―;
   wherein L₅ and L_{5'} are each independently selected from a bond, C₁-C₆alkylene, C₂-C₆ alkenylene or C₂-C₆alkynylene, and are each independently optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'};
   wherein R_{D} and R_{D'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆ alkyl, C₁-C₆alkylcarbonyloxy, C₁-C₆alkylcarbonyloxyC₁-C₆alkyl and C₁-C₆ alkylaminoC₁-C₆alkyl; and
   R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆― O―R₈, ―L₆―C(O)R₈, ―C(O)OR₈, ―OC(O)R₈, ―C(O)NR₈R₉, ―N(R₉)C(O)OR₈, ―C(O)―L_{6'}―O―R₈, ―C(O)― L_{6'}―NR₈R₉, ―C(O)―L_{6'}―N(R₉)C(O)OR₈, ―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'} and ―L_{D}―C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene, and wherein R_{D} and R_{D'} are as defined immediately above in this embodiment;
   or R₄ and R₅, together with the N attached thereto, form a heterocyclyl (e.g., 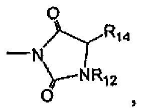 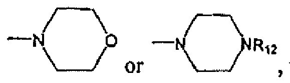 wherein R₁₂ is selected from the group consisting of N-protecting group,
   hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆―O―R₈, ―L₆―C(O)R₈, ―L₆―C(O)OR₈, ―L₆―OC(O)R₈, ―L₆―C(O)NR₈R₉, ―N(R₉)C(O)OR₈, ―L₆―C(O)―L_{6'}―NR₈R₉, ―L₆―C(O)―L₆―N(R₉)C(O)OR₈, ―L₆―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, and j, R₈, R₉, R₁₀, L₆ and L_{6'} are as defined immediately above in this embodiment, and wherein R₁₄ is hydrogen, C₁-C₆alkyl, C₂-C₆alkenyl or C₂-C₆alkynyl);
   wherein at each occurrence R₄ and R₅ (or L₁, Rv, L_{D}, L₆, L_{6'}, L₉, R₂, R₃, R₄, R₅, R₆, R₈, R₉, R₁₀, R₁₂, R₁₄, R_{A2}, R_{D} and R_{D'}) are each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A₂, Z, and N(R₄R₅) is independently selected at each occurrence from 5-, 6- or 7-membered carbocyclyls (e.g., cyclopentyl, cyclopentenyl, cyclohexyl or phenyl), and each heterocyclyl moiety in A₂, Z, and N(R₄R₅) is independently selected at each occurrence from 5-, 6- or 7-membered heterocyclyls (e.g., dioxanyl, dithianyl, dihydrofuranyl, tetrahydrofuranyl, furanyl, furazanyl, thiazolyl, imidazolyl, isoxazolyl, isoxazolinyl, isoxazolidinyl, isothiazolyl, morpholinyl, 3-oxo-morpholinyl, oxazolyl, oxazolinyl, oxadiazolyl, oxazolidinyl, piperidinyl, piperazinyl, piperidyl, pyrimidinyl, pyrazinyl pyrazolyl, pyridyl, pyridazinyl, pyrrolidinyl, pyridinyl, pyrrolyl, tetrazolyl, tetrahydropyranyl, thiadiazolyl, thiadiazolidinyl, thiazolinyl, thiazolidinyl, thienyl, triazinyl, or triazolyl); and
   wherein each carbocyclyl and heterocyclyl moiety in N(R₄R₅) (or in A₂, Z, and N(R₄R₅)) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{H}―O―R_{K}, ―L_{H}―S―R_{K}, ―L_{H}―C(O)R_{K}, ―L_{H}―OC(O)R_{K}, ―L_{H}―C(O)OR_{K}, ―L_{H}―NR_{K}R_{K'}, ―L_{H}― S(O)R_{K}, ―L_{H}―SO₂R_{K}, ―L_{H}―C(O)NR_{K}R_{K'}, ―L_{H}―N(R_{K})C(O)R_{K'}, ―L_{H}―NR_{K}SO₂R_{K'}, and ―L_{H}―NR_{K}SO₂NR_{K'}R_{K''}, wherein L_{H} is independently selected at each occurrence from a bond, C₁-C₆alkylene, C₂-C₆alkenylene or C₂-C₆alkynylene, and R_{K}, R_{K'} and R_{K''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl.

In a non-limiting example, k is 0, p is 1, and L₃ is ―L₅―W―L_{5'}―, wherein L₅ and W are bonds, and L_{5'} is C₁-C₆alkylene, C₂-C₆alkenylene or C₂-C₆alkynylene, wherein R₄ and R₅, together with the N attached thereto, form a heterocyclyl (e.g., 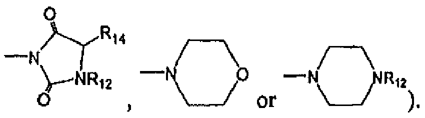 wherein R₆ is hydrogen, C₁-C₆alkyl, C₂-C₆alkenyl or C₂-C₆alkynyl;

In another non-limiting example, R₁ is thiazolyl, L₁ is ―CH₂―, R_{A2} is H, k is 0, R₂ and R₃ are benzyl, p is 1, and L₃ is ―L₅―W―L_{5'}―, wherein L₅ and W are bonds, and L_{5'} is C₁-C₆alkylene, wherein R₄ and R₅, together with the N attached thereto, form a heterocyclyl selected from 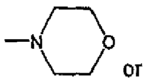 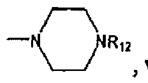 wherein R₁₂ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―C(O)R₈, ―C(O)OR₈, ―C(O)NR₈R₉, ―C(O)―L_{6'}―NR₈R₉, ―C(O)―L₆―N(R₉)C(O)OR₈, ―C(O)―L_{6'}―N(R₉)C(O)NR₈R₁₀, ―S(O)ⱼR₈, and ―S(O)ⱼNR₈R₉, and wherein j, R₈, R₉, R₁₀ and L_{6'} are as defined immediately above in this embodiment. Each carbocyclyl and heterocyclo moiety in R₁₂ can be optionally substituted with at least one moiety selected from the group consisting of halogen, oxo, thioxo, hydroxy, nitro, cyano, amino, formyl, C₁-C₆alkyl, C₂-C₆alkenyl, C₂-C₆alkynyl, ―L_{H}―O―R_{K}, ―L_{H}―S―R_{K}, ―L_{H}―C(O)R_{K}, ―L_{H}―OC(O)R_{K}, ―L_{H}―C(O)OR_{K}, ―L_{H}―NR_{K}R_{K'}, ―L_{H}―S(O)R_{K}, ―L_{H}―SO₂R_{K}, ―L_{H}―C(O)NR_{K}R_{K'}, ―L_{H}―N(R_{K})C(O)R_{K'}, ―L_{H}― NR_{K}SO₂R_{K'} and ―L_{H}―NR_{K}SO₂NR_{K'}R_{K''}, wherein L_{H}, R_{K}, R_{K'} and R_{K''} are as defined immediately above in this embodiment.

Non-limiting examples of the compounds of this embodiment include:
1,3-thiazol-5-ylmethyl 1,1-dibenzyl-3-[4-(4-nitrophenyl)piperazin-1-yl]propylcarbamate;
1,3-thiazol-5-ylmethyl 1,1-dibenzyl-3-morpholin-4-ylpropylcarbamate;
1,3-thiazol-5-ylmethyl 4-(3-benzyl-4-phenyl-3-{[(1,3-thiazol-5-ylmethoxy)carbonyl]amino} butyl)piperazine-1-carboxylate;
1,3-thiazol-5-ylmethyl 1,1-dibenzyl-3-{4-[(4-methylphenyl)sulfonyl]piperazin-1-yl}propyl carbamate;
ethyl 4-[4-(3-benzyl-4-phenyl-3-{[(1,3-thiazol-5-ylmethoxy)carbonyl]amino} butyl)piperazin-1-yl]benzoate; and
1,3-thiazol-5-ylmethyl 1,1-dibenzyl-3-piperazin-1-ylpropylcarbamate.

In yet another embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, ―Lₛ―O―Rₛ, ―Lₛ―S―Rₛ, ―Lₛ―C(O)Rₛ, ―Lₛ―OC(O)Rₛ, ―Lₛ―C(O)ORₛ, ―Lₛ―NRₛR_{s'}, ―Lₛ―S(O)Rₛ, ―Lₛ―SO₂Rₛ, ―Lₛ―C(O)NRₛR_{s'}, ―Lₛ―N(Rₛ)C(O)R_{s'}, ―Lₛ―NRₛSO₂R_{s'} and ―Lₛ―NRₛSO₂NR_{s'}R_{s''}, wherein Lₛ is independently selected at each occurrence from a bond, C₁-C₁₀ alkylene, C₂-C₁₀alkenylene or C₂-C₁₀alkynylene, and Rₛ, R_{s'} and R_{s''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is ―O―L_{A1}―, wherein L_{A1} is a bond;
X is O;
A₂ is ―L_{A2}―N(R_{A2})―, wherein L_{A2} is a bond, and R_{A2} is hydrogen, C₁₋C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents ―L₉―V―L_{9'}―, wherein L₉ is independently selected from a bond, C₁-C₆alkylene, C₂-C₆alkenylene or C₂-C₆alkynylene, L_{9'} is a bond, and V is selected from the group consisting of a bond or ―C(O)N(Rᵥ)―, and wherein Rᵥ is selected from hydrogen, C₁-C₆alkyl, C₂-C₆alkenyl or C₂-C₆alkynyl;
Z, taken together with (L₃)ₚ and N(R₄R₅), forms 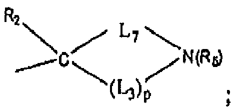 R₂ is selected from carbocycloC₁-C₆alkyl (e.g., C₆-C₁₀arylC₁-C₆alkyl , such as benzyl) or heterocyclylC₁-C₆alkyl;
L₇ is C₁-C₄alkylene, C₂-C₄alkenylene or C₂-C₄alkynylene, and is optionally substituted with at least one substituent selected from the group halogen, oxo, thioxo, hydroxy, nitro, cyano, amino, -O-R_{D}, ―S―R_{D}, ―C(O)R_{D}, ―OC(O)R_{D}, ―C(O)OR_{D}, ―NR_{D}R_{D'} and ―C(O)NR_{D}R_{D'}, wherein R_{D} and R_{D'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
p is 0 or 1;
L₃ is C₁-C₄alkylene, C₂-C₄alkenylene or C₂-C₄alkynylene, and are each independently optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'};
R₅ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆―O―R₈, ―L₆―C(O)R₈, ―C(O)OR₈, ―OC(O)R₈, ―C(O)NR₈R₉, ―N(R₉)C(O)OR₈, ―C(O)―L₆―O―R₈, ―C(O)―L_{6'}―NR₈R₉, ―C(O)―L_{6'}― N(R₉)C(O)OR₈, ―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆ alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'} and ―L_{D}― C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene, and wherein R_{D} and R_{D'} are as defined immediately above in this embodiment;
wherein R₅ (or at each occurrence L₁, Rᵥ, L_{D}, L₆, L_{6'}, L₉, R₂, R₅, R₈, R₉, R₁₀, R_{A2}, R_{D} and R_{D'}) is each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆ thioalkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆ alkylaminoC₁-C₆alkyl;
wherein each carbocyclyl moiety in A₂, Z, and N(R₄R₅) is independently selected at each occurrence from 5-, 6- or 7-membered carbocyclyls (e.g., cyclopentyl, cyclopentenyl, cyclohexyl or phenyl), and each heterocyclyl moiety in A₂, Z, and N(R₄R₅) is independently selected at each occurrence from 5-, 6- or 7-membered heterocyclyls; and
wherein each carbocyclyl and heterocyclyl moiety in R₅ (or in A₂, R₂, and 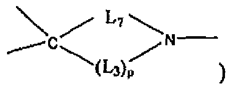 is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆alkynylene, and R_{K}, R_{K'} and R_{K"} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl and C₁-C₆alkylaminoC₁-C₆alkyl.

In a non-limiting example, k is 0, p is 1, L₇ is C₁-C₃alkylene, and L₃ is C₁-C₃alkylene.

In another non-limiting example, R₁ is thiazolyl, L₁ is -CH₂-, R_{A2} is H, k is 0, R₂ is benzyl, p is 1, L₇ is C₁-C₃alkylene (e.g., -CH₂-CH₂-), and L₃ is C₁-C₃alkylene (e.g., -CH₂-CH₂-).

In any of the above examples, R₅ preferably includes at least one carbocyclyl or heterocyclyl moiety. For instance, R₅ can be, without limitation, carbocyclylC₁-C₆alkyl (e.g., benzyl), heterocycloC₁-C₆alkyl, -C(O)R₈, -C(O)OR₈, -C(O)NR₈R₉, -C(O)-L_{6'}-NR₈R₉, -C(O)-L_{6'}-N(R₉)C(O)OR₈, -C(O)-L_{6'}-N(R₉)C(O)NR₈R₁₀, -S(O)ⱼR₈, or -S(O)ⱼNR₈R₉, wherein R₈ is carbocyclyl, carbocyclylC₁-C₆alkyl, heterocyclyl or heterocycloC₁-C₆alkyl, and j, R₉, R₁₀, and L_{6'} are as defined immediately above in this embodiment. In one specific instance, R₅ is thiazolylC₁-C₆alkoycarbonyl, such as thiazolylmethoxycarbonyl.

Non-limiting examples of the compounds of this embodiment include:
1,3-thiazol-5-ylmethyl 4-benzyl-1-[(4-methylphenyl)sulfonyl]piperidin-4-ylcarbamate;
1,3-thiazol-5-ylmethyl 4-benzyl-4-{[(1,3-thiazol-5-ylmethoxy)carbonyl]amino} piperidine-1-carboxylate; and
1,3-thiazol-5-ylmethyl 1,4-dibenzylpiperidin-4-ylcarbamate.

In still another embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, -Lₛ-O-Rₛ, -Lₛ-S-Rₛ, -Lₛ-C(O)Rₛ, -Lₛ-OC(O)Rₛ, -Lₛ-C(O)ORₛ, -Lₛ-NRₛR_{s'}, -Lₛ-S(O)Rₛ, -Lₛ-SO₂Rₛ, -Lₛ-C(O)NRₛR_{s'}, -Lₛ-N(Rₛ)C(O)R_{s'}, -Lₛ-NRₛSO₂R_{s'} and -Lₛ-NRₛSO₂NR_{s'}R_{s"}, wherein Lₛ is independently selected at each occurrence from a bond, C₁-C₁₀ alkylene, C₂-C₁₀alkenylene or C₂-C₁₀alkynylene, and Rₛ, R_{s'} and R_{s"} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is -O-L_{A1}-, wherein L_{A1} is a bond;
X is O;
A₂ is -L_{A2}-N(R_{A2})-, wherein L_{A2} is a bond, and R_{A2} is hydrogen, C₁-C₆alkyl, C₂-C₆alkenyl, C₂-C₆ alkynyl, carbocyclylC₁-C₆alkyl (e.g., phenylC₁-C₆alkyl, such as benzyl) or heterocycloC₁-C₆alkyl;
k is 0 or 1;
L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(Rᵥ)-, and wherein Rv is selected from hydrogen, C₁-C₆alkyl, C₂-C₆alkenyl or C₂-C₆alkynyl;
Z is -C(R₂R₃)-, wherein R₂ is carbocyclylC₁-C₆alkyl (e.g., phenylC₁-C₆alkyl, such as benzyl) or heterocycloC₁-C₆alkyl, and R₃ is hydrogen, C₁-C₆alkyl, C₂-C₆alkenyl or C₂-C₆alkynyl;
p is 0 or 1;
L₃ is C₁-C₆alkylene, C₂-C₆alkenylene or C₁-C₆alkynylene, and is optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D'} and -C(O)NR_{D}R_{D'}, wherein R_{D} and R_{D'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkyxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl; and
R₅ is R_{E}―carbocyclylC₁-C₆alkyl or R_{E}―heterocycloC₁-C₆alkyl, wherein R_{E} is carbocyclyl, heterocyclyl, carbocyclylC₁-C₆alkyl or heterocycloC₁-C₆alkyl;
R₄ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆-O-R₈, -L₆-C(O)R₈, -C(O)OR₈, -OC(O)R₈, -C(O)NR₈R₉, -N(R₉)C(O)OR₈, -C(O)-L₆-O-R₈, -C(O)-L_{6'}-NR₈R₉, -C(O)-L₆-N(R₉)C(O)OR₈, -C(O)-L₆-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆ alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene, and wherein R_{D} and R_{D'} are as defined immediately above in this embodiment;
wherein at each occurrence R₄ and R₅ (or L₁, R_{V}, L_{D}, L₆, L_{6'}, L₉, R₂, R₃, R₄, R_{5,} R₈, R₉, R₁₀, R_{A2}, R_{E}, R_{D} and R_{D'}) are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
wherein each carbocyclyl moiety in A₂, Z, and N(R₄R₅) is independently selected at each occurrence from 5-, 6- or 7-membered carbocyclyls, and each heterocyclyl moiety in A₂, Z, and N(R₄R₅) is independently selected at each occurrence from 5-, 6- or 7-membered heterocyclyls; and
wherein each carbocyclyl and heterocyclyl moiety in N(R₄R₅) (or in A₂, Z, and N(R₄R₅)) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{R}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl.

In a non-limiting example, R₂ is carbocyclylC₁-C₆alkyl (e.g., benzyl), and R₅ is heterocyclocarbocyclylC₁-C₆alkyl (e.g., pyridylbenzyl).

In another non-limiting example, R₁ is thiazolyl, L₁ is -CH₂-, R_{A2} is H, k is 0, R₂ is benzyl, p is 1, R₅ is pyridylbenzyl, and L₃ is C₁-C₃alkylene (e.g., -CH₂-CH₂-) optionally substituted with halogen, oxo, thioxo, hydroxy, nitro, cyano, amino or formyl.

Non-limiting examples of the compounds of this embodiment include:
1,3-thiazol-5-ylmethyl 1-benzyl-2-hydroxy-3-[isobutyl(4-pyridin-2-ylbenzyl)amino]propyl carbamate; and
tert-butyl 2-(2-hydroxy-4-phenyl-3-{[(1,3-thiazol-5-ylmethoxy)carbonyl]amino}butyl)-2-(4-pyridin-2-ylbenzyl)hydrazinecarboxylate.

Preferred compounds of this embodiment include, but are not limited to:
1,3-thiazol-5-ylmethyl (1S,2R)-1-benzyl-2-hydroxy-3-[isobutyl(4-pyridin-2-ylbenzyl)amino] propylcarbamate; and
tert-butyl 2-((2S,3S)-2-hydroxy-4-phenyl-3-{[(1,3-thiazol-5-ylmethoxy)carbonyl]amino} butyl)-2-(4-pyridin-2-ylbenzyl)hydrazinecarboxylate.

In another embodiment, the present invention features compounds of formula I, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆ alkenyl, C₂-C₆alkynyl, -Lₛ-O-Rₛ, -Lₛ-S-Rₛ, -Lₛ-C(O)Rs, -Lₛ-OC(O)Rₛ, -Lₛ-C(O)ORₛ, -Ls-NRₛR_{s'}, -Lₛ-S(O)Rₛ, -Lₛ-SO₂Rₛ, -Lₛ-C(O)NRₛR_{s'}, -Lₛ-N(Rₛ)C(O)R_{s'}, -Lₛ-NRₛSO₂R_{s'} and -Lₛ-NRₛSO₂NR_{s'}R_{s"}, wherein Lₛ is independently selected at each occurrence from a bond, C₁-C₁₀alkylene, C₂-C₁₀alkenylene or C₂-C₁₀alkynylene, and Rₛ, R_{s'} and R_{s"} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is -O-L_{A1}-, wherein L_{A1} is a bond;
X is O;
A₂ is -L_{A2}-N(R_{A2})-, wherein L_{A2} is a bond, and R_{A2} is selected from the group consisting of hydrogen, C₁-C₆alkyl, C₂-C₆alkenyl, C₂-C₆alkynyl, carbocyclyl, carbocyclylC₁-C₆alkyl (e.g., benzyl, cyclohexylmethyl or naphthylmethyl), heterocyclyl, heterocycloC₁-C₆alkyl (e.g., pyridyl, triazolyl or quinolinyl), -L_{F}-O-R_{F}, -L_{F}-S-R_{F}, -L_{F}-C(O)R_{F}, -L_{F}-C(O)OR_{F}, -L_{F}-OC(O)R_{F}, -L_{F}-NR_{F}R_{F'}, -L_{F}-S(O)R_{F}, -L_{F}-SO₂R_{F}, -L_{F}-C(O)NR_{F}R_{F'}, -L_{F}-N(R_{F})C(O)R_{F'}, -L_{F}-N(R_{F})SO₂R_{F'}, -L_{F}-(R_{F})SO₂NR_{F'}R_{F"}, -L_{E}-Carbocyclyl-L₄-Y-L_{4'}-R_{E} and -L_{E}-heterocyclyl-L₄-Y-L_{4'}-R_{E}, wherein L_{F}, L_{E}, L₄ and L_{4'} are each independently selected at each occurrence from a bond, C₁-C₆alkylene, C₂-C₆alkenylene or C₂-C₆ alkynylene, and R_{F}, R_{F'} and R_{F"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆ alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₆ alkylene, C₂-C₆alkenylene, C₂-C₆alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆ alkenyl or C₂-C₆alkynyl, and wherein R_{E} is independently selected at each occurrence from the group consisting of carbocyclyl, heterocyclyl, carbocyclylC₁-C₆alkyl, heterocycloC₁-C₆alkyl, C₁-C₆alkylene, C₂-C₆alkenylene and C₂-C₆alkynylene;
k is 0 or 1;
L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(R_{V})-, and wherein R_{V} is selected from hydrogen, C₁-C₆alkyl, C₂-C₆alkenyl or C₂-C₆alkynyl;
Z is a bond;
p is 1, 2, or 3;
L₃ at each occurrence independently represents -L₅-W-L_{5'}-, wherein at each occurrence W is independently selected from the group consisting of a bond, C₁-C₆alkylene, C₂-C₆alkenylene, C₂-C₆ alkynylene, -S-, -O- and -C(O)-, and wherein L₅ and L_{5'} are each independently selected at each occurrence from a bond, C₁-C₆alkylene, C₂-C₆alkenylene or C₂-C₆alkynylene, and are each independently optionally substituted with at least one substituent selected from the group consisting of halogen, oxo, thioxo, hydroxy, nitro, cyano, amino, -O-R_{M}, -S-R_{M}, -C(O)R_{M}, -OC(O)R_{M}, -C(O)OR_{M}, -NR_{M}R_{M'}, N(R_{M})C(O)R_{M'}, N(R_{M})C(O)OR_{M'}, and -C(O)NR_{M}R_{M'}, wherein R_{M} and R_{M'} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆ alkylcarbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl;
R₄ is selected from the group consisting of hydrogen, C₁-C₆alkyl, C₂-C₆alkenyl, C₂-C₆alkynyl, carbocyclyl, carbocyclylC₁-C₆alkyl (e.g., benzyl), heterocyclyl, heterocycloC₁-C₆alkyl, -L_{F}-O-R_{F}, -L_{F}-S-R_{F}, -L_{F}-C(O)R_{F}, -L_{F}-C(O)OR_{F}, -L_{F}-OC(O)R_{F}, -L_{F}-NR_{F}R_{F'}, -L_{F}-S(O)R_{F}, -L_{F}-SO₂R_{F}, -L_{F}-C(O)NR_{F}R_{F'}, -L_{F}-N(R_{F})C(O)R_{F'}, -L_{F}-N(R_{F})SO₂R_{F'}, -L_{F}-N(R_{F})SO₂NR_{F'}R_{F"}, -L_{E}-carbocyclyl-L₄-Y-L₄-R_{E} and -L_{E}-heterocyclyl-L₄-Y-L_{4'}-R_{E}, wherein L_{F}, L_{E}, L₄, L_{4'}, R_{F}, R_{F'}, R_{F"}, Y and R_{E} are as defined immediately above in this embodiment;
R₅ is selected from the group consisting of hydrogen, C₁-C₆alkyl, C₂-C₆alkenyl, C₆-C₆alkynyl,
carbocyclyl, carbocyclylC₁-C₆alkyl, heterocyclyl, heterocycloC₁-C₆alkyl, -L_{E}-carbocyclyl-L₄-Y-L_{4'}-R_{E}, -L_{E}-heterocyclyl-L₄-Y-L_{4'}-R_{E}, -L₆-O-R₈, -L₆-C(O)R₈, -C(O)OR₈, -OC(O)R₈, -C(O)NR₈R₉, -N(R₉)C(O)OR₈, -C(O)-L_{6'}-O-R₈, -C(O)-L_{6'}-NR₈R₉, -C(O)-L_{6'}-N(R₉)C(O)OR₈, -C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆ alkylene, C₂-C₆alkenylene or C₂-C₆alkynylene, wherein R_{D} and R_{D'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl, and wherein L_{E}, L₄, L_{4'}, Y and R_{E} are as defined immediately above in this embodiment;
wherein at each occurrence R₄ and R₅ (or L₁, R_{V}, L_{D}, L₆, L_{6'}, L₉, L_{F}, L_{E}, L₄, L_{4'}, W, Y, R₄, R₅, R₈, R₉, R₁₀, R_{A2}, R_{E}, R_{F}, R_{F'}, R_{F"}, R_{D} and R_{D'}) are each independently optionally substituted with at least one substituent selected from the group consisting of halogen, oxo, thioxo, hydroxy, nitro, cyano, amino, -O- R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
wherein each carbocyclyl moiety in R_{A2}, L₃, R₄ and R₅ is independently selected at each occurrence from 5-, 6-, 7-, 8-, 9- or 10-membered carbocyclyls (e.g., C₅-C₁₀cycloalkyl, C₅₋C₁₀ cycloalkenyl or C₆-C₁₀aryl, such as cyclohexyl, phenyl or naphthyl), and each heterocyclyl moiety in R_{A2}, L₃, R₄ and R₅ is independently selected at each occurrence from 5-, 6-, 7-, 8-, 9- or 10-membered heterocyclyls (e.g., H₅-H₁₀heteroaryl, H₅-H₁₀heterocycloalkyl or H₅-H₁₀heterocycloalkenyl, such as pyridyl, triazolyl or quinolinyl); and
wherein each carbocyclyl and heterocyclyl moiety in R₄ and R₅ (or in R_{A2}, L₃, R₄ and R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K},-L_{H}-S-R_{K}, -L_{H}-C(O)R_{K}, -L_{H}-OC(O)R_{K}, ₋L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-(R_{K})C(O)R_{K'}, -L_{H}-NR_{K}SO₂R_{K'}, and -L_{H}-NR_{K}SO₂NR_{K'}R_{K"}, wherein L_{H} is independently selected at each occurrence from a bond, C₁-C₆alkylene, C₁-C₆alkenylene or C₂-C₆alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl.

In a non-limiting example, R_{A2} and R₄ are each independently selected from the group consisting of C₅-C₁₀carbocyclylC₁-C₆alkyl (e.g., benzyl, cyclohexylmethyl or naphthylmethyl), H₅-H₁₀ heterocycloC₁-C₆alkyl (e.g., pyridyl, triazolyl or quinolinyl), -L_{G}-C₅-C₇carbocyclyl-L₁₀-U-L_{10'}-R_{H} (e.g., benzoylbenzyl, benzyloxybenzyl, 1H-triazolylbenzyl, biphenylmethyl or pyridylbenzyl) and -L_{G}-H₅-H₇ heterocyclyl-L₁₀-U-L_{10'}-R_{H}, wherein at each occurrence L_{G} is independently C₁-C₃alkylene, L₁₀ and L_{10'} are each independently selected at each occurrence from a bond or C₁-C₃alkylene, and U is independently selected at each occurrence from the group consisting of a bond, -S-, -O-, -C(O)-, -C(O)O- and -OC(O)-, and wherein at each occurrence R_{H} is independently C₅-C₇carbocyclyl, H₅-H₇heterocyclyl, C₁-C₆alkyl, C₂-C₆alkenyl or C₂-C₆alkynyl. R_{A2} and R₄ can be the same or different.

In another non-limiting example, p is 1, L₅ and W are bonds, and L_{5'} is C₂-C₄alkylene (e.g., - CH₂-CH₂-) which is optionally substituted with at least one substituent selected from the group consisting of halogen, oxo, thioxo, hydroxy, nitro, cyano and amino.

In still another non-limiting example, R₁ is thiazolyl, L₁ is -CH₂-, k is 0, p is I, L₅ and W are bonds, and L_{5'} is C₂-C₄alkylene (e.g., -CH₂-CH₂-) which is optionally substituted with at least one substituent selected from the group consisting of halogen, oxo, thioxo, hydroxy, nitro, cyano and amino, wherein R_{A2} and R₄ are each independently selected from the group consisting of C₅-C₁₀carbocyclylC₁-C₆alkyl (e.g., benzyl, cyclohexylmethyl or naphthylmethyl), H₅-H₁₀heterocycloC₁-C₆alkyl (e.g., pyridyl, triazolyl or quinolinyl), -L_{G}-C₅-C₇carbocyclyl-L₁₀-U-L_{10'}-R_{H} (e.g., benzoylbenzyl, benzyloxybenzyl, 1H-triazolylbenzyl, biphenylmethyl or pyridylbenzyl) and -L_{G}-H₅-H₇heterocyclyl-L₁₀-U-L_{10'}-R_{H}, and wherein U, L_{G}, L₁₀, L_{10'} and R_{H} are as defined immediately above.

In yet another non-limiting example, R₁ is thiazolyl, L₁ is -CH₂-, k is 0, p is 1, L₅ and W are bonds, and L₅, is C₂-C₄alkylene (e.g., -CH₂-CH₂-) which is optionally substituted with at least one substituent selected from the group consisting of NR_{M}R_{M'}, N(R_{M})C(O)R_{M'} and N(R_{M})C(O)OR_{M'}, wherein at each occurrence R_{M} is independently hydrogen, C₁-C₆alkyl, C₂-C₆alkenyl or C₂-C₆alkynyl, and at each occurrence R_{M'} is independently C₁-C₆alkyl, C₂C₆alkenyl, C₂-C₆alkynyl, C₅-C₁₀carbocyclyl (e.g., phenyl), C₅-C₁₀carbocyclylC₁-C₆alkyl, H₅-H₁₀heterocyclyl or H₅-H₁₀heterocycloC₁-C₆alkyl, wherein R_{A2} and R₄ are each independently selected from the group consisting of C₅-C₁₀carbocyclylC₁-C₆alkyl (e.g., benzyl, cyclohexylmethyl or naphthylmethyl), H₅-H₁₀heterocycloC₁-C₆alkyl (e.g., pyridyl, triazolyl or quinolinyl), -L_{G}C₅-C₇carbocyclyl-L₁₀-U-L₁₀-R_{H} (e.g., benzoylbenzyl, benzyloxybenzyl, 1H-triazolylbenzyl, biphenylmethyl or pyridylbenzyl) and -L_{G}-H₅-H₇heterocyclyl-L_{10'}-U-L₁₀-R_{H}, and wherein U, L_{G}, L₁₀, L₁₀ and R_{H} are as defined immediately above.

In any of the above examples, R₅ can be, without limitation, (H₅-H₆heterocyclo)C₁-C₆ alkoxycarbonyl, wherein the H₅-H₆heterocyclyl moiety comprises at least one nitrogen ring atom. For instance, R₅ or R₄ can be thiazolylC₁-C₆alkoycarbonyl, such as thiazolylmethoxycarbonyl. R₅ can also be, without limitation, carbocyclylC₁-C₆alkyl (e.g., benzyl or naphthylmethyl) or heterocycloC₁-C₆ₐₗkyl (e,g., pyridylmethyl, thienylmethyl or furylmethyl).

Non-limiting examples of the compounds of this embodiment include:
1,3-thiazol-5-ylmethyl benzyl(3-{benzyl[(1,3-thiazol-5-ylmethoxy)carbonyl]amino}propyl) carbamate;
1,3-thiazol-5-ylmethyl benzyl(3-{benzyl[(1,3-thiazol-5-ylmethoxy)carbonyl]amino}-2-hydroxy propyl)carbamate;
1,3-thiazol-5-ylmethyl benzyl(3-{benzyl[(1,3-thiazol-5-ylmethoxy)carbonyl]amino}-2-oxopropyl)carbamate;
1,3-thiazol-5-ylmethyl benzyl[9-benzyl-10-oxo-12-(1,3-thiazol-5-yl)-3,6,11-trioxa-9-azadodec-1-yl]carbamate;
1,3-thiazol-5-ylmethyl cyclohexylmethyl(3-{(cyclohexylmethyl)[(1,3-thiazol-5-ylmethoxy) carbonyl]amino}propyl)carbamate;
methyl 4-({(3-{[4-(methoxycarbonyl)benzyl][(1,3-thiazol-5-ylmethoxy)carbonyl]amino} propyl)[(1,3-thiazol-5-y)methoxy)carbonyl]amino}methyl)benzoate;
1,3-thiazol-5-ylmethyl 4-benzoylbenzyl(3-{(4-benzoylbenzyl)[(1,3-thiazol-5-ylmethoxy) carbonyl]amino}propyl)carbamate;
1,3-thiazol-5-ylmethyl quinolin-3-ylmethyl(3-{(quinolin-3-ylmethyl)[(1,3-thiazol-5-ylmethoxy) carbonyl]amino}propyl)carbamate;
1,3 -thiazol-5-ylmethyl 4-pyridin-2-ylbenzyl(3-{(4-pyridin-2-ylbenzyl)[(1,3-thiazol-5-ylmethoxy) carbonyl]amino}propyl)carbamate
1,3-thiazol-5-ylmethyl 4-(benzyloxy)benzyl(3-{[4-(benzyloxy)benzyl][(1,3-thiazol-5-ylmethoxy) carbonyl]amino}propyl)carbamate;
1,3-thiazol-5-ylmethyl 3-{[(1,3-thiazol-5-ylmethoxy)carbonyl][4-(1H-1,2,4-triazol-1-yl)benzyl] amino}propyl[4-(1H-1,2,4-triazol-1-yl)benzyl]carbamate;
1,3-thiazol-5-ylmethyl 4-methoxybenzyl(3-{(4-methoxybenzyl)[(1,3-thiazol-5-ylmethoxy) carbonyl]amino)propyl)carbamate;
tert-butyl 4-methoxybenzyl(3-{(4-methoxybenzyl)[(1,3-thiazol-5-ylmethoxy)carbonyl]amino} propyl)carbamate;
1,3-thiazol-5-ylmethyl 4-tert-butylbenzyl(3-{(4-tert-butylbenzyl)[(1,3-thiazol-5-ylmethoxy) carbonyl]amino}propyl)carbamate;
*tert*-butyl 4-benzoylbenzyl(3-{(4-benzoylbenzyl)[(1,3-thiazol-5-ylmethoxy)carbonyl]amino} propyl)carbamate;
1,3-thiazol-5-ylmethyl 1,1'-biphenyl-4-ylmethyl(3-{(1,1'-biphenyl-4-ylmethyl)[(1,3-thiazol-5-ylmethoxy)carbonyl]amino}propyl)carbamate;
1,3-thiazol-5-ylmethyl benzyl{3-[benzyl(pyridin-4-ylmethyl)amino]propyl}carbamate;
ethyl N-(3-{(ethoxycarbonylmethyl)[(1,3-thiazol-5-ylmethoxy)carbonyl]amino}propyl)-N-[(1,3-thiazol-5-ylmethoxy)carbonyl]glycinate;
1,3-thiazol-5-ylmethyl benzyl(3-{benzyl[(5-methylthien-2-yl)methyl]amino} propyl)carbamate;
1,3-thiazol-5-ylmethyl benzyl{3-[benzyl(2-furylmethyl)amino]propyl}carbamate;
1,3-thiazol-5-ylmethyl benzyl{3-[benzyl(2-naphthylmethyl)amino]propyl}carbamate;
methyl 4-({(3-{(tert-butoxycarbonyl)[4-(methoxycarbonyl)benzyl]amino}propyl)[(1,3-thiazol-5-ylmethoxy)carbonyl]amino}methyl)benzoate;
1,3-thiazol-5-ylmethyl benzyl{3-[benzyl(neopentyl)amino]propyl}carbamate;
tert-butyl benzyl(3-{benzyl[(1,3-thiazol-5-ylmethoxy)carbonyl]amino}propyl)carbamate;
benzyl benzyl(3-{benzyl[(1,3-thiazol-5-ylmethoxy)carbonyl]amino}propyl)carbamate;
methyl benzyl(3-{benzyl[(1,3-thiazol-5-ylmethoxy)carbonyl)amino}propyl)carbamate;
1,3-thiazol-5-ylmethyl benzyl[3-(benzyl{4-[3-(dimethylamino)propoxy]benzyl}amino)propyl] carbamate;
1,3-thiazol-5-ylmethyl benzyl{3-[benzyl(4-pyridin-2-ylbenzyl)amino]propyl}carbamate;
tert-butyl 3-{[(1,3-thiazol-5-ylmethoxy)carbonyl](4-(1H-1,2,4-triazol-1-yl)benzyl] amino}propyl[4-(1H-1,2,4-triazol-1-yl)benzyl]carbamate;
5-{benzyl[(1,3-thiazol-5-ylmethoxy)carbonyl]amino}-2-[(tert-butoxycarbonyl)amino]-1,2,3,5-tetradeoxy-1-phenyl-D-glycero-pentitol;
*tert*-butyl 1,1'-biphenyl-4-ylmethyl(3-{(1,1'-biphenyl-4-ylmethyl)[(1,3-thiazol-5-ylmethoxy) carbonyl]amino}propyl)carbamate;
tert-butyl 4-pyridin-2-ylbenzyl(3-{(4-pyridin-2-ylbenzyl)[(1,3-thiazol-5-ylmethoxy)carbonyl] amino}propyl)carbamate;
ethyl N-[(1,3-thiazol-5-ylmethoxy)carbonyl]-N-(3-{[(1,3-thiazol-5-ylmethoxy)carbonyl]amino} propyl)glycinate;
1,3-thiazol-5-ylmethyl benzyl{3-{benzyl[(1,3-thiazol-5-ylmethoxy)carbonyl]amino}-2-(phenoxycarbonyl)amino]propyl}carbamate; and
1,3-thiazol-5-ylmethyl benzyl[3-{benzyl[(1,3-thiazol-5-ylmethoxy)carbonyl]amino}-2-(isobutyrylamino)propyl]carbamate.

The present invention also features compounds of formula 11, 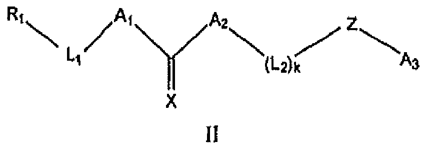 or pharmaceutically acceptable salts, solvates or prodrugs thereof, wherein
R₁ is a 3-, 4-, 5-, 6-, 7-, 8-, 9-, or 10-membered heterocyclyl comprising at least one nitrogen ring atom;
L₁ is a bond, C₁-C₁₀alkylene, C₂-C₁₀alkenylene or C₂-C₁₀alkynylene;
A₁ is a bond or selected from the group consisting of-O-L_{A1}-, -S-L_{A1}-, and -N(R_{A1})-L_{A1}-, wherein L_{A1} is a bond, C₁-C₁₀alkylene, C₂-C₁₀alkenylene or C₂-C₁₀alkynylene, and R_{A1} is hydrogen, C₁-C₆alkyl, C₂-C₆alkenyl or C₂-C₆alkynyl;
X is O or S;
A₂ is a bond or selected from the group consisting of ―L_{A2}―O―, ―L_{A2}―S― and ―L_{A2}―N(R_{A2})―, wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, ―L_{D}―O―R_{D}, ―L_{D}―S―R_{D}, ―L_{D}― C(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}SO₂R_{D}, ―L_{D}―C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, carbocyclyl, carbocyclylC₁-C₆alkyl, heterocyclyl, heterocycloC₁-C₆alkyl, ―L_{E}―carbocyclyl―L₄―Y―L_{4'}―R_{E}, and ―L_{E}―heterocyclyl―L₄―Y―L_{4'}―R_{E}, wherein L_{D}, L_{E}, L₄ and L_{4'} are each independently selected at each occurrence from a bond, C₁-C₁₀ alkylene, C₂-C₁₀alkenylene or C₂-C₁₀alkynylene, wherein R_{D}, R_{D'} and R_{D''} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₁₀alkylene, C₂-C₁₀alkenylene, C₂-C₁₀alkynylene, ―S―, ―O―, ―C(O)―, ―N(R_{Y})C(O)―, ―C(O)N(R_{Y})―, ―C(O)O― and ―OC(O)―, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆ alkyl;
k is 0 or 1, and at each occurrence L₂ independently represents ―L₉―V―L_{9'}―, wherein L₉ and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, ―S―, ―O―, ―C(O)―, ―N(R_{V})C(O)―, ―C(O)N(R_{V})―, ―C(O)O― and ―OC(O)―, wherein R_{V} is independently selected at each occurrence from hydrogen, C₁-C₆ alkyl, C₂-C₆alkenyl or C₂-C₆alkynyl;
Z is ―C(R₂R₃)―, =C(R₂)― or ―C(R₂)=, wherein R₂ is selected from the group consisting of carbocyclyl, carbocyclylC₁-C₆alkyl, heterocyclyl, heterocycloC₁-C₆alkyl, ―L_{D}―O―R_{E}, ―L_{D}―S―R_{E}, ―L_{D}― C(O)R_{E}, ―L_{D}―OC(O)R_{E}, ―L_{D}―C(O)OR_{E}, ―LD―N_{D}R_{E}R_{D}, ―L_{D}―S(O)R_{E}, ―L_{D}―SO₂R_{E}, ―L_{D}―C(O)NR_{D}R_{E}, ―L_{D}―N(R_{D})C(O)R_{E}, ―L_{D}―N(R_{D})SO₂R_{E}, ―L_{D}―N(R_{D)}SO₂NR_{D'}R_{E}, ―L_{E}―carbocyclyl―L₄―Y―L_{4'}―R_{E} and ―L_{E}― heterocyclyl―L₄―Y―L_{4'}―R_{E}, wherein R₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L_{D}― O―R_{D}, ―L_{D}―S―R_{D}, ―L_{D}―(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}― SO₂R_{D}, ―L_{D}―C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, ―L_{E}― carbocyclyl―L₄―Y―L_{4'}―R_{E} and ―L_{E}―heterocyclyl―L₄―Y―L_{4'}―R_{E}, and wherein L₄, L_{4'}, Y, L_{D}, L_{E}, R_{E}, R_{D}, R_{D'} and R_{D''} are as defined immediately above in this aspect;
or Z is selected from the group consisting of 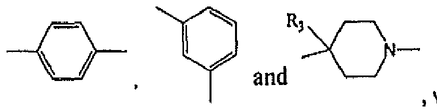 wherein R₃ is as defined immediately above in this aspect;
A₃ is selected from the group consisting of hydrogen, C₁-C₆alkyl, C₂-C₆alkenyl, C₂-C₆alkynyl,
carbocyclyl, carbocyclylC₁-C₆alkyl, heterocyclyl, heterocycloC₁-C₆alkyl, ―L_{E}―carbocyclyl―L₄―Y―L_{4'}―R_{E}, ―L_{E}―heterocyclyl―L₄―Y―L_{4'}―R_{E}, ―L₆―O―R₈, ―L₆―C(O)R₈, ―L₆―C(O)OR₈, ―L₆―OC(O)R₈, ―L₆―C(O)NR₈R₉, ―L₆―N(R₈)C(O)R₉, ―L₆―N(R₉)C(O)OR₈, ―L₆―NR₈R₉, ―L₆―C(O)―L_{6'}―NR₈R₉, ―L₆― C(O)―L_{6'}―N(R₉)C(O)OR₈, ―L₆―C(O)―L_{6'}―N(R₉)C(O)NR₈R₁₀, ―L₆―S(O)ⱼR₈, ―L₆―N(R₉)S(O)ⱼR₈, ―L₆― S(O)ⱼNR₈R₉ and ―L₆―N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₂-C₁₀alkynylene, wherein R₈, R₉ and R₁₀ are each independently selected at each occurrence from the group consisting of hydrogen, C₁-C₆alkyl, C₂-C₆ alkenyl, C₂-C₆alkynyl, carbocyclyl, carbocyclylC₁-C₆alkyl, heterocyclyl, heterocycloC₁-C₆alkyl, ―L_{D}―O― R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}―SO₂R_{D}, ―L_{D}―C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, ―L_{E}―carbocyclyl― L₄―Y―L_{4'}―R_{E} and ―L_{E}―heterocyclyl―L₄―Y―L_{4'}―R_{E}, and wherein L_{D}, R_{D}, R_{D'}, R_{D''}, L_{E}, L₄, L_{4'}, Y and R_{E} are as defined immediately above in this aspect;
wherein at each occurrence L₁, L_{A1}, R_{A1}, Y, V, R_{Y}, R_{V}, L_{A2}, R_{A2}, L_{D}, L_{E}, L₄, L_{4'}, L₆, L_{6'}, L₉, L_{9'}, R₂, R₃, R₈, R₉, R₁₀, R_{E}, R_{D}, R_{D'}, R_{D''} and A₃ are each independently optionally substituted with at least one substituent selected from the group consisting of halogen, oxo, thioxo, hydroxy, nitro, cyano, amino, formyl, carbocyclyl, heterocyclyl, ―O―R_{L}, ―S―R_{L}, ―C(O)R_{L}, ―OC(O)R_{L}, ―C(O)OR_{L}, ―NR_{L}R_{L'}, ―S(O)R_{L}, ―SO₂R_{L}, ―C(O)NR_{L}R_{L'}, ―N(R_{L})C(O)R_{L'}, ―N(R_{L})SO₂R_{L'} and ―N(R_{L})SO₂NR_{L'}R_{L''}, and wherein R_{L}, R_{L'} and R_{L''} are each independently selected at each occurrence from the group consisting of hydrogen, C₁-C₆ alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkyl carbonyl, C₁-C₆alkylcarbonylC₁-C₆alkyl, C₁-C₆alkoxycarbonyl, C₁-C₆alkoxycarbonylC₁-C₆alkyl, C₁-C₆ alkylcarbonyloxy, C₁-C₆alkylcarbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl;
wherein each carbocyclyl moiety in L₁, A₁, A₂, (L₂)ₖ, Z and A₃ is independently selected at each occurrence from 3-, 4-, 5-, 6-, 7-, 8-, 9- or 10-membered carbocyclyls, and each heterocyclyl moiety in L₁, A₁, A₂, (L₂)ₖ, Z and A₃ is independently selected at each occurrence from 3-, 4-, 5-, 6-, 7-, 8-, 9- or 10-membered heterocyclyls; and
wherein each carbocyclyl and heterocyclyl moiety in the compound (e.g., in L₁, A₁, A₂, (L₂)ₖ, Z and A₃, including optional substitution carbocyclyl or heterocyclyl) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{S}-O-R_{S}, ―L_{S}―S―R_{S}, ―L_{S}―C(O)R_{S}, ―L_{S}―OC(O)R_{S}, ―L_{S}―C(O)OR_{S}, ―L_{S}―NR₅R_{S'}, ―L_{S}―S(O)R_{S}, ―L_{S}―SO₂R_{S}, ―L_{S}― C(O)NR_{S}R_{S'}, ―L_{S}―N(R_{S})C(O)R_{S'}, ―L_{S}―N(R_{S})SO₂R_{S'}, ―L_{S}―N(R_{S})SO₂NR_{S'}R_{S''}, carbocyclyl, carbocyclylC₁-C₆alkyl, heterocyclyl and heterocycloC₁-C₆alkyl, wherein at each occurrence L_{S} is independently selected from a bond, C₁-C₁₀alkylene, C₂-C₁₀alkenylene or C₂-C₁₀alkynylene, and R_{S}, R_{S'} and R_{S''} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆alkylaminoC₁-C₆alkyl, C₃-C₁₀carbocyclyl, C₃-C₁₀carbocyclylC₁-C₆alkyl, H₃-H₁₀heterocyclyl and H₃-H₁₀heterocycloC₁-C₆alkyl;
with the proviso that said compound is not ritonavir.

In one embodiment, the present invention features compounds of formula II, or pharmaceutically acceptable salts, solvates or prodrugs thereof, wherein
R₁ is a 5- or 6-membered heterocyclyl comprising at least one nitrogen ring atom (e.g., thiazolyl, imidazolyl, oxazolyl, or pyridyl), and is optionally substituted with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{S}―O―R_{S}, ―L_{S}―S―R_{S}, ―L_{S}―C(O)R_{S}, ―L_{S}―OC(O)R_{S}, ―L_{S}―C(O)OR_{S}, -Ls-NR_{S}R_{S'}, ―L_{S}―S(O)R_{S}, ―L_{S}―SO₂R_{S}, ―L_{S}―C(O)NR_{S}R_{S'}, ―L_{S}―N(R_{S})C(O)R_{S'}, ―L_{S}―NR_{S}SO₂R_{S'} and ―L_{S}― NR_{S}SO₂NR_{S'}R_{S''}, wherein L_{S} is independently selected at each occurrence from a bond, C₁-C₁₀alkylene, C₂-C₁₀alkenylene or C₂-C₁₀alkynylene, and R_{S}, R_{S'} and R_{S''} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl;
L₁ is a bond, C₁-C₆alkylene, C₂-C₆alkenylene or C₂-C₆alkynylene;
A₁ is ―O―L_{A1}―, wherein L_{A1} is a bond;
X is O;
A₂ is ―L_{A2}―N(R_{A2})―, wherein L_{A2} is a bond, and R_{A2} is selected from the group consisting of hydrogen, C₁-C₆alkyl, C₂-C₆alkenyl, C₂-C₆alkynyl, ―C(O)R_{F}, ―C(O)OR_{F}, carbocyclylC₁-C₆alkyl and heterocycloC₁-C₆alkyl, wherein R_{F} is independently selected at each occurrence from hydrogen, C₁-C₆ alkyl, C₂-C₆alkenyl, C₂-C₆alkynyl, carbocyclyl, heterocyclyl, carbocyclylC₁-C₆alkyl or heterocycloC₁-C₆ alkyl;
k is 0 or 1;
L₂ represents ―L₉―V―L_{9'}―, wherein L₉ and L_{9'} are each independently selected from a bond, C₁-C₆alkylene, C₂-C₆alkenylene or C₂-C₆alkynylene, and wherein V is selected from the group consisting of a bond, ―S―, ―O―, ―C(O)― and ―C(O)N(R_{V})―, and R_{V} is selected from hydrogen, C₁-C₆alkyl, C₂-C₆alkenyl or C₂-C₆alkynyl;
Z is ―C(R₂R₃)―, wherein R₂ is carbocyclylC₁-C₆alkyl (e.g., phenylC₁-C₆alkyl, such as benzyl), heterocycloC₁-C₆alkyl, R_{E}―carbocyclylC₁-C₆alkyl― or R_{E}―heterocyclylC₁-C₆alkyl―, and R₃ is hydrogen, C₁-C₆alkyl, C₂-C₆alkenyl, C₂-C₆alkynyl, carbocyclylC₁-C₆alkyl (e.g., phenylC₁-C₆alkyl, such as benzyl), heterocycloC₁-C₆alkyl, R_{E}―carbocyclylC₁-C₆alkyl― or R_{E}―heterocyclylC₁-C₆alkyl―, wherein R_{E} is independently selected at each occurrence from carbocyclyl, heterocyclyl, carbocyclylC₁-C₆alkyl or heterocycloC₁-C₆alkyl;
A₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L_{E}―carbocyclyl―L₄―Y―L_{4'}―R_{E}, ―L_{E}―heterocyclyl―L₄―Y―L_{4'}―R_{E}, ―L₆―O―R₈, ―L₆―C(O)R₈, ―L₆―C(O)OR₈, ―L₆―OC(O)R₈, ―L₆―C(O)NR₈R₉, ―L₆―N(R₈)―C(O)R₉, ―L₆―N(R₉)C(O)OR₈, ―L₆―NR₈R₉, ―L₆―C(O)―L_{6'}―NR₈R₉, ―L₆― C(O)―L_{6'}―N(R₉)C(O)OR₈, ―L₆―C(O)―L_{6'}―(R₉)C(O)NR₈R₁₀, ―L₆―S(O)ⱼR₈, ―L₆―N(R₉)S(O)ⱼR₈, ―L₆― S(O)ⱼNR₈R₉ and ―L₆―N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₆alkylene, C₂-C₆alkenylene or C₂-C₆alkynylene, wherein R₈, R₉ and R₁₀ are each independently selected at each occurrence from the group consisting of hydrogen, C₁-C₆alkyl, C₂-C₆ alkenyl, C₂-C₆alkynyl, carbocyclyl, carbocyclylC₁-C₆alkyl, heterocyclyl, heterocycloC₁-C₆alkyl, ―L_{D}―O― R_{D}, ―L_{D}―S―R_{D}, ―L_{D}―C(O)R_{D}, ―L_{D}―OC(O)R_{D}, ―L_{D}―C(O)OR_{D}, ―LD―NR_{D}R_{D'}, ―L_{D}―S(O)R_{D}, ―L_{D}―SO₂R_{D}, ―L_{D}―C(O)NR_{D}R_{D'}, ―L_{D}―(R_{D})C(O)R_{D'}, ―L_{D}―N(R_{D})SO₂R_{D'}, ―L_{D}―N(R_{D})SO₂NR_{D'}R_{D''}, ―L_{E}―carbocyclyl― L₄―Y―L_{4'}―R_{E} and ―L_{E}―heterocyclyl―L₄―Y―L₄―R_{E}, wherein L_{D}, L_{E}, L₄ and L_{4'} are each independently selected at each occurrence from a bond, C₁-C₆alkylene, C₂-C₆alkenylene or C₂-C₆alkynylene, wherein Y is independently selected at each occurrence from the group consisting of a bond, C₁-C₁₀alkylene, C₂-C₁₀ alkenylene, C₂-C₁₀alkynylene, -S-, ―O―, ―C(O)―, ―N(R_{Y})C(O)―, ―C(O)N(R_{Y})―, ―C(O)O― and ―OC(O)―, and R_{Y} is independently selected at each occurrence from hydrogen, C₁-C₆alkyl, C₂-C₆alkenyl or C₂-C₆ alkynyl, wherein R_{D}, R_{D'}, and R_{D''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl, C₁-C₆alkyl aminoC₁-C₆alkyl, carbocyclyl, carbocyclylC₁-C₆alkyl, heterocyclyl and heterocycloC₁-C₆alkyl, and wherein R_{E} is as defined immediately above in this embodiment;
wherein at each occurrence L₁, L₄, L_{4'}, L₆, L_{6'}, L₉, L_{9'}, L_{D}, L_{E}, L_{F}, R₂, R₃, R₈, R₉, R₁₀, R_{A2}, R_{D}, R_{D'}, R_{D''}, R_{E}, R_{F}, R_{V}, R_{Y} and Y are each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
wherein each carbocyclyl moiety in A₂, Z and A₃ is independently selected at each occurrence from 5-, 6- or 7-membered carbocyclyls (e.g., C₅-C₇cycloalkyl, C₅-C₇cycloalkenyl or phenyl), and each heterocyclyl moiety in A₂, Z and A₃ is independently selected at each occurrence from 5-, 6- or 7-membered heterocyclyls (e.g., H₅-H₇heteroaryl, H₅-H₇heterocycloalkyl or H₅-H₇heterocycloalkenyl); and
wherein each carbocyclyl and heterocyclyl moiety in A₂, Z and A₃ is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{S}―O―R_{S}, ―L_{S}―S―R_{S}, ―L_{S}―C(O)R_{S}, ―L_{S}―OC(O)R_{S}, ―L_{S}―C(O)OR_{S}, ―L_{S}―NR_{S}R_{S'}, ―L_{S}―S(O)R_{S}, ―L_{S}―SO₂R_{S}, ―L_{S}― C(O)NR_{S}R_{S'}, ―L_{S}―(R_{S})C(O)R_{S'}, ―L_{S}―NR_{S}SO₂R_{S'} and ―L_{S}―NR_{S}SO₂NR_{S'}R_{S''}, wherein L_{S}, R_{S}, R_{S'} and R_{S''} are as defined immediately above in this embodiment.

In a non-limiting example, R, is thiazolyl, L₁ is -CH₂-, R_{A2} is hydrogen, k is 0, R₂ is carbocyclylC₁-C₆alkyl (e.g., benzyl), and A₃ is C₁-C₆alkyl, C₂-C₆alkenyl, C₂-C₆alkynyl, ―L₆―O―R₈, ―L₆― S―R₈, ―L₆―C(O)R₈, ―L₆―C(O)OR₈, or ―L₆―OC(O)R₈, wherein L₆ is a bond, C₁-C₆alkyl, C₂-C₆alkenyl or C₂-C₆alkynyl,, and R₈ is hydrogen, C₁-C₆alkyl, C₂-C₆alkenyl or C₂-C₆alkynyl.

Non-limiting examples of the compounds of this embodiment include:
methyl N-[(1,3-thiazol-5-ylmethoxy)carbonyl]phenylalaninate (e.g., methyl N-[(1,3-thiazol-5-ylmethoxy)carbonyl]-L-phenylalaninate); and
1,3-thiazol-5-ylmethyl 1,1-dibenzylbut-3-enylcarbamate.

Terminology

Compounds of the present invention are generally described herein using standard nomenclature. For a recited compound having asymmetric center(s), it should be understood that all of the stereoisomers of the compound and mixtures thereof are encompassed in the present invention unless otherwise specified. Non-limiting examples of stereoisomers include enantiomers, diastereomers, and cis-transisomers. Where a recited compound exists in various tautomeric forms, the compound is intended to encompass all tautomeric forms. Certain compounds are described herein using general formulas that include variables (e.g., R₁, A₁, L₁, X, or Z). Unless otherwise specified, each variable within such a formula is defined independently of any other variable, and any variable that occurs more than one time in a formula is defined independently at each occurrence. If substituents are described as being "independently selected" from a group, each substituent is selected independently from the other. Each substituent therefore can be identical to or different from the other substituent(s).

The number of carbon atoms in a hydrocarbyl substituent can be indicated by the prefix "Cₓ-C_{y}," where x is the minimum and y is the maximum number of carbon atoms in the substituent. A prefix attached to a multiple-component substituent only applies to the first component that immediately follows the prefix. To illustrate, the term "alkylaryl" contains two components: alkyl and aryl. Thus, C₁-C₆alkylaryl refers to a C₁-C₆alkyl appended to the parent molecular moiety through an aryl group. Likewise, alkylC₆-C₁₀aryl refers to an alkyl group appended to the parent molecular moiety through a C₆-C₁₀aryl group. Similarly, the prefix "halo" on haloalkoxyalkyl indicates that only the alkoxy component is substituted with one or more halogen radicals, and the prefix "halo" on alkoxyhaloalkyl indicates that only the alkyl component is substituted with one or more halogen radicals.

When words are used to describe a linking element between two other elements of a depicted chemical structure, the leftmost-described component of the linking element is the component that is bound to the left element in the depicted structure. To illustrate, if the chemical structure is X-L-Y and L is described as methylarylethyl, then the chemical would be X-methyl-aryl-ethyl-Y.

When a chemical formula is used to describe a substituent, the dash on the right (or left) side of the formula indicates the portion of the substituent that has the free valence.

If a substitute is described as being "substituted," a non-hydrogen radical is in the place of one or more hydrogen radials on a carbon or nitrogen of the substituent. Thus, for example, a substituted alkyl substituent is an alkyl substituent wherein at least one non-hydrogen radical is in the place of a hydrogen radical(s) on the alkyl substituent. It should be recognized that if there are two or more substitutions on a substituent, each non-hydrogen radical may be identical or different unless otherwise stated.

The term "alkyl" (alone or in combination with another term(s)) refers to a straight- or branched-chain saturated hydrocarbyl substituent typically containing from 1 to 20 carbon atoms (C₁-C₂₀ alkyl), more typically from 1 to 10 carbon atoms (C₁-C₁₀alkyl), and even more typically from 1 to 6 carbon atoms (C₁-C₆alkyl). For instance, an alkyl substituent can have 1, 2, 3, 4, 5, 6, 7, 8,9, or 10 carbon atoms. Representative examples of alkyl include, but are not limited to, methyl, ethyl, propyl, n-propyl, isopropyl, butyl, n-butyl, iso-butyl, sec-butyl, tert-butyl, 2-methylpropyl, 2,2-dimethylpropyl, 3-methylbutyl, 2-ethylbutyl, pentyl, hexyl, 3-methylhexyl, 3,5,5-trimethylhexyl, 2,2-dimethylpentyl, 2,3-dimethylpentyl, heptyl, octyl, nonyl and decyl.

The term "alkenyl" (alone or in combination with another term(s)) refers to a straight- or branched-chain hydrocarbyl substituent containing at least one carbon-carbon double bond and typically from 2 to 20 carbon atoms (C₂-C₂₀alkenyl), more typically from 2 to 10 atoms (C₂-C₁₀alkenyl), and even more typically from 2 to 6 carbon atoms (C₂-C₆alkenyl). For instance, an alkenyl moiety can have 2, 3, 4, 5, 6, 7, 8, 9 or 10 carbon atoms and 1 or more carbon-carbon double bonds. Representative examples of alkenyl include, but are not limited to, ethenyl, 2-propenyl, 3-propenyl, 2-methyl-2-propenyl, 1-butenyl, 2-butenyl, 3-butenyl, 4-pentenyl, 2,2-dimethyl-4-pentenyl, 5-hexenyl, 2-heptenyl, 2-methyl-1-heptenyl, and 3-decenyl. The carbon-carbon double bond can have either cis or trans geometry within the alkenyl moiety, relative to groups substituted on the double bond carbons.

The term "alkenylene" refers to a divalent unsaturated hydrocarbon group which may be linear or branched and which has at least one carbon-carbon double bond. An alkenylene group typically contains 2 to 20 carbon atoms (i.e., C₂-C₂₀alkenylene), more typically from 2 to 10 carbon atoms (i.e., C₂-C₁₀alkenylene), and even more typically from 2 to 6 carbon atoms (i.e., C₂-C₆alkenylene). Representative alkenylene groups include, by way of example, ethene-1,2-diyl, prop-1-ene-1,2-diyl, prop-1-ene-1,3-diyl, and but-2-ene-1,4-diyl.

The term "alkoxy" (alone or in combination with another term(s)) refers to an alkyl group appended to the parent molecular moiety through an oxy moiety. For instance, C₁-C₂₀alkoxy, C₁-C₁₀alkoxy and C₁-C₆alkoxy refer to C₁-C₂₀alkyl, C₁-C₁₀alkyl or C₁-C₆alkyl appended to the parent molecular moiety through an oxy moiety, respectively. Representative examples of alkoxy include, but are not limited to, methoxy, ethoxy, propoxy, 2-propoxy, butoxy, tert-butoxy, pentyloxy, and hexyloxy.

The term "alkoxyalkyl" (alone or in combination with another term(s)) refers to an alkyl group to which is appended an alkoxy group. For instance, C₁-C₂₀alkoxyC₁-C₆alkyl, C₁-C₁₀alkoxyC₁-C₆alkyl and C₁-C₆alkoxyC₁-C₆alkyl refer to C₁-C₆alkyl to which is appended a C₁-C₂₀alkoxy, C₁-C₁₀alkoxy or C₁-C₆alkoxy group, respectively. Representative examples of alkoxyalkyl include, but are not limited to, tert-butoxymethyl, 2-ethoxyethyl, 2-methoxyethyl, and methoxymethyl.

The term "alkoxycarbonyl" (alone or in combination with another term(s)) refers to an alkoxy group appended to the parent molecular moiety through a carbonyl group. For instance, C₁-C₂₀alkoxycarbonyl, C₁-C₁₀alkoxycarbonyl and C₁-C₆alkoxycarbonyl refer to C₁-C₂₀alkoxy, C₁-C₁₀alkoxy or C₁-C₆alkoxy appended to the parent molecular moiety through a carbonyl, respectively. Representative examples of alkoxycarbonyl include, but are not limited to, methoxycarbonyl, ethoxycarbonyl, and tert-butoxycarbonyl.

The term "alkoxycarbonylalkyl" (alone or in combination with another term(s)) refers an alkoxycarbonyl group appended to the parent molecular moiety through an alkylene group. For instance, C₁-C₂₀alkoxycarbonylC₁-C₁₀alkyl, C₁-C₁₀alkoxycarbonylC₁-C₁₀alkyl and C₁-C₆alkoxycarbonylC₁-C₁₀alkyl refer to C₁-C₂₀alkoxycarbonyl, C₁-C₁₀alkoxycarbonyl or C₁-C₆alkoxycarbonyl appended to the parent molecular moiety through a C₁-C₁₀alkylene, respectively. Representative examples of alkoxycarbonylalkyl include, but are not limited to, 2-methoxy-2-oxoethyl, 2-ethoxy-2-oxoethyl, 3-methoxy-3-oxopropyl, 3-ethoxy-3-oxopropyl, 4-ethoxy-2-(ethoxycarbonyl)-4-oxobutyl, 5-methoxy-5-oxopentyl, and 6-methoxy-6-oxohexyl.

The term "alkylamino" refers to-NR₁R₂, wherein R₁ is an alkyl and R₂ is hydrogen or an alkyl (e.g., C₁-C₁₀alkylamino, in which R1 is C₁-C₁₀alkyl, and R₂ is hydrogen or C₁-C₁₀alkyl).

The term "alkylcarbonyl" (alone or in combination with another term(s)) refers to an alkyl group appended to the parent molecular moiety through a carbonyl group. For instance, C₁-C₂₀ alkylcarbonyl, C₁-C₁₀alkylcarbonyl and C₁-C₆alkylcarbonyl refer to C₁-C₂₀alkyl, C₁-C₁₀alkyl or C₁-C₆ alkyl appended to the parent molecular moiety through a carbonyl moiety, respectively. Representative examples of alkylcarbonyl include, but are not limited to, acetyl, 1-oxopropyl, 2,2-dimethyl-1-oxopropyl, 1-oxobutyl, and 1-oxopentyl.

The term "alkylcarbonylalkyl" (alone or in combination with another term(s)) refers to an alkylcarbonyl group appended to the parent molecular moiety through an alkylene group. Fro instance, C₁-C₂₀alkylcarbonylC₁-C₁₀alkyl, C₁-C₁₀alkylcarbonylC₁-C₁₀alkyl and C₁-C₆alkylcarbonylC₁-C₁₀alkyl refer to C₁-C₂₀alkylcarbonyl, C₁-C₁₀alkylcarbonyl or C₁-C₆alkylcarbonyl appended to the parent molecular moiety through a C₁-C₁₀alkylene, respectively. Representative examples of alkylcarbonylalkyl include, but are not limited to, 2-oxopropyl, 3,3-dimethyl-2-oxopropyl, 3-oxobutyl, and 3-oxopentyl.

The term "alkylcarbonyloxy" (alone or in combination with another term(s)) refers to an alkylcarbonyl group appended to the parent molecular moiety through an oxy moiety. For instance, C₁-C₂₀alkylcarbonyloxy, C₁-C₁₀alkylcarbonyloxy and C₁-C₆alkylcarbonyloxy refer to C₁-C₂₀alkylcarbonyl, C₁-C₁₀alkylcarbonyl or C₁-C₆alkylcarbonyl appended to the parent molecular moiety through an oxy moiety, respectively. Representative examples of alkylcarbonyloxy include, but are not limited to, acetyloxy, ethylcarbonyloxy, and tert-butylcarbonyloxy.

The term "alkylcarbonyloxyalkyl" (alone or in combination with another term(s)) refers to an alkylcarbonyloxy group appended to the parent molecular moiety through an alkylene moiety. For instance, C₁-C₂₀alkylcarbonyloxyC₁-C₁₀alkyl, C₁-C₁₀alkylcarbonyloxyC₁-C₁₀alkyl and C₁-C₆alkyl carbonyloxyC₁-C₁₀alkyl refer to C₁-C₂₀alkylcarbonyloxy, C₁-C₁₀alkylcarbonyloxy or C₁-C₆alkylcarbonyl oxy appended to the parent molecular moiety through a C₁-C₁₀lkylene moiety. Representative examples of alkylcarbonyloxyalkyl include, but are not limited to, 2-(acetyloxy)ethyl, 3-(acetyloxy)propyl, and 3-(propionyloxy)propyl.

The terms "alkylene" or "alkylenyl" (alone or in combination with another term(s)) denote a divalent group derived from a straight or branched saturated hydrocarbon chain typically containing from 1 to 20 carbon atoms (i.e., C₁-C₂₀alkylene), more typically from 1 to 10 carbon atoms (i.e., C₁-C₁₀ alkylene), and even more typically from 1 to 6 carbon atoms (i.e., C₁-C₆alkylene). For instance, an alkylene group can consist of 1, 2, 3, 4, 5, 6, 7, 8, 9 or 10 carbon atoms. Representative examples of alkylene include, but are not limited to, -CH₂-, -CH₂CH₂-, -CH₂CH₂CH₂-, -CH₂CH₂CH₂CH₂-, and -CH₂CH(CH₃)CH₂-.

The term "alkynyl" refers to a straight- or branched-chain hydrocarbyl substituent containing at least one triple bond and typically from 2 to 20 carbon atoms (i.e., C₂-C₂₀alkynyl), more typically from 2 to 10 carbon atoms (i.e., C₂-C₁₀alkynyl), and even more typically from 2 to 6 carbon atoms (i.e., C₂-C₆ alkynyl). For instance, an alkynyl group can have 2, 3, 4, 5, 6, 7, 8, 9 or 10 carbon atoms and containing 1 or more carbon-carbon triple bonds. Representative examples of alkynyl include, but are not limited to, ethynyl, 1-propynyl, 2-propynyl, 3-propynyl, -butynyl, 3-butynyl, 2-pentynyl, and decynyl.

The terms "alkynylene" (alone or in combination with another term(s)) refers to a divalent unsaturated hydrocarbon group which may be linear or branched and which has at least one carbon-carbon triple bonds. An alkynylene group typically contains from 2 to 20 carbon atoms (i.e., C₂-C₂₀ alkynylene), more typically from 2 to 10 carbon atoms (i.e., C₂-C₁₀alkynylene), and even more typically from 2 to 6 carbon atoms (i.e., C₂-C₆alkynylene). Representative alkynylene groups include, by way of example, ethyne-1,2-diyl, prop-1-yne-1,2-diyl, prop-1-yne-1,3-diyl, and but-2-yne-1,4-diyl.

The term "aryl" (alone or in combination with another term(s)) refers to an aromatic carbocyclyl containing from 6 to 14 carbon ring atoms. For instance, C₆-C₁₄aryl, C₆-C₁₀aryl and C₆-C₈ aryl refer to an aromatic carbocyclyl containing from 6 to 14, from 6 to 10 or from 6 to 8 carbon ring atoms, respectively. Non-limiting examples of aryls include phenyl, naphthalenyl, anthracenyl, and indenyl. An aryl group of the present invention can be substituted or unsubstituted, and connected to the parent molecular moiety through any substitutable carbon atom of the group.

The term "arylalkoxy" (alone or in combination with another term(s)) refers to an alkoxy group to which is appended an aryl group. For instance, arylC₁-C₂₀alkoxy, arylC₁-C₁₀alkoxy and arylC₁-C₆alkoxy refer to an aryl group appended to the parent molecular moiety through C₁-C₂₀alkoxy, C₁-C₁₀ alkoxy or C₁-C₆alkoxy, respectively. Representative examples of arylalkoxy include, but are not limited to, 2-phenylethoxy, 3-naphth-2-ylpropoxy, and 5-phenylpentyloxy.

The term "arylalkoxyalkyl" (alone or in combination with another term(s)) refers to an arylalkoxy group appended to the parent molecular moiety through an alkylene group. For instance, arylC₁-C₂₀alkoxyC₁-C₆alkyl, arylC₁-C₁₀alkoxyC₁-C₆alkyl and arylC₁-C₆alkoxyC₁-C₆alkyl refer to arylC₁-C₂₀alkoxy, arylC₁-C₁₀alkoxy or arylC₁-C₆alkoxy appended to the parent molecular moiety through a C₁-C₆alkylene group, respectively. Representative examples of arylalkoxyalkyl include, but are not limited to, benzyloxymethyl, 2-(benzyloxy)ethyl, and (2-phenylethoxy)methyl.

The term "arylalkoxycarbonyl" (alone or in combination with another term(s)) refers to an arylalkoxy group appended to the parent molecular moiety through a carbonyl group. For instance, arylC₁-C₂₀alkoxycarbonyl, arylC₁-C₁₀alkoxycarbonyl and arylC₁-C₆alkoxycarbonyl refer to arylC₁-C₂₀ alkoxy, arylC₁-C₁₀alkoxy or arylC₁-C₆alkoxy appended to the parent molecular moiety through a carbonyl group, respectively. Representative examples of arylalkoxycarbonyl include, but are not limited to, benzyloxycarbonyl, and naphth-2-ylmethoxycarbonyl.

The term "arylalkyl" (alone or in combination with another term(s)) refers to an aryl group appended to the parent molecular moiety through an alkylene group. For instance, arylC₁-C₂₀alkyl, arylC₁-C₁₀alkyl and arylC₁-C₆alkyl refer to an aryl appended to the parent molecular moiety through a C₁-C₂₀alkylene, C₁-C₁₀alkylene or C₁-C₆alkylene, respectively Representative examples of substituted/ unsubstituted arylalkyl include, but are not limited to, benzyl, 4-(benzyloxy)benzyl, 4-methoxybenzyl, 4-hydroxybenzyl, 3-(1,3-benzodioxol-5-yl)-2-methylpropyl, 3-(phenoxy)benzyl, 3-(1,3-benzodioxol-5-yl) propyl, 2-phenylethyl, 3-phenylpropyl, 2-naphthylmethyl, 3,5-ditert-butyl-2-hydroxybenzyl, 3-methoxy benzyl, 3,4-dimethoxybenzyl, 4-(dimethylamino)benzyl, 4-[3-(dimethylamino)propoxy]benzyl, (6-methoxy-2-napthyl)methyl, and 2-naphth-2-ylethyl.

The term "arylalkylcarbonyl" (alone or in combination with another term(s)) refers to an arylalkyl group appended to the parent molecular moiety through a carbonyl group. For instance, arylC₁-C₂₀alkylcarbonyl, arylC₁-C₁₀alkylcarbonyl and arylC₁-C₆alkylcarbonyl refer to arylC₁-C₂₀alkyl, arylC₁-C₁₀ alkyl or arylC₁-C₆alkyl appended to the parent molecular moiety through a carbonyl group, respectively. Representative examples of arylalkylcarbonyl include, but are not limited to, 2-naphthylacetyl, and phenylacetyl.

The term "arylcarbonyl" (alone or in combination with another term(s)) refers to an aryl group appended to the parent molecular moiety through a carbonyl group. Representative examples of arylcarbonyl include, but are not limited to, benzoyl, and naphthoyl.

The term "aryloxy" (alone or in combination with another term(s)) refers to an aryl group appended to the parent molecular moiety through an oxy moiety. Representative examples of substituted/unsubstituted aryloxy include, but are not limited to, phenoxy, naphthyloxy, 3-bromophenoxy, 4-chlorophenoxy, 4-methylphenoxy, and 3,5-dimethoxyphenoxy.

The term "aryloxyalkyl" (alone or in combination with another term(s)) refers to an aryloxy group appended to the parent molecular moiety through an alkylene group. For instance, aryloxyC₁-C₂₀alkyl, aryloxyC₁-C₁₀alkyl and aryloxyC₁-C₆alkyl refer to an aryloxy group appended to the parent molecular moiety through a C₁-C₂₀alkylene, C₁-C₁₀alkylene or C₁-C₆alkylene group, respectively. Representative examples of aryloxyalkyl include, but are not limited to, 2-phenoxyethyl, 3-naphth-2-yl oxypropyl, and phenoxymethyl.

The term "aryloxycarbonyl" (alone or in combination with another term(s)) refers to an aryloxy group appended to the parent molecular moiety through a carbonyl group.

The term "arylthio" (alone or in combination with another term(s)) refers to an aryl group appended to the parent molecular moiety through a sulfur atom. Representative examples of arylthio include, but are not limited to, phenylthio, naphthalen-1-ylthio, and naphthalen-2-ylthio.

The term "arylthioalkoxy" (alone or in combination with another term(s)) refers to a thioalkoxy group to which is appended an aryl group. For instance, arylC₁-C₂₀thioalkoxy, arylC₁-C₁₀ thioalkoxy and arylC₁-C₆thioalkoxy refer to an aryl group appended to the parent molecular moiety through a C₁-C₂₀thioalkoxy, C₁-C₁₀thioalkoxy or C₁-C₆thioalkoxy group, respectively. Representative examples of arylthioalkoxy include, but are not limited to, (phenylmethyl)thio, (2-phenylethyl)thio, and (naphthalen-1-ylmethyl)thio.

The term "arylthioalkoxyalkyl" (alone or in combination with another term(s)) refers to an arylthioalkoxy group appended to the parent molecular moiety through an alkylene group. For instance, arylC₁-C₂₀thioalkoxyC₁-C₆alkyl, arylC₁-C₁₀thioalkoxyC₁-C₆alkyl and arylC₁-C₆thioalkoxyC₁-C₆alkyl refer to an arylC₁-C₂₀thioalkoxy, arylC₁-C₁₀thioalkoxy or arylC₁-C₆thioalkoxy group appended to the parent molecular moiety through a C₁-C₆ alkylene group, respectively. Representative examples of arylthioalkoxy include, but are not limited to, (phenylmethyl)thiomethyl, (2-phenylethyl)thiomethyl, and (naphthalen-1-ylmethyl)thiomethyl.

The term "arylthioalkyl" (alone or in combination with another term(s)) refers to an arylthio group appended to the parent molecular moiety through an alkylene group. For instance, arylthioC₁-C₂₀ alkyl, arylthioC₁-C₁₀alkyl and arylthioC₁-C₆alkyl refer to an arylthio group appended to the parent molecular moiety through a C₁-C₂₀alkylene, C₁-C₁₀alkylene or C₁-C₆alkylene group, respectively. Representative examples of arylthioalkyl include, but are not limited to, (phenylthio)methyl, 2-(phenylthio)ethyl, and 3-(phenylthio)propyl.

The term "dialkylamino" refers to-NRR', wherein R and R' are independently selected from alkyl groups (e.g., di-(C₁-C₁₀alkyl)amino, in which R and R' are independently selected from C₁-C₁₀alkyl groups).

The term "dialkylaminocarbonyl" refers to a dialkylamino group appended to the parent molecular moiety through a carbonyl group.

The term "carbonyl" (alone or in combination with another term(s)) refers to a -C(O)- group.

The term "carboxy" (alone or in combination with another term(s)) refers to a -CO₂H group.

The term "carboxyalkyl" (alone or in combination with another term(s)) refers to a carboxy group appended to the parent molecular moiety through an alkylene group. For instance, carboxyC₁-C₂₀ alkyl, carboxyC₁-C₁₀alkyl and carboxyC₁-C₆alkyl refer to a carboxy group appended to the parent molecular moiety through a C₁-C₂₀alkylene, C₁-C₁₀alkylene or C₁-C₆alkylene group, respectively. Representative examples of carboxyalkyl include, but are not limited to, carboxymethyl, 2-carboxyethyl, and 3-carboxypropyl.

The term "cyano" (alone or in combination with another term(s)) refers to a -CN group.

The terms "carbocycle" or "carbocyclic" or "carbocyclyl" (alone or in combination with another term(s)) refer to a saturated (e.g., "cycloalkyl"), partially saturated (e.g., "cycloalkenyl") or completely unsaturated (e.g., "aryl") ring system typically containing from 3 to 14 carbon ring members (i.e., C₃-C₁₄carbocyclyl) and zero heteroatom ring member. "Ring atoms" or "ring members" are the atoms bound together to form the ring or rings of a cyclic substituent. In many cases, a carbocyclyl has I ring or 2 or 3 fused or spiro rings, and contains from 3 to 10 ring members (i.e., C₃-C₁₀carbocyclyl, such as C₃-C₁₀cycloalkyl), from 3 to 8 ring members (i.e., C₃-C₈carbocyclyl, such as C₃-C₈cycloalkyl), from 3 to 6 ring members (i.e., C₃-C₆carbocyclyl, such as C₃-C₆cycloalkyl), from 4 to 10 ring members (i.e., C₄-C₁₀carbocyclyl, such as C₄-C₁0cycloalkyl and C₄-C₁₀cycloalkenyl), from 4 to 8 ring members (i.e., C₄-C₈ carbocyclyl, such as C₄-C₈cycloalkyl and C₄-C₈cycloalkenyl), or from 5 to 7 ring members (i.e., C₅-C₇ carbocyclyl, such as C₅-C₇cycloalkyl, C₅-C₇cycloalkenyl and phenyl). A substituted cycloalkyl may have either cis or trans geometry. Representative examples of carbocyclyl groups include, but are not limited to, cyclopropyl, cyclobutyl, cyclopentyl, cyclohexyl, cycloheptyl, cyclooctyl, adamantyl, decahydro-naphthalenyl, octahydro-indenyl, cyclohexenyl, phenyl, naphthyl, fluorenyl, indanyl, or 1,2,3,4-tetrahydro-naphthyl. A carbocyclyl group of the present invention can be unsubstituted or substituted, and attached to the parent molecular moiety through any substitutable carbon atom of the group.

The term "cycloalkenyl" (alone or in combination with another term(s)) refers to a nonaromatic, partially unsaturated carbocyclyl typically having from 4 to 14 carbon ring members and zero heteroatom ring member (e.g., C₄-C₁₀cycloalkenyl, C₄-C₈cycloalkenyl, C₄-C₆cycloalkenyl, or C₅-C₇ cycloalkenyl). Representative examples of cycloalkenyl groups include, but not limited to, cyclobutenyl, cyclopentenyl, cyclohexenyl, and octahydronaphthalenyl.

The term "cycloalkyl" (alone or in combination with another term(s)) refers to a saturated carbocyclyl group typically containing from 3 to 14 carbon ring members and zero heteroatom ring member (e.g., C₃-C₁₀cycloalkyl, C₃-C₈cycloalkyl, C₃-C₆cycloalkyl, or C₅-C₇cycloalkyl). Non-limiting examples of cycloalkyls include cyclopropyl, cyclobutyl, cyclopentyl, cyclohexyl, cycloheptyl, and cyclooctyl.

The term "carbocyclylalkyl" (alone or in combination with another term(s)) refers to a carbocyclyl group appended to the parent molecular moiety through an alkylene group. C₃-C₁₀ carbocyclylC₁-C₆alkyl, C₄-C₈carbocyclylC₁-C₆alkyl and C₅-C₇carbocyclylC₁-C₆alkyl refer to a C₃-C₁₀ carbocyclyl, C₄-C₈carbocyclyl or C₅-C₇carbocyclyl group appended to the parent molecular moiety through a C₁-C₆alkylene group, respectively. Non-limiting examples of carbocyclylalkyl include C₃-C₁₀ cycloalkylC₁-C₆alkyl, C₃-C₈cycloalkylC1-C6alkyl, C₃-C₆cycloalkylC₁-C₆alkyl, C₄-C₁₀cycloalkenylC₁-C₆ alkyl, C₄-C₈cycloalkenylC₁-C₆alkyl, or C₄-C₆cycloalkenylC₁-C₆alkyl.

The term "carbocyclylalkoxy" (alone or in combination with another term(s)) refers to an alkoxy group to which is appended an carbocyclyl group. For instance, C₃-C₁₀carbocyclylC₁-C₆alkoxy, C₄-C₈gcarbocyclylC₁-C₆alkoxy and C₅-C₇carbocyclylC₁-C₆alkoxy refer to a C₃-C₁₀carbocyclyl, C₄-C₈ carbocyclyl or C₅-C₇carbocyclyl group appended to the parent molecular moiety through a C₁-C₆alkoxy group, respectively. Non-limiting examples of carbocyclylalkoxy include C₃-C₁₀cycloalkylC1-C₆alkoxy, C₃.C₈cycloalkylC₁-C₆alkoxy, C₃-C₆cycloalkylC₁-C₆alkoxy, C₄-C₁₀cycloalkenylC1-C₆alkoxy, C₄-C₈ cycloalkenylC₁-C₆alkoxy, or C₄-C₆cycloalkenylC₁-C₆alkoxy.

The term "carbocyclylalkoxyalkyl" (alone or in combination with another term(s)) refers to an carbocyclylalkoxy group appended to the parent molecular moiety through an alkylene group. For instance, C₃-C₁₀carbocyclylC₁-C₆alkoxyC₁-C₆alkyl, C₄-C₈carbocyclylC₁-C₆alkoxyC₁-C₆alkyl and C₅-C₇carbocyclylC₁-C₆alkoxyC₁-C₆alkyl refer to C₃-C₁₀carbocyclylC₁-C₆alkoxy, C₄-C₈carbocyclylC₁-C₆ alkoxy or C₅-C₇carbocyclylC₁-C₆alkoxy appended to the parent molecular moiety through a C₁-C₆ alkylene group, respectively.

The term "carbocyclylalkoxycarbonyl" (alone or in combination with another term(s)) refers to an carbocyclylalkoxy group appended to the parent molecular moiety through a carbonyl group. For instance, C₃-C₁₀carbocyclylC₁-C₆alkoxycarbonyl, C₄-C₈carbocyclylC₁-C₆alkoxycarbonyl and C₅-C₇ carbocyclylC₁-C₆alkoxycarbonyl refer to C₃-C₁₀carbocyclylC₁-C₆alkoxy, C₄-C₈carbocyclylC₁-C₆alkoxy or C₅-C₇carbocyclylC₁-C₆alkoxy appended to the parent molecular moiety through a carbonyl group, respectively.

The term "carbocyclylalkylcarbonyl" (alone or in combination with another term(s)) refers to an carbocyclylalkyl group appended to the parent molecular moiety through a carbonyl group. For instance, C₃-C₁₀carbocyclylC₁-C₆alkylcarbonyl, C₄-C₈carbocyclylC₁-C₆alkylcarbonyl and C₅-C₇ carbocyclylC₁-C₆alkylcarbonyl refer to C₃-C₁₀carbocyclylC₁-C₆alkyl, C₄-C₈carbocyclylC₁-C₆alkyl or C₅-C₇carbocyclylC₁-C₆alkyl appended to the parent molecular moiety through a carbonyl group, respectively.

The term "carbocyclylcarbonyl" (alone or in combination with another term(s)) refers to an carbocyclyl group appended to the parent molecular moiety through a carbonyl group. For instance, C₃-C₁₀carbocyclylcarbonyl, C₄-C₈carbocyclylcarbonyl and C₅-G₇carbocyclylcarbonyl refer to a C₃-C₁₀ carbocyclyl, C₄-C₈carbocyclyl and C₅-C₇carbocyclyl group appended to the parent molecular moiety through a carbonyl group, respectively.

The term "carbocyclyloxy" (alone or in combination with another term(s)) refers to an carbocyclyl group appended to the parent molecular moiety through an oxy moiety (e.g., C₃-C₁₀ carbocyclyloxy, C₄-C₈carbocyclyloxy and C₅-C₇carbocyclyloxy).

The term "carbocyclyloxyalkyl" (alone or in combination with another term(s)) refers to an carbocyclyloxy group appended to the parent molecular moiety through an alkylene group. For instance, C₃-C₁₀carbocyclyloxyC1-C6alkyl, C₄-C₈carbocyclyloxyC₁-C₆alkyl and C₅-C₇carbocyclyloxyC₁-C₆alkyl refer to a C₃-C₁₀carbocyclyloxy, C₄-C₈carbocyclyloxy or C₅-C₇carbocyclyloxy group appended to the parent molecular moiety through a C₁-C₆alkylene group, respectively.

The term "carbocyclyloxycarbonyl" (alone or in combination with another term(s)) refers to an carbocyclyloxy group appended to the parent malecular moiety through a carbonyl group. For instance, C₃-C₁₀carbocyclyloxycarbonyl, C₄-C₈carbocyclyloxycarbonyl and C₅-C₇carbocyclyloxycarbonyl refer to a C₃-C₁₀carbocyclyloxy, C₄-C₈carbocyclyloxy or C₅-C₇carbocyclyloxy appended to the parent molecular moiety through a carbonyl group, respectively.

The term "carbocyclylthio" (alone or in combination with another term(s)) refers to an carbocyclyl group appended to the parent molecular moiety through a sulfur atom (e.g., C₃-C₁₀ carbocyclylthio, C4-C8carbocyclylthio, and C₅-C₇carbocyclylthio).

The term "carbocyclylthioalkoxy" (alone or in combination with another term(s)) refers to a thioalkoxy group to which is appended an carbocyclyl group. For instance, C₃-C₁₀carbocyclylC₁-C₆ thioalkoxy, C₄-C₈carbocyclylC₁-C₆thioalkoxy and C₅-C₇carbocyclylC₁-C₆thioalkoxy refer to a C₃-C₁₀ carbocyclyl, C₄-C₈carbocyclyl or C₅-C₇carbocyclyl group appended to the parent molecular moiety through a C₁-C₆thioalkoxy group, respectively.

The term "carbocyclylthioalkoxyalkyl" (alone or in combination with another term(s)) refers to an carbocyclylthioalkoxy group appended to the parent molecular moiety through an alkylene group. For instance, C₃-C₁₀carbocylylC₁-C₆thioalkoxyC₁-C₆alkyl, C₄-C₈carbocyclylC₁-C₆thioalkoxyC₁-C₆alkyl and C₅-C₇carbocyclylC₁-C₆thioalkoxyC₁-C₆alkyl refer to a C₃-C₁₀carbocyclylC₁-C₆thioalkoxy, C₄-C₈ carbocyclylC₁-C₆thioalkoxy or C₅-C₇carbocyclylC₁-C₆thioalkoxy group appended to the parent molecular moiety through a C₁-C₆ alkylene group, respectively.

The term "carbocyclylthioalkyl" (alone or in combination with another term(s)) refers to an carbocyclylthio group appended to the parent molecular moiety through an alkylene group. For instance, C₃-C₁₀carbocyclylthioC1-C6alkyl, C₄-C₈carbocyclylthioC1-C6alkyl and C₅-C₇carbocyclylthioC₁-C₆alkyl refer to a C₃-C₁₀carbocyclylthio, C₄-C₈carbocyclylthio or C₅-C₇carbocyclylthio group appended to the parent molecular moiety through a C₁-C₆alkylene group, respectively.

The term "carbocyclylcarbocyclylalkyl" refers to a carbocyclylalkyl group to which is appended a carbocyclyl group.

The term "carbocyclylalkoxycarbocyclylalkyl" refers to a carbocyclylalkyl group to which is appended a carbocyclylalkoxy group.

The term "(carbocyclylalkyl)carbocyclylalkyl" refers to a carbocyclylalkyl to which another carbocyclylalkyl group is appended. The former carbocyclylalkyl group can be identical to or different from the latter carbocyclylalkyl group.

The term "carbocyclylalkoxyheterocycloalkyl" refers to a heterocycloalkyl to which is a carbocyclylalkoxy group is appended (e.g., arylalkoxyheterocycloalkyl or (cycloalkylalkoxy)heterocycloalkyl).

The term "carbocyclylcarbonylheterocycloalkyl" refers to a heterocycloalkyl to which is a carbocyclylcarbonyl group is appended (e.g., arylcarbonylheterocycloalkyl or (cycloalkylcarbonyl)heterocycloalkyl).

The term "carbocyclylheterocycloalkyl" refers to a heterocycloalkyl to which is a carbocyclyl group is appended (e.g., arylheterocycloalkyl or cycloalkylheterocycloalkyl).

The term "carbocyclylcarbonylcarbocyclylalkyl" refers to a carbocyclylalkyl group to which is appended a carbocyclylcarbonyl group.

The term "(carbocyclylalkyl)heterocycloalkyl" refers to a heterocycloalkyl group to which a carbocyclylalkyl group is appended,

The term "cycloalkylcarbonyl" (alone or in combination with another term(s)) refers to a cycloalkyl group appended to the parent molecular moiety through a carbonyl group. For instance, C₃-C₁₀cycloalkylcarbonyl, C₃-C₈cyloalkylcarbonyl and C₃-C₆cycloalkylcarbonyl refer to a C₃-C₁₀cycloalkyl, C₃-C₈cycloalkyl or C₃-C₆cycloalkyl group appended to the parent molecular moiety through a carbonyl group, respectively, Representative examples of cycloalkylcarbonyl include, but are not limited to, cyclopropylcarbonyl, 2-cyclobutylcarbonyl, and cyclohexylcarbonyl.

The term "formyl" (alone or in combination with another term(s)) refers to a -C(O)H group.

The term "halo" or "halogen" (alone or in combination with another term(s)) refers to -Cl, -Br, -I or -F.

The term "haloalkoxy" (alone or in combination with another term(s)) refers to an alkoxy group, as defined herein, in which at least one hydrogen atom is replaced with a halogen (e.g., C₁-C₁₀ haloalkoxy, C₁-C₈haloalkoxy, or C₁-C₆haloalkoxy). Representative examples of haloalkoxy include, but are not limited to, chloromethoxy, fluoromethoxy, difluromethoxy, 2-fluoroethoxy, trifluoromethoxy, and pentafluoroethoxy.

The term "haloalkyl" (alone or in combination with another term(s)) refers to an alkyl group in which at least one hydrogen atom is replaced with a halogen (e.g., C₁-C₁₀haloalkyl, C₁-C₈haloalkyl, or C₁-C₆haloalkyl). Representative examples of haloalkyl include, but are not limited to, chloromethyl, fluoromethyl, difluoromethyl, 2-fluoroethyl, trifluoromethyl, pentafluoroethyl, and 2-chloro-3-fluoropentyl.

The terms "heterocycle" or "heterocyclo" or "heterocyclyl" (alone or in combination with another term(s)) refer to a saturated (e.g., "heterocycloalkyl"), partially unsaturated (e.g., "heterocycloalkenyl" or "heterocycloalkynyl") or completely unsaturated (e.g., "heteroaryl") ring system typically containing from 3 to 14 ring atoms, where at least one of the ring atoms is a heteroatom (i.e., nitrogen, oxygen or sulfur), with the remaining ring atoms being independently selected from the group consisting of carbon, nitrogen, oxygen and sulfur. In many embodiments, a heterocyclyl group of the present invention includes I ring, or 2 or 3 fused or spiro rings at least one of which is a heterocyclyl ring. Each heterocyclic ring may comprise, without limitation, from 3 to 8 ring members (such as 3, 4, 5, 6, 7 or 8 ring members) and 1or more heteroatoms. Certain heterocyclyls comprise a sulfur atom as a ring member; and in some cases, the sulfur atom is oxidized to SO or SO₂. A heterocyclyl group may be optionally substituted with a variety of substituents, and can be linked to the parent molecular moiety via any substitutable carbon or nitrogen atom in the group, provided that a stable molecule results. The nitrogen heteroatom(s) in a heterocyclyl may or may not be quaternized, and may or may not be oxidized to N-oxide. In addition, the nitrogen heteroatom(s) may or may not be N-protected.

As used herein, the number of ring atoms in a heterocyclyl moiety can be identified by the prefix "Hₓ-H_{y}," where x is the minimum and y is the maximum number of ring atoms in the heterocyclyl moiety.

Representative examples of monocyclic, bicyclic or tricyclic heterocyclyls include, but are not limited to, aziridinyl, azetidinyl, azepanyl, azepinyl, azocinyl, benzimidazolyl, benzimidazolinyl, benzisothiazolyl, benzisoxazolyl, benzodioxinyl, benzofuranyl, benzofuryl, benzopyranyl, benzoxazolyl, benzothiazolyl, benzothienyl, benzothiopyranyl, benzoxadiazolyl, benzotriazolyl, chromanyl, chromenyl, cinnolinyl, decahydroquinolinyl, diazepinyl, dihydroisoquinoline, dihydrofuranyl, tetrahydrofuranyl, 2,3-dihydroindolyl, 1,3-dioxolanyl, dioxanyl, dithianyl, furanyl, furazanyl, imidazolinyl, imidazolidinyl, imidazolyl, imidazopyridinyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolinyl, isothiazolidinyl, isothiazolyl, isoxazolyl, isoxazolinyl, isoxazolidinyl, morpholinyl, 3-oxo-morpholinyl, naphthyridinyl, octahydroisoquinolinyl, oxadiazolinyl, oxadiazolyl, oxazolyl, oxadiazolidinyl, oxazolinyl, 2-oxo-oxazolinyl, oxazolidinyl, pentazolyl, phenazinyl, phthalazinyl, piperidinyl, piperidonyl, piperazinyl, piperidyl, pteridinyl, purinyl, pyranopyridinyl, pyranyl, pyrazinyl, pyrazolyl, pyrazolinyl, pyrazolidinyl, pyridazinyl, pyridoimidazolyl, pyridooxazolyl, pyridopyrimidinyl, pyridothiazolyl, pyridazinyl, pyridyl, pyrimidyl, pyrimidinyl, pyrrolidonyl, pyrrolyl, pyrrolinyl, pyrrolidinyl, quinazolinyl, quinolinyl, quinolyl, quinoxalinyl, quinuclidinyl, tetrahydrofuranyl, tetrahydroquinolyl, tetrahydroisoquinolinyl, tetrahydroquinolinyl, tetrazolyl, tetrahydropyranyl, tetrahydropyridinyl, tetrahydrothienyl, thiadiazolinyl, thiadiazinyl, thiadiazolyl, thiazolyl, thiadiazolidinyl, thiazolinyl, thiazolidinyl, thienothiazolyl, thienooxazolyl, thienoimidazolyl, thienopyridinyl, thienyl, thiophenyl, thiomorpholinyl, 1,1-dioxidothiomorpholinyl (thiomorpholine sulfone), thiopyranyl, 1,4-diazepanyl, thioanthrenyl, thioxanthenyl, triazinyl, triazolyl, trithianyl, xanthenyl, and variants of the foregoing in which one or more sulfur atoms comprised therein are oxidized.

The term "heterocycloalkoxy" (alone or in combination with another term(s)) refers to an alkoxy group to which is appended a heterocyclyl. For instance, heterocycloC₁-C₆alkoxy means a heterocyclyl group appended to the parent molecular moiety through a C₁-C₆alkoxy group.

The term "heterocycloalkoxyalkyl" (alone or in combination with another term(s)) refers to a heterocycloalkoxy group appended to the parent molecular moiety through an alkylene group (e.g., heterocycloC₁-C₆alkoxyC₁-C₆alkyl).

The term "heterocycloalkoxycarbonyl" (alone or in combination with another term(s)) refers to a heterocycloalkoxy group appended to the parent molecular moiety through a carbonyl group. For instance, heterocycloC₁-C₆alkoxycarbonyl means a heterocycloC₁-C₆alkoxy group appended to the parent molecular moiety through a carbonyl group.

The term "heterocycloalkyl" (alone or in combination with another term(s)) refers to a heterocyclyl appended to the parent molecular moiety through an alkylene group (e.g., heterocycloC₁-C₆ alkyl).

The term "heterocycloalkylcarbonyl" (alone or in combination with another term(s)) refers to a heterocycloalkyl group appended to the parent molecular moiety through a carbonyl group (e.g., heterocycloC₁-C₆alkylcarbonyl, in which heterocycloC₁-C₆alkyl is appended to the parent molecular moiety through a carbonyl group).

The term "heterocyclocarbonyl" (alone or in combination with another term(s)) refers to a heterocyclyl appended to the parent molecular moiety through a carbonyl group.

The terms "heterocyclyloxy" or "(heterocyclo)oxy" (alone or in combination with another term(s)) refers to a heterocyclyl group appended to the parent molecular moiety through an oxy moiety.

The term "(heterocyclyo)oxyalkyl" (alone or in combination with another term(s)) refers to a heterocyclyloxy group appended to the parent molecular moiety through an alkylene group.

The term "(heterocyclo)oxycarbonyl" (alone or in combination with another term(s)) refers to a (heterocyclo)oxy group appended to the parent molecular moiety through a carbonyl group.

The term "heterocyclothio" (alone or in combination with another term(s)) refers to a heterocyclyl appended to the parent molecular moiety through a sulfur atom.

The term "heterocyclothioalkoxy" (alone or in combination with another term(s)) refers to a thioalkoxy group to which is appended a heterocyclyl.

The term "heterocyclothioalkoxyalkyl" (alone or in combination with another term(s)) refers to a heterocyclothioalkoxy group appended to the parent molecular moiety through an alkylene group.

The term "heterocyclothioalkyl" (alone or in combination with another term(s)) refers to a heterocyclothio group appended to the parent molecular moiety through an alkylene group.

The term "heterocyclocarbocyclyl" refers to a heterocyclyl appended to the parent molecular moiety through a carbocyclyl group (e.g., heterocycloaryl or heterocyclocycloalkyl, in which a heterocyclyl appended to the parent molecular moiety through an aryl or cycloalkyl group, respectively).

The term "heterocyclocarbocyclylalkyl" refers to a heterocyclocarbocyclyl group appended to the parent molecular moiety through an alkylene group (e.g., heterocyclocarbocyclylC₁-C₆alkyl, such as heterocycloarylC₁-C₆alkyl or heterocyclocycloalkylC₁-C₆alkyl).

The term "(heterocyclo)alkoxycarbocyclylalkyl" refers to a carbocyclylalkyl to which a heterocycloalkoxy group is appended.

The term "(heterocyclo)carbonylcarbocyclylalkyl" refers to a carbocyclylalkyl to which a heterocyclocarbonyl group is appended.

The term "(heterocyclo)heterocycloalkyl" refers to a heterocycloalkyl to which a heterocyclyl group is appended.

The term "(heterocyclo)alkoxyheterocycloalkyl" refers to a heterocycloalkyl group to which a heterocycloalkoxy group is appended.

The term "(heterocyclo)carbonylheterocycloalkyl" refers to a heterocycloalkyl to which a heterocyclocarbonyl is appended.

The term "(heterocycloalkyl)carbocyclylalkyl" refers to a carbocyclylalkyl to which a heterocycloalkyl group is appended.

The term "(heterocycloalkyl)heterocycloalkyl" refers to a heterocycloalkyl to which another heterocycloalkyl group is appended. The former heterocycloalkyl group can be identical to or different from the latter heterocycloalkyl group.

The term "hydroxy" (alone or in combination with another term(s)) refers to an -OH group.

The term "heteroaryl" (alone or in combination with another term(s)) refers to an aromatic heterocyclyl typically containing from 5 to 14 ring atoms. A heteroaryl can be a single ring or two or three fused rings. Representative examples of heteroaryls include, but are not limited to, benzimidazolyl, benzothiazolyl, benzothienyl, benzoxazolyl, benzofyuranyl, benzoxadiazolyl, dibenzothienyl, dibenzofuranyl, furyl, imidazopyridinyl, imidazolyl, indazolyl, indolyl, isoindolyl, isoquinolinyl, isothiazolyl, isoxazolyl, naphthyridinyl, oxadiazolyl, oxadiazolyl, oxazolyl, pyrazinyl, pyrazolyl, pyridinyl, pyridoimidazolyl, pyrimidinyl, pyridazinyl, pyrrolyl, quinolinyl, quinolizinyl, quinoxalinyl, quinazolinyl, thiazolyl, thienopyridinyl, thienyl, triazinyl, and triazolyl. The heteroaryl groups of the present invention can be independently substituted or unsubstituted, and connected to the parent molecular moiety through any substitutable carbon or nitrogen atom in the groups. In addition, the nitrogen heteroatom may or may not be quaternized, and may or may not be oxidized to the N-oxide. Also, the nitrogen containing rings may or may not be N-protected.

The term "heteroarylalkoxy" (alone or in combination with another term(s)) refers to an alkoxy group to which is appended a heteroaryl group (e.g., heteroarylC₁-C₆alkoxy). Representative examples of heteroarylalkoxy include, but are not limited to, 2-pyridin-3-ylethoxy, 1,3-thiazol-5-ylmethoxy, 3-quinolin-3-ylpropoxy, and 5-pyridin-4-ylpentyloxy.

The term "heteroarylalkoxyalkyl" (alone or in combination with another term(s)) refers to a heteroarylalkoxy group appended to the parent molecular moiety through an alkylene group (e.g., heteroarylC₁-C₆alkoxyC₁-C₆alkyl). Representative examples of heteroarylalkoxyalkyl include, but are not limited to, (2-pyridin-3-ylethioxy)methyl, (3-quinolin-3-ylpropoxy)methyl, (1,3-thiazol-5-ylmethoxy)methyl, and 2-(5-pyridin-4-ylpentyloxy)ethyl.

The term "heteroarylalkoxycarbonyl" (alone or in combination with another term(s)) refers to a heteroarylalkoxy group appended to the parent molecular moiety through a carbonyl group (e.g., heteroarylC₁-C₆alkoxycarbonyl, in which a heteroarylC₁-C₆alkoxy is appended to the parent molecular moiety through a carbonyl group). Representative examples of heteroarylalkoxycarbonyl include, but are not limited to, (2-pyridin-3-ylethoxy)carbonyl, (3-quinolin-3-ylpropoxy)carbonyl, 2-(1,3-thiazol-5-ylmethoxy)carbonyl, and (5-pyridin-4-ylpentyloxy)carbonyl.

The term "heteroarylalkyl" (alone or in combination with another term(s)) refers to a heteroaryl group appended to the parent molecular moiety through an alkylene group (e.g., heteroarylC₁-C₆alkyl). Representative examples of heteroarylalkyl include, but are not limited to, 3-quinolinylmethyl, 3-pyridinylmethyl, 4-pyridinylmethyl, 1H-imidazol-4-ylmethyl, 1H-pyrrol-2-ylmethyl, pyridin-3-ylmethyl, and 2-pyrimidin-2-ylpropyl.

The term "heteroarylalkylcarbonyl" (alone or in combination with another term(s)) refers to a heteroarylalkyl group appended to the parent molecular moiety through a carbonyl group (e.g., heteroarylC₁-C₆alkylcarbonyl, in which a heteroarylC₁-C₆alkyl is appended to the parent molecular moiety through a carbonyl group). Representative examples of heteroarylalkylcarbonyl include, but are not limited to, ((2,5-dimethoxytetrahydro-3-furanyl)methyl)carbonyl, (3-quinolinylmethyl)carbonyl, (3-pyridinylmethyl)carbonyl, (4-pyridinylmethyl)carbonyl, (1H-imidazol-4-ylmethyl)carbonyl, (1H-pyrrol-2-ylmethyl)carbonyl, (pyridin-3-ylmethyl)carhonyl, and (2-pyrimidin-2-ylpropyl)carbonyl.

The term "heteroarylcarbonyl" (alone or in combination with another term(s)) refers to a heteroaryl group appended to the parent molecular moiety through a carbonyl group. Representative examples of heteroarylcarbonyl include, but are not limited to, pyridin-3-ylcarbonyl, (1,3-thiazol-5-yl)carbonyl, and quinolin-3-ylcarbonyl.

The term "heteroaryloxy" (alone or in combination with another term(s)) refers to a heteroaryl group appended to the parent molecular moiety through an oxy moiety. Representative examples of heteroaryloxy include, but are not limited to, pyridin-3-yloxy, and quinolin-3-yloxy.

The term "heteroaryfoxyalkyl" (alone or in combination with another term(s)) refers to a heteroaryloxy group appended to the parent molecular moiety through an alkylene group (e.g., heteroaryloxyC₁-C₆alkyl). Representative examples of heteroaryloxyalkyl include, but are not limited to, pyridin-3-yloxymethyl, and 2-quinolin-3-yloxyethyl,

The term "heteroaryloxycarbonyl" (alone or in combination with another term(s)) refers to a heteroaryloxy group appended to the parent molecular moiety through a carbonyl group.

The term "heteroarylthio" (alone or in combination with another term(s)) refers to a heteroaryl group appended to the parent molecular moiety through a sulfur atom. Representative examples of heteroarylthio include, but are not limited to, (3-quinolinyl)thio, (3-pyridinyl)thio, and (4-pyridinyl)thio.

The term "heteroarylthioalkoxy" (alone or in combination with another term(s)) refers to a thioalkoxy group to which is appended a heteroaryl group. Representative examples of heteroarylthioalkoxy include, but are not limited to, 2-pyridin-3-ylethylthio, 1,3-thiazol-5-ylmethylthio, 3-quinolin-3-ylpropylthio, and 5-pyridin-4-ylpentylylthio.

The term "heteroarylthioalkoxyalkyl" (alone or in combination with another term(s)) refers to a heteroarylthioalkoxy group appended to the parent molecular moiety through an alkylene group. Representative examples of heteroarylthioalkoxyalkyl include, but are not limited to, (2-pyridin-3-ylethylthio)methyl, (3-quinolin-3-ylpropylthio)methyl, (1,3-thiazol-5-ylmethylthio)methyl, and 2-(5-pyridin-4-ylpentylthio)ethyl.

The term "heteroarylthioalkyl" (alone or in combination with another term(s)) refers to a heteroarylthio group appended to the parent molecular moiety through an alkylene group. Representative examples of heteroarylthioalkyl include, but are not limited to, (3-quinolinyl)thiomethyl, (3-pyridinyl)thiomethyl, (4-pyridinyl)thiomethyl, and 2-((4-pyridinyl)thio)ethyl.

The term "N-protecting group" or "N-protected" refers to those groups capable of protecting an amino group against undesirable reactions. Commonly used N-protecting groups are described in Greene and Wuts, PROTECTING GROUPS IN CHEMiCAL SYNTHESIS (3rd ed., John Wiley & Sons, NY (1999), which is incorporate herein by reference in its entirety. Non-limiting examples ofN-protecting groups include acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, benzoyl, 4-chlorobenzoyl, 4-bromobenzoyl, or 4-nitrobenzoyl; sulfonyl groups such as benzenesulfonyl or p-toluenesulfonyl; sulfenyl groups such as phenylsulfenyl (phenyl-S-) or triphenylmethylsulfenyl (trityl-S-); sulfinyl groups such as p-methylphenylsulfinyl (p-methylphenyl-S(O)-) or t-butylsulfinyl (t-Bu-S(O)-); carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2-trichloro-ethoxycarbonyl, phenoxycarbonyl, 4-nitro-phenoxycarbonyl, fluorenyl-9-methoxycarbonyl, cyclopentyloxycarbonyl, adamantyloxycarbonyl, cyclohexyloxycarbonyl, or phenylthiocarbonyl; alkyl groups such as benzyl, p-methoxybenzyl, triphenylmethyl, or benzyloxymethyl; p-methoxyphenyl; and silyl groups such as trimethylsilyl. Preferred N-protecting groups include formyl, acetyl, benzoyl, pivaloyl, t-butylacetyl, phenylsulfonyl, benzyl, t-butyloxycarbonyl (Boc) and benzyloxycarbonyl (Cbz).

The term "hydroxyalkyl" (alone or in combination with another term(s)) refers to an alkyl group to which is appended at least one hydroxy. Representative examples of hydroxyalkyl include, but are not limited to, hydroxymethyl, 2-hydroxyethyl, 3-hydroxypropyl, and 2-ethyl-4-hydroxyheptyl.

The term "nitro" (alone or in combination with another term(s)) refers to a -NO₂ group.

The term "oxo" (alone or in combination with another term(s)) refers to a =O moiety.

The term "oxy" (alone or in combination with another term(s)) refers to a -O- moiety.

The term "sulfonyl" (alone or in combination with another term(s)) refers to a -(O)₂- group.

The term "thioalkoxy" (alone or in combination with another term(s)) refers to an alkyl group appended to the parent molecular moiety through a sulfur atom. For instance, C₁₋C₂₀thioalkoxy, C₁-C₁₀thioalkoxy and C₁C₆thioalkoxy refer to C₁-C₂₀alkyl, C₁₋C₁₀alkyl or C₁-C₆alkyl appended to the parent molecular moiety through a sulfur atom, respectively. Representative examples of thioalkoxy include, but are not limited to, methylthio, ethylthio, and butylthio.

The term "pharmaceutical acceptable" is used adjectivally to mean that the modified noun is appropriate for use as a pharmaceutical product or as a part of a pharmaceutical product.

The term "prodrug" refers to derivatives of the compounds of the invention which have chemically or metabolically cleavable groups and become, by solvolysis or under physiological conditions, the compounds of the invention which are pharmaceutically active *in vivo*. A prodrug of a compound can be formed in a conventional manner with a functional group of the compound (e.g., an amino, hydroxy, sulfhydryl, or carboxy group). A prodrug derivative form often offers advantages of solubility, tissue compatibility, or delayed release in a mammalian organism. Examples of prodrugs include, but are not limited to, acetate, formate, benzoate or other acylated derivatives of alcohol and amine functional groups within the compounds of the invention. Prodrugs also include acid derivatives, such as esters prepared from reaction of the parent acidic compound with a suitable alcohol, or amides prepared by reaction of the parent acid compound with a suitable amine.

The term "solvate" refers to the physical association of a compound of the invention with one or more solvent molecule, whether organic or inorganic. This physical association often includes hydrogen bonding. In certain instances, the solvate is capable of isolation, for example, when one or more solvate molecules are incorporated in the crystal lattice of the crystalline solid. "Solvate" encompasses both solution-phase and isolable solvates. Exemplary solvates include hydrates, ethanolates, and methanolates.

The following abbreviations are used in the description of the Schemes and Examples:
AcOH for acetic acid;
atm for atmospheres;
Boc for tert-butoxycarbonyl;
CDI for 1,1'-carbonyldiimidazole;
DCE for 1,2-dichloroethane;
DEAD for diethyl azodicarboxylate;
DMAP for 4-Dimethylaminopyridine
DMF for N,N-dimethylformamide;
DMSO for dimethylsulfoxide;
EDCI for (N-ethyl-N'-(3-dimethylaminopropyl)carbodiimide hydrochloride;
Et₃N for triethylamine;
EtOAc for ethyl acetate;
EtOH for ethanol;
Fmoc chloride for 9-fluorenylmethyl chloroformate;
HOBt for N-Hydroxybenzotriazole;
IPA for isopropyl alcohol;
MeOH for methanol;
MsCl for methanesulfonyl chloride;
TFA for trifluoroacetic acid;
THF for tetrahydrofuran; and
TLC for thin layer chromatography.

Stereoisomers

The compounds of the invention can comprise asymmetrically substituted carbon atoms known as chiral centers. These chiral centers are designated as "R" or "S" depending on the configuration of substituents around the chiral carbon atom. The terms "R" and "S" used herein are configurations as defined in Nomenclature of Organic Chemistry, Section E: Stereochemistry, Recommendations 1974, PURE APPL. CHEM., 45:11-30 (1976), The compounds of this invention may exist as single stereoisomers (e.g., single enantiomers or single diastereomer), mixtures of Stereoisomers (e.g. any mixture of enantiomers or diastereomers) or racemic mixtures. All such single stereoisomers, mixtures and racemates are encompassed within the scope of the invention. Compounds identified herein as single stereoisomers are meant to describe compounds that are present in a form that is substantially free from other enantiomers or diastereomers. By "substantially free," it means that at least 80% of the compound in a composition is the desired enantiomer or diastereomer; preferably, at least 90% of the compound in a composition is the desired enantiomer or diastereomer; and more preferably, at least 95%, 96%, 97%, 98% or 99% of the compound in a composition is the desired enantiomer or diastereomer. Where the stereochemistry of the chiral carbon(s) present in a chemical structure is not specified, the chemical structure is intended to encompass compounds containing either stereoisomer of each chiral center present in the chemical structure.

Individual stereoisomers of the compounds of this invention can be prepared using many methods known in the art. These methods include, but are not limited to, stereospecific synthesis, chromatographic separation of diastereomers, chromatographic resolution of enantiomers, conversion of enantiomers in an enantiomeric mixture to diastereomers followed by chromatographically separation of the diastereomers and regeneration of the individual enantiomers, and enzymatic resolution.

Stereospecific synthesis typically involves the use of appropriate optically pure (enantiomerically pure) or substantial optically pure materials and synthetic reactions that do not cause racemization or inversion of stereochemistry at the chiral centers. Mixtures of stereoisomers of compounds, including racemic mixtures, resulting from a synthetic reaction may be separated by chromatographic techniques as appreciated by those of ordinary skill in the art. Chromatographic resolution of enantiomers can be accomplished on chiral chromatography resins, many of which are commercially available. In a non-limiting example, racemate is placed in solution and loaded onto the column containing a chiral stationary phase. Enantiomers can then be separated by HPLC.

Resolution of enantiomers can also be accomplished by converting enantiomers in a mixture to diastereomers by reaction with chiral auxiliaries. The resulting diastereomers can be separated by column chromatography or crystallization/re-crystallization. This technique is useful when the compounds to be separated contain a carboxyl, amino or hydroxyl group that will form a salt or covalent bond with the chiral auxiliary. Non-limiting examples of suitable chiral auxiliaries include chirally pure amino acids, organic carboxylic acids or organosulfonic acids. Once the diastereomers are separated by chromatography, the individual enantiomers can be regenerated. Frequently, the chiral auxiliary can be recovered and used again.

Enzymes, such as esterases, phosphatases or lipases, can be useful for the resolution of derivatives of enantiomers in an enantiomeric mixture. For example, an ester derivative of a carboxyl group in the compounds to be separated can be treated with an enzyme which selectively hydrolyzes only one of the enantiomers in the mixture. The resulting enantiomerically pure acid can then be separated from the unhydrolyzed ester.

Alternatively, salts of enantiomers in a mixture can be prepared using any method known in the art, including treatment of the carboxylic acid with a suitable optically pure base such as alkaloids or phenethylamine, followed by precipitation or crystallization/re-crystallization of the enantiomerically pure salts. Methods suitable for the resolution/separation of a mixture of stereoisomers, including racemic mixtures, can be found in ENANTIOMERS, RACEMATES, AND RESOLUTIONS (Jacques et al., 1981, John Wiley and Sons, New York, NY).

A compound of this invention may possess one or more unsaturated carbon-carbon double bonds. All double bond isomers, such as the cis (Z) and trans (E) isomers, and mixtures thereof are intended to be encompassed within the scope of the present invention. In addition, where a compound exists in various tautomeric forms, a recited compound is not limited to any one specific tautomer, but rather is intended to encompass all tautomeric forms.

Pharmaceutical Compositions

The present invention features pharmaceutical compositions comprising the compounds of the present invention. Typically, a pharmaceutical composition of the present invention comprises one or more compounds of the present invention (e.g., any compound described above or in Example 1) and a pharmaceutically acceptable carrier or excipient. Non-limiting examples of suitable pharmaceutically acceptable carriers or excipients include sugars (e.g., lactose, glucose or sucrose), starches (e.g., corn starch or potato starch), cellulose or its derivatives (e.g., sodium carboxymethyl cellulose, ethyl cellulose or cellulose acetate), oils (e.g., peanut oil, cottonseed oil, safflower oil, sesame oil, olive oil, corn oil or soybean oil), glycols (e.g., propylene glycol), buffering agents (e.g., magnesium hydroxide or aluminum hydroxide), agar, alginic acid, powdered tragacanth, malt, gelatin, talc, cocoa butter, pyrogen-free water, isotonic saline, Ringer's solution, ethanol, or phosphate buffer solutions. Lubricants, coloring agents, releasing agents, coating agents, sweetening, flavoring or perfuming agents, preservatives, or antioxidants can also be included in a pharmaceutical composition of the present invention, as appreciated by those of ordinary skill in the art.

The present invention also features pharmaceutical compositions comprising pharmaceutically acceptable salts, solvates, or prodrugs of the compounds of the present invention. Pharmaceutically acceptable salts can be zwitterions or derived from pharmaceutically acceptable inorganic or organic acids or bases. Preferably, a pharmaceutically acceptable salt of a compound retains the biological effectiveness of the free acid or base of the compound without undue toxicity, irritation, or allergic response, has a reasonable benefit/risk ratio, and is effective for their intended use and not biologically or otherwise undesirable. Non-limiting examples of pharmaceutically acceptable salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. The basic nitrogen-containing groups can also be quatemized with such agents as loweralkyl halides (e.g., methyl, ethyl, propyl or butyl chlorides, bromides or iodides), dialkyl sulfates (e.g., dimethyl, diethyl, dibutyl or diamyl sulfates), long chain halides (e.g., decyl, lauryl, myristyl or stearyl chlorides, bromides or iodides), aralkyl halides (e.g., benzyl or phenethyl bromides). Other salts that can be used in the present invention include salts with alkali or alkaline earth metals, such as sodium, potassium, calcium or magnesium, or with organic bases. Examples of acids which can be used to form pharmaceutically acceptable acid addition salts include, but are not limited to, hydrochloric acid, sulphuric acid, phosphoric acid, oxalic acid, maleic acid, succinic acid, citric acid, or other suitable inorganic or organic acids.

In addition, the present invention features pharmaceutical compositions comprising a compound of the present invention (or a salt, solvate or prodrug thereof) and a therapeutic agent. Preferably, a pharmaceutical composition of the present invention comprises a compound of the present invention (or a salt, solvate or prodrug thereof) and a drug that is metabolizable by a CYP enzyme (e.g., CYP3A4, CYP2D6 or CYP2C9). CYPs are known to be involved in the metabolism of a wide range of drugs, including but not limited to, immunomodulators (e.g., cyclosporine or FK-506), anti-cancer or chemotherapeutic agents (e.g., taxol or taxotere), antibiotics (e,g., clarithromycin, erythromycin, or telithromycin), antivirals (e.g., indinavir, lopinavir, nelfinavir, or saquinavir), antihistamines (e.g., astemizole, chlorpheniramine,or terfenidine), calcium channel blockers (e.g., amlodipine, diltiazem, felodipine, lercanidipine, nifedipine, nisoldipine, nitrendipine, or verapamil), beta blockers (e.g., carvedilol, S-metoprolol, propafenone, or timolol), and antidepressants (e.g., amitriptyline, clomipramine, desipramine, imipramine, or paroxetine). The inhibition of CYPs by a compound of the present invention can improve the pharmacokinetics of the co-administered drug, leading to an improved Cₘₐₓ (the maximum plasma level), Tₘₐₓ (time to maximum plasma level), AUC (area under the plasma concentration curve), half-life, or blood/liver/tissue level of the drug.

In one embodiment, a pharmaceutical composition of the present invention comprises a compound of the present invention (or a salt, solvate or prodrug thereof) and a drug that can be metabolized by CYP3A4. In another embodiment, a pharmaceutical composition of the present invention comprises a compound of the present invention (or a salt, solvate or prodrug thereof) and a drug that can be metabolized by CYP2D6. In still another embodiment, a pharmaceutical composition of the present invention comprises a compound of the present invention (or a salt, solvate or prodrug thereof) and a drug that can be metabolized by CYP2C9.

In one example, a pharmaceutical composition of the present invention comprises a compound of the invention (or a salt, solvate or prodrug thereof) and a drug selected from the group consisting of an immunomodulator, an anti-cancer or chemotherapeutic agent, an antibiotic agent, an antiviral agent, an antihistamine, a calcium channel blocker, a beta blocker, and an antidepressant, In another example, a pharmaceutical composition of the present invention comprises a compound of the invention (or a salt, solvate or prodrug thereof) and a drug selected from the group consisting of cyclosporine, FK-506, taxol, taxotere, clarithromycin, erythromycin, telithromycin, indinavir, lopinavir, nelfinavir, saquinavir, astemizole, chlorpheniramine, terfenidine, amlodipine, diltiazem, felodipine, lercanidipine, nifedipine, nisoldipine, nitrendipine, verapamil, carvedilol, S-metoprolol, propafenone, timolol, amitriptyline, clomipramine, desipramine, imipramine, and paroxetine.

In still another example, a pharmaceutical composition of the present invention comprises a compound of the invention (e.g., a compound selected from Example 1) or a salt, solvate or prodrug thereof, and an antiviral agent. In yet another example, a pharmaceutical composition of the present invention comprises a compound of the invention (e.g., a compound selected from Example 1) or a salt, solvate or prodrug thereof, and an anti-hepatitis C virus (HCV) agent. In still yet another example, a pharmaceutical composition of the present invention comprises a compound of the invention (e.g., a compound selected from Example 1) or a salt, solvate or prodrug thereof, and an anti-human immunodeficiency virus (HIV) agent.

In a further example, a pharmaceutical composition of the present invention comprises a compound of the invention (e.g., a compound selected from Example 1) or a salt, solvate or prodrug thereof, and 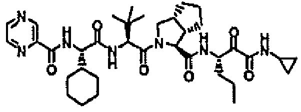 (hereinafter compound VX-950, Vertex Pharmaceuticals Inc.) or a salt, solvate or prodrug thereof. In another example, a pharmaceutical composition of the present invention comprises a compound of the invention (e.g., a compound selected from Example 1) or a salt, solvate or prodrug thereof, and 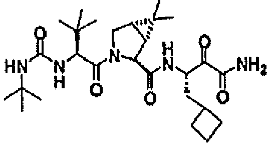 (hereinafter compound SCH503034, Schering-Plough Co.) or a salt, solvate or prodrug thereof. In still another example, a pharmaceutical composition of the present invention comprises a compound of the invention (e.g., a compound selected from Example 1) or a salt, solvate or prodrug thereof, and lopinavir (i.e., [1S-[1R*,(R*),3R*,4R*]]-N-[4-[[(2,6-dimethylphenoxy)acetyl]amino]-3-hydroxy-5-phenyl-1-(phenylmethyl) pentyl]tetrahydroalpha-(1-methylethyl)-2-oxo-1(2H)-pyrimidineacetamide). In a further example, a pharmaceutical composition of the present invention comprises a compound of the invention (e.g., a compound selected from Example 1) or a salt, solvate or prodrug thereof, and 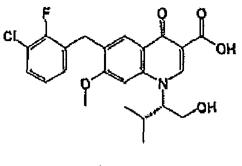 (hereinafter compound GS9137, Gilead Sciences, Inc., Foster City, CA) or a salt, solvate or prodrug thereof.

Any compound described herein (e.g., the compounds listed in Example 1), or a salt, solvate or prodrug thereof, can be included in a pharmaceutical composition of the present invention.

A pharmaceutical composition of the present invention can be administered to a subject in need thereof via a variety of routes, such as orally, parenterally, sublingually, rectally, topically or by inhalation spray. Topical administration may involve the use of transdermal administration such as transdermal patches or iontophoresis devices. Parenteral administration includes, but is not limited to, subcutaneous, intravenous, intramuscular or intrasternal injections, and infusion techniques.

The pharmaceutical compositions of the present invention can be formulated based on their routes of administration using methods well known in the art. For example, a sterile injectable preparation can be prepared as a sterile injectable aqueous or oleagenous suspension using suitable dispersing or wetting agents and suspending agents. Suppositories for rectal administration can be prepared by mixing drugs with a suitable nonirritating excipient such as cocoa butter or polyethylene glycols which are solid at ordinary temperatures but liquid at the rectal temperature and will therefore melt in the rectum and release the drugs. Solid dosage forms for oral administration can be capsules, tablets, pills, powders or granules. In such solid dosage forms, the active compounds can be admixed with at least one inert diluent such as sucrose lactose or starch. Solid dosage forms may also comprise other substances in addition to inert diluents, such as lubricating agents. In the case of capsules, tablets and pills, the dosage forms may also comprise buffering agents. Tablets and pills can additionally be prepared with enteric coatings. Liquid dosage forms for oral administration can include pharmaceutically acceptable emulsions, solutions, suspensions, syrups or elixirs containing inert diluents commonly used in the art. Liquid dosage forms may also comprise wetting, emulsifying, suspending, sweetening, flavoring, or perfuming agents. The pharmaceutical compositions of the present invention can also be administered in the form of liposomes, as described in U.S. Patent No. 6,703,403.

A compound of the present invention (or a salt, solvent or prodrug thereof) can be administered to a human or animal host in a single dose or divided doses. A typical daily dosage can range from 0.001 to 300 mg/kg body weight, such as from 0.1 to 25 mg/kg, body weight. Preferably, each dosage contains a sufficient amount of a compound of the present invention that is effective in inhibiting CYP enzyme(s) in the host or improving the pharmacokinetics of the co-administered drug. The amount of active ingredients that are combined to produce a single dosage form may vary depending upon the host treated and the particular mode of administration. It will be understood that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

In many cases, a pharmaceutical composition of the present invention comprises a compound of the invention in such an amount that is effective in improving the pharmacokinetics of the co-administered drug, but is ineffective by itself for the treatment of any disease.

Inhibition of Cytochrome P450 enzymes

The present invention features methods of using the compounds of the invention to inhibit cytochrome P450 oxidases, such as CYP3A4, CYP2D6 or CYP2C9. The methods comprise contacting a compound of the present invention (or a salt, solvate or prodrug thereof) with a CYP enzyme (such as CYP3A4, CYP2D6 or CYP2C9), thereby inhibiting the metabolizing activity of the CYP enzyme. As used herein, "inhibiting" means significantly reducing, or abolishing, the original activity of an enzyme. For instance, a compound of the present invention inhibits the activity of a CYP enzyme if the compound can reduce the metabolizing activity of the enzyme by at least 10%, 20%, 30%, 40%, 50%, 60%, 70%, 80%, 90%, 95% or more. Methods suitable for measuring the metabolizing activity of a CYP enzyme are well known in the art, Any compound of the present invention can be used to inhibit the metabolizing activity of a CYP enzymes (e.g., CYP3A4, CYP2D6 or CYP2C9).

In one embodiment, the present invention features methods of using the compounds of the invention to inhibit CYP3A4. The methods comprise contacting a compound of the invention (e.g., a compound listed in Example 1), or a salt, solvate or prodrug thereof, with CYP3A4, thereby inhibiting the metabolizing activity of CYP3A4. In another embodiment, the present invention features additional methods of using the compounds of the invention to inhibit CYP3A4. The methods comprise contacting a compound of the invention (e.g., a compound listed in Example 1), or a salt, solvate or prodrug thereof, with cells comprising CYP3A4, thereby inhibiting the metabolizing activity of CYP3A4 in the cells. In yet another embodiment, the present invention features methods of using the compounds of the invention to inhibit CYP2D6. The methods comprise contacting a compound of the invention (e.g., a compound listed in Example 1), or a salt, solvate or prodrug thereof, with CYP2D6, thereby inhibiting the metabolizing activity of CYP2D6. In still yet another embodiment, the present invention features additional methods of using the compounds of the invention to inhibit CYP2D6. The methods comprise contacting a compound of the invention (e.g., a compound listed in Example 1), or a salt, solvate or prodrug thereof, with cells comprising CYP2D6, thereby inhibiting the metabolizing activity of CYP2D6 in the cells, In a further embodiment, the present invention features methods of using the compounds of the invention to inhibit CYP2C9, The methods comprise contacting a compound of the invention (e.g., a compound listed in Example 1), or a salt, solvate or prodrug thereof, with CYP2C9, thereby inhibiting the metabolizing activity of CYP2C9. In still yet another embodiment, the present invention features additional methods of using the compounds of the invention to inhibit CYP2C9. The methods comprise contacting a compound of the invention (e.g., a compound listed in Example 1), or a salt, solvate or prodrug thereof, with cells comprising CYP2C9, thereby inhibiting the metabolizing activity of CYP2C9 in the cells.

The present invention also features methods of using the compounds of the invention to inhibit a CYP enzyme *in vivo.* The methods comprise administering an effective amount of a compound of the present invention (or a salt, solvate or prodrug thereof) to a subject of interest, thereby inhibiting the metabolizing activity of a CYP enzyme (e.g., CYP3A4, CYP2D6 or CYP2C9) in the subject. As used herein, a subject of interest can be a human or mammal. Because cytochrome P450 sequence homology has been observed among almost all lineages of life, including birds, fish, insects, worms, sea squirts, sea urchins, plants, fungi, slime molds, bacteria and archaea, the present invention also contemplate the use of the compounds of the invention to inhibit the metabolizing activities of CYP enzymes in other animals or organisms.

The present invention further features methods of using the compounds of the invention to improve the pharmacokinetics of drugs that are metabolized by CYP enzymes (e.g., CYP3A4, CYP2D6, or CYP2C9). The methods comprise administering a drug which is metabolizable by a CYP enzyme (e.g., CYP3A4, CYP2D6, or CYP2C9) and an effective amount of a compound of the present invention (or a salt, solvate or prodrug thereof) to a subject in need thereof. The compound of the present invention, or the salt, solvate or prodrug thereof, is administered in such an amount that it effectively inhibits the CYP enzyme and therefore the metabolism of the co-administered drug. In many instances, the inhibition of the CYP enzyme leads to an increased Cₘₐₓ, Tₘₐₓ, AUC, half-life, or the blood, liver or tissue level of the co-administered drug. Drugs suitable for co-administration with a compound of the present invention include a wide array of drugs that are metabolizable by CYP3A4, CYP2D6, CYP2C9 or other CYP enzymes, such as many immunomodulators, anti-cancer or chemotherapeutic agents, antibiotics, antivirals (e.g., anti-HIV or anti-HCV agents), antihistamines, calcium channel blockers, beta blockers or antidepressants. Specific examples of these drugs include, but are not limited to, compound VX-950, compound SCH503034, compound GS9137, cyclosporine, FK-506, taxol, taxotere, clarithromycin, erythromycin, telithromycin, indinavir, lopinavir, nelfinavir, saquinavir, astemizole, chlorpheniramine, terfenidine, amlodipine, diltiazem, felodipine, lercanidipine, nifedipine, nisoldipine, nitrendipine, verapamil, carvedilol, S-metoprolol, propafenone, timolol, amitriptyline, clomipramine, desipramine, imipramine, and paroxetine.

In one embodiment, the present invention features methods for improving the pharmacokinetics of an antiviral agent. The methods comprise administrating an antiviral agent, and an effective amount of compound of the invention (e.g., a compound listed in Example 1) or a salt, solvate or prodrug thereof, to a subject in need thereof, thereby improving the pharmacokinetics of the anti-viral agent in the subject. In many cases, the compound of the invention increases the blood or liver level of the co-administered drug. Many anti-HIV or anti-HCV agents can be boosted by a compound of the invention. Non-limiting examples of these agents include HIV or HCV protease inhibitors that are metabolized by CYP3A4, CYP2D6 or CYP2C9.

In another embodiment, the present invention features methods for improving the pharmacokinetics of compound VX-950, SCH503034 or GS9137. The methods comprise administrating compound VA-950, SCH503034 or GS9137, and an effective amount of a compound of the invention (e.g., a compound listed in Example 1) or a salt, solvate or prodrug thereof, to a subject in need thereof, thereby improving the pharmacokinetics of VA-950, SCH503034 or GS9137 in the subject (e.g., increasing the blood or liver level of VA-950, SCH503034 or GS9137 in the subject).

A compound of the present invention (or a salt, solvate or prodrug thereof) and another drug can be combined in a single formulation and administered simultaneously to a subject of interest. They can also be administered simultaneously but in different formulations, or administered substantially simultaneously (e.g., less than 5 minutes apart) or sequentially (e.g., at least 5 minutes apart). By improving the pharmacokinetics of the co-administered drug, a compound of the present invention can improve the potency or effectiveness of the drug in the treatment of a targeted disease, such as viral infection, cancer, high blood pressure or mental disorder. As used herein, two agents are co-administered if one agent can affect the action of the other agent, including the pharmacokinetics of the other agent, regardless of whether these agents are administered simultaneously or sequentially.

The present invention also features methods of using the compounds of the invention and therapeutic agents for the treatment of diseases. The methods comprise administering a therapeutic agent which is metabolizable by a CYP enzyme (e,g., CYP3A4, CYP2D6, or CYP2C9) and an effective amount of a compound of the present invention (or a salt, solvate or prodrug thereof) to a subject in need thereof, where the therapeutic agent is effective in treating a disease in the subject, and the compound of the present invention (or the salt, solvate or prodrug thereof) improves the pharmacokinetics of the therapeutic agent, leading to an increased blood, liver or tissue level, half-life, Cₘₐₓ, Tₘₐₓ, AUC of the therapeutic agent. Preferably, the compound of the invention increases the blood or liver level of the co-administered therapeutic agent. As used herein, the term "treating" refers to reversing, alleviating, inhibiting the progress of, or preventing the disorder or condition, or one or more symptoms of such disorder or condition to which such term applies. The term "treatment" refers to the act of treating. Therapeutic agents suitable for co-administration with a compound of the invention can be any drug that is metabolizable by a CYP enzyme, such as immunomodulators (e.g., cyclosporine or FK-506), anti-cancer or chemotherapeutic agents (e.g., taxol or taxotere), antibiotics (e.g., clarithromycin, erythromycin, or telithromycin), antivirals (e.g., indinavir, lopinavir, nelfinavir, or saquinavir), antihistamines (e.g., astemizole, chlorpheniramine,or terfenidine), calcium channel blockers (e.g., amlodipine, diltiazem, felodipine, lercanidipine, nifedipine, nisoldipine, nitrendipine, or verapamil), beta blockers (e.g., carvedilol, S-metoprolol, propafenone, or timolol), or antidepressants (e.g., amitriptyline, clomipramine, desipramine, imipramine, or paroxetine). Preferably, the therapeutic agent co-administered with a compound of the invention is metabolized by CYP3A4, CYP2D6, or CYP2C9.

In one embodiment, the present invention features methods of using a compound of the present invention (or a salt, solvate or prodrug thereof) and an antiviral agent for the treatment of viral infection. The methods comprise administering an antiviral agent which is metabolizable by a CYP enzyme (e.g., CYP3A4, CYP2D6, or CYP2C9) and a compound of the present invention (or a salt, solvate or prodrug thereof) to a subject in need thereof, where the antiviral agent is effective in treating viral infection in the subject, and the compound of the present invention (or a salt, solvate or prodrug thereof) increases the blood or liver level or otherwise improves the pharmacokinetics of the co-administered antiviral agent. Non-limiting examples of suitable antiviral agents include anti-HIV agents (e.g., lopinavir, compound GS9137, or other HIV protease, integrase, reverse transcriptase or fusion inhibitors) or anti-HCV agents (compounds VX-950 or SCH503034, or other HCV protease or polymerase inhibitors). In one example, the present invention features methods which comprise administering an HCV inhibitor (e.g., an HCV protease inhibitor such as compound VX-950 or SCH503034), and a compound of the present invention (e.g., a compound described in Example 1) or a salt, solvate or prodrug thereof, to an HCV patient, wherein the HCV inhibitor is effective in treating HCV infection in the patient, and the compound of the invention (or the salt, solvate or prodrug thereof) increases the blood or liver level (or otherwise improves the pharmacokinetics) of the HCV inhibitor (e.g., compound VX-950 or SCH503034) in the patient. In another example, the present invention features methods which comprise administering a cocktail of anti-HCV agents (e.g., a cocktail including compound VX-950 or SCH503034), and a compound of the present invention (e.g., a compound described in Example 1) or a salt, solvate or prodrug thereof, to an HCV patient, wherein the cocktail of anti-HCV agents is effective in treating HCV infection in the patient, and the compound of the invention (or the salt, solvate or prodrug thereof) increases the blood or liver level (or otherwise improves the pharmacokinetics) of at least one anti-HCV agent in the cocktail (e.g., compound VX-950 or SCH503034) in the patient. In yet another example, the present invention features methods which comprise administering an HIV inhibitor (e.g., an HIV protease or integrase inhibitor such as lopinavir or compound GS9137), and a compound of the present invention (e.g., a compound described in Example 1) or a salt, solvate or prodrug thereof, to an HIV patient, wherein the HIV inhibitor is effective in treating HIV infection in the patient, and the compound of the invention (or the salt, solvate or prodrug thereof) increases the blood or liver level (or otherwise improves the pharmacokinetics) of the HIV inhibitor (e.g., lopinavir or compound GS9137) in the patient. In still another example, the present invention features methods which comprise administering a cocktail of anti-HIV agents (e.g., a cocktail comprising lopinavir or compound GS9137), and a compound of the present invention (e.g., a compound described in Example 1) or a salt, solvate or prodrug thereof, to an HIV patient, wherein the cocktail is effective in treating HIV infection in the patient, and the compound of the invention (or the salt, solvate or prodrug thereof) increases the blood or liver level (or otherwise improves the pharmacokinetics) of at least one anti-HIV agent in the cocktail (e.g., lopinavir or compound GS9137).

Any compound described herein (e.g., the compounds described in Example 1), or a salt, solvate or prodrug thereof, can be used in a method of the present invention. Preferably, a compound of the invention is administered in such an amount that is effective in improving the pharmacokinetics of the co-administered drug, but is ineffective by itself for the treatment of any disease.

The present invention further features the use of the compounds of the present invention, or salts, solvates or prodrugs thereof, for the manufacture of a medicament for the treatment of a disease. The medicament includes a compound of the present invention and a drug suitable for the treatment of the disease. Preferably, the drug is metabolizable by a CYP enzyme, such as CYP3A4, CYP2D6 or CYP2C9. Non-limiting examples of drugs that are metabolizable by CYP enzymes include many immunomodulators, anti-cancer or chemotherapeutic agents, antibiotic agents, antiviral agents, antihistamines, calcium channel blockers, beta blockers, and antidepressants. Specific examples of these drugs include, but are not limited to, cyclosporine, FK-506, taxol, taxotere, clarithromycin, erythromycin, telithromycin, indinavir, lopinavir, nelfinavir, saquinavir, astemizole, chlorpheniramine, terfenidine, amlodipine, diltiazem, felodipine, lercanidipine, nifedipine, nisoldipine, nitrendipine, verapamil, carvedilol, S-motoprolol, propafenone, timolol, amitriptyline, clomipramine, desipramine, imipramine, paroxetine, compound VX-950, compound GS9137, and compound SCH503034.

In one example, the present invention features the use of a compound of the present invention (e.g., a compound described in Example 1) or a salt, solvate or prodrug thereof, and a drug that is metabolizable by CYP3A4, for the manufacture of a medicament for the treatment of a disorder. In another example, the present invention features the use of a compound of the present invention (e.g., a compound described in Example 1) or a salt, solvate or prodrug thereof, and a drug that is metabolizable by CYP2D6, for the manufacture of a medicament for the treatment of a disorder. In still another example, the present invention features the use of a compound of the present invention (e.g., a compound described in Example 1) or a salt, solvate or prodrug thereof, and a drug that is metabolizable by CYP2C9, for the manufacture of a medicament for the treatment of a disorder.

Compound Preparation

The following synthetic Schemes illustrate the general methods by which the compounds of the present invention can be prepared. Starting materials can be obtained from commercial sources or prepared using methods well known to those of ordinary skill in the art. By way of example, a synthetic route similar to that shown hereinbelow may be used, together with synthetic methods known in the art of synthetic organic chemistry, or variations thereon as appreciated by those skilled in the art.

The present invention is intended to encompass compounds prepared by either synthetic processes or metabolic processes. Metabolic processes include those occurring in the human or animal body (*in vivo*), or those occurring *in vitro,*

If a substituent described herein is not compatible with the synthetic methods of this invention, the substituent may be protected with a suitable protecting group that is stable to the reaction conditions used in these methods. The protecting group may be removed at a suitable point in the reaction sequence to provide a desired intermediate or target compound. Suitable protecting groups and methods for protecting or deprotecting substituents are well know in the art, examples of which can be found in Greene and Wuts, *supra.*

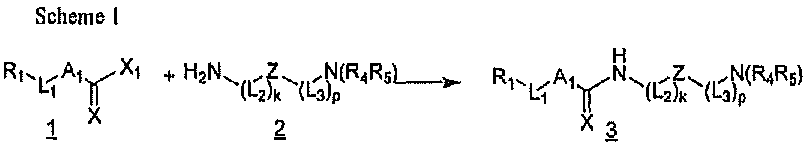

Compounds of formula 3 which are representative of compounds of formula (I) can be prepared according to Scheme 1. Compounds of formula 1, wherein R₁, L₁, A₁, and X are as defined in formula (I) and X₁ is halo, para-nitrophenol or another leaving group known to those skilled in the art, when treated with compounds of formula 2, wherein L₂, L₃, Z, k, p, R₄ and R₅ are as defined in formula (I), in a solvent such as but not limited to tetrahydrofuran, DMF, acetonitrile and the like, in the presence or absence of a base such as but not limited to diisopropylethylamine, triethylamine, N-methyl morpholine, sodium, potassium or cesium carbonate, at a temperature of from about 25 °C to about 55 °C, will provide compounds of formula 3.

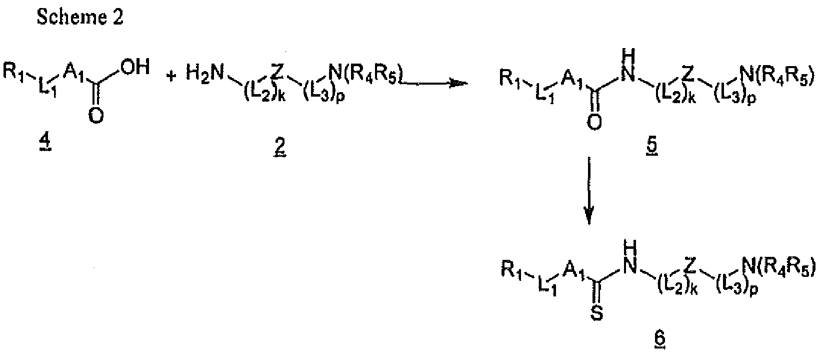

Similarly, compounds of formula 5 and compounds of formula 6 which are both representative of compounds of formula (I) can be prepared according to Scheme 2. Compounds formula 4, wherein R₁, L₁ and A₁ are as defined in formula (1), when treated with compounds of formula 2, wherein L₂, L₃, Z, k, p, R₄ and R₅ are as defined in formula (I), along with an acid coupling reagent commonly known to those skilled in the art, will provide compounds of formula 5. Typical conditions for acid coupling include mixing the acid and the amine components with reagents such as but not limited to 1-(3-dimethylaminopropyl)-3-ethylcarbodiimide hydrochloride (EDCI), 1,3-dicyclohexylcarbodiimide (DCC), bis(2-oxo-3-oxazolidinyl)phosphinic chloride (BOPCI), O-(7-azabenzotriazol-1-yl)-N,N,N',N'-tetramethyluronium hexafluorophosphate (HATU) or O-benzotriazol-1-yl-N,N,N',N'-tetramethyluronium tetrafluoroborate (TBTU) or similar coupling reagent, in the presence or absence of 1-hydroxybenzo triazole, with or without an organic base such as N-methylmorpholine or diisopropylethylamine in tetrahydrofuran or dichloromethane, at temperatures of from about 0 °C to about 25 °C. Alternatively, the acid of formula 4 can be converted to the corresponding acid chloride by treatment with thionyl chloride under heated conditions or by treatment with oxalyl chloride in dichloromethane containing a catalytic amount of DMF. The newly formed acid chloride when treated with the amine of formula 2 in solvents including tetrahydrofuran will provide compounds of formula 5. The treatment of compounds of formula 5 with Lawesson's Reagent (CAS No. 19172-47-5; available from Sigma-Aldrich Co. (Catalog No. 22,743-9)) in a solvent such as tetrahydrofuran or dichloromethane at a temperature of from about 25 °C to about 55 °C will provide compounds of formula 6.

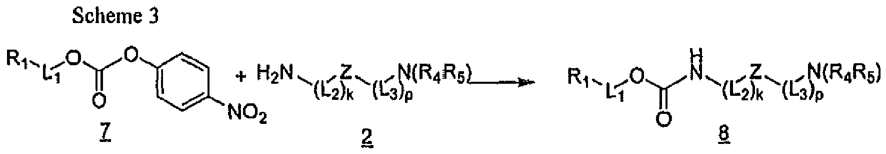

Compounds of formula 8, which are representative of compounds of formula (1) wherein A₁ is -0-, can be prepared according to Scheme 3. Compounds of formula 7, (which can be prepared by the treatment of compounds of formula R₁-L₁-OH, wherein R₁ and L₁ are as defined in formula (I), with paranitro phenol and carbonyl diimidazole), when treated with compounds of formula 2, wherein L₂, L₃, Z, k, p, R₄ and R₅ are as defined in formula (I), in solvents such as tetrahydrofuran, dichloromethane or acetonitrile, will provide compounds of formula 8. The reaction is typically carried out at temperatures of from about 25 °C to about 65 °C.

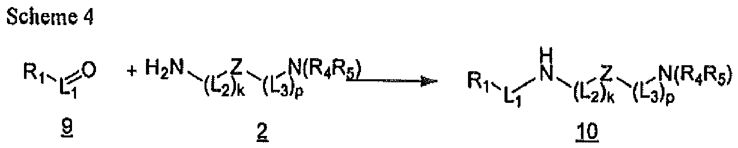

As outlined in Scheme 4, compounds of formula 10 which are representative of compounds of formula (I) wherein A₁ is a bond are prepared accordingly. Compounds of formula 9, wherein R₁ and L₁ are defined in formula (I), when treated with compounds of formula 2, wherein L₂, L₃, Z, k, p, R₄ and R₅ are defined in formula (I), in the presence or absence of sodium sulfate followed by the addition of sodium cyanoborohydride or sodium borohydride, will provide compounds of formula 10. Typical solvents include tetrahydrofuran and the reactions can be conducted at temperatures of from about 25 °C to about 45 °C.

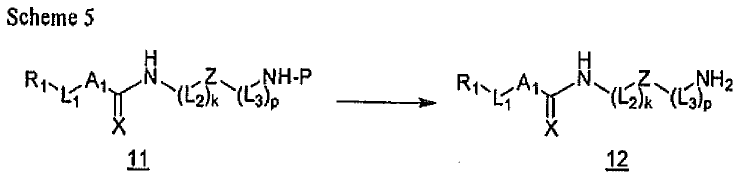

Alternatively, compounds of formula 11 wherein R_{1,} L₁, A₁, X, L₂, Z, L₃, k and p are as defined in formula (I), can be prepared according to the procedures outlined in Scheme 1-4. The replacement of R₄ or R₅ with a nitrogen protecting group in compounds of formula 2 in any of the procedures outline in Scheme I-4 will provide compounds of formula 11. Typical nitrogen protecting groups include tert-butyloxycarbonyl and benzyloxycarbonyl. Compounds of formula 11 when treated according to conditions known to one skilled in the art or as disclosed in Greene and Wuts, *supra,* will provide compounds of formula 12. For example, when compounds of formula 11 contain a tert-butyloxycarbonyl protecting group, treatment with trifluoroacetic acid in dichloromethane will provide compounds of formula 12.

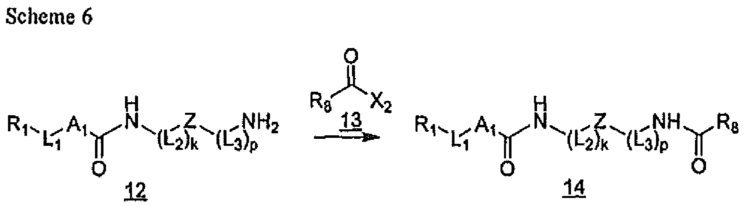

Compounds of formula 14 which are representative of compounds of formula (I) wherein R₄ or R₅ is R₈-C(O)- can be prepared according to Scheme 6. The treatment of compounds of formula 12 wherein R₁, L₁, A₁, X, L₂, Z, L₃, k and p are defined in formula (I), with compounds of formula 13 wherein R₈ is defined in formula (I) and X₂ is halo, in the presence of a base such as but not limited to diisoprapylathylamine or triethylamine, will provide compounds of formula 14. Typical solvents include but are not limited to tetrahydrofuran and DMF.

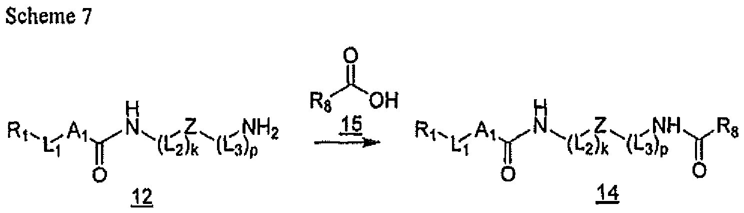

Alternatively, compounds of formula 14 which are representative of compounds of formula (I) can be prepared according to the methods outlined in Scheme 7. When the compounds of formula 12 wherein R₁, L₁, A₁, X, L₂, Z, L₃, k and p are defined in formula (I), and the compounds of formula 15 wherein R₈ is defined in formula (I), are treated with an acid coupling reagent commonly known to those skilled in the art, compounds of formula 14 will be achieved. Acid coupling reagents described in Scheme 2 can be utilized for this transformation. In addition, compounds of formula 15 can be converted to the corresponding acid chloride utilizing conditions outlined in Scheme 2, followed by treatment with compounds of formula 12 in the presence of a base such as but not limited to diisopropylethylamine or triethylamine in tetrahydrofuran or dichloromethane to provide compounds of formula 14.

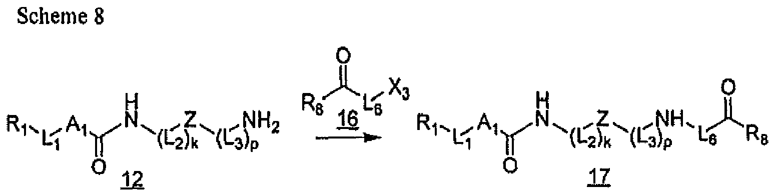

As outlined in Scheme 8, compounds of formula 17 which are representative of compounds of formula (I) wherein R₄ or R₅ is R₈-C(O)-L₆- can be prepared accordingly. The treatment of compounds of formula 12 wherein R₁, L_{1,} A₁, L₂, Z, L₃, k and p are defined in formula (I), and compounds of formula 16 wherein R₈ is defined in formula (I), L₆ is a bond and X₃ is halo, methanesulfonyl, trifluoromethanesulfonyl, para-toluenesulfonyl or a similar leaving group, in the presence of a base, will provide compounds of formula 17. Typical base and solvent conditions useful for this transformation include but are not limited to sodium, potassium or cesium carbonate in acetonitrile, or sodium hydride in tetrahydrofuran or DMF or sodium hydroxide and a phase transfer catalyst such as but not limited to tributyl ammonium benzyl bromide and aqueous sodium hydroxide in the presence or absence of an organic solvent. Depending on the conditions, heating may or may not be needed to effect the transformation. Alternatively, when formula 16 is R₈-C(O)O-X₃ wherein X₃ is para-nitrophenyl, the transformation can be carried out in tetrahydrofuran at a temperature of from about 25 °C to about 60°C.

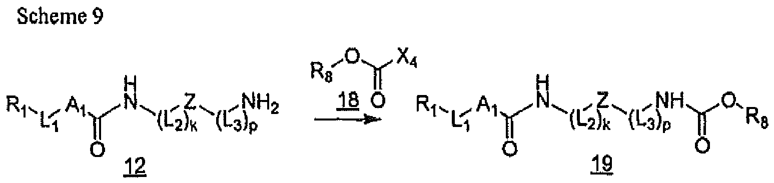

Compounds of formula 19, which are representative of compounds of formula (1) wherein R₄ or R₅ is R₈-O-C(O)- and R₈ is defined in formula (I), can be prepared according to Scheme 9. Compounds of formula 12 wherein R₁, L₁, A₁, L₂, Z, L₃, k and p are defined in formula (I), when treated with compounds of formula 18 wherein R₈ is defined in formula (I) and X₄ is halo or para-nitrophenol or a similar leaving group, in the presence or absence of a base, will provide compounds of formula 19. Typical conditions include heating the mixture of compounds at a temperature of from about 25 °C to about 64 °C in solvents such as but not limited to tetrahydrofuran or acetonitrile.

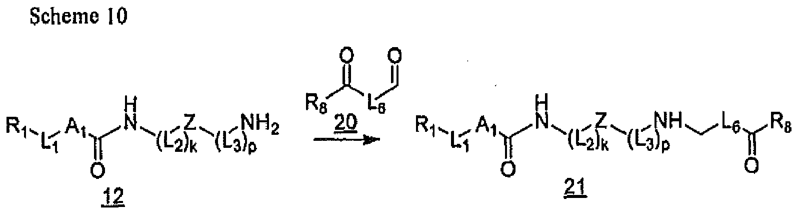

As outlined in Scheme 10, compounds of formula 21 which are representative of compounds of formula (I) wherein R₄ or R₅ is R₈-C(O)-L₆- and R₈ and L₆ are defined in formula (I) can be prepared accordingly. Compounds of formula 12 wherein R₁, L₁, A₁, L₂, Z, L₃, k and p are defined in formula (I), when treated with compounds of formula 20 wherein R₈ and L₆ are defined in formula (I) in the presence or absence of sodium sulfate in a solvent such as but not limited to tetrahydrofuran, followed by the addition of sodium cyanoborohydride, sodium borohydride or sodium tri-acetoxyborohydride, will provide compounds of formula 21.

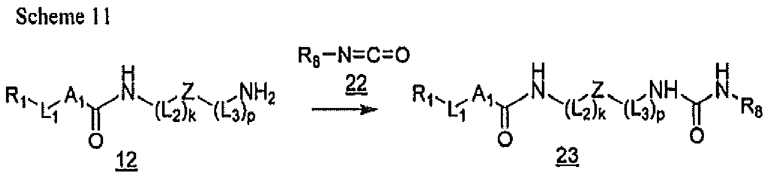

Compounds of formula **23** which are representative of compounds of formula (I) wherein R₄ is R₈-NH-C(O)- can be prepared according to Scheme 11. Compounds of formula **12** wherein R₁, L₁, A₁, L₂, Z, L₃, k and p are defined in formula (I), when treated with isocyanates of formula **22** in a solvent such as but not limited to tetrahydrofuran, will provide compounds of formula **23.**

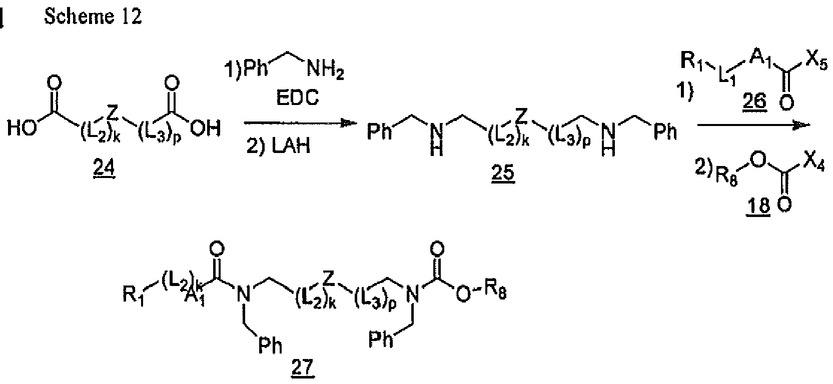

Compounds of formula **27** which are representative of compounds of formula (I) can be prepared according to Scheme 12. Compounds of formula **24,** wherein L₂, Z, L₃, k and p are defined in formula (I), are treated with at least two equivalents of benzyl amine and acid coupling conditions outlined in Scheme 2, followed by treating the product with a reducing agent such as lithium aluminum hydride in tetrahydrofuran, will provide di-amine compounds of formula **25,** Compounds of formula **25,** when treated with compounds of formula **26** wherein R₁, L₁ and A₁ are defined in formula (I) and X₅ is halo or para-nitrophenol in tetrahydrofuran, in the presence or absence of a base such as but not limited to diisopropylethylamine or triethylamine, followed by treatment with compounds of formula **18** wherein R₈ is defined in formula (I) and X₄ is halo or para-nitrophenol in tetrahydrofuran in the presence or absence of a base, will provide compounds of formula **27.**

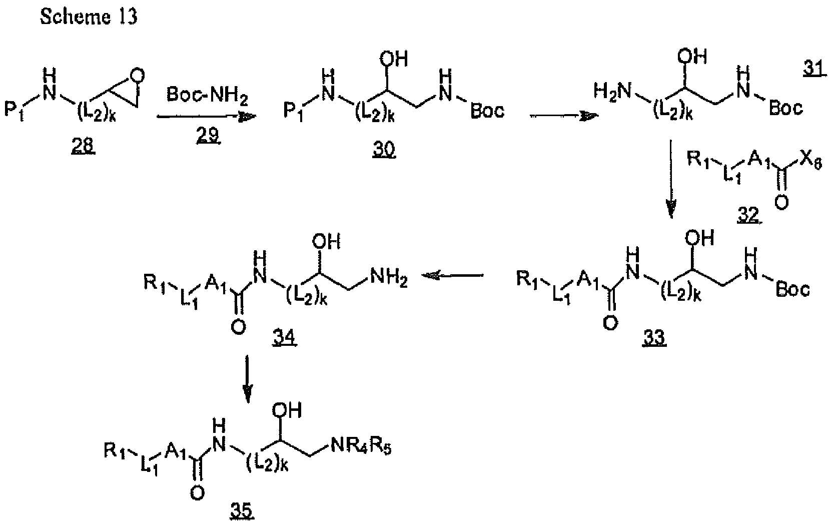

Compounds of formula **35** which are representative of compounds of formula (I) can prepared according to Scheme 13. Compounds of formula **28,** wherein L₂ and k are defined in formula (I) and P₁ is a benzyl or benzyloxycarbonyl (CBZ) protecting group, when treated with compounds of formula **29,** will provide compounds of formula **30.** This transformation can be carried out under heated conditions or in the presence of a lewis acid catalyst. Compounds of formula 30 can be selectively deprotected to provide compounds of formula **31.** The selective deprotection of the benzyl or CBZ protecting group can be achieved using standard conditions known to one skilled in the art such as stirring or shaking the compound in the presence of 1-5 atmospheres of hydrogen and a palladium catalyst such as 5-10 % Pd on carbon in a variety of solvents including tetrahydrofuran or alcoholic solvents or mixtures thereof. The treatment of compounds of formula **31** with compounds of formula **32** wherein R₁, L₁ and A₁ are defined in formula **(I)** and X₆ is halo or para-nitrophenol in solvents such as but not limited to tetrahydrofuran will provide compounds of formula **33.** The deprotection of compounds of formula **33** using standard Boc deprotection conditions including trifluoroacetic acid in dichloromethane will provide compounds of formula **34.** Compounds of formula **34** can be converted into compounds of formula **35** according to the methods outlined in Schemes 6-11.

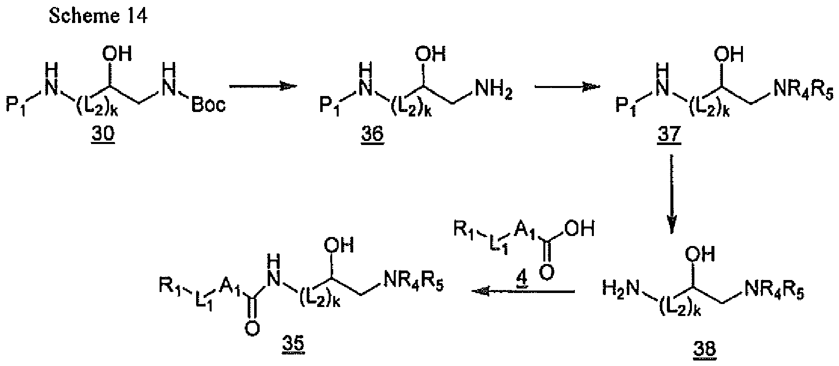

Alternatively, compounds of formula **30** wherein L₂ and k are defined in formula **(I)** and P₁ is benzyl or benzyloxycarbonyl can be treated according to conditions that will deprotect the Boc protecting group which include but are not limited to trifluoroacetic acid in dichloromethane to provide compounds of formula **36.** Compounds of formula **36** when subjected to conditions outlined in Schemes 6-11 will provide compounds of formula **37.** Compounds of formula **37** when treated with conditions that will remove a benzyl or benzyloxycarbonyl protecting group, such as but not limited to stirring or shaking the compound in the presence of 1-5 atmospheres of hydrogen and a palladium catalyst such as 5-10 % Pd on carbon in a variety of solvents including tetrahydrofuran or alcoholic solvents or mixtures thereof, will provide compounds of formula **38.** The treatment of compounds of formula **38** and compounds of formula **4** wherein R₁, L₁ and A₁ are defined in formula (I) according to acid coupling conditions as described in Scheme 2 will provide compounds of formula **35.**

It should be understood that the above-described embodiments and the following examples are given by way of illustration, not limitation. Various changes and modifications within the scope of the present invention will become apparent to those skilled in the art from the present description.

**Example 1**. Inhibition of CYP Isozymes

The inhibitory effects of the following representative compounds of the present invention on the metabolizing activity of CYP3A4, CYP2D6 or CYP2C9 were evaluated using standard methods as described in Yun et al., DRUG METABOLISM & DISPOSITION. 21: 403-407 (1993):
1,3-thiazol-5-ylmethyl (1*S*,3*S*,4*S*)-3-hydroxy-4-{[N-(methoxycarbonyl)-3-methyl-L-valyl] amino}-5-phenyl-1-(4-pyridin-2-ylbenzyl)pentylcarbamate;
methyl (1*S*)-1-({[(1*R*,3*S*,4*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl)pentyl] amino}carbonyl)-2,2-dimethylpropylcarbamate;
methyl (1*S*)-1-({[(1*S*,3*S,*4*S*)-3-hydroxy-4-({3-methyl-*N*-[(1,3-thiazol-5-ytmethoxy)carbonyl]-L-valyl}amino)-5-phenyl-1-(4-pyridin-2-ylbenzyl)pentyl]amino}carbonyl)-2,2-dimethylpropylcarbamate;
*tert*-butyl (1*S*,3*S*,4*S*)-3-hydroxy-5-phenyl-1-(4-pyridin-2-ylbenzyl)-4-{[(1,3-thiazol-5-ylmethoxy)carbonyl]amino} pentylcarbamate;
*tert-*butyl (1*S*,3*S*,4*S*)-3-hydroxy-4-({3-methyl-*N-*[(1,3-thiazol-5-ylmethoxy)carbonyl]-L-valyl} amino)-5-phenyl-1-(4-pyridin-2-ylbenzyl)pentylcarbamate;
*tert*-butyl (1*S*,3*S*,4*S*)-3-hydroxy-5-phenyl-1-(4-pyridin-2-ylbenzyl)-4-({*N*-[(1,3-thiazol-5-yl methoxy)carbonyl]-L-alanyl}amino)pentylcarbamate;
methyl (1*S*,3*S*,4*S*)-3-hydroxy-4-({3-methyl-*N*-[(1,3-thiazol-5-ylmethoxy)carbonyl]-L-valyl} amino)-5-phenyl-1-(4-pyridin-2-ylbenzyl)pentylcarbamate;
methyl (1*S*,3*S*,4*S*)-3-hydroxy-5-phenyl-1-(4-pyridin-2-ylbenzyl)-4-({*N*-[(1,3-thiazol-5-yl methoxy)carbonyl]-L-alanyl}amino)pentylcarbamate;
N¹-((1*S*,3*S*,4*S*)-3-hydroxy-5-phenyl-1-(4-pyridin-2-ylbenzyl)-4-{[(1,3-thiazol-5-ylmethoxy) carbonyl]amino}pentyl)-*N*²-(methoxycarbonyl)-3-methyl-L-valinamide;
1,3-thiazol-5-ylmethyl (1*S*,3*S*,4*S*)-1-benzyl-3-hydroxy-5-phenyl-4-{[(1,3-thiazol-5-ylmethoxy) carbonyl]amino}pentylcarbamate;
N¹-{(1S,2S,4S)-1-benzyl-4-[(tert-butoxycarbonyl)amino]-2-hydroxy-5-phenylpentyl}-3-methyl-N²-[(1,3-thiazol-5-ylmethoxy)carbonyl]-L-valinamide;
N¹-((1S,2S,4S)-1-benzyl-2-hydroxy-5-phenyl-4-{[(1,3-thiazol-5-ylmethoxy)carbonyl]amino} pentyl)-N²-(methoxycarbonyl)-3-methyl-L-valinamide;
1,3-thiazol-5-ylmethyl (1S,2S,4S)-4-(acetylamino)-1-benzyl-2-hydroxy-5-phenylpentyl carbamate;
1,3-thiazol-5-ylmethyl (1S,2S,4S)-4-amino-1-benzyl-2-hydroxy-5-phenylpentylcarbamate;
(1S,3S)-3-(acetylamino)-4-phenyl-1-((1S)-2-phenyl-1-{[(1,3-thiazol-5-ylmethoxy)carbonyl] amino}ethyl)butyl acetate;
1,3-thiazol-5-ylmethyl (1S,2S,4S)-1-benzyl-2-hydroxy-4-[(methylsulfonyl)amino]-5-phenyl pentylcarbamate;
1,3-thiazol-5-ylmethyl (1S,2S,4S)-1-benzyl-4-{[(dimethylamino)carbonyl]amino}-2-hydroxy-5-phenylpentylcarbamate;
methyl (1*S*,3*S*,4*S*)-1-benzyl-3-hydroxy-5-phenyl-4-{[(1,3-thiazol-5-ylmethoxy)carbonyl]amino} pentylcarbamate,
1,3-thiazol-5-ylmethyl (1S,2S,4S)-1-benzyl-4-{[(dimethylamino)sulfonyl]amino}-2-hydroxy-5-phenylpentylcarbamate;
*tert*-butyl (1*S*,3*S*,4*S*)-1-benzyl-3-hydroxy-5-phenyl-4-{[(1,3-thiazol-5-ylmethoxy)carbonyl] amino}pentylcarbamate;
1,3-thiazol-5-ylmethyl (1S,2S,4S)-1-benzyl-2-hydroxy-4-{[(2S)-3-methyl-2-(2-oxotetra hydropyrimidin-1(2H)-yl)butanoyl]amino}-5-phenylpentylcarbamate;
isobutyl (1*S*,3*S*,4*S*)-1-benzyl-3-hydroxy-5-phenyl-4-{[(1,3-thiazol-5-ylmethoxy)carbonyl]amino} pentylcarbamate;
isopropyl (1*S*,3*S*,4*S*)-1-benzyl-3-hydroxy-5-phenyl-4-{[(1,3-thiazol-5-ylmethoxy)carbonyl] amino}pentylcarbamate;
*tert*-butyl (1*S*,3*R*,4*S*)-1-benzyl-3-hydroxy-5-phenyl-4-{[(1,3-thiazol-5-ylmethoxy)carbonyl] amino}pentylcarbamate;
1,3-thiazol-5-ylmethyl (4S,5S)-4-benzyl-5-{(2S)-2-[(4S)-4-isopropyl-2,5-dioxoimidazolidin-1-yl]-3-phenylpropyl}-2-oxo-1,3-oxazolidine-3-carboxylate;
N¹-((1S,3S,4S)-1-benzyl-3-hydroxy-5-phenyl-4-{[(1,3-thiazol-5-ylmethoxy)carbonyl]amino} pentyl)-L-valinamide;
N¹-((1S,3S,4S)-1-benzyl-3-hydroxy-5-phenyl-4-{[(1,3-thiazol-5-ylmethoxy)carbonyl]amino} pentyl)-N²-[(dimethylamino)carbonyl]-L-valinamide;
N¹-((1S,3S,4S)-1-benzyl-3-hydroxy-5-phenyl-4-{[(1,3-thiazol-5-ylmethoxy)carbonyl]amino} pentyl)-N²-[(methylamino)carbonyl]valinamide;
N¹-((1S,3S,4S)-1-benzyl-3-hydroxy-5-phenyl-4-{[(1,3-thiazol-5-ylmethoxy)carbonyl]amino} pentyl)-N²-[(2-hydroxypropoxy)carbonyl]-L-valinamide;
1,3-thiazol-5-ylmethyl (1S)-1-[(4S,6S)-4-benzyl-2-oxo-1,3-oxazinan-6-yl]-2-phenylethyl carbamate;
N¹-((1S,3S,4S)-1-benzyl-3-hydroxy-5-phenyl-4-{[(1,3-thiazol-5-ylmethoxy)carbonyl]amino} pentyl)-N²-[(2,3-dihydroxypropoxy)carbonyl]-L-valinamide;
1,3-thiazol-5-ylmethyl (1S,2S,4S)-1-benzyl-2-hydroxy-5-phenyl-4-{[(4-phenylpiperazin-1-yl) carbonyl]amino}pentylcarbamate;
*N*¹-((1*S*,3*S*,4*S*)-1-benzyl-3-hydroxy-5-phenyl-4-{[(1,3-thiazol-4-ylmethoxy)carbonyl]amino} pentyl)-*N*²-{[[(2-isopropyl-1,3-thiazol-4-yl)methyl](methyl)amino]carbonyl}-L-valinamide;
1,3-thiazol-4-ylmethyl (1*S*,3*S*,4*S*)-1-benzyl-3-hydroxy-5-phenyl-4-{[(1,3-thiazol-4-ylmethoxy) carbonyl]amino}pentylcarbamate;
1,3-thiazol-5-ylmethyl (1S,2S,4S)-1-benzyl-2-hydroxy-4-[(4S)-4-isopropyl-2,5-dioxoimidazo lidin-1-yl]-5-phenylpentylcarbamate;
1,3-thiazol-5-ylmethyl (1S,2S,4S)-1-benzyl-2-hydroxy-4-{[(2-isopropyl-1,3-thiazol-4-yl) acetyl]amino}-5-phenylpentylcarbamate;
1,3-thiazol-5-ylmethyl (1S,2S,4S)-1-benzyl-4-{[(2,6-dimethylphenoxy)acetyl]amino}-2-hydroxy-5-phenylpentylcarbamate;
1,3-thiazol-5-ylmethyl (1S,2S,4S)-1-benzyl-4-{[(tert-butylamino)carbonyl]amino}-2-hydroxy-5-phenylpentylcarbamate;
N¹-((1S,3S,4S)-1-benzyl-3-hydroxy-5-phenyl-4-{[(1,3-thiazol-5-ylmethoxy)carbonyl]amino} pentyl)-N²-(methoxycarbonyl)-3-methyl-L-valinamide;
1,3-thiazol-5-ylmethyl (1S,3S,4S)-1-benzyl-4-{[(2,6-dimethylphenoxy)acetyl]amino}-3-hydroxy-5-phenylpentylcarbamate;
*tert-*butyl (1*S*,2*R*)-1-benzyl-2-hydroxy-3-{isobutyl[(1,3-thiazol-5-ylmethoxy)carbonyl]amino} propylcarbamate;
N,N-dimethyl-N-[(1,3-thiazol-5-ylmethoxy)carbonyl]-L-phenylalaninamide;
*N-*[(1*S*)-1-benzyl-2-morpholin-4-yl-2-oxoethyl]-*N*-[(1,3-thiazol-5-ylmethoxy)carbonyl]amine;
N,N-diisobutyl-N-[(1,3-thiazol-5-ylmethoxy)carbonyl]-L-phenylalaninamide;
N-isobutyl-N-[(1,3-thiazol-5-ylmethoxy)carbonyl]-L-phenylalaninamide;
N,N-dibenzyl-N-[(1,3-thiazol-5-ylmethoxy)carbonyl]-L-phenylalaninamide;
N-benzyl-N-(2-phenylethyl)-N-[(1,3-thiazol-5-ylmethoxy)carbonyl]-L-phenylalaninamide;
*tert*-butyl (1*S*,2*S*)-1-benzyl-3-{benzyl[(1,3-thiazol-5-ylmethoxy)carbonyl]amino}-2-hydroxy propylcarbamate;
*tert-*butyl (1*S*,2*R*)-1-benzyl-3-{benzyl[(1,3-thiazol-5-ylmethoxy)carbonyl]amino}-2-hydroxy propylcarbamate;
*tert-*butyl benzyl((2*R*,3*S*)-2-hydroxy-4-phenyl-3-{[(1,3-thiazol-5-ylmethoxy) carbonyl]amino} butyl)carbamate;
1,3-thiazol-5-ylmethyl 4-[benzyl(pyridin-4-ylacetyl)amino]phenylcarbamate;
benzyl benzyl(4-{[(1,3-thiazol-5-ylmethoxy)carbonyl]amino}phenyl)carbamate;
*tert-*butyl 3-{[(1,3-thiazol-5-ylmethoxy)carbonyl]amino}phenylcarbamate,
1,3-thiazol-5-ylmethyl 4-{benzyl[(3-chlorophenyl)acetyl]amino}phenylcarbamate;
1,3-thiazol-5-ylmethyl 3-{[(3-chlorophenyl)acetyl]amino}phenylcarbamate;
1,3-thiazol-5-ylmethyl 3-[(pyridin-4-ylacetyl)amino]phenylcarbamate;
N²-(tert-butoxycarbonyl)-N²-methyl-N¹-(3-{[(1,3-thiazol-5-ylmethoxy)carbonyl] amino}phenyl)-L-valinamide;
1,3-thiazol-5-ylmethyl 3-({[(3-methylphenyl)amino]carbonyl}amino)phenylcarbamate;
[3-((2S)-2-tert-Butoxycarbonylamino-3-phenyl-propionylamino)-phenyl]-carbamic acid thiazol-5-ylmethyl ester;
1,3-thiazol-5-ylmethyl 3-({(2S)-2-[(tert-butoxycarbonyl)amino]-2-phenylethanoyl}amino) phenylcarbamate;
1,3-thiazol-5-ylmethyl 3-[(3,3-dimethylbutanoyl)amino]phenylcarbamate;
N²-(tert-butoxycarbonyl)-N¹-(3-{[(1,3-thiazol-5-ylmethoxy)carbonyl]amino}phenyl)-L-isoleucinamide;
N²-(tert-butoxycarbonyl)-N¹-(3-{[(1,3-thiazol-5-ylmethoxy)carbonyl]amino}phenyl)-L-valinamide;
N²-(tert-butoxycarbonyl)-3-methyl-N¹-(3-{[(1,3-thiazol-5-ylmethoxy)carbonyl]amino}phenyl)-L-valinamide;
1,3-thiazol-5-ylmethyl 3-({(2S)-2-[(tert-butoxycarbonyl)amino]-2-cyclohexylethanoyl}amino) phenylcarbamate;
dibenzyl 2-(4-benzyl-4-{[(1,3-thiazol-5-ylmethoxy)carbonyl]amino}piperidin-1-yl)ethylimido dicarbonate;
1,3-thiazol-5-ylmethyl 4-benzyl-1-(2-{[(4-methylphenyl)sulfonyl]amino}ethyl)piperidin-4-yl carbamate;
1,3-thiazol-5-ylmethyl 4-benzyl-1-(2-{[(1,3-thiazol-5-ylmethoxy)carbonyl]amino} ethyl) piperidin-4-ylcarbamate;
1,3-thiazol-5-ylmethyl 4-benzyl-1-[2-(dibenzylamino)ethyl]piperidin-4-ylcarbamate;
1,3,thiazol-5-ylmethyl 1-(2-aminoethyl)-4-benzylpiperidin-4-ylcarbamate;
1,3-thiazol-5-ylmethyl 1,1-dibenzyl-3-[4-(4-nitrophenyl)piperazin-1-yl]propylcarbamate;
1,3-thiazol-5-ylmethyl 1,1-dibenzyl-3-morpholin-4-ylpropylcarbamate;
1,3-thiazol-5-ylmethyl 4-(3-benzyl-4-phenyl-3-{[(1,3-thiazol-5-ylmethoxy)carbonyl]Amino} butyl)piperazine-1-carboxylate;
1,3-thiazol-5-ylmethyl 1,1-dibenzyl-3-{4-[(4-methylphenyl)sulfonyl]piperazin-1-yl}propyl carbamate;
ethyl 4-[4-(3-benzyl-4-phenyl-3-{[(1,3-thiazol-5-ylmethoxy)carbonyl]amino} butyl)piperazin-1-yl]benzoate;
1,3-thiazol-5-ylmethyl 1,1-dibenzyl-3-piperazin-1-ylpropylcarbamate;
1,3-thiazol-5-ylmethyl 4-benzyl-1-[(4-methylphenyl)sulfonyl]piperidin-4-ylcarbamate;
1,3-thiazol-5-ylmethyl 4-benzyl-4-{[(1,3-thiazol-5-ylmethoxy)carbonyl]amino}piperidine-1-carboxylate;
1,3-thiazol-5-ylmethyl 1,4-dibenzylpiperidin-4-ylcarbamate;
1,3-thiazol-5-ylmethyl benzyl(3-{benzyl[(1,3-thiazol-5-ylmethoxy)carbonyl]amino} propyl) carbamate;
1,3-thiazol-5-ylmethyl benzyl(3-{benzyl[(1,3-thiazol-5-ylmethoxy)carbonyl]amino}-2-hydroxypropyl)carbamate;
1,3-thiazol-5-ylmethyl benzyl(3-{benzyl[(1,3-thiazol-5-ylmethoxy)carbonyl]amino}-2-oxopropyl)carbamate;
1,3-thiazol-5-ylmethyl benzyl[9-benzyl-10-oxo-12-(1,3-thiazol-5-yl)-3,6,11-trioxa-9-azadodec-1-yl]carbamate;
1,3-thiazol-5-ylmethyl cyclohexylmethyl(3-{(cyclohexylmethyl)[(1,3-thiazol-5-ylmethoxy) carbonyl]amino} propyl)carbamate;
methyl-4-({(3-{[4-(methoxycarbonyl)benzyl][(1,3-thiazol-5-ylmethoxy)carbonyl]amino} propyl)[(1,3-thiazol-5-ylmethoxy)carbonyl]amino}methyl)benzoate;
1,3-thiazol-5-ylmethyl 4-benzoylbenzyl(3-{(4-benzoylbenzyl)[(1,3-thiazol-5-ylmethoxy) carbonyl]amino}propyl)carbamate;
1,3-thiazol-5-ylmethyl quinolin-3-ylmethyl(3-{(quinolin-3-ylmethyl)[(1,3-thiazol-5-ylmethoxy) carbonyl]amino} propyl)carbamate;
1,3-thiazol-5-ylmethyl 4-pyridin-2-ylbenzyl(3-{(4-pyridin-2-ylbenzyl)[(1,3-thiazol-5-ylmethoxy) carbonyl]amino}propyl)carbamate;
1,3-thiazol-5-ylmethyl 4-(benzyloxy)benzyl(3-{[4-(benzyloxy)benzyl][(1,3-thiazol-5-ylmethoxy) carbonyl]amino}propyl)carbamate;
1,3-thiazol-5-ylmethyl 3-{[(1,3-thiazol-5-ylmethoxy)carbonyl][4-(1*H*-1,2,4-triazol-1-yl)benzyl] amino}propyl[4-(1*H*-1,2,4-triazol-1-yl)benzyl]carbamate;
1,3-thiazol-5-ylmethyl 4-methoxybenzyl(3-{(4-methoxybenzyl)[(1,3-thiazol-5-ylmethoxy) carbonyl]amino} propyl)carbamate;
*tert*-butyl 4-methoxybenzyl(3-{(4-methoxybenzyl)[(1,3-thiazol-5-ylmethoxy)carbonyl]amino} propyl)carbamate;
1,3-thiazol-5-ylmethyl 4-*tert*-butylbenzyl(3-{(4-*tert*-butylbenzyl)[(1,3-thiazol-5-ylmethoxy) carbonyl]amino}propyl)carbamate;
*tert*-butyl 4-benzoylbenzyl(3-{(4-benzoylbenzyl)[(1,3-thiazol-5-ylmethoxy) carbonyl]amino} propyl)carbamate;
1,3-thiazol-5-ylmethyl 1,1'-biphenyl-4-ylmethyl(3-{(1,1'-biphenyl-4-ylmethyl)[(1,3-thiazol-5-ylmethoxy)carbonyl]amino}propyl)carbamate;
1,3-thiazol-5-ylmethyl benzyl{3-[benzyl(pyridin-4-ylmethyl)amino]propyl}carbamate;
ethyl *N-*(3-{(ethoxycarbonylmethyl)[(1,3-thiazol-5-ylmethoxy)carbonyl]amino}propyl)-N-[(1,3-thiazol-5-ylmethoxy)carbonyl]glycinate;
1,3-thiazol-5-ylmethyl benzyl(3-{benzyl[(5-methylthien-2-yl)methyl]amino}propyl)carbamate;
1,3-thiazol-5-ylmethyl benzyl{3-[benzyl(2-furylmethyl)amino]propyl}carbamate;
1,3-thiazol-5-ylmethyl benzyl{3-[benzyl(2-naphthylmethyl)amino]propyl}carbamate;
methyl 4-({(3-{(tert-butoxycarbonyl)[4-(methoxycarbonyl)benzyl]amino}propyl)[(1,3-thiazol-5-ylmethoxy)carbonyl]amino}methyl)benzoate;
1,3-thiazol-5-yimethyl benzyl{3-[benzyl(neopentyl)amino]propyl}carbamate;
*tert*-butyl benzyl(3-{benzyl[(1,3-thiazol-5-ylmethoxy)carbonyl]amino}propyl)carbamate;
benzyl benzyl(3-{benzyl[(1,3-thiazol-5-ylmethoxy)carbonyl]amino}propyl)carbamate;
methyl benzyl(3-{benzyl[(1,3-thiazol-5-ylmethoxy)carbonyl]amino}propyl)carbamate;
1,3-thiazol-5-ylmethyl benzyl[3-(benzyl{4-[3-(dimethylamino)propoxy]benzyl}amino)propyl) carbamate;
1,3-thiazol-5-ylmethyl benzyl{3-[benzyl(4-pyridin-2-ylbenzyl)amino]propyl}carbamate;
*tert*-butyl 3-{[(1,3-thiazol-5-ylmethoxy)carbonyl][4-(1*H*-1,2,4-triazol-1-yl)benzyl]amino} propyl[4-(1*H*-1,2,4-triazol-1-yl)benzyl]carbamate;
5-{benzyl[(1,3-thiazol-5-ylmethoxy)carbonyl]amino}-2-[(tert-butoxycarbonyl)amino]-1,2,3,5-tetradeoxy-1-phenyl-D-glycero-pentitol;
*tert*-butyl 1,1'-biphenyl-4-ylmethyl(3-{(1,1'-biphenyl-4-ylmethy)[(1,3-thiazol-5-ylmethoxy) carbonyl]amino}propyl)carbamate;
*tert*-butyl 4-pyridin-2-ylbenzyl(3-{(4-pyridin-2-ylbenzyl)[(1,3-thiazol-5-ylmethoxy)carbonyl] amino}propyl)carbamate;
ethyl N-[(1,3-thiazol-5-ylmethoxy)carbonyl]-N-(3-{[(1,3-thiazol-5-ylmethoxy)carbonyl]amino} propyl)glycinate;
1,3-thiazol-5-ylinothyl (1S,2R)-1-benzyl-2-hydroxy-3-[isobutyl(4-pyridin-2-ylbenzyl)amino] propylcarbamate;
tert-butyl 2-((2S,3S)-2-hydroxy-4-phenyl-3-{[(1,3-thiazol-5-ylmethoxy)carbonyl]amino} butyl)-2-(4-pyridin-2-ylbenzyl)hydrazinecarboxylate;
methyl N-[(1,3-thiazol-5-ylmethoxy)carbonyl]-L-phenylalaninate;
1,3-thiazol-5-ylmethyl 1,1-dibenzylbut-3-enylcarbamate;
1,3-thiazol-5-ylmethyl benzyl{3-{benzyl[(1,3-thiazol-5-ylmethoxy)carbonyl]amino}-2-[(phenoxycarbonyl)amino]propyl}carbamate; and
1,3-thiazol-5-ylmethyl benzyl[3-{benzyl[(1,3-thiazol-5-ylmethoxy)carbonyl]amino}-2-(isobutyrylamino)propyl]carbamate.

These compounds inhibited human CYP3A4, CYP2D6 and CYP2C9 with IC₅₀s (the concentration required for 50% inhibition) ranging from about 0.04 µM to about 10 µM. When tested with terfenadine as the probe substrate (Yun et al., DRUG METABOLISM & DISPOSITION, 21: 403-407 (1993)), many compounds of the present invention inhibited the terfenadine hydroxylase activity, which represents the most abundant form of cytochrome P450 (CYP3A4) in human liver, with IC₅₀s ranging from about 0.05 µM to about 3 µM.

**Example 2.** Improvement of the Pharmacokinetics of Drugs

The pharmacokinetics boosting effects of the compounds of the present invention were evaluated by measuring the compounds' protective effects on the metabolism of lopinavir, an HIV protease inhibitor. Lopinavir were mixed with human microsomes containing CYP3A4 in the presence of a compound of the invention. The amount of unmetabolized lopinavir after incubation indicated how well the compound prevented lopinavir from being metabolized and thereby improved the drug's pharmacokinetics. When used at 4 µM ("% LPV Remaining (4µM)") as compared to 0.4 µM ("% LPV Remaining (0.4µM)"), representative compounds of the present invention substantially increased the amount of unmetabolized lopinavir. See Table 1. Significant pharmacokinetics boosting effects were also observed for some compounds at 0.4 µM ("% LPV Remaining (0.4µM)").

**Table 1. Pharmacokinetics Boosting Effect of the Compounds of the Invention**

| **Representative Compounds** | **% LPV Remaining (0.4µM)** | **% LPV Remaining (4µM)** |
|---|---|---|
| 1,3-thiazol-5-ylmethyl (1*S*,3*S,*4*S*)-3-hydroxy-4-{[*N*-(methoxycarbonyl)-3-methyl-L-valyl]amino}-5-phenyl-1-(4-pyridin-2-ylbenzyl)pentylcarbamate | 4.2 | 81 |
| methyl (1*S*)-1-({[(1*S*,3*S*,4*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl) pentyl]amino}carbonyl)-2,2-dimethylpropylcarbamate | 60 | 100 |
| tert-butyl 2-((2S,3S)-2-hydroxy-4-phenyl-3-{[(1,3-thiazol-5-ylmethoxy) carbonyl]amino}butyl)-2-(4-pyridin-2-ylbenzyl)hydrazinecarboxylate | 32 | 100 |
| *N*¹-((1*S*,3*S*,4*S*)-3-hydroxy-5-phenyl-1-(4-pyridin-2-ylbenzyl)-4-{[(1,3-thiazol-5-ylmethoxy)carbonyl]amino}pentyl)-*N*²-(methoxycarbonyl)-3-methyl-L-valinamide | 27 | 100 |
| 1,3-thiazol-5-ylmethyl (1*S*,3*S*,4*S*)-1-benzyl-3-hydroxy-5-phenyl-4-{[(1,3-thiazol-5-ylmethoxy)carbonyl]amino} pentylcarbamate | 23.0 | 69 |
| N¹-((1S,2S,4S)-1-benzyl-2-hydroxy-5-phenyl-4-{[(1,3-thiazol-5-yl methoxy)carbonyl]amino}pentyl)-N²-(methoxycarbonyl)-3-methyl-L-valinamide | 22 | 100 |
| 1,3-thiazol-5-ylmethyl (1S,2S,4S)-4-(acetylamino)-1-benzyl-2-hydroxy-5-phenylpentylcarbamate | 17 | 74 |
| 1,3-thiazol-5-ylmethyl (1S,2S,4S)-4-amino-1-benzyl-2-hydroxy-5-phenyl pentylcarbamate | 4.5 | 62 |
| (1S,3S)-3-(acetylamino)-4-phenyl-1-((1S)-2-phenyl-1-{[(1,3-thiazol-5-yl methoxy)carbonyl]amino}ethyl)butyl acetate | 16 | 78 |
| 1,3-thiazol-5-ylmethyl(1S,2S,4S)-1-benzyl-2-hydroxy-4-[(methylsulfonyl) amino]-5-phenylpentylcarbamate | 33 | 88 |
| 1,3-thiazol-5-ylmethyl (1S,2S,4S)-1-benzyl-4-{[(dimethylamino)carbonyl] amino}-2-hydroxy-5-phenylpentylcarbamate | 29 | 84 |
| methyl (1*S,*3*S,*4*S*)-1-benzyl-3-hydroxy-5-phenyl-4-{[(1,3-thiazol-5-yl methoxy)carbonyl]amino}pentylcarbamate | 30 | 86 |
| 1,3-thiazol-5-ylmethyl(1S,2S,4S)-1-benzyl-4-{[(dimethylamino)sulfonyl] amino}-2-hydroxy-5-phenylpentylcarbamate | 34 | 88 |
| *tert*-butyl (1*S*,3*S*,4*S*)-1-benzyl-3-hydroxy-5-phenyl-4-{[(1,3-thiazol-5-ylmethoxy)carbonyl]amino}pentylcarbamate | 80 | 100 |
| 1,3-thiazol-5-ylmethyl (1S,2S,4S)-1-benzyl-2-hydroxy-4-{[(2S)-3-methyl-2-(2-oxotetrahydropyrimidin-1 (2H)-yl)butanoyl]amino}-5-phenylpentyl carbamate | 21 | 61 |
| isobutyl (1*S,*3*S*,4*S*)-1-benzyl-3-hydroxy-5-phenyl-4-{[(1,3-thiazol-5-yl methoxy)carbonyl]amino} pentylcarbamate | 33 | 97 |
| isopropyl (1*S*,3*S*,4*S*)-1-benzyl-3-hydroxy-5-phenyl-4-{[(1,3-thiazol-5-yl methoxy)carbonyl]amino}pentylcarbamate | 19 | 75 |
| *tert*-butyl (1*S*,3*R*,4*S*)-1-benzyl-3-hydroxy-5-phenyl-4-{[(1,3-thiazol-5-yl methoxy)carbonyl]amino}pentylcarbamate | 1.9 | 71 |
| 1,3-thiazol-5-ylmethyl (4S,5S)-4-benzyl-5-{(2S)-2-[(4S)-4-isopropyl-2,5-dioxoimidazolidin-1-yl]-3-phenylpropyl}-2-oxo-1,3-oxazolidine-3-carboxylate | 7.5 | 89 |
| N¹-((1S,3S,4S)-1-benzyl-3-hydroxy-5-phenyl-4-{[(1,3.thiazol-5-yl methoxy)carbonyl]amino}pentyl)-L-valinamide | 53 | 84 |
| N¹-((1S,3S,4S)-1-benzy)-3-hydroxy-5-phenyl-4-{[(1,3-thiazol-5-yl methoxy)carbonyl]amino}pentyl)-N²-[(dimethylamino)carbonyl]-L-valinamide | 28 | 69 |
| N¹-((1S,3S,4S)-1-benzyl-3-hydroxy-5-phenyl-4-{[(1,3-thiazol-5-yl methoxy)carbonyl]amino}pentyl)-N²-[(methylamino)carbonyl]valinamide | 29 | 70 |
| N¹-((1S,3S,4S)-1-benzyl-3-hydroxy-5-phenyl-4-{[(1,3-thiazol-5-yl methoxy)carbonyl]amino}pentyl)-N²-[(2-hydroxypropoxy)carbonyl]-L-valinamide | 19 | 76 |
| 1,3-thiazol-5-ylmethyl (1S)-1-[(4S,6S)-4-benzyl-2-oxo-1,3-oxazinan-6-yl]-2-phenylethylcarbamate | 6.2 | 68 |
| N¹-((1S,3S,4S)-1-benzyl-3-hydroxy-5-phenyl-4-{[(1,3-thiazol-5-yl methoxy)carbonyl]amino}pentyl)-N²-[(2,3-dihydroxypropoxy)carbonyl]-L-valinamide | 3.1 | 44 |
| 1,3-thiazol-5-ylmethyl (1S,2S,4S)-1-benzyl-2-hydroxy-4-[(4S)-4-isopropyl-2,5-dioxoimidazolidin-1-yl]-5-phenylpentylcarbamate | 44 | 83 |
| 1,3-thiazol-5-ylmethyl (1S,2S,4S)-1-benzyl-2-hydroxy-4-{[(2-isopropyl-1,3-thiazol-4-yl)acetyl]amino}-5-phenylpentylcarbamate | 61 | 90 |
| 1,3-thiazol-5-ylmethyl (1S,2S,4S)-1-benzyl-4-{[(2,6-dimethylphenoxy) acetyl]amino}-2-hydroxy-5-phenylpentylcarbamate | 72 | 100 |
| 1,3-thiazol-5-ylmethyl (1S,2S,4S)-1-benzyl-4-{[(tert-butylamino)carbonyl] amino}-2-hydroxy-5-phenylpentylcarbamate | 56 | 78 |
| N¹-((1S,3S,4S)-1-benzyl-3-hydroxy-5-phenyl-4-{[(1,3-thiazol-5-yl methoxy)carbonyl]amino}pentyl)-N²-(methoxycarbonyl)-3-methyl-L-valinamide | 34 | 82 |
| 1,3-thiazol-5-ylmethyl (1S,3S,4S)-1-benzyl-4-{[(2,6-dimethylphenoxy) acetyl]amino}-3-hydroxy-5-phenylpentylcarbamate | 12 | 100 |
| N,N-diisobutyl-N-[(1,3-thiazol-5-ylmethoxy)carbonyl]-L-phenyl alaninamide | 2 | 77 |
| N-isobutyl-N-[(1,3-thiazol-5-ylmethoxy)carbonyl]-L-phenylalaninamide | 1.9 | 16 |
| N,N-dibenzyl-N-[(1,3-thiazol-5-ylmethoxy)carbonyl]-L-phenylalaninamide | 1.9 | 78 |
| N-benzyl-N-(2-phenylethyl)-N-[(1,3-thiazol-5-ylmethoxy)carbonyl]-L-phenylalaninamide | 1.8 | 83 |
| *tert*-butyl (1*S*,2*S*)-1-benzyl-3-{benzyl[(1,3-thiazol-5-ylmethoxy) carbonyl]amino}-2-hydroxypropylcarbamate | 25 | 100 |
| 1,3-thiazol-5-ylmethyl 1,1-dibenzyl-3-[4-(4-nitrophenyl)piperazin-1-yl] propylcarbamate | 47 | 75 |
| 1,3-thiazol-5-ylmethyl 1,1-dibenzyl-3-morpholin-4-ylpropylcarbamate | 5.2 | 78 |
| dibenzyl 2-(4-benzyl-4-{[(1,3-thiazol-5-ylmethoxy)carbonyl]amino}piperidin-1-yl)ethylimidodicarbonate | 1.6 | 63 |
| 1,3-thiazol-5-ylmethyl benzyl(3-{benzyl[(1,3-thiazol-5-ylmethoxy) carbonyl]amino}propyl)carbamate | 5.5 | 100 |
| 1,3-thiazol-5-ylmethyl benzyl(3-{benzyl[(1,3-thiazol-5-ylmethoxy) carbonyl]amino}-2-hydroxypropyl)carbamate | 6.0 | 100 |
| 1,3-thiazol-5-ylmethyl benzyl(3-{benzyl[(1,3-thiazol-5-ylmethoxy) carbonyl]amino}-2-oxopropyl)carbamate | 1.7 | 94 |
| 1,3-thiazol-5-ylmethyl benzyl[9-benzyl-10-oxo-12-(1,3-thiazol-5-yl)-3,6,11-trioxa-9-azadodec-1-yl]carbamate | 4.6 | 100 |
| 1,3-thiazol-5-ylmethyl cyclohexylmethyl(3-{(cyclohexylmethyl)[(1,3-thiazol-5-ylmethoxy)carbonyl]amino}propyl)carbamate | 2.9 | 98 |
| methyl 4-({(3-{[4-(methoxycarbonyl)benzyl][(1,3-thiazol-5-ylmethoxy) carbonyl]amino}propyl)[(1,3-thiazol-5-ylmethoxy)carbonyl]amino} methyl)benzoate | 2 | 40 |
| 1,3-thiazol-5-ylmethyl 4-benzoylbenzyl(3-{(4-benzoylbenzyl)[(1,3-thiazol-5-ylmethoxy)carbonyl]amino}propyl)carbamate | 2.9 | 93 |
| 1,3-thiazol-5-ylmethyl quinolin-3-ylmethyl(3-{(quinolin-3-ylmethyl)[(1,3-thiazol-5-ylmethoxy)carbonyl]amino} propyl)carbamate | 7.0 | 73 |
| 1,3-thiazol-5-ylmethyl 4-pyridin-2-ylbenzyl(3-{(4-pyridin-2-ylbenzyl) [(1,3-thiazol-5-ylmethoxy)carbonyl]amino}propyl)carbamate | 8.0 | 86 |
| 1,3-thiazol-5-ylmethyl 4-(benzyloxy)benzyl(3-{[4-(benzyloxy)benzyl] [(1,3-thiazol-5-ylmethoxy)carbonyl]amino}propyl)carbamate | 12.0 | 45 |
| 1,3-thiazol-5-ylmethyl 3-{[(1,3-thiazol-5-ylmethoxy)carbonyl][4-(1*H*-1,2,4-triazol-1-yl)benzyl]amino}propyl[4-(1*H-*1,2,4-triazol-1-yl)benzyl] carbamate | 4.8 | 69 |
| 1,3-thiazol-5-ylmethyl 4-methoxybenzyl(3-{(4-methoxybenzyl)[(1,3-thiazol-5-ylmethoxy)carbonyl]amino}propyl)carbamate | 1.7 | 91 |
| 1,3-thiazol-5-ylmethyl 4-*tert*-butylbenzyl(3-{(4-*tert*-butylbenzyl)[(1,3-thiazol-5-ylmethoxy)carbonyl]amino}propyl)carbamate | 1.9 | 63 |
| *tert*-butyl 4-benzoylbenzyl(3-{(4-benzoylbenzyl)[(1,3-thiazol-5-yl methoxy)carbonyl]amino}propyl)carbamate | 2 | 77 |
| 1,3-thiazol-5-ylmethyl 1,1'-biphenyl-4-ylmethyl(3-{(1,1'-biphenyl-4-yl methyl)[(1,3-thiazol-5-ylmethoxy)carbonyl]amino}propyl)carbamate | 2.6 | 33 |
| 1,3-thiazol-5-ylmethyl benzyl{3-{benzyl[(1,3-thiazol-5-ylmethoxy) carbonyl]amino}-2-[(phenoxycarbonyl)amino]propyl}carbamate | 53 | 100 |
| 1,3-thiazol-5-ylmethyl benzyl[3-{benzyl[(1,3-thiazol-5-ylmethoxy)carbonyl]amino}-2-(isobutyrylamino)propyl]carbamate | 66 | 100 |

**Example 3.** Preparation of tert-butyl (1S,3S,4S)-1-benzyl-3-hydroxy-5-phenyl-4-{[(1,3-thiazol-5-ylmethoxy)carbonyl]amino} pentylcarbamate

A suspension of *tert*-butyl (1*S*,3*S*,4*S*)-1-benzyl-3-hydroxy-5-phenyl-4-amino-pentyl carbamate (12 g, 24 mmol, Kempf et al., J. MED. CHEM., 33: 2687-2689 (1990)), carbonic acid 5-methyl thiazole ester 4-nitrophenyl ester hydrochloride (7.93 g, 1.04 equivalents, U.S. Patent No. 5,773,625), and N-methyl morpholine (11.82 mL, 4.5 equivalents) in DMF (24 mL) was stirred for 2 days at 25°C. The resulting slurry was quenched with saturated sodium bicarbonate (100 mL), extracted with EtOAc (100 mL), washed with 10% citric acid, dried (Na₂SO₄), and concentrated *in vacuo.* Column chromatography on silica (90% EtOAc/hexane) followed by crystallization in hot 50% EtOAc/hexane gave a white solid (9.5 g, 75.6%). 1H NMR (300 MHz, DMSO-D6) δ ppm 1.29 (s, 9 H), 1.45 (m, 2 H), 2.55 - 2.62 (m, 2 H), 2.63 - 2.81 (m, 2 H), 3.57 (m, 1 H), 3.87 (m, 2 H), 4.61 (d, *J*=6.62 Hz, 1 H), 4.97 - 5.28 (m, 2 H), 6.62 (d, *J*=9.19 Hz, 1 H), 6.89 (d, *J*=9.56 Hz, 1 H), 7.05 - 7.36 (m, 10 H), 7.86 (s, I H), 9.04 (s, 1 H); MS (-ESI) m/z 560.3 (M+Cl)'.

**Example 4.** Preparation of 1,3-thiazol-5-ylmethyl (1S,2S,4S)-4-amino-1-benzyl-2-hydroxy-5-phenylpentylcarbamate

A solution of tert-butyl (1S,3S,4S)-1-benzyl-3-hydroxy-5-phenyl-4-{[(1,3-thiazol-5-yl methoxy)carbonyl]amino}pentylcarbamate (1.2 g, 6.2 mmol) in 4N HCl/ dioxane (12 mL) was stirred at room temperature for 3 h. The reaction mixture was concentrated *in vacuo,* trituated with EtOAc, and filtered to give a white solid (0.93 g, 98.2%). 1H NMR (300 MHz, DMSO-D6) δ ppm 1.58 (t, *J*=6.43 Hz, 2 H), 2.54 - 2.68 (m, 1 H), 2.72 - 2.93 (m, 3 H), 3.41 - 3.55 (m, 1 H), 5.13 (s, 2 H), 7.04 - 7.47 (m, 11 H), 7.84 (s, 1 H), 9.07 (s, 1 H); MS(ESI) m/z 426.1 (M+H)⁺.

**Example 5**. Preparation of 1,3-thiazol-5-ytmethyl(1S,2S,4S)-1-benzyl-2-hydroxy-4-{[(2S)-3-methyl-2-(2-oxotetrahydropyrimidin-1(2H)-yl)butanoyl]amino}-5-phenylpentylcarbamate

A solution of 1,3-thiazol-5-ylmethyl (1S,2S,4S)-4-amino-1-benzyl-2-hydroxy-5-phenyl pentyl carbamate (50 mg, 0.1 mmol), 1-(3-dimethylaminopropyl)-3-ethylcarbodiimide hydrochloride (29.7 mg, 1.5 equivalents), and 1-hydroxybenzotriazole (20.4 mg, 1.5 equivalents) in N,N-dimethylformamide (0.5 mL) was stirred for 5 minutes at room temperature. To this mixture was added 3-methyl-2-(2-oxo-tetrahydro-pyrimidin-1-yl)-butyric acid (22.2 mg, 1.1 equivalents) followed by N-methylmorpholine (50 µL, 4.5 equivalents) and the solution was stirred for 16 hours, The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (45 mg, 73,8%). 1H NMR (300 MHz, DMSO-D6) δ ppm 0,74 (t, *J*=6.43 Hz, 6 H), 1.40-1.56 (m, 2 H), 1.49 - 1.65 (m, 1 H), 1.94 - 2.05 (m, 1 H), 2.59 - 2.77 (m, 4 H), 2.77 - 2.92 (m, 2 H), 2.95 - 3.13 (m, 2 H), 3.46 - 3.61 (m, 1 H), 3.88 - 3.98 (m, I H), 4.14 - 4.22 (m, 1 H), 4.30 (d, *J=*11.03 Hz, 1 H), 4.61 (d, *J*=5.88 Hz, 1 H), 5.16 (dd, 2 H), 6.28 (s, 1 H), 6.90 (d, *J*=9.56 Hz, 1 H), 7.05 - 7.31 (m, 10 H), 7.49 (d, *J*=8.82 Hz, 1 H), 7.86 (s, 1 H), 9.05 (s, 1 H); MS(ESI) m/z 608.4 (M+H)⁺.

**Example 6.** Preparation of 1,3-thiazol-5-ylmethyl(1S,2S,4S)-1-benzyl-4-{[(2,6-dimethyl phenoxy)acetyl]amino}-2-hydroxy-5-phenylpentylcarbamate

A solution of 1,3-thiazol-5-ylmethyl (1S,2S,4S)-4-amino-1-benzyl-2-hydroxy-5-phenylpentyl carbamate (100 mg, 0.2 mmol), 1-(3-dimethylaminopropyl)-3-ethylcarbodiimide hydrochloride (60 mg, 1.5 equivalents), and 1-hydroxybenzotriazole (40.8 mg, 1.5 equivalents) in N,N-dimethylformamide (1 mL) was stirred for 5 minutes at room temperature. To this mixture was added (2,6-Dimethyl-phenoxy)-acetic acid (38 mg, 1.05 equivalents) followed by N-methylmorpholine (100 µL, 4.5 equivalents) and the solution was stirred for 16 hours, The reaction mixture was quenched with saturated sodium bicarbonate, extracted with EtOAc, washed with 10% citric acid, dried (Na₂SO₄), and concentrated *in vacuo.* Column chromatography on silica (80% EtOAc/hexane) gave a white foam (60 mg, 50.9%). 1H NMR (300 MHz, DMSO-D6) δ ppm 1.59 (m, 2 H), 2.13 (s, 6 H), 2.64 - 2.84 (m, 4 H), 3.59 (m, 1 H), 3.84 - 4.16 (m, 3 H), 4.70 (d, *J*=6.25 Hz, 1 H), 5.04 - 5.32 (m, 2 H), 6.86 - 7.07 (m, 4 H), 7.08 - 7.29 (m, 10 H), 7.80 (d, *J*=9.19 Hz, 1 H), 7.88 (s, 1 H); MS(ESI) m/z 558.3 (M+H)⁺

**Example 7.** Preparation of N¹-((1S,3S,4S)-1-benzyl-3-hydroxy-5-phenyl-4-{[(1,3-thiazol-5-ylmethoxy)carbonyl]amino}pentyl)-N²-(methoxycarbonyl)-3-methyl-L-valinamide

A solution of 1,3-thiazol,5-ylmethyl (1S,2S,4S)-4-amino-1-benzyl-2-hydroxy-5-phenylpentyl carbamate (50 mg, 0.1 mmol), 1-(3-dimethylaminopropyl)-3-ethylcarbodiimide hydrochloride (29.7 mg, 1.5 equivalents), and 1-hydroxybenzotriazole (20.4 mg, 1.5 equivalents) in N,N-dimethylformamide (0.5 mL) was stirred for 5 minutes at room temperature. To this mixture was added L-tert-leucine methyl carbamate (21 mg, 1.1 equivalents) followed by N-methylmorpholine (50 µL, 4.5 equivalents) and the solution was stirred for 16 hours. The reaction mixture was quenched with saturated sodium bicarbonate, extracted with EtOAc, washed with 10% citric acid, dried (Na₂SO₄), and concentrated *in vacuo.* Column chromatography on silica (8% MeOH/ CH₂Cl₂) gave a white foam (44 mg, 73.5%). 1H NMR (300 MHz, DMSO-D6) δ ppm 0.81 (s, 9 H), 1.39 - 1.52 (m, 2 H), 2.55 - 2.71 (m, 4 H), 3.48 - 3.66 (m, 4 H), 3.82 (d, *J*=9.56 Hz, 2 H), 4.05 - 4.23 (m, 1 H), 4.64 (d, *J*=6.25 Hz, 1 H), 5.04 - 5.30 (m, 2 H), 6.69 (d, *J*=9.56 Hz, 1 H), 6.90 (d, *J*=9.56 Hz, 1 H), 7.05 - 7.29 (m, 10 H), 7.71 - 7.82 (m, 1 H), 7.86 (s, 1 H), 9.05 (s, 1 H); MS(ESI) m/z 597.3 (M+H)⁺.

Example 8. Preparation of 1,3-thiazol-5-ylmethyl (1S,2S,4S)-4-(acetylamino)-1-benzyl-2-hydroxy-5-phenylpentylcarbamate and (1S,3S)-3-(acetylamino)-4-phenyl-1-((1S)-2-phenyl-1-{[(1,3-thiazol-5-ylmethoxy)carbonyl]amino}ethyl)butyl acetate

To a solution of 1,3-thiazol-5-ylmethyl (1S,2S,4S)-4-amino-1-benzyl-2-hydroxy-5-phenyl pentylcarbamate (100 mg, 0.2 mmol), DMAP (54 mg, 2.2 equivalents) in DMF (1 mL) was added acetyl chloride (19 µL, 1.3 equivalents) and stirred at 25°C for 20 min. The reaction mixture was quenched with saturated sodium bicarbonate, extracted with EtOAc, washed with 10% citric acid, dried (Na₂SO₄), and concentrated in vacuo. Column chromatography on silica (10% MeOH/ CH₂Cl₂) gave two compounds, major 1,3-thiazol-5-ylmethyl (1S,2S,4S)-4-(acetylamino)-1-benzyl-2-hydroxy-5-phenylpentylcarbamate (50 mg, 53.3%) and minor (1S,3S)-3-(acetylamino)-4-phenyl-1-((1S)-2-phenyl-1-{[(1,3-thiazol-5-yl methoxy)carbonyl]amino}ethyl)butyl acetate (15 mg, 14.7%). The former compound: 1H NMR (300 MHz, DMSO-D6) 8 ppm 1.36 - 1.48 (m, 2 H,) 1.65 (s, 3 H), 2.53 - 2.78 (m, 4 H), 3.46 - 3.59 (m, 1 H), 3.73 - 3.88 (m, 1 H), 4.62 (d, *J*=5.88 Hz, 1 H), 5.10 - 5.29 (m, 2 H), 6.90 (d, *J*=9.19 Hz, 1 H), 7.05 - 7.34 (m, 10 H), 7.59 (d, *J*=8.82 Hz, 1 H), 7.87 (s, 1 H), 9.05 (s, 1 H); MS(ESI) m/z 468.2 (M+H)⁺; the latter compound: 1H NMR (300 MHz, DMSO-D6) δ ppm 1.44 - 1.65 (m, 2 H), 1.68 (s, 3 H), 2.03 (s, 3 H), 2.52 - 2.75 (m, 4 H), 3.99 - 4.24 (m, 2 H), 4.85 - 4.99 (m, 1 H), 5.05 - 5.29 (m, 2 H), 7.08 - 7.42 (m, 11 H), 7.73 (d, *J*=9.19 Hz, 1 H), 7.86 (s, 1 H), 9.06 (s, 1 H); MS(ESI) m/z 510.2 (M+H)⁺.

**Example 9.** Preparation of methyl (1S,3S,4S)-1-benzyl-3-hydroxy-5-phenyl-4-{[(1,3-thiazol-5-ylmethoxy)carbonyl]amino} pentylcarbamate

To a solution of 1,3-thiazol-5-ylmethyl (1S,2S,4S)-4-amino-1-benzyl-2-hydroxy-5-phenyl pentylcarbamate (50 mg, 0.1 mmol), DMAP (54 mg, 4.4 equivalents) in DMF (0.5 mL) was added methyl chloroformate (24.6 µL, 3 equivalents) and stirred at 25°C for 30 min. The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (11 mg, 22.7%). 1H NMR (300 MHz, DMSO-D6) δ ppm 1.46 (t, *J*=6.80 Hz, 2 H), 2.52 - 2.83 (m, 4 H), 3.41 (s, 3 H), 3.50 - 3.61 (m, 1 H), 3.78 - 3.92 (m, 2 H), 4.65 (d, *J*=6.25 Hz, 1 H), 5.07 - 5.26 (m, 2 H), 6.87 - 6.98 (m, 2 H), 7.03 - 7.35 (m, 9 H), 7.86 (s, 1 H), 9.04 (s, 1 H); MS(ESI) m/z 484.3 (M+H)⁺.

Example 10. Preparation of isopropyl (1S,3S,4S)-1-benzyl-3-hydroxy-5-phenyl-4-{[(1,3-thiazol-5-ylmethoxy)carbonyl]amino}pentylcarbamate

To a solution of 1,3-thiazol-5-ylmethyl (1S,2S,4S)-4-amino-1-benzyl-2-hydroxy-5-phenyl pentylcarbamate (200 mg, 0.4 mmol), DMAP (200 mg, 4 equivalents) in DMF (1.5 mL) was added 1M isopropyl chloroformate/ toluene (1.1 mL, 2.7 equivalents) and stirred at 25°C for 16 h. The reaction mixture was quenched with saturated sodium bicarbonate, extracted with EtOAc, washed with 10% citric acid, dried (Na₂SO₄), and *concentrated in vacuo.* Crystallization in hot 50% EtOAc/hexane gave a white solid (95 mg, 46.3%). 1H NMR (300 MHz, DMSO-D6) δ ppm 1.02 (d, *J*=6.25 Hz, 3 H), 1.09 (d, *J*=6.25 Hz, 3 H), 1.42 - 1.53 (m, 2 H), 2.53 - 2.78 (m, 4 H), 3.50 - 3.61 (m, I H), 3.78 - 3.95 (m, 2 H), 4.52 - 4.69 (m, 2 H), 5.10 - 5.24 (m, 2 H), 6.80 (d, *J*=8.46 Hz, I H), 6.92 (d, *J*=9.56 Hz, 1 H), 7.07 - 7.30 (m, 10 H), 7.86 (s, 1 H), 9.05 (s, 1 H); MS(ESI) m/z 512.3 (M+H)⁺.

**Example 11.** Preparation of 1,3-thiazol-5-ylmethyl (1S,2S,4S)-1-benzyl-4-{[(tert-butyl amino)carbonyl]amino}-2-hydroxy-5-phenylpentylcarbamate

To a solution of 1,3-thiazol-5-ylmethyl (1S,2S,4S)-4-amino-1-benzyl-2-hydroxy-5-phenyl pentylcarbamate (100 mg, 0.2 mmol), DMAP (108 mg, 4.4 equivalents) in DMF (1 mL) was added tert-butyl isocyanate (69 µL, 3 equivalents) and stirred at 25°C for 1 h. The reaction mixture was quenched with saturated sodium bicarbonate, extracted with EtOAc, washed with 10% citric acid, dried (Na₂SO4), and concentrated *in vacuo.* Column chromatography on silica (90% EtOAc/hexane) gave a white solid (58 mg, 55.1%). 1H NMR (300 MHz, DMSO-D6) δ ppm 1.18 (s, 9 H), 1.21 - 1.50 (m, 2 H), 2.58 - 2.78 (m, 4 H), 3.57 (m, 1 H), 3.77 - 3.90 (m, 1 H), 3.89 - 4.02 (m, I H), 4.67 (d, *J*=6.25 Hz, 1 H), 5.06 - 5.20 (m, 2 H), 5.42 (d, *J*=8.82 Hz, 1 H), 5.53 (s, 1 H), 6.90 (d, *J*=9.56 Hz, I H), 7.02 - 7.30 (m, 11 H), 7.86 (s, I H), 9.05 (s, 1 H); MS(ESI) m/z 525.3 (M+H)⁺.

**Example 12.** Preparation of 1,3-thiazol-5-ylmethyl(1S,2S,4S)-1-benzyl-4-{[(dimethylamino) carbonyl]amino}-2-hydroxy-5-phenylpentylcarbamate

To a solution of 1,3-thiazol-5-ylmethyl (1S,2S,4S)-4-amino-1-benzyl-2-hydroxy-5-phenyl pentylcarbamate (50 mg, 0.1 mmol), DMAP (54 mg, 4.4 equivalents) in DMF (0.5 mL) was added dimethylcarbamoyl chloride (27.7 µL, 3 equivalents) and stirred at 25°C for 3 h. The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (12 mg, 24.1%). 1H NMR (300 MHz, DMSO-D6) δ ppm 1.48 (t, *J*=6.99 Hz, 2 H), 2.57 - 2.80 (m, 10 H), 3.47 - 3.62 (m, I H), 3.78 - 3.87 (m, 1 H), 3.89 - 4.02 (m, 1 H), 4.66 (d, *J*=6.25 Hz, 1 H), 5.08 - 5.22 (m, 2 H), 5.89 (d, *J*=8.09 Hz, 1 H), 6.88 (d, *J*=9.56 Hz, 1 H), 7.06 - 7.32 (m, 10 H), 7.87 (s, 1 H), 9.06 (s, 1 H); MS(ESI) m/z 497.2 (M+H)⁺.

**Example 13.** Preparation of 1,3-thiazol-5-ylmethyl(1S,2S,4S)-1-benzyl-2-hydroxy-4-[(methylsulfonyl) amino]-5-phenylpentylcarbamate

To a solution of 1,3-thiazol-5-ylmethyl (1S,2S,4S)-4-amino-1-benzyl-2-hydroxy-5-phenyl pentylcarbamate (50 mg, 0.1 mmol), DMAP (54 mg, 4.4 equivalents) in DMF (0.5 mL) was added methansulfonyl chloride (23.3 µL, 3 equivalents) and stirred at 25°C for 6 h. The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (16 mg, 31.7%). 1 H NMR (300 MHz, DMSO-D6) δ ppm 1.51 (m, 2 H), 2.13 (s, 3 H), 2.64 - 2.77 (m, 4 H), 3.56 - 3.66 (m, 1 H), 3.69 - 3.80 (m, 1 H), 3.81 - 3.96 (m, 1 H), 4.77 (d, *J*=5.88 Hz, 1 H), 5.03 - 5,27 (m, 2 H), 7.01 (d, *J*=9.56 Hz, 1 H), 7.10 (d, *J*=8.82 Hz, 1 H), 7.13 - 7.35 (m, 10 H), 7.85 (s, 1 H), 9.03 (s, 1 H); MS(ESI) m/z 504.1 (M+H)⁺.

**Example 14.** Preparation of 1,3-thiazol-5-ylmethyl(1S,2S,4S)-1-benzyl-4-{[(dimethylamino) sulfonyl]amino}-2-hydroxy-5-phenylpentylcarbamate

To a solution of 1,3-thiazol-5-ylmethyl (1S,2S,4S)-4-amino-1-benzyl-2-hydroxy-5-phenyl pentylcarbamate (50 mg, 0.1 mmol), DMAP (54 mg, 4.4 equivalents) in DMF (0.5 mL) was added dimethyl sulfamoyl chloride (30 µL, 3 equivalents) and stirred at 25°C for 5 h. The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (10 mg, 18.7%). 1H NMR (300 MHz, DMSO-D6) δ ppm 1.35 - 1.71 (m, *J*=36.03 Hz, 2 H), 2.34 (s, 6 H), 2.57 - 2.84 (m, 4 H), 3.50 - 3.65 (m, 1 H), 3.66 - 3.85 (m, 2 H), 4.75 (d, *J*=6.62 Hz, 1 H), 5.13 (s, 2 H), 6.96 (d, *J*= 9.38 Hz, 2 H), 7.06 - 7.33 (m, 10 H), 7.84 (s, 1 H), 9.04 (s, 1 H); MS(ESI) m/z 533.2 (M+H)⁺.

**Example 15.** Preparation of 1,3-thiazol-5-ylmethyl (1S,3S,4S)-1-benzyl-3-hydroxy-5-phenyl-4-{[(1,3-thiazol-5-ylmethoxy)carbonyl]amino}pentylcarbamate

A solution of 1,3-thiazol-5-ylmethyl (1S,2S,4S)-4-amino-1-benzyl-2-hydroxy-5-phenyl pentylcarbamate (50 mg, 0.1 mmol), carbonic acid 5-methylthiazole ester 4-nitrophenyl ester hydrochloride (35 mg, 1.1 equivalents), and N-methyl morpholine (55 µL, 5 equivalents) in DMF (0.5 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (10% MeOH/ CH₂Cl₂) gave a white solid (20 mg, 35.2%). 1H NMR (300 MHz, DMSO-D6) δ ppm 1.47 (t, *J*=6.99 Hz, 2 H), 2.56 - 2.79 (m, 4 H), 3.47 - 3.61 (m, 1 H), 3.79 - 3.97 (m, *J*=1.47 Hz, 2 H), 4.66 (d, *J*=5.88 Hz, 1 H), 5.07 - 5.29 (m, 4 H), 6.93 (d, *J*=9.19 Hz, 2 H), 7.04 - 7.27 (m, 10 H), 7.86 (s, 2 H), 9.05 (d, *J*=7.35 Hz, 2 H); MS(ESI) m/z 567.3 (M+H)⁺.

**Example 16.** Preparation of 3-methyl-N-[(1,3-thiazol-5-ylmethoxy)carbonyl]-L-valine

A solution of L-tert-leucine (2.4 g, 18.3 mmol), carbonic acid 5-methylthiazole ester 4-nitrophenyl ester hydrochloride (6.07 g, 1.05 equivalents), and N-methyl morpholine (6.4 mL, 3.2 equivalents) in DMF (30 mL) was stirred for 3 days at 25°C. The reaction mixture was quenched with 10% citric acid, extracted with EtOAc, dried (Na₂SO₄), and concentrated *in vacuo.* The resulting solid was washed with MeOH, and filtered. The filtrate was concentrated *in vacuo,* washed with hot 80% EtOAc/hexane and filtered. Yield of the combined solid (1.5 g, 30.1%). 1H NMR (300 MHz, DMSO-D6) δ ppm 0.94 (s, 9 H), 3.83 (d, *J*=8.82 Hz, 1 H), 5.28 (s, 2 H), 7.52 (d, *J*=8.82 Hz, 1 H), 7.94 (s, 1 H), 9.09 (s, 1 H), 12.56 (s, 1 H); MS(ESI) m/z 273.0 (M+H)⁺.

**Example 17.** Preparation of N¹-{(1S,2S,4S)-1-benzyl-4-[(tert-butoxycarbonyl)amino]-2-hydroxy-5-phenylpentyl}-3-methyl-N²-[(1,3-thiazol-5-ylmethoxy)carbonyl]-L-valinamide,

A solution of the compound of Example 16 (133 mg, 0.49 mmol), *tert*-butyl (1S,3S,4S)-1-benzyl-3-hydroxy-5-phenyl-4-amino-pentylcarbamate (198 mg, 1.05 equivalents), 1-(3-dimethylamino propyl)-3-ethylcarbodiimide hydrochloride (144.6 mg, 1.5 equivalents), and 1-hydroxybenzotriazole (99.4 mg, 1.5 equivalents) in N,N-dimethylformamide (1 mL) was stirred for 10 minutes at room temperature. To this mixture was added N-methylmorpholine (134 µL, 2.5 equivalents) and the solution was stirred for 16 hours. The reaction mixture was quenched with saturated sodium bicarbonate, extracted with EtOAc, washed with 10% citric acid, dried (Na₂SO₄), and concentrated *in vacuo.* Column chromatography on silica (6% MeOH/ CH₂Cl₂) gave a white solid (240 mg, 76.7%). 1H NMR (300 MHz, DMSO-D6) δ ppm 0.82 (s, 9 H), 1.26 (s, 9 H), 1.40 - 1.58 (m, 2 H), 2.53 - 2.79 (m, *J*=3.31 Hz, 4 H), 3.53 - 3.63 (m, 1 H), 3.71 - 3.83 (m, 1 H), 3.96 (d, 1 H), 4.05 - 4.19 (m, 1 H), 4.78 (d, 1 H), 5.28 (s, 2 H), 6.56 (d, *J*=9.19 Hz, 1 H), 6.98 - 7.31 (m, 11 H), 7.57 (s, 1 H), 7.57 (d, 1 H), 7.94 (s, 1 H), 9.09 (s, 1 H); MS m/z 639.4 (M+H)⁺.

**Example 18.** Preparation of tert-butyl (1S,3S,4S)-1-benzyl-4-{[(2,6-dimethylphenoxy)acetyl] amino}-3-hydroxy-5-phenylpentylcarbamate

A solution of *tert*-butyl (1*S*,3*S*,4*S*)-1-benzyl-3-hydroxy-5-phenyl-4-amino-pentylcarbamate (38.5 mg, 0.1 mmol), (2,6-dimethyl-phenoxy)-acetic acid (18.9 mg, 1.05 equivalents), 1-(3-dimethyl aminopropyl)-3-ethylcarbodiimide hydrochloride (29.7 mg, 1.5 equivalents), and 1-hydroxybenzotriazole (20.4 mg, 1.5 equivalents) in N,N-dimethylformamide (1 mL) was stirred for 4 minutes at room temperature. To this mixture was added N-methylmorpholine (27.5 µL, 2.5 equivalents) and the solution was stirred for 16 hours. The reaction mixture was quenched with saturated sodium bicarbonate, extracted with EtOAc, washed with 10% citric acid, dried (Na₂SO₄), and concentrated *In vacuo.* Column chromatography on silica (3% MeOH/ CH₂Cl₂) gave a white solid (240 mg, 76.7%). 1H NMR (300 MHz, DMSO-D6) δ ppm 1.31 (s, 9 H), 1.39 - 1.55 (m, *J*=6.99 Hz, 2 H), 2.14 (s, 6 H), 2.61 (d, *J*=6.99 Hz, 2 H), 2.80 (d, *J*=7.35 Hz, 2 H), 3.61 - 3.70 (m, 1 H), 3.84 (m, 1 H), 4.00 - 4.11 (m, 2 H), 4.20 - 4.38 (m, I H), 4.99 (d, 1 H), 6.66 (d, *J*=9.19 Hz, 1 H), 6.88 - 7.28 (m, 13 H), 7.43 (d, *J*=9.56 Hz, 1 H); MS m/z 547.4 (M+H)⁺.

**Example 19.** Preparation of 1,3-thiazol-5-ylmethyl(1S,3S,4S)-1-benzyl-4-{[(2,6-dimethyl phenoxy)acetyl]amino}-3-hydroxy-5-phenylpentylcarbamate

A solution of the compound of Example 18 (42 mg, 0.08 mmol) in 4N HCL/ dioxane (3 mL) was stirred at room temperature for 6 h. The reaction mixture was concentrated *in vacuo,* trituated with Et₂O, and filtered to give a white solid (36 mg, 97.1 %) which was used for the next step without further purification.

A solution of the above amine intermediate (36 mg, 0.075 mmol), carbonic acid 5-methyl thiazole ester 4-nitrophenyl ester hydrochloride (27.1 mg, 1.15 equivalents), and N-methyl morpholine (24.7 µL, 3 equivalents) in DMF (0.5 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (6% MeOH/ CH₂Cl₂) gave a white solid (22 mg, 50.2%).1H NMR (300 MHz, DMSO-D6) δ ppm 1.40 - 1.60 (m, 2 H), 2.13 (s, 8 H), 2.58 - 2.67 (m, 2 H), 2.81 (d, *J*=7.35 Hz, 2 H), 3.60 - 3.68 (m, 1 H), 3.85 - 3.98 (m, 1 H), 4.06 (s, 2 H), 4.22 - 4.37 (m, 1 H), 5.01 (d, *J*=5.88 Hz, 1 H), 5.16 (d, *J*=2.21 Hz, 2 H), 6.89 - 7.31 (m, 13 H), 7.46 (d, *J*=9.19 Hz, 1 H), 7.88 (s, 1 H), 9.07 (s, 1 H); MS (ESI) m/z 588.4(M+H)⁺.

**Example 20.** Preparation of N¹-((1S,2S,4S)-1-benzyl-2-hydroxy-5-phenyl-4-{[(1,3-thiazol-5-ylmethoxy)carbonyl]amino} pentyl)-N²-(methoxycarbonyl)-3-methyl-L-valinamide

A mixture of methyl (1S)-1-({[(1S,2S,4S)-4-amino-1-benzyl-2-hydroxy-5-phenylpentyl] amino}carbonyl)-2,2-dimethylpropylcarbamate (see Example 8B of WO 2005/058841) (100 mg, 0.22 mmol), carbonic acid 5-methylthiazole ester 4-nitrophenyl ester hydrochloride (72.5 mg, 1.05 equivalents), and N-methyl morpholine (72 µL, 3 equivalents) in DMF (0.5 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (4% MeOH/ CH₂Cl₂) gave a white solid (90 mg, 68.7%). 1H NMR (300 MHz, DMSO-D6) δ ppm 0.82 (s, 9 H), 1.38 - 1.56 (m, 2 H), 2.55 - 2.83 (m, 4 H), 3.54 (s, 4 H), 3.80 - 3.93 (m, 2 H), 4.07 - 4.23 (m, 1 H), 4.75 - 4.86 (m, 1 H), 5.11 (s, 2 H), 6.84 (d, *J*=9.56 Hz, 1 H), 7.04 - 7.24 (m, 11 H), 7.52 (dd, 1 H), 7.84 (s, 1 H), 9.06 (s, 1 H); MS (ESI) m/z 597.4 (M+H)⁺.

**Example 21.** Preparation of tert-butyl (1S,3S,4S)-4-(dibenzylamino)-3-hydroxy-5-phenyl-1-(4-pyridin-2-ylbenzyl) pentylcarbamate

A mixture of (2S,3S,5S)-5-amino-2-(dibenzylamino)-1-phenyl-6-(4-pyridin-2-ylphenyl) hexan-3-ol (see Example 2-2 of WO 2005/058841) (5.2 g, 9.6 mmol), and di-*ter*t-butyl dicarbonate (2.1 g, 1 equivalents) in THF (70 mL) and IN NaHCO₃ solution (20mL) was stirred for 16 h at 25°C The THF solvent was evaporated *in vacuo,* the resulting slurry was extracted with EtOAc, washed with 10% citric acid, dried (Na₂SO₄), and concentrated *in vacuo.* Column chromatography on silica (40% EtOAc/hexane) gave a white solid (5.75 g, 93.3%). 1 H NMR (300 MHz, DMSO-D6) δ ppm 1.28 (s, 9 H), 1.44 - 1.59 (m, I H), 1.68 - 1.87 (m, 1 H), 2.52 - 2.76 (m, 2 H), 2.81 - 3.05 (m, 2 H), 3.47 (d, *J*=13.97 Hz, 2 H), 3.50 - 3.58 (m, 2 H), 3.98 - 4.12 (m, 2 H), 4.69 (d, *J*=3.68 Hz, 1 H), 6,49 (d, 1 H), 7.04 - 7.39 (m, 15 H), 7.79 - 7.99 (m, 3 H), 8.65 (d, *J*=4.78 Hz, 1 H); MS (ESI) m/z 642.4 (M+H)⁺.

**Example 22.** Preparation of tert-butyl (1S,3S,4S)-4-amino-3-hydroxy-5-phenyl-1-(4-pyridin-2-ylbenzyl)pentylcarbamate

A mixture of the compound of Example 21 (5.75 g, 9 mmol) and 20% wt Pearlman's Catalyst (2.5 g) in EtOAc (35 mL) and MeOH (35 mL) was hydrogenated at 1 atm for 3 days. The solution was filtered and the filtrate was concentrated *in vacuo.* Column chromatography on silica (8% MeOH/ CH₂Cl₂ with 4% NH₄OH) afforded a white solid (3.02 g, 73%). 1H NMR (300 MHz, DMSO-D6) δ ppm 1.29 (s, 9 H), 1.49 - 1.71 (m, 2 H), 2.35 - 2.88 (m, 4 H), 3.40 - 3.46 (m, 1 H), 3.74 - 3.85 (m, 2 H), 4.45 (d, I H), 6.69 (d, *J*=8.82 Hz, 1 H), 7.13 - 7.41 (m, 9 H), 7.78 - 7.92 (m, 2 H), 7.97 (d, *J*=8.46 Hz, 2 H), 8.64 (d, *J*=4.78 Hz, 1 H); MS m/z 462.3 (M+H)⁺.

**Example 23.** Preparation of tert-butyl (1S,3S,4S)-3-hydroxy-5-phenyl-1-(4-pyridin-2-ylbenzyl)-4-{[(1,3-thiazol-5-ylmethoxy)carbonyl]amino}pentylcarbamate

A solution of the compound of Example 22 (2.2 g, 4.76 mmol), carbonic acid 5-methyl thiazole ester 4-nitrophenyl ester hydrochloride (1.58 g, 1.05 equivalents), and N-methyl morpholine (1.2 mL, 2.3 equivalents) in DMF (15 mL) was stirred for 3 days at 25°C The reaction mixture was quenched with saturated sodium bicarbonate, extracted with EtOAc, washed with 10% citric acid, dried (Na₂SO₄), and concentrated *in vacuo.* Column chromatography on silica (5% MeOH/ CH₂Cl₂) gave a white solid (1.95 g, 68%). 1H NMR (300 MHz, DMSO-D6) δ ppm 1.29 (s, 9 H), 1.41 - 1.53 (m, 2 H), 2.56 - 2.81 (m, 4 H), 3.54 - 3.64 (m, 1 H), 3.80 - 4.00 (m, 2 H), 4.64 (d, *J*=6.25 Hz, 1 H), 5.14 (s, 2 H), 6.68 (d, *J*=8.82 Hz, 1 H), 6.91 (d, *J*=9.19 Hz, 1 H), 7.09 - 7.40 (m, 9 H), 7.79 - 7.94 (m, 3 H), 7.96 (d, *J*=8.09 Hz, 2 H), 8.64 (d, *J*=4.78 Hz, 1 H), 9.03 (s, 1 H); MS (ESI) m/z 603.3 (M+H)⁺.

**Example 24.** Preparation of tert-butyl (1S,3S,4S)-3-hydroxy-4-({3-methyl-N-[(1,3-thiazol-5-ylmethoxy)carbonyl]-L-valyl}amino)-5-phenyl-1-(4-pyridin-2-ylbenzyl)pentylcarbamate

A solution of the compound of Example 22 (410 mg, 0.88 mmol), the compound of Example 16 (266 mg, 1.1 equivalents), 1-(3-dimethylaminopropyl)-3-ethylcarbodiimide hydrochloride (262 mg, 1.5 equivalents), and 1-hydroxybenzotriazole (118 mg, 1.5 equivalents) in N,N-dimethylformamide (3 mL) was stirred for 10 minutes at room temperature. To this mixture was added N-methylmorpholine (240 µL, 2.5 equivalents) and the solution was stirred for 16 hours. The reaction mixture was quenched with saturated sodium bicarbonate, extracted with EtOAc, washed with 10% citric acid, dried (Na₂SO₄), and concentrated *in vacuo.* Column chromatography on silica (1% MeOH/ EtOAc) gave a white solid (450 mg, 70.7%). 1H NMR (300 MHz, DMSO-D6) δ ppm 0.81 (s, 9 H), 1.27 (s, 9 H), 1.47 - 1.61 (m, 2 H), 2.57 - 2.78 (m, 4 H), 3.60 (m, 1 H), 3.77 - 3.89 (m, 1 H), 3.96 (d, 1 H), 4.06 - 4.19 (m, 1 H), 4.80 (d, 1 H), 5.28 (s, 2 H), 6.62 (d, *J*=8.82 Hz, 1 H), 6.97 - 7.39 (m, 10 H), 7.55 (d, 1 H), 7.76 - 8.01 (m, 5 H), 8.64 (d, *J*=4.78 Hz, 1 H), 9.09 (s, 1 H); MS m/z 716.5 (M+H)⁺.

**Example 25.** Preparation of methyl (1S,3S,4S)-3-hydroxy-4-({3-methyl-N-[(1,3-thiazol-5-ylmethoxy)carbonyl]-L-valyl}amino)-5-phenyl-1-(4-pyridin-2-ylbenzyl)pentylcarbamate

A solution of tert-butyl (1S,3S,4S)-3-hydroxy-4-({3-methyl-N-[(1,3-thiazol-5-ylmethoxy) carbonyl]-L-valyl}amino)-5-phenyl-1-(4-pyridin-2-ylbenzyl)pentylcarbamate (340 mg, 0.47 mmol) in CH₂Cl₂ (2 mL), MeOH (2 mL), and TFA (2 mL) was stirred at room temperature for 16 h. The reaction mixture was concentrated *in vacuo,* added water, extracted with EtOAc. The aqeous layer was basified with saturated sodium bicarbonate, extracted with EtOAc, dried (Na₂SO₄), and concentrated *in vacuo* to give a white solid (196 mg, 67%) which was used for the next step without further purification.

To a solution of the above intermediate (196 mg, 0.32 mmol), DMAP (171 mg, 4.4 equivalents) in DMF (2 mL) was added methyl chloroformate (61.5 µL, 2.5 equivalents) and stirred at 25°C for 1 h. The reaction mixture was quenched with saturated sodium bicarbonate, extracted with EtOAc, washed with 10% citric acid, dried (Na₂SO₄), and concentrated *in vacuo.* Column chromatography on silica (2% MeOH/ EtOAc) gave a white solid (110 mg, 51.3%). 1H NMR (300 MHz, DMSO-D6) δ ppm 0.82 (s, 9 H), 1.46 - 1.59 (m, 2 H), 2.55 - 2.86 (m, 4 H), 3.39 (s, 3 H), 3.53 - 3.65 (m, 1 H), 3.80 - 3.87 (m, 1 H), 3.95 (d, *J*=9.93 Hz, 1 H), 4,05 - 4.14 (m, 1 H), 4.85 (d, 1 H), 5.27 (s, 2 H), 6.95 (d, *J*=8.82 Hz, 1 H), 7.02 - 7.38 (m, 10 H), 7.61 (d, 1 H), 7.79 - 8.03 (m, 5 H), 8.64 (d, *J*=4.41 Hz, 1 H), 9.09 (s, 1 H); MS(ESI) m/z 674.4 (M+H)⁺.

**Example 26.** Preparation of methyl (S)-1((28,4S,5S)-5-amino-4-hydroxy-6-phenyl-1-(4-(pyridin-2-yl)phenyl)hexan-2-ylamino)-3,3-dimethyl-1-oxobutan-2-ylcarbamate

A solution of tert-butyl (1S,2S,4S)-1-benzyl-2-hydroxy-4-({(2S)-2-[(methoxycarbonyl) amino]-3,3-dimethylbutanoyl}amino)-5-[4-(2-pyridinyl)phenyl]pentylcarbamate (see Example 2B of WO 2005/058841) (2 g, 3.16 mmol) in 50/50 TFA/CH₂Cl₂ (8 mL) was stirred at room temperature for 16 h. The reaction mixture was concentrated *in vacuo,* added water, extracted with EtOAc. The aqeous layer was basified with saturated sodium bicarbonate, extracted with EtOAc, dried (Na₂SO₄), and concentrated *in vacuo* to give a white solid (1.52 g, 90.3%) which was used for the next step without further purification.

**Example 27.** Preparation of N¹-((1S,3S,4S)-3-hydroxy-5-phenyl-1-(4-pyridin-2-ylbenzyl)-4-{[(1,3-thiazol-5-ylmethoxy)carbonyl]amino}pentyl)-N²-(methoxycarbonyl)-3-methyl-L-valinamide

A mixture of the compound of Example 26 (100 mg, 0.19 mmol), carbonic acid 5-methyl thiazole ester 4-nitrophenyl ester hydrochloride (62.5 mg, 1.05 equivalents), and N-methyl morpholine (61.5 µL, 1.05 equivalents) in DMF (0.5 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (4% MeOH/ CH₂Cl₂) gave a white solid (64 mg, 50.6%). 1H NMR (300 MHz, DMSO-D6) δ ppm 0.82 (s, 9 H), 1.41 - 1.56 (m, 2 H), 2.55 - 2.83 (m, 4 H), 3.50 (s, 3 H), 3.57 - 3.65 (m, 1 H), 3.84 (d, *J*=9.93 Hz, 2 H), 4.07 - 4.26 (m, 1 H), 4.67 (d, *J*=6.25 Hz, 1 H), 5.13 (s, 2 H), 6.68 (d, *J*=9.56 Hz, 1 H), 6.92 (d, *J*=9.56 Hz, 1 H), 7.08 - 7.37 (m, 9 H), 7.76 - 7.94 (m, 6 H), 8.63 (d, *J*=4.41 Hz, 1 H), 9.04 (s, 1 H); MS (ESI) m/z 674.4 (M+H)⁺.

**Example 28.** Preparation of methyl (S)-1-((2S,4S,5S)-5-((S)-2-amino-3,3-dimethylbutanamido)-4-hydroxy-6-phenyl-1-(4-(pyridin-2-yl)phenyl)hexan-2-ylamino)-3,3-dimethyl-1-oxobutan-2-ylcarbamate

A solution of the compound of Example 26 (500 mg, 0.94 mmol), Boc-L-tert-leucine (326 mg, 1.5 equivalents), 3-(diethoxyphosphoryloxy)-1,2,3-benzotriazin-4(3H)-one (560 mg, 2 equivalents), and Et₃N (0.32 mL, 2.5 equivalents) in THF (5 mL) was stirred for 7 hours at room temperature. The reaction mixture was quenched with saturated sodium bicarbonate, extracted with EtOAc, washed with 10% citric acid, dried (Na₂SO₄), and concentrated *in vacuo* to gave a white solid (690 mg) which was used for the next step without further purification.

A solution of the above intermediate (690 mg) in 80/20 TFA/CH₂Cl₂ (10 mL) was stirred at room temperature for 16 h. The reaction mixture was concentrated *in vacuo,* and the resulting residue was purified by column chromatography on silica (15% MeOH/ CH₂Cl₂ with 1% NH₄OH) afforded a white solid (510 g, 84.1% overall). 1H NMR (300 MHz, DMSO-D6) δ ppm 0.79 (s, 9 H), 1.35 - 1.62 (m, 2 H), 2.52 - 2.78 (m, 4 H), 3.49 (s, 3 H), 3.58 - 3.67 (m, 1 H), 3.82 (d, *J*=9.93 Hz, 1 H), 3.97 - 4.24 (m, 2 H), 4.96 (d, *J*=5.15 Hz, 1 H), 6.64 (d, *J*=9.93 Hz, 1 H), 7.06 - 7.37 (m, 8 H), 7.44 - 7.58 (m, 2 H), 7.74 - 8.00 (m, 6 H), 8.63 (d, *J*=4.78 Hz, 1 H); MS(APCI) m/z 646.0 (M+H)⁺.

**Example 29.** Preparation of methyl (1S)-1-({[(1S,3S,4S)-3-hydroxy-4-({3-methyl-N-[(1,3-thiazol-5-ylmethoxy)carbonyl]-L-valyl}amino)-5-phenyl-1-(4-pyridin-2-ylbenzyl)pentyl]amino} carbonyl)-2,2-dimethylpropylcarbamate

A mixture of the compound of Example 28 (30 mg, 0.046 mmol), carbonic acid 5-methyl thiazole ester 4-nitrophenyl ester hydrochloride (16.2 mg, 1.1 equivalents), and N-methyl morpholine (11.3 µL, 2.2 equivalents) in DMF (0.5 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (9% MeOH/ CH₂Cl₂) gave a white solid (16 mg, 43.8%). 1H NMR (300 MHz, DMSO-D6) δ ppm 0.79 (s, 9 H), 0.82 (s, 9 H), 1.22-1.66 (m, 2 H), 2.62 - 2.85 (m, 4 H), 3.49 (s, 3 H), 3.57 - 3.68 (m, 1 H), 3.82 (d, *J*= 10.30 Hz, 1 H), 3.96 (d, *J*=9.56 Hz, 1 H), 4.01 - 4.21 (m, 2 H), 4.86 (d, *J*=5.88 Hz, 1 H), 5.28 (s, 2 H), 6.60 (d, 1 H), 7.00 (d, *J*=9.56 Hz, 1 H), 7.05 - 7.27 (m, 6 H), 7.26 - 7.33 (m, 1 H), 7.59 (d, 1 H), 7.80 (d, 1 H), 7.83 - 7.93 (m, 4 H), 7.95 (s, 1 H), 8.63 (d, *J*=4.41 Hz, 1 H), 9.10 (s, 1 H); MS (ESI) m/z 787.4 (M+H)⁺.

**Example 30.** Preparation of (S)-methyl 2-((thiazol-5-ylmethoxy)carbonylamino)propanoate

A suspension of L-alanine methyl ester hydrochloride (2.5 g, 17.9 mmol), carbonic acid 5-methylthiazole ester 4-nitrophenyl ester hydrochloride (6 g, 1.06 equivalents), and N-methyl morpholine (6.3 mL, 3.2 equivalents) in DMF (40 mL) was stirred for 16 h at 25°C The reaction mixture was quenched with saturated sodium bicarbonate, extracted with EtOAc, washed with 10% citric acid, dried (Na₂SO₄), and concentrated *in vacuo.* Column chromatography on silica (60% EtOAc/hexane) gave a white solid (1.79 g, 41.3%). 1H NMR (300 MHz, DMSO-D6) δ ppm 1.26 (d, *J*=7.35 Hz, 3 H), 3.62 (s, 3 H), 4.10 (q, *J*=7.35 Hz, 1 H), 5.27 (s, 2 H), 7.82 (d, *J=*7.35 Hz, 1 H), 7.93 (s, I H), 9.09 (s, 1 H); MS (ESI) m/z 244.9 (M+H)⁺.

**Example 31.** Preparation of tert-butyl (1S,3S,4S)-3-hydroxy-5-phenyl-1-(4-pyridin-2-ylbenzyl)-4-({N-[(1,3-thiazol-5-ylmethoxy)carbonyl]-L-alanyl}amino)pentylcarbamate

A solution of the compound of Example 30 (1.79 g, 7.3 mmol), LiOH-H₂O (0.77 g, 2.5 equivalents) in H₂O (9.5 mL) and 1,4-dioxane (19 mL) was stirred at 25°C for 7 hours. The reaction was acidified with 10% citric acid to pH 5, extracted with EtOAc, dried (Na₂SO₄), and concentrated *in vacuo* to afford a white solid (1.59 g, 93%) which was used for the next step without further purification.

A solution of the above intermediate (225 mg, 1.1 equivalents), the compound of Example 22 (410 mg, 0.89 mmol), 1-(3-dimethylaminopropyl)-3-ethylcarbodiimide hydrochloride (262 mg, 1.5 equivalents), and 1-hydroxybenzotriazole (180 mg, 1.5 equivalents) in N,N-dimethylformamide (4 mL) was stirred for 10 minutes at room temperature. To this mixture was added N-methylmorpholine (0.24 mL, 2.5 equivalents) and the solution was stirred for 16 hours. The reaction mixture was quenched with saturated sodium bicarbonate, extracted with EtOAc, washed with 10% citric acid, dried (Na₂SO₄), and **concentrated** *in vacuo.* Column chromatography on silica (3% MeOH/ EtOAc) gave a white solid (490 mg, 81.8%). 1H NMR (300 MHz, DMSO-D6) δ ppm 1.06 (d, *J*=6.99 Hz, 3 H), 1.27 (s, 9 H), 1.35 - 1.58 (m, 2 H), 2.53 - 2.85 (m, 4 H), 3.53 - 3.68 (m, I H), 3.75 - 3.89 (m, 1 H), 3.96 - 4.17 (m, 2 H), 4.86 (d, *J*=5.52 Hz, 1 H), 5.25 (dd, *J*=4.04 Hz, 2 H), 6.62 (d, *J=*8.82 Hz, 1 H), 7.09 - 7.35 (m, 7 H), 7.37 (d, *J*=8.09 Hz, 1 H), 7.45 (d, 1 H), 7.77 - 8.01 (m, 5 H), 8.63 (d, *J*=4.78 Hz, 1 H), 9.08 (s, 1 H); MS (ESI) m/z 674.4 (M+H)⁺_{.}

**Example 32.** Preparation of methyl (1S,3S,4S)-3-hydroxy-5-phenyl-1-(4-pyridin-2-ylbenzyl)-4-({N-[(1,3-thiazol-5-ylmethoxy)carbonyl]-L-alanyl}amino)pentylcarbamate

A solution of tert-butyl (1S,3S,4S)-3-hydroxy-5-phenyl-1-(4-pyridin-2-ylbenzyl)-4-({N-[(1,3-thiazol-5-ylmethoxy)carbonyl]-L-alanyl}amino)pentylcarbamate (400 mg, 0.59 mmol) in CH₂Cl₂ (2 mL), MeOH (2 mL), and TFA (2 mL) was stirred at room temperature for 16 h. The reaction mixture was concentrated *in vacuo,* added water, extracted with EtOAc. The aqeous layer was basified with saturated sodium bicarbonate, extracted with EtOAc, dried (Na₂SO₄), and concentrated *in vacuo* to give a white solid (150 mg, 44%) which was used for the next step without further purification.

To a solution of the above intermediate (150 mg, 0.26 mmol), DMAP (140 mg, 4.4 equivalents) in DMF (2 mL) was added methyl chloroformate (50.5 µL, 2.5 equivalents) and stirred at 25°C for I h. The reaction mixture was quenched with saturated sodium bicarbonate, extracted with EtOAc, washed with 10% citric acid, dried (Na₂SO₄), and concentrated *in vacuo.* Column chromatography on silica (3% MeOH/ EtOAc) gave a white solid (102 mg, 61.7%). 1H NMR (300 MHz, DMSO-D6) δ ppm 1.07 (d, 3 H), 1.34 - 1.59 (m, 2 H), 2.61 - 2.81 (m, 4 H), 3.39 (s, 3 H), 3.51 - 3.66 (m, 1 H), 3.79 - 3.93 (m, 1 H), 3.93 - 4.12 (m, 2 H), 4.89 (d, 1 H), 5.25 (dd, *J=*4.41 Hz, 2 H), 6.93 (d, I H), 7.10 - 7.35 (m, 9 H), 7.39 (d, 1 H), 7.46 (d, 1 H), 7.79 - 8.01 (m, 5 H), 8.64 (d, *J*=4.78 Hz, 1 H), 9.08 (s, 1 H); MS(ESI) m/z 632.3 (M+H)⁺.

**Example 33.** Preparation of 1,3-thiazol-5-ylmethyl benzyl[9-benzyl-10-oxo-12-(1,3-thiazol-5-yl)-3,6,11-trioxa-9-azadodec-1-yl]carbamate

A suspension 1,2-bis(2-benzylaminoethoxy)ethane (40 mg, 0.12 mmol), carbonic acid 5-methylthiazole ester 4-nitrophenyl ester hydrochloride (81 mg, 2.1 equivalents), and Et₃N (51 µL, 3 equivalents) in DMF (0.5 mL) was stirred for 16 h at 25°C The reaction mixture was quenched with saturated sodium bicarbonate, extracted with EtOAc, dried (Na₂SO₄), and concentrated *in vacuo.* Column chromatography on silica (5% MeOH/ CH₂Cl₂) gave an oil (17 mg, 22.9%). 1H NMR (300 MHz, DMSO-D6) δ ppm 3.22 - 3.43 (m, 8 H), 3.45 - 3.53 (m, 4 H), 4.46 (s, 2 H), 4.48 (s, 2 H), 5.32 (s, 2 H), 5.35 (s, 2 H), 7.06 - 7.39 (m, 10 H), 7.88 (s, 1 H), 7.95 (s, 1 H), 9.07 (s, 1 H), 9.09 (s, 1 H); MS (ESI) m/z 611.3 (M+H)⁺.

**Example 34.** Preparation of methyl N-[(1,3-thiazol-5-ylmethoxy)carbonyl]-L-phenylalaninate

To a suspension of carbonic acid 4-nitro-phenyl ester thiazol-5-ylmethyl ester hydrochloride salt (see U.S. Patent No. 5,773,625) (0.81 g, 2.6 mmol) in EtOAc (10 mL) was added an solution of NaHCO₃ (0.21 g, 2.6 mmol) in H₂O (5 mL), shaked, and separated the layers. The organic layer was dried (Na₂SO₄), filtered, and then added to a suspension of (S)-2-amino-3-phenyl-propionic acid methyl ester (0.5 g, 2.3 mmol) in EtOAc (10 mL) followed by addition of Et₃N (0.32 mL, 2.3 mmol) and DMAP (0.31 g, 2.6 mmol). The mixture was stirred at room temperature for 18 h. After which, the solution was diluted with EtOAc (20 mL) and washed with sat. NaHCO₃ (20 mL), H₂O (20 mL), and brine (20 mL). The organic layer was dried (Na₂SO₄) and concentrated *in vacuo.* Column chromatography on silica (20% to 50% EtOAc/hexanes gradient) afforded the title compound as a colorless oil (0.40 g, 54%). ¹H NMR (300 MHz, DMSO-D6) δ ppm 9.08 (s, 1 H), 7.81 - 7.98 (m, 2 H), 7.11 - 7.34 (m, 5 H), 5.13 - 5.28 (m, 2 H), 4.18 - 4.32 (m, 1 H), 3.62 (s, 3 H), 2.96 - 3.10 (m, 1 H), 2.78 - 2.92 (m, *J*=13.79, 10.11 Hz, I H); MS m/z (M+H)⁺.

**Example 35.** Preparation of (S)-3-phenyl-2-((thiazol-5-yloxy)carbonylamino)propanoic acid

To a solution of methyl N-[(1,3-thiazol-5-ylmethoxy)carbonyl]-L-phenylalaninate in THF (8 mL) was added a solution of LiOH●HCl (0.14g, 3.4 mmol) in H₂O (4 mL). The reaction mixture was stirred at room temperature for 18 h. After which, the solution was acidified with 0.1 N HCI and extracted with 3:1 CH₂Cl₂:*i*PrOH solvent mixture (3x10 mL). The organic extracts were combined and concentrated *in vacuo.* The resulting residue was washed with CH₂Cl₂ and dried under high vacuum to afford the title compound as an off-white solid (0.27 g, 79%). ¹H NMR (300 MHz, DMSO-D6) δ ppm 9.07 (s, 1 H), 7.88 (s, 1 H), 7.64 (d, *J*=8.46 Hz, 1 H), 7.12 - 7.32 (m, 5 H), 5.13 - 5.28 (m, 2 H), 4.06 - 4.25 (m, 1 H), 3.06 (dd, *J*=13.79,4.60 Hz, 1 H), 2.82 (dd, *J*=13.79, 10.48 Hz, 1 H); MS m/z 307.0 (M+H)⁺.

**Example 36.** Preparation of N-benzyl-N-(2-phenylethyl)-N-[(1,3-thiazol-5-ylmethoxy) carbonyl]-L-phenylalaninamide

A mixture of the compound of Example 35 (25 mg, 0.08 mmol), EDCI (23 mg, 0.12 mmol), HOBt (17 mg, 0.12 mmol), and 4-methylmorpholine (27 µL, 0.24 mmol) in DMF (0.5 mL) was stirred at RT for 15 min, then *N*-benzyl-2-phenethylamine (19 µL, 9.0 mmol) was added. The reaction mixture was stirred at room temperature for 18 h. Subsequently, solution was diluted with EtOAc (2 mL) and washed with H₂O (2 mL) and brine (2 mL). The organic layer was dried (Na₂SO₄) and concentrated *in vacuo.* Column chromatography on silica (30%→50% EtOAc/hexanes) afforded the title compound as a colorless oil (24 mg, 58%). ¹H NMR (300 MHz, DMSO-D6) δ ppm 9.07 (s, 1 H), 7.86-8.07 (m, 1 H), 6.98 - 7.41 (m, 16 H), 5.17 - 5.28 (m, 1 H), 4.65 - 4.77 (m, *J*=8.09 Hz, 1 H), 4.37 - 4.62 (m, 2 H), 3.70 (s, 1 H), 3.45 - 3.59 (m, 1 H), 3.35 - 3.43 (m, 1 H), 2.64 - 2.94 (m, 4 H); MS m/z 500.2 (M+H)⁺.

**Example 37.** Preparation of N,N-dimethyl-N-[(1,3-thiazol-5-ylmethoxy)carbonyl]-L-phenylalaninamide

The procedure of Example 36 was followed, except substituting dimethylamine for *N-*benzyl-2-phenethylamine. The title compound was prepared as a colorless oil (12 mg, 44%). 1H NMR (300 MHz, DMSO-D6) δ ppm 9.07 (s, I H), 7.87 (s, 1 H), 7.73 (d, *J*=8.09 Hz, 1 H), 7.12 - 7.34 (m, 5 H), 5.18 (s, 2 H), 4.50 - 4.68 (m, 1 H), 3.32 (s, 2 H), 2.90 (s, 3 H), 2.78 (s, 3 H); MS m/z 334.1 (M+H)⁺.

**Example 38.** Preparation of N-[(1S)-1-benzyl-2-morpholin-4-yl-2-oxoethyl]-N-[(1,3-thiazol-5-ylmethoxy)carbonyl]amine

The procedure of Example 36 was followed, except substituting morpholine for *N*-benzyl-2-phenethylamine. The title compound was prepared as a colorless oil (19 mg, 61%). ¹H NMR (300 MHz, DMSO-D6) δ ppm 9.08 (s, 1 H), 7.88 (s, 1 H), 7.80 (d, *J*=8.09 Hz, 1 H), 7.16 - 7.35 (m, 5 H), 5.20 (s, 2 H), 4.57 - 4.70 (m, 1 H), 3.35 - 3.55 (m, 8 H), 2.76 - 2.95 (m, 2 H); MS m/z 376.0 (M+H)⁺.

**Example 39.** Preparation of N,N-diisobutyl-N-[(1,3-thiazol-5-ylmethoxy)carbonyl]-L-phenylalaninamide

The procedure of Example 36 was followed, except substituting diisobutylamine for N-benzyl-2-phenethylamine. The title compound was prepared as a colorless oil (6 mg, 18%). ¹H NMR (300 MHz, DMSO-D6) δ ppm 9.06 (s, 1 H), 7.86 (s, 1 H), 7.78 (d, *J=*8.46 Hz, 1 H), 7.12 - 7.33 (m, 5 H), 5.10 - 5.28 (m, 2 H), 4.51 - 4.68 (m, 1 H), 2.69 - 3.29 (m, 6 H), 1.73 - 1.98 (m, 2 H), 0.67 - 0.87 (m, 12 H); MS m/z 418.1 (M+H)⁺.

**Example 40,** Preparation of N-isobutyl-N-[(1,3-thiazol-5-ylmethoxy)carbonyl]-L-phenyl alaninamide

The procedure of Example 36 was followed, except substituting isobutylamine for *N*-benzyl-2-phenethylamine. The title compound was prepared as a white solid (16 mg, 55%). ¹H NMR (300 MHz, DMSO-D6) δ ppm 9.06 (s, 1 H), 7.96 (t, *J*=5.70 Hz, 1 H), 7.87 (s, 1 H), 7.54 (d, *J=*8.82 Hz, 1 H), 7.09 - 7.33 (m, 5 H), 5.17 (s, 2 H), 4.14 - 4.30 (m, I H), 2.66 - 2.99 (m, 4 H), 1.55 - 1.71 (m, 1 H), 0.79 (dd, *J=*6.62, 2.94 Hz, 6 H); MS m/z 362.1 (M+H)⁺.

**Example 41.** Preparation of N,N-dibenzyl-N-[(1,3-thiazol-5-ylmethoxy)carbonyl]-L-phenyl alaninamide

The procedure of Example 36 was followed, except substituting dibenzylamine for *N*-benzyl-2-phenethylamine. The title compound was prepared as a colorless oil (12 mg, 31%). ¹H NMR (300 MHz, DMSO-D6) δ ppm 9.08 (s, 1 H), 8.01 (d, *J*=8.46 Hz, 1 H), 7.89 (s, 1 H), 6.94 - 7.41 (m, 15 H), 5.12 - 5.29 (m, 2 H), 4.34 - 4.75 (m, 5 H), 2.76 - 2.93 (m, 2 H); MS m/z 486.2 (M+H)⁺.

**Example 42**. Preparation of 1,3-Bis-benzylamino-propan-2-ol

A solution of 1,3-diamino-2-propanol (0.5 g, 5.5 mmol) in CH₂Cl₂ (15 mL) was stirred rapidly and solid Na₂SO₄ (1.6 g, 11.1 mmol) was added followed by benzaldehyde (1.1 mL, 11.1 mmol). Stirring was continued at room temperature for 18 hours after which time it was filtered and the resulting filtrate concentrated. The residue was dissolved in EtOH (10 mL) and cooled to 0°C. Solid NaBH₄ (0.52 g, 13.9 mmol) was added in portions over 10 minutes and the resulting mixture stirred at 0°C for 1 hour. After this time H₂O (2 mL) was added and the solution was concentrated, dissolved in EtOAc (10 mL) and washed with 1N HCl (2 x 10 mL). The acid washes were combined and the pH was adjusted with 3N NaOH to alkaline pH. The solution was extracted with CH₂Cl₂ (3 x 10 mL), the extracts combined, dried over MgSO₄, filtered and the resulting solution concentrated to give 0.93 g, 62% of the compound of this Example (1,3-Bis-benzylamino-propan-2-ol).

**Example 43.** Preparation of N,N'-dibenzylpropane-1,3-diamine

Following the same procedure as for the compound of Example 42, using 1,3-diamino propane (0.5 mL, 6 mmol), Na₂SO₄ (1.7 g, 12 mmol), benzaldehyde (1.2 mL, 12 mmol) and NaBH₄ (0.57g, 15 mmol), gave 1.5 g, 99% of the compound of this Example (N,N'-dibenzylpropane-1,3-diamine).

**Example 44.** Preparation of N,N'-Bis-(4-pryidin-2-yl-benzyl)-propane-1,3-diamine

Following the same procedure as for the compound of Example 42, using 1,3-diamino propane (27 µL, 0.32 mmol), Na₂SO₄ (91 mg, 0.64 mmol), 2-pyridylbenzaldehyde (118 mg, 0.64 mmol) and NaBH₄ (31 mg, 0.81 mmol), gave 100 mg, 76% of the compound of this Example (N,N'-Bis-(4-pryidin-2-yl-benzyl)-propane-1,3-diamine).

**Example 45.** Preparation of N,N'-Bis-quinolin-3-ylmethyl-propane-1,3-diamine

Following the same procedure as for the compound of Example 42, using 1,3-diamino propane (40 µL, 0.48 mmol), Na₂SO₄ (136 mg, 0.95 mmol), 3-quinolinecarboxaldehyde (150 mg, 0.95 mmol) and NaBH₄ (45 mg, 1.2 mmol), gave 110 mg, 65% of the compound of this Example (N,N'-Bis-quinolin-3-ylmethyl-propane-1,3-diamine).

**Example 46.** Preparation of N,N'-Bis-(4-benzyloxybenzyl)-propane-1,3-diamine

Following the same procedure as for the compound of Example 42, using 1,3-diamino propane (28 µL, 0.33 mmol), Na₂SO₄ (95 mg, 0.67 mmol), 4-benzyloxybenzaldehyde (142 mg, 0.67 mmol), and NaBH₄ (32 mg, 0.84 mmol), gave 101 mg, 65% of the compound of this Example (N,N'-Bis-(4-benzyloxybenzyl)-propane-1,3-diamine).

**Example 47.** Preparation of N,N'-Bis-cyclohexylmethyl-propane-1,3-diamine

To a 1:1 mixture of benzene and methanol (2 mL) was added 1,3-diaminopropane (50 µL, 0.6 mmol) and cyclohexanecarboxaldehyde (145 µL, 1.2 mmol) and the solution was heated at 50°C for 2 hours after which time it was cooled to room temperature, NaBH₄ (91 mg, 2.4 mmol) was added and the resulting solution was stirred at room temperature for 1 hour. To this solution was added 10% NaHCO₃ (8 mL) and the resulting mixture was extracted with EtOAc (3 x 8 mL), the organic extracts combined, washed with brine (1 x 8 mL), dried over Na₂SO₄, the drying agent filtered off and the solvent removed in *vacuo* to give the compound of this Example (N,N'-Bis-cyclohexylmethyl-propane-1,3-diamine).

**Example 48.** Preparation of NN'-Bis-(4-methylbenzoate)methyl-propane-1,3-diamine

Following the procedure for preparing the compound of Example 17, using 1,3-diamino propane (50 µL, 0.6 mmol), methyl-4-formyl benzoate (197 mg, 1.2 mmol), and NaBH₄ (91 mg, 2.4 mmol), gave the compound of this Example (N,N'-Bis-(4-methylbenzoate)methyl-propane-1,3-diamine).

**Example 49.** Preparation af tert-butyl 4-pyridin-2-ylbenzyl(3-{(4-pyridin-2-ylbenzyl) [(1,3-thiazol-5-ylmethoxy)carbonyl]amino}propyl)carbamate

To a solution of the compound of Example 44 (110 mg, 0.27 mmol) in THF (2 mL) was added Et₃N (45 µL, 0.32 mmol) and carbonic acid 4-nitro-phenyl ester thiazol-5-ylmethyl ester hydrochloride salt (see U.S. Patent No. 5,773,625) (75 mg, 0.27 mmol) and the solution was stirred at room temperature 18 hours after which time the solvent was removed *in vacuo* and the residue dissolved in THF (4 mL). To this solution was added a solution of NaHCO₃ (45 mg, 0.54 mmol) in H₂O (1 mL) followed by di-tert-butyl dicarbonate (74 µL, 0.32 mmol) and the resulting solution was stirred at room temperature for 3 hours. H₂O (10 mL) was added and the pH was adjusted by adding 1N HCl until the pH<7 and the resulting solution was extracted with EtOAc (3 x 10 mL), the organic extracts combined, washed with brine (1 x 10 mL) and dried over Na₂SO₄. The drying agent was filtered off and the solvent was removed from the resulting filtrate *in vacuo* and the residue was purified by column chromatography on silica gel (1% CH₃OH/CHCl₃) to give 95 mg, 54% of the title compound. ¹NMR (CDCl3) δ ppm 8.61 - 8.87 (m, 2 H) 7.81 - 8.06 (m, 5 H) 7.63 - 7.80 (m, 4 H) 7.13 - 7.40 (m, 7 H) 5.36 (s, 2 H) 4.23 - 4.57 (m, 4 H) 2.97 - 3.38 (m, 4 H) 1.53 - 1.86 (m, 2 H) 1.32 - 1.53 (m, 9 H); MS M+H⁺= 650.

**Example 50.** Preparation of 1,3-thiazol-5-ylmethyl benzyl(3-{benzyl[(1,3-thiazol-5-ylmethoxy)carbonyl]amino}-2-hydroxypropyl)carbamate

To a solution of the compound of Example 42 (0.93 g, 3.4 mmol) in THF (15 mL) was added diisopropylethylamine (2.4 mL, 13.8 mmol) and carbonic acid 4-nitro-phenyl ester thiazol-5-ylmethyl ester hydrochloride salt (see U.S. Patent No. 5,773,625) (2.2 g, 6.9 mmol) and the resulting solution was refluxed for 18 hours after which time the solution was cooled and diluted with EtOAc (30 mL). This solution was washed with 5% K₂CO₃ (5 x 30 mL) and brine (1 x 30 mL), dried over Na₂SO₄, filtered, and the solvent removed from the filtrate *in vacuo* to give a crude residue that was purified by column chromatography on silica gel (2% CH₃OH/CH₂Cl₂) to give the title compound. NMR (*d6-*DMSO) δ ppm 9.10 (d, 2H) 7.93 (d, 2H) 6.99 - 7.50 (m, 10H) 5.31 (s, 4H) 4.26 - 4.70 (m, 4H) 3.80 - 4.10 (m, 1H) 3.08 - 3.37 (m, 2H) 2.78 - 3.09 (m, 2H); MS M + H⁺ = 554.

Example 51. Preparation of 1,3-thiazol-5-ylmethyl benzyl(3-{benzyl[(1,3-thiazol-5-yl methoxy)carbonyl]amino}propyl)carbamate

Following the same procedure as in Example 50, using the compound of Example 43 (1.5 g, 6 mmol), diisopropylethylamine (4.2 mL, 24 mmol) and carbonic acid 4-nitro-phenyl ester thiazol-5-ylmethyl ester hydrochloride salt (3.8 g, 12 mmol), gave the title compound. NMR (*d6*-DMSO) δ ppm 9.11 (s, 2 H) 7.94 (d, 2 H) 7.00 - 7.49 (m, 10 H) 5.33 (s, 4 H) 4.19 - 4.51 (m, 4 H) 2.94 - 3.25 (m, 4 H) 1.45 - 1.76 (m, 2 H); MS M + H⁺ = 537.

**Example 52.** Preparation of 1,3-thiazol.5-ylmethyl 4-pyridin-2-ylbenzyl(3-{(4-pyridin-2-ylbenzyl)[(1,3-thiazol-5-ylmethoxy)carbonyl]amino}propyl)carbamate

Following the same procedure as in Example 50, using compound of Example 44 (95 mg, 0.23 mmol), diisopropylethylamine (162 µL, 0.93 mmol) and carbonic acid 4-nitro-phenyl ester thiazol-5-ylmethyl ester hydrochloride salt (147 mg, 0.46 mmol), gave 11 mg, 7% of the title compound. NMR (*d6-*DMSO) δ ppm 8.97 - 9.18 (m, 2 H) 8.60 - 8.73 (m, 2 H) 7.78 - 8.13 (m, 10 H) 7.09 - 7.43 (m, 6 H) 5.34 (s, 4 H) 4.27 - 4.53 (m, 4 H) 3.04 - 3.29 (m, 4 H) 1.52 - 1.79 (m, 2 H); MS M + H⁺ = 691.

**Example 53.** Preparation of 1,3-thiazol-5-ylmethyl quinolin-3-ylmethyl(3-{(quinolin-3-yl methyl)[(1,3-thiazol-5-ylmethoxy)carbonyl]amino}propyl)carbamate

Following the same procedure as in Example 50, using the compound of Example 45 (105 mg, 0.29 mmol), diisopropylethylamine (205 µL, 1.2 mmol), and carbonic acid 4-nitro-phenyl ester thiazol-5-ylmethyl ester hydrochloride salt (186 mg, 0.59 mmol), gave 21 mg, 12% of the title compound. NMR (CDC13) δ ppm 8.58 - 8.92 (m, 3 H) 8.72 (s, I H) 8.06 - 8.25 (m, 2 H) 7.43 - 8.08 (m, 10 H) 5.36 (s,4H) 4.34 - 4.73(m,4H)3.04-3.44(m.4H) 1.59-1.96(m,2H);MS M+H⁺=639.

**Example 54.** Preparation of 1,3-thiazol-5-ylmethyl 4-(benzyloxy)benzyl(3-{[4-(benzyloxy) benzyl][(1,3-thiazol-5-ylmethoxy)carbonyl]amino}propyl)carbamate

Following the same procedure as in Example 50, using the compound of Example 46 (101 mg, 0.22 mmol), diisopropylethylamine (155 µL, 0.89 mmol), and carbonic acid 4-nitro-phenyl ester thiazol-5-ylmethyl ester hydrochloride salt (144 mg, 0.45 mmol), gave 83 mg, 5 1% of the title compound. NMR (*d6*-DMSO) δ ppm 9.07 (s, 2 H) 7.84 - 8.01 (m, 2 H) 7.21 - 7.51 (m, 9 H) 6.83 - 7.20 (m, 9 H) 5.32 (s, 4 H) 5.06 *(*s*,* 4H) 4.11 - 4.36 (m, 4 H) 2.91 - 3.17 (m, 4H) 1.46 - 1.70 (m, 2 H); MS M + H⁺= 749.

**Example 55.** Preparation of 1,3-thiazol-5-ylmethyl cyclohexylmethyl(3-{(cyclohexylmethyl) [(1,3-thiazol-5-ylmethoxy)carbonyl]amino}propyl)carbamate

Following the same procedure as in Example 50, using the compound of Example 47 (0.3 mmol), diisopropylethylamine (209 µL, 1.20 mmol), and carbonic acid 4-nitro-phenyl ester thiazol-5-ylmethyl ester hydrochloride salt (190 mg, 0.6 mmol), gave 43 mg, 26% of the title compound. NMR (*d6*-DMSO) δ ppm 9.08 (s, 2 H) 7.92 (s, 2 H) 5.28 (s, 4 H) 2.84 - 3.26 (m, 8 H) 1.39 - 1.80 (m, 14 H) 0.98 - 1.30 (m, 6 H) 0.66 - 0.96 (m, 4 H); MS M + H⁺ = 549.

**Example 56.** Preparation of methyl 4-({(3-{[4-(methoxycarbonyl)benzyl][(1,3-thiazol-5-yl methoxy)carbonyl]amino} propyl)[(1,3-thiazol-5-ylmethoxy)carbonyl]amino}methyl) benzoate

Following the same procedure as in Example 50, using the compound of Example 48 (0.3 mmol), diisopropylethylamine (209 µL, 1.20 mmol), and carbonic acid 4-nitro-phenyl ester thiazol-5-ylmethyl ester hydrochloride salt (190 mg, 0.6 mmol), gave the title compound. NMR (CD30D) δ ppm 8.81 - 9.10 (m, 2 H) 7.78 - 8.06 (m, 6 H) 7.08 - 7.40 (m, 4 H) 5.36 (s, 4 H) 4.30 - 4.58 (m, 4 H) 3.31 (s, 6H) 3.04 - 3.39 (m,4H) 1.53 - 1.84 (m, 2H); MS M+H⁺=653.

**Example 57.** Preparation of 1,3-thiazol-5-ylmethyl benzyl(3- {benzyl[(1,3-thiazol-5-yl methoxy)carbonyl]amino}-2-oxopropyl)carbamate

To a solution of 2M oxalyl chloride in CH₂Cl₂ (1.1 mL, 2.2 mmol) at-78°C was added a solution of DMSO (0.21 mL, 2.9 mmol) in CH₂Cl₂ (2 mL) and stirring was continued for 20 minutes. To this solution was added a solution of 1,3-thiazol-5-ylmethyl benzyl(3-{benzyl[(1,3-thiazol-5-yl methoxy) carbonyl]amino}-2-hydroxypropyl)carbamate (0.80 g, 1.45 mmol) in CH₂Cl₂ (2 mL) and stirring was continued for 20 minutes at-78°C. To this solution was added a solution of Et₃N (0.81 mL, 5.8 mmol) in CH₂Cl₂ (2 mL) and stirring was continued at-78°C for 20 minutes after which time the temperature was allowed to warm to room temperature and H₂O (5 mL) was added. The solution was washed with H₂O (3 x 5 mL), dried over Na₂SO₄, filtered and the solvent removed *in vacuo* to yield a crude residue which was purified by column chromatography on silica gel (2% CH₃OH/CH₂Cl₂) to give the title compound. NMR (*d6*-DMSO) δ ppm 9.10 (s, 2 H) 7.83 -7.98 (m, 2 H) 7.03 - 7.43 (m, 10 H) 5.17 - 5.43 (m, 4 H) 4.24 - 4.48 (m, 4 H) 3.97 - 4.24 (m, 4 H); MS M + H⁺ = 551.

**Example 58.** Preparation of 1,3-thiazol-5-ylmethyl benzyl(3-{benzyl[(1,3-thiazol-5-yl methoxy)carbonyl]amino}-2-aminopropyl)carbamate

To a solution of 1,3-thiazol-5-ylmethyl benzyl(3-{benzyl[(1,3-thiazol-5-ylmethoxy)carbonyl] amino}-2-hydroxypropyl)carbamate (1.94 g, 3.5 mmol) in CH₂Cl₂ at 0°C was added Et₃N (0.73 mL, 5.3 mmol) and methanesulfonyl chloride (0.33 mL, 4.2 mmol) and stirring was continued at 0°C for 1 hour after which time the solution was allowed to warm to room temperature over 30 minutes, washed with 1N HCl (1 x 15 mL), H₂O (1 x 15 mL), 10% NaHCO₃ (1 x 15 mL) and brine (1 x 15 mL), dried over Na₂SO₄, filtered and the solvent removed *in vacuo.* A portion of this residue (0.32 g, 0.5 mmol) was dissolved in DMF (3 mL) and NaN₃ (0.33 g, 5.1 mmol) was added and the solution was stirred at 80°C for 24 hours, after which time the solution was cooled, diluted with H₂O (8 mL) and extracted with EtOAc (3 x 10 mL). The combined organic extracts were dried over Na₂SO₄, filtered and the solvent removed *in vacuo.* The residue was dissolved in CH₃OH (4 mL) and tin (II) chloride dihydrate (126 mg, 0.56 mmol) was added and the solution was stirred vigorously for I hour at room temperature. Another 126 mg of tin (II) chloride dihydrate was added and the solution was stirred at room temperature for 18 hours after which time 10% NaHCO₃ (1 mL) was added and the solution was filtered through Celite and the solvent removed *in vacuo.* The crude residue was dissolved in CH₂Cl₂ (5 mL), washed with brine (1 x 5 mL), dried over Na₂SO₄, filtered and the solvent removed *in vacuo* to give 29 mg, 10% of the compound of this Example.

**Example 59.** Preparation of 1,3-thiazol-5-ylmethyl benzyl{3-{benzyl[(1,3-thiazol-5-yl methoxy)carbonyl]amino}-2-[(phenoxycarbonyl)amino]propyl}carbamate

To a solution of the compound of Example 58 (29 mg, 0.04 mmol) in CH₂Cl₂ (1 mL) was added diisopropylethylamine (36 µL, 0.21 mmol) followed by phenylacetyl chloride (6 µL, 0.05 mmol) and stirring was continued at room temperature 18 hours, after which time the solvent was removed *in vacuo,* the residue was dissolved in EtOAc (5 mL), washed with brine (1 x 5mL), dried over Na₂SO₄, filtered and the solvent removed *in vacuo* to leave a crude residue which was purified by column chromatography on silica gel (1% CH₃OH/.CH₂Cl₂) to give 12 mg, 43% of the title compound. NMR (CDCl3) δ ppm 8.74 (s, 2 H) 7.83 (s, 2 H) 7.10 - 7.41 (m, 12 H) 6.89 - 7.11 (m, 3 H) 5.09 - 5.36 (m, 4 H) 4.42 (q, 2 H) 3.91 - 4.31 (m, 3 H) 3.42 - 3.58 (m, 1 H) 3.29 - 3.42 (m, 2 H) 3.06 - 3.26 (m, 2 H); MS M+H⁺ = 670.

**Example 60.** Preparation of 1,3-thiazol-5-ylmethyl benzyl[3-{benzyl[(1,3-thiazol-5-yl methoxy)carbonyl]amino}-2-(isobutyrylamino)propyl]carbamate

Following the same procedure as in Example 59, using the compound of Example 58 (29 mg, 0.04 mmol), diisopropylethylamine (36 µL, 0.21 mmol) and isobutyryl chloride (5 µL, 0.05 mmol), gave 7 mg, 27% of the title compound. NMR (CDCl3) δ ppm 8.75 (s, 2 H) 7.83 (s, 2 H) 7.16 - 7.42 (m, 7 H) 6.92 - 7.17 (m, 3 H) 5.32 (s, 4 H) 4.04 - 4.69 (m, 4 H) 3.48 - 3.68 (m, 1 H) 3.05 - 3.43 (m, 2 H) 1.60 (s, 3 H) 0.93 - 1.13 (m, 6 H); MS (M + H⁺) = 622.

**Example 61.** Preparation of 1,3-thiazol-5-ylmethyl benzyl[3-{benzyl[(1,3-thiazol-5-yl methoxy)carbonyl]amino}-propyl]carbamate

Following the same procedure as in Example 50, using 1,3-diaminopropane (100 µL, 1.2 mmol), diisopropylethylamine (0.84 mL, 4.8 mmol) and carbonic acid 5-methylthiazole ester 4-nitrophenyl ester hydrochloride (759 mg, 2.4 mmol), gave 184 mg, 43% of the compound of the Example. NMR (*d6*-DMSO) δ ppm 9.08 (s, 2 H) 7.92 (s, 2 H) 7.28 (t, 2 H) 5.24 (s, 4 H) 2.87 - 3.08 (m, 4 H) 1.43 - 1.61 (m, 2 H); MS (M + H⁺) = 357.

**Example 62,** Preparation of 1,3-thiazol-5-ylmethyl 4-benzoylbenzyl(3-{(4-benzoylbenzyl) [(1,3-thiazol-5-ylmethoxy)carbonyl]amino}propyl)carbamate

To a solution of the compound of Example 61 (35 mg, 0.10 mmol) in DMF (1 mL) was added 4-(bromomethyl)benzophenone (62 mg, 0.23 mmol). Sodium hydride (10 mg, 0.24 mmol) was added and the solution stirred at room temperature for 18 hours, after which time was poured into 1:1 EtOAc:1N HCl (10 mL). The layers were separated and the organic layer was washed with 1N HCl (1 x 5 mL) and brine (1 x 5 mL), dried over Na₂SO₄, filtered and the solvent removed *in vacuo* to leave a crude residue that was purified by column chromatography on silica gel (1-2% CH₃OH/CH₂Cl₂) to give 11 mg, 15% of the title compound. NMR (CD3OD) δ ppm 8.91- 9.06 (m, 2 H) 7.15 - 8.00 (m, 20 H) 5.43(s, 2 H) 5.38 (s, 2 H) 4.36 - 4.64 (m, 4 H) 3.11 - 3.44 (m, 4 H) 1.61 - 1.86 (m, 2 H); MS (M + H⁺) = 745.

**Example 63.** Preparation of 1,3-thiazol-5-ylmethyl 4-methoxybenzyl(3-{(4-methoxybenzyl) [(1,3-thiazol-5-ylmethoxy)carbonyl]amino}propyl)carbamate

Following the same procedure as in Example 62, using the compound of Example 61 (30 mg, 0.08 mmol), sodium hydride (8 mg, 0.19 mmol) and 4-methoxybenzyl bromide (27 µL, 0.19 mmol), gave 22.6 mg, 45% of the title compound. NMR (CDCl3) δ ppm 8.78 (s, 2 H) 7.87 (s, 2 H) 6.72 - 7.20 (m, 8 H) 5.34 (s, 4 H) 4.15 - 4.42 (m, 4 H) 2.95 - 3.29 (m, 4 H) 1.61 (s, 6 H) 1.45 - 1.79 (m, 2 H); MS (M + H⁺) = 597.

**Example 64.** Preparation of 1,3-thiazol-5-ylmethyl 4-tert-butylbenzyl(3-{(4-tert-butylbenzyl) [(1,3-thiazol-5-ylmethoxy)carbonyl]amino}propyl)carbamate

Following the same procedure as in Example 62, using the compound of Example 61 (30 mg, 0.08 mmol), sodium hydride (8 mg, 0.19 mmol) and 4-*tert*-butylbenzyl bromide (34 µL, 0.19 mmol), gave 17.3 mg, 32% of the title compound. NMR (CDCI3) δ ppm 8.78 (s, 2 H) 7.86 (s, 2 H) 6.90 - 7.39 (m, 8 H) 5.34 (s,4H) 4.16-4.45 (m,4H) 2.97-3.29 (m,4H) 1.60 (s,2H) 1.30 (s,18H); MS (M+H⁺)= 649.

**Example 65.** Preparation of 1,3-thiazol-5-ylmethyl 1,1'-biphenyl-4-ylmethyl(3-{(1,1'-biphenyl-4-ylmethyl)[(1,3-thiazol-5-ylmethoxy)carbonyl]amino}propyl)carbamate

Following the same procedure as in Example 62, using the compound of Example 61 (30 mg, 0.08 mmol), sodium hydride (8 mg, 0.19 mmol) and 4-phenylbenzyl bromide (46 mg, 0.19 mmol), gave 14.6 mg, 25% of the title compound. NMR (CDCI3) δ ppm 8.76 (s, 2 H) 7.87 (s,2 H) 7.03 - 7.65 (m, 18 H) 5.36 (s, 4 H) 4.25 - 4.56 (m, 4 H) 3.03 - 3.40 (m, 4 H) 1.61 (s, 2 H); MS (M + H⁺) =:: 689.

Example 66. Preparation of 1,3-thiazol-5-ylmethyl 3-{[(1,3-thiazol-5-ylmethoxy)carbonyl] [4-(1H-1,2,4-triazol-1-yl)benzyl]amino}propyl[4-(1H-1,2,4-triazol-1-yl)benzyl]

Following the same procedure as in Example 62, using the compound of Example 61 (30 mg, 0.08 mmol), sodium hydride (8 mg, 0.19 mmol) and 1-[4-(bromomethyl)phenyl]-1H-1,2,4-triazole (44 mg, 0.19 mmol), gave 25 mg, 44% of the title compound. NMR (CDCI3) δ ppm 8.85 (s, 2 H) 8.63 (s, 2 H) 8.15 (s, 2 H) 7.84-7.96 (m, 2 H) 7.55 - 7.70 (m, 4 H) 7.10 - 7.43 (m, 4 H) 5.36 (s, 4 H) 4.29 - 4.58 (m,4H) 3.07-3.38 (m,4H) 1,58-1.87 (m,2H); MS (M+H⁺)= 671.

**Example 67.** Preparation of ethyl N-[(1,3.thiazol-5-ylmethoxy)carbonyl]-N-(3-{[(1,3-thiazol-5-ylmethoxy)carbonyl]amino}propyl)glycinate

Following the same procedure as in Example 62, using the compound of Example 61 (31 mg, 0.08 mmol), sodium hydride (8 mg, 0.19 mmol) and ethyl bromoacetate (21 µL, 0.19 mmol), gave a mixture of the title compound and ethyl N-(3-{(ethoxycarbonylmethyl)[(1,3-thiazol-5-ylmethoxy)carbonyl]amino}propyl)-N-[(1,3-thiazol-5-ylmethoxy)carbonyl]glycinate, which were separated by prep HPLC on a C18 silica gel column (7:3 CH₃OH:NH₄OH, 0.4 mL,min). The title compound (9.6 mg, 25%): NMR (CDCI3) δ ppm 8.79 - 8.80 (m, 2 H) 7.85 - 7.88 (m, 2 H) 5.21- 5.41 (m, 4 H) 4.05 - 4.27 (m, 2 H) 3.83 - 4.00 (m, 2 H) 3.12 - 3,47 (m, 4 H) 1.55 - 1.85 (m, 2 H) 1.12 -1.32 (m, 3 H); MS (M + H⁺) = 443.

**Example 68.** Preparation of ethyl N-(3-{(ethoxycarbonylmethyl)[(1,3-thiazol-5-yl methoxy)carbonyl]amino}propyl-N-[(1,3-thiazol-5-ylmethoxy)carbonyl]glycinate

The title compound (10.1 mg, 22%) was isolated by HPLC as described in Example 67. NMR (CDCI3) δ ppm 8.79 - 8.80 (m, 2 H) 7.79 - 7.92 (m, 2 H) 5.30 (s, 1 H) 5.35 (s, 1 H) 4.06 - 4.31 (m, 4 H) 3.94 (t, 4 H) 3.22 - 3.45 (m, 4 H) 1,61 - 1.90 (m, 4 H) 1.14 - 1.35 (m, 6 H); MS (M + H⁺) = 529,

**Example 69.** Preparation of tert-butyl (3-{(1,3-thiazol-5-ylmethoxy)carbonylamino}propyl) carbamate

Following the same procedure as for the compound of Example 61, using N-Boc-1,3-propanediamine (0.47 g, 2.7 mmol), diisopropylethylamine (0.47 mL, 2.7 mmol), and carbonic acid 5-methylthiazole ester 4-nitrophenyl ester hydrochloride (0.76 g, 2.7 mmol), gave 0.32 g, 38% of the compound of this Example.

**Example 70.** Preparation of tert-butyl benzyl(3-{benzyl[(1,3-thiazol-5-ylmethoxy)carbonyl] amino}propyl)carbamate

Following the same procedure as in Example 62, using the compound of Example 69 (100 mg, 0.32 mmol), sodium hydride (26 mg, 0.63 mmol) and benzyl bromide (75 µL, 0.63 mmol), gave 66 mg, 42% of the title compound. NMR (CDCI3) δ ppm 8.78 (s, I H) 7.87 (s, 1 H) 7.04 - 7.45 (m, 10 H) 5.35 (s, 2 H) 4.19 - 4.51 (m,4 H) 2.94 - 3.33 (m, 4 H) 1.51 - 1.82 (m, 2 H) 1.43 (s, 9 H); MS (M + H⁺)= 496.

**Example 71.** Preparation of tert-butyl 3-{[(1,3-thiazol-5-ylmethoxy)carbonyl][4-(1H-1,2,4-triazol-1-yl)benzyl]amino}propyl[4-(1H-1,2,4-triazol-1-yl)benzyl]carbamate

Following the same procedure as in Example 62, using the compound of Example 69 (44 mg, 0.14 mmol), sodium hydride (12 mg, 0.31 mmol) and 1-[4-(bromomethyl)-phenyl]-1H-1,2,4-triazole (73 mg, 0.31 mmol) gave 42 mg, 47% of the title compound. NMR (*d6*-DMSO) δ ppm 9.26 (s, 2 H) 8.98 - 9.16 (m, 1 H) 8.23 (s, 2 H) 7.87 - 8.02 (m, 1 H) 7.70 - 7.89 (m, 4 H) 7.20 - 7.50 (m, 4 H) 5.36 (s, 2H) 4.21 - 4.55 (m, 4 H) 2.94 - 3.26 (m, 4 H) 1.55 -1.77 (m, 2 H) 1.33 (s, 9 H); MS (M + H⁺) = 630.

**Example 72.** Preparation of tert-butyl 4-methoxybenzyl(3-{(4-methoxybenzyl)[(1,3-thiazol-5-ylmethoxy)carbonyl]amino}propyl)carbamate

Following the same procedure as in Example 62, using the compound of Example 69 (47 mg, 0.15 mmol), sodium hydride (13 mg, 0.30 mmol) and 4-methoxybenzyl bromide (47 µL, 0.30 mmol) gave the title compound. NMR (CDCI3) δ ppm 8.79 (s, 1 H) 7.87 (s, 1H) 6.96 - 7.23 (m, 4 H) 6.73 - 6.96 (m, 4 H) 5.35 (s, 2 H) 4.12 - 4.43 (m, 4 H) 2.92 - 3.26 (m, 4 H) 1.58 (s, 6 H) 1.43 (s, 9 H) 1.16 -1.79 (m, 2 H); MS (M + H⁺) = 556.

**Example 73.** Preparation of tert-butyl 4-benzoylbenzyl(3-{(4-benzoylbenzyl)[(1,3-thiazol-5-ylmethoxy)carbonyl]amino}propyl)carbamate

Following the same procedure as in Example 62, using the compound of Example 69 (49 mg, 0.16 mmol), sodium hydride (14 mg, 0.32 mmol) and 4-(bromomethyl)-benzophenone (95 mg, 0.32 mmol) gave 31 mg, 28% of the title compound. NMR(CDCI3) δ ppm 8.72 - 8.85 (m, 1 H) 7.13 - 7.93 (m, 19 H) 5.32 - 5.43 (m, 2 H) 4.31 - 4.60 (m, 4 H) 3.05 - 3.37 (m, 4 H) 1.62 - 1.87 (m, 2 H) 1.57 (s, 9 H); MS (M + H⁺) = 704.

**Example 74.** Preparation of methyl 4-({(3-{(tert-butoxycarbonyl)[4-(methoxycarbonyl) benzyl]amino}propyl)[(1,3-thiazol-5-ylmethoxy)carbonyl]amino}methyl)benzoate

Following the same procedure as in Example 62, using the compound of Example 69 (39 mg, 0.13 mmol), sodium hydride (11 mg, 0.26 mmol) and methyl 4-(bromomethyl)benzoate (63 mg, 0.26 mmol) gave 10 mg, 13% of the title compound. NMR (CDCI3) δ ppm 8,73- 8.85 (m, 1 H) 7.78 - 8.12 (m, 7 H) 7.38 - 7.51 (m, 2 H) 5.29 - 5.42 (m, 2 H) 4.22 - 4.55 (m, 4 H) 3.00 - 3.34 (m, 4 H) 1.59 (s, 6 H) 1.51 - 1.83 (m, 2H) 1.40 (s, 9H); MS (M+H⁺) =611.

**Example 75.** Preparation of tert-butyl 1,1'-biphenyl-4-ylmethyl(3-{(1-1'-biphenyl-4-yl methyl) [(1,3-thiazol-5-ylmethoxy)carbonyl]amino}propyl)carbamate

Following the same procedure as in Example 62, using the compound of Example 69 (64 mg, 0.20 mmol), sodium hydride (18 mg, 0.45 mmol) and 4-phenylbenzyl bromide (111 mg, 0.45 mmol) gave 63 mg, 48% of the title compound. NMR (CDCI3) δ ppm 8.76 (s, 1 H) 7.87 (s, 1 H) 7.10 - 7.67 (m, 18 H) 5.37 (s, 2 H) 4.24 - 4.55 (m, 4 H) 3.02 - 3.37 (m, 4 H) 1.60 - 1.88 (m, 2 H) 1.44 (s, 9 H); MS (M + H⁺) = 648.

**Example 76.** Preparation of benzyl 3-{benzyl[(1,3-thiazol-5-ylmethoxy)carbonyl]amino} propane

To a solution of tert-butyl benzyl(3-{benzyl[(1,3-thiazol-5-ylmethoxy)carbonyl]amino} propyl)carbamate (66 mg, 0.13 mmol) in THF (2 mL) was added 4M HCl/dioxane (2 mL) and the solution was stirred at room temperature for 1 hour, after which time the solvent was removed *in vacuo* and the residue dissolved in EtOAc (5 mL), washed with 10% NaHCO₃ (2 x 5 mL), brine (1 x 5 mL), dried over Na₂SO₄, filtered and the solvent removed *in vacuo* to give the compound of this Example.

**Example 77.** Preparation of benzyl benzyl(3-{benzyl[(1,3-thiazol-5-ylmethoxy)carbonyl] amino}propyl)carbamate

To a solution of the compound of Example 76 (0.04 mmol) in THF (2 mL) was added diisopropylethylamine (13 µL, 0.07 mmol) and benzyl chloroformate (7 µL, 0.05 mmol) and the solution was stirred at room temperature for 2 hours, after which time it was poured into EtOAc, (5 mL), washed with 1N HCl (1 x 5 mL), brine (1 x 5 mL), dried over Na₂SO₄, filtered and the solvent removed *in vacuo* to give a crude residue which was purified by column chromatography on silica gel (5% CH₃OH/CH₂Cl₂) to give 7 mg, 37% of the title compound. NMR (*d6*-DMSO) δ ppm 9.07 (s, 1H) 7.82 - 8.01 (m, 1 H) 6.98 - 7.51 (m, 15 H) 5.32 (s, 2 H) 5.08 (s, 2 H) 4.18-4.50 (m, 4 H) 2.96-3.23 (m, 4 H) 1.48-1.77 (m, 2 H); MS (M + H⁺) = 530.

**Example 78.** Preparation of methyl benzyl(3-{benzyl[(1,3-thiazol-5-ylmethoxy)carbonyl] amino}propyl)carbamate

Following the same procedure as in Example 77, using tert-butyl 1,1'-biphenyl-4-ylmethyl(3-{(1,1'-biphenyl-4-ylmethyl)[(1,3-thiazol-5-ylmethoxy)carbonyl]amino}propyl)carbamate (0.04 mmol), diisopropylethylamine (13 µL, 0.07 mmol) and methyl chloroformate (3 µL, mmol), gave 9 mg, 54% of the title compound. NMR (CDCI3) δ ppm 8.78 (s, 1 H) 7.76 - 7.94 (m, 1H) 6.98 - 7.47 (m, 10 H) 5.35 (s, 2 H) 4.22 - 4.52 (m, 4 H) 2.95 - 3.34 (m, 4 H) 1.48 - 1.86 (m, 2 H) 1.58 (s, 3 H); MS (M + H⁺) = 454.

**Example 79.** Preparation of 1,3-thiazol-5-ylmethyl benzyl(3-[bonzyl(pyridin-4-ylmethyl) amino]propyl) carbamate

To a solution of tert-butyl 1,1'-biphenyl-4-ylmethyl(3-{(1,1'-biphenyl-4-ylmethyl) [(1,3-thiazol-5-ylmethoxy)carbonyl]amino}propyl)carbamate (0.04 mmol) in 1,2-dichloroethane (2 mL) was added 4-pyridinecarboxaldehyde (8 µL, 0.08 mmol) followed by sodium triacetoxyborohydride (23 mg, 0.11 mmol) and acetic acid (5 µL, 0.08 mmol) and the solution was stirred for 18 hours at room temperature, after which time was added 10% NaHCO₃ (5 mL) and the reaction extracted with CH₂CL₂ (3 x 5 mL), the organic extracts combined, washed with brine (1 x 10 mL), dried over Na₂SO₄, filtered and the solvent removed *in vacuo* to yield a crude residue that was purified by column chromatography on silica gel (5% CH₃OH/CH₂Cl₂) to give 14 mg, 77% of the title compound. NMR (*d6*-DMSO) δ ppm 9.08 (s, 1 H) 8.40 - 8.57 (m, 2 H) 7.89 (s, 1 H) 7.06 - 7.50 (m, 13 H) 5.32 (s, 2 H) 4.26 - 4.45 (m, 2 H) 3.36 - 3.56 (m, 4 H) 3.03-3.23 (m, 2 H) 2.15 - 2.39 (m, 2 H) 1.50 - 1.73 (m, 2 H); MS (M + H⁺) = 487.

**Example 80.** Preparation of 1,3-thiazol-5-ylmethyl benzyl[3-(benzyl{4-[3-(dimethylamino) propoxy]benzyl}amino)propyl]carbamate

Following the same procedure as in Example 79, using tert-butyl 1,1'-biphenyl-4ylmethyl(3-{(1,1'-biphenyl-4-ylmethyl) [(1,3-thiazol-5-ylmethoxy)carbonyl]amino}propy)carbamate (0.04 mmol), 4-[3-(dimethylamino)propoxy]benzaldehyde (17 µL, 0.08 mmol), NaHB(OAc)₃ (23 mg, 0.11 mmol) and HOAc (5 µL, 0.08 mmol), gave 5 mg, 23% of the title compound. NMR (*d6*-DMSO) δ ppm 9.86 (s, 2 H) 9.08 (s, 1H) 7.76 - 7,98 (m, 3 H) 7.02 - 7.39 (m, 10 H) 5.32 (s, 2 H) 4.23 - 4.43 (m, 2 H) 4.12 (t, 2 H) 3.95 (t, 2 H) 2.95 - 3.41 (m, 6 H) 2.38 (t, 2 H) 2.16 (s, 6 H) 1.72 - 1.96 (m, 2 H) 1.48 - 1.72 (m, 2 H); MS (M + H⁺) = 587,

**Example 81.** Preparation of 1,3-thiazol-5-ylmethyl benzyl{3-[benzyl(4-pyridin-2-ylbenzyl) amino]propyl}carbamate

Following the same procedure as in Example 79, using tert-butyl 1,1'-biphenyl-4-ylmethyl(3-{(1,1'-biphenyl-4-ylmethyl) [(1,3-thiazol-5-ylmethoxy)carbonyl]amino}propyl)carbamate (0.04 mmol), 4-(2-pyridyl)benzaldehyde (15 mg, 0.08 mmol), NaHB(OAc)₃ (23 mg, 0.11 mmol) and HOAc (5 µL, 0.08 mmol), gave 17 mg, 84% of the title compound. NMR (*d6*-DMSO) δ ppm 9.06 (s, 1 H) 8.57 - 8.76 (m, 1 H) 7.78 - 8.09 (m, 5 H) 7.02 - 7.49 (m, 13 H) 5.32 (s, 2 H) 4.25 - 4.46 (m, 2 H) 3.38 - 3.63 (m, 4 H) 3.02 - 3.24 (m, 2 H) 2.19 - 2.42 (m, 2 H) 1.48 - 1.79 (m, 2 H); MS (M + H⁺) = 563.

**Example 82.** Preparation of 1,3-thiazol-5-ylmethyl benzyl{3-[benzyl(neopentyl)amino] propyl}carbamate

Following the same procedure as in Example 79, using tert-butyl 1,1'-biphenyl-4-ylmethyl(3-{(1,1'-biphenyl-4-ylmethyl) [(1,3-thiazol-5-ylmethoxy)carbonyl]aminolpropyl)carbamate (25 mg, 0.06 mmol), trimethylacetaldehyde (16 µL, 0.14 mmol), NaHB(OAc)₃ (40 mg, 0.19 mmol) and HOAc (8 µL, 0.14 mmol), gave 5 mg, 17% of the title compound, NMR (CDCI3) δ ppm 8.82 (s, I H) 7.89 (s, 1 H) 7.06 - 7.52 (m, 10H) 5.39 (s,2H)4,35-4.60 (m,2H) 4.06-4.37 (m,2H) 3,19-3.39 (m,2H) 2.56-3.20 (m, 2 H) 1.53 - 2.21 (m, 4 H) 1.00 (s, 9 H); MS (M + H⁺) = 466.

**Example 83.** Preparation of 1,3-thiazol-5-ylmethyl benzyl{3-[benzyl(2-naphthylmethyl amino]propyl}carbamate

Following the same procedure as in Example 79, using tert-butyl 1,1'-biphenyl-4-ylmathyl(3-{(1,1'-biphenyl-4-ylmethyl) [(1,3-thiazol-5-ylmethoxy)carbonyl]amino}propyl)carbamate (25 mg, 0.06 mmol), 2-naphthaldehyde (23 mg, 0.14 mmol), NaHB(OAc)₃ (40 mg, 0.19 mmol) and HOAc (8 µL, 0.14 mmol), gave 5 mg, 15% of the title compound. NMR (CDCI3) δ ppm 8.69 - 8.85 (m, 1 H) 7.76 - 8.01 1 (m, 6 H) 7.14 - 7.68 (m, 12 H) 5.28 (s, 2 H) 4.05 - 4.48 (m, 6 H) 3.04 - 3.26 (m, 2 H) 2.66 - 2.98 (m, 2 H) 1.43 - 2.19 (m, 2 H); MS (M + H⁺) = 536.

**Example 84.** Preparation of 1,3-thiazol-5-ylmethyl benzyl{3-[benzyl(2-furylmethyl)amino] propyl}carbamate

Following the same procedure as in Example 79, using tert-butyl 1,1'-biphenyl-4-ylmethyl(3-{(1,1'-biphenyl-4-ylmethyl) [(1,3-thiazol-5-ylmethoxy)carbonyl]amino}propyl)carbamate (25 mg, 0.06 mmol), 2-furaldehyde (12 µL, 0.14 mmol), NaHB(OAc)₃(40 mg, 0.19 mmol) and HOAc (8 µL, 0.14 mmol), gave 5 mg, 17% of the title compound. NMR (CDCI3) δ ppm 8.81 (s, 1 H) 7.88 (s, 1 H) 7.00 - 7.58 (m, 13 H) 5.36 (s, 2 H) 4.31 - 4.53 (m, 2 H) 4.14 (t, 4 H) 3.11 - 3.34 (m, 2 H) 2.59-2.99 (m, 2 H) 1.87 - 2.22 (m, 2 H); MS (M + H⁺) = 476.

**Example 85.** Preparation of 1,3-thiazol-5-ylmethyl benzyl(3-{benzyl[(5-methylthien-2-yl) methyl]amino}propyl)carbamate

Following the same procedure as in Example 79, using tert-butyl 1,1'-biphenyl-4-ylmethyl(3-{(1,1'-biphenyl-4-ylmethyl) [(1,3-thiazol-5-ylmethoxy)carbonyl]amino}propyl)carbamate (25 mg, 0.06 mmol), 5-methyl-2-thiophenecarboxaldehyde (16 µL, 0.14 mmol), NaHB(OAc)₃(40 mg, 0.19 mmol) and HOAc (8 µL, 0.14 mmol), gave 5 mg, 16% of the title compound. NMR (CDCI3) δ ppm 8.81 (s, 1 H) 7.88 (s, 1 H) 6.67 - 7.60 (m, 12 H) 5.36 (s, 2 H) 4.29 - 4.50 (m, 2 H) 3.97 - 4.33 (m, 4 H) 3.09 - 3.29 (m, 2 H) 2.65-2.97 (m, 2 H) 2.51 (s, 3H) 1.41 - 2.13 (m, 2 H); MS (M + H⁺)= 506.

**Example 86.** Preparation of tert-butyl (1S,2R)-1-benzyl-2-hydroxy-3-{isobuty[(1,3-thiazol-5-ylmethoxy)carbonyl]amino}propylcarbamate

Following the same procedure as in Example 50, using (1-benzyl-2-hydroxy-3-isobutylamino propyl) carbamic acid *tert*-butyl ester) (46 mg, 0.14 mmol, WO 2005061487), Et₃N (23 µL, 0.17 mmol) and carbonic acid 5-methylthiazole ester 4-nitrophenyl ester hydrochloride (48 mg, 0.15 mmol), gave 39.6mg,61% of the title compound. NMR (CDCl3) δ ppm 8.81 (s, 1H) 7.88 (s, 1 H) 7.09-7.41 (m, 5 H) 5.34 (s, 2 H) 4.13 - 4.72 (m, 2 H) 3.77 (s,2 H) 2.72 - 3.62 (m, 6 H) 1.35 (s, 9 H) 0.80 - 0.82 (d, 6 H); MS (M + H+) = 478.

**Example 87.** Preparation of tert-butyl (1S,2S)-1-benzyl-3-(benzyl[(1,3-thiazol-5-ylmethoxy) carbonyl]amino)-2-hydroxypropylcarbamate

Following the same procedure as in Example 50, using (1-benzyl-2-hydroxy-3-isobutylamino propyl) carbamic acid *tert*-butyl ester) (0.19 mmol), Et₃N (32 µL, 0.23 mmol) and carbonic acid 5-methylthiazole ester 4-nitrophenyl ester hydrochloride (66 mg, 0.21 mmol), gave the title compound. NMR (CDCl3) δ ppm 8.80 (s, 1H) 7.85 (s, 1H) 6.97 - 7.38 (m, 10 H) 5.37 (s, 2 H) 4.67 - 5.01 (m, 1 H) 4.29 - 4.61 (m, 2 H) 3.49 - 3.79 (m, 2 H) 2.98 - 3.30 (m, 1 H) 2.66 - 2.96 (m, 2 H) 1.37 (s, 9 H); MS (M +H+) = 512.

**Example 88.** Preparation of tert-butyl (1S,2R)-1-benzyl-3-{benzyl[(1,3-thiazol-5-ylmethoxy) carbonyl] amino}-2-hydroxypropylcarbamate

Following the same procedure as in Example 50, using (1-benzyl-2-hydroxy-3-isobutyl aminopropyl) carbamic acid *tert*-butyl ester) (0.19 mmol), Et₃N (32 µL, 0.23 mmol) and carbonic acid 5-methylthiazole ester 4-nitrophenyl ester hydrochloride (59 mg, 0.21 mmol), gave 42 mg, 41% of the title compound. NMR (CDCl3) δ ppm 8.80 (s, I H) 7.88 (s, 1 H) 7.00-7.38 (m, 10 H) 5.40 (s, 2 H) 4.34 - 4.61 (m, 3H) 3.63-3.87 (m, 2 H) 3.27-3.50 (m, 2 H) 2.64-3.02 (m, 2 H) 1.33 (s, 9 H); MS (M + H+) =512.

**Example 89.** Preparation of tert-butyl (1S,2R)-1-benzyl-2-hydroxy-3-[isobutyl(4-pyridin-2-ylbenzyl)amino]propylcarbamate

Following the same procedure as in Example 79, using (1-benzyl-2-hydroxy-3-isobutyl aminopropyl) carbamic acid *tert*-butyl ester) (66 mg, 0.20 mmol), 2-pyridylbenzaldehyde (83 mg, 0.45 mmol), sodium triacetoxyborohydride (126 mg, 0.59 mmol) and acetic acid (26 µL, 0.45 mmol), gave 40 mg, 40% of the compound of this Example. NMR (CDCI3) δ ppm 8.63 - 8.76 (m, 1 H) 7.87 - 8.09 (m, 2 H) 7.66-7.82 (m, 2 H) 7.08 - 7.47 (m, 9 H) 4.42 - 4.62 (m, 1 H) 3.50 - 3.89 (m, 3 H) 3.30 - 3.49 (m, 1 H) 2.69-2.99 (m, 2 H) 2.37-2.66 (m, 2 H) 2.14-2.29 (m, 2 H) 1.72-1.95 (m, 1 H) 1.34 (s, 9 H) 0.75 - 0.98 (m, 6 H); MS (M + H+) = 504.

**Example 90.** Preparation of 1,3-thiazol-5-ylmethyl (1S,2R)-1-benzyl-2-hydroxy-3-[isobutyl (4-pyridin-2-ylbenzyl)amino]propylcarbamate

To the compound of Example 89 (39 mg, 0.08 mmol) was added 4M HCl in dioxane (2 mL) and the solution was stirred at room temperature for 1 hour, after which time the solvent was removed *in vacuo* and the residue was dissolved in THF (2 mL) and to this solution was added Et₃N (13 µL, 0.09 mmol) and carbonic acid 5-methylthiazole ester 4-nitrophenyl ester hydrochloride (24 mg, 0.09 mmol) and the solution treated in the same manner as in the preparation of the title compound of Example 50 to give 24 mg, 59% of the title compound. NMR (CDCI3) δ ppm 8.77 (s, 1 H) 8.66 - 8.75 (m, 1 H) 7.88-8.05 (m, 2 H) 7.64 - 7.86 (m, 3 H) 7.04 - 7.47 (m, 9 H) 5.19 (s, 2 H) 3.73 - 3.93 (m, 2 H) 3.53 - 3.76 (m, 2 H) 3.30 - 3.48 (m, I H) 2.70-2.97 (m, 2 H) 2.34-2.64 (m, 2 H) 2.14-2.32 (m, 2 H) 1.69 - 1.94 (m, 1 H) 0.75 - 0.99 (m, 6 H); MS (M + H+) = 545.

**Example 91.** Preparation of Fluorenylmethyl benzyl((2R,3S)-2-hydroxy-4-phenyl-3-{[(tert-butyl oxy)carbonyl]amino} butyl)carbamate

To a solution of (1-benzyl-2-hydroxy-3-isobutylaminopropyl) carbamic acid *tert*-butyl ester) (0.19 mmol) in THF (3 mL) was added Et₃N (32 µL, 0.23 mmol) and Fmoc chloride (49 mg, 0.19 mmol) and the solution was stirred at room temperature for 3 hours, after which time the solvent was removed *in vacuo* and the crude residue was purified by column chromatography on silica gel (1% CH₃OH/CHCl₃) to give 108 mg, 96% of the compound of this Example. NMR (CDCI3) δ ppm 6.89-7.82 (m, 18 H) 4.06-4.64 (m, 6 H) 3.56 - 3.85 (m, 2 H) 3.22-3.51(m, 2 H) 2.72-3.03 (m, 2 H) 1.32 (s, 9 H); MS (M+H+) =593.

**Example 92.** Preparation of Fluorenylmethyl benzyl((2R,3S)-2-hydroxy-4-phenyl-3-{[(1,3-thiazol-5-ylmethoxy)carbonyl]amino} butyl)carbamate

Following the same procedure as in Example 90, using the compound of Example 91 (100 mg, 0.17 mmol), Et₃N (28 µL, 0.20 mmol) and carbonic acid 5-methylthiazole ester 4-nitrophenyl ester hydrochloride (52 mg, 0.19 mmol), gave 89 mg, 83% of the compound of this Example.

**Example 93.** Preparation of tert-butyl benzyl((2R,3S)-2-hydroxy-4-phenyl-3-{[(1,3-thiazol-5-ylmethoxy)carbonyl]amino}butyl)carbamate

To a solution of the compound of Example 92 (89 mg, 0.14 mmol) in CH₃CN (2 mL) was added diethylamine (290 µL, 2.8 mmol) and the solution was stirred at room temperature for 4 hours, after which time the solvent was removed *in vacuo* and the residue was dissolved in THF (4 mL) and H₂O (1 mL). Solid NaHCO₃ (24 mg, 0.28 mmol) was added followed by di-*tert*-butyl dicarbonate (39 µL, 0.17 mmol) and the solution was stirred at room temperature for 18 hours, after which time it was poured into H₂O (10 mL) and 1N HCl was added until the pH was acidic. The solution was extracted with EtOAc (3x10 mL), the organic extracts were combined, washed with brine (1 x 10 mL), dried over Na₂SO₄, filtered and the solvent removed *in vacuo* to give a crude residue which was purified by column chromatography on silica gel (1% CH₃OH/CHCl₃) to give 53 mg, 74% of the title compound. NMR (CDCI3) δ ppm 8.75 (s, 1 H) 7.79 (s, 1H)7.04-7.42 (m, 10 H) 5.06 - 5.29 (m, 2 H) 4.73 - 4.91 (m, 1 H) 4.30 - 4.55 (m, 3 H) 3.40 - 3.92 (m, 3 H) 3.09 - 3.3 1(m, 1 H) 2.74 - 3.00 (m, 2 H) 1.47 (s, 9 H); MS (M+H+)=512.

**Example 94.** Preparation of tert-butyl 2-((2S,3S)-2-hydroxy-4-phenyl-3-{[(benzyloxy) carbonyl]amino}butyl)-2-(4-pyridin-2-ylbenzyl)hydrazinecarboxylate

To a solution of (1-oxiranyl-2-phenyl-ethyl)-carbamic acid benzyl ester (0.75 g, 2,5 mmol, WO 2005061487) in 2-propanol (10 mL) was added N-(4-pyridin-2-ylbenzyl)hydrazine carboxylic acid *tert*-butyl ester (0.75 g, 2.5 mmol, WO 2005061487) and the solution was refluxed for 18 hours, after which time it was cooled, solvent was removed *in vacuo* and the crude residue was purified by column chromatography on silica gel (10% EtOAc/hexane) to give 100 mg, 7% of the compound of this Example. NMR (CDCI3) δ ppm 8.62-8.76 (m, 1 H) 7.86-8.06 (m, 2 H) 7.65-7.82 (m, 2 H) 7.09-7.51 (m, 14 H) 5.18 - 5.46 (m, 2 H) 5.00 - 5.10 (m, 2 H) 3.52 - 4.11 (m, 4 H) 2.87-3.02 (m, 2 H) 2.72-2.87 (m, 1 H) 1.33 (s, 9 H); MS (M + H+) = 597.

**Example 95.** Preparation of tert-butyl 2-((2S,3S)-2-hydroxy-4-phenyl-3-(amino)butyl)-2-(4-pyridin-2-ylbenzyl)hydrazinecarboxylate

To a solution of the compound of Example 94 (100 mg, 0.17 mmol) in CH₃OH (5 mL) was added 10% Pd/C (10 mg) and the solution was stirred at room temperature under H₂ gas for 18 hours, after which time the solution was filtered through Celite, the catalyst washed with CH₃OH (10 mL) and the solvent removed *in vacuo* to give the compound of this Example.

**Example 96.** Preparation of tert-butyl 2-((2S,3S)-2-hydroxy-4-phenyl-3-{[(1,3-thiazol-5-ylmethoxy)carbonyl]amino}butyl)-2-(4-pyridin-2-ylbenzyl)hydrazinecarboxylate

Following the same procedure as in Example 50, using the compound of Example 95 (78 mg, 0.17 mmol), Et₃N (28 µL, 0.20 mmol) and carbonic acid 5-methylthiazole ester 4-nitrophenyl ester hydrochloride (59 mg, 0.18 mmol), gave 42 mg, 41% of the title compound. NMR (CDCI3) δ ppm 8.77 (s, 1 H) 8.68 - 8.70 (d, 1 H) 7.92-7.95 (d, 2 H) 7.62-7.87 (m, 2 H) 7.02-7.49 (m, 9 H) 5.14 - 5.44 (m, 4 H) 3.51-4.10 (m, 4 H) 2.84-3.03 (m, 2 H) 2.69-2.86 (m, 1 H) 2.39-2.58 (m, 1 H) 1.33 (s, 9 H); MS(M+H+)=604.

**Example 97.** Preparation of 5-{benzylamino}-2-[(tert-butoxycarbonyl)amino]-1,2,3,5-tetradeoxy-1-phenyl-D-glycero-pentitol

To a solution of (1-benzyl-2-oxiranylethyl) carbamic acid tert-butyl ester (69 mg, 0.25 mmol, WO 2005061487) in 2-propanol (4 mL) was added benzyl amine (270 µL, 2.5 mmol) and the solution was stirred at 50°C for 18 hours, after which time in was poured into H₂0 (10 mL), extracted with EtOAc (3 x 10 mL), the organic extracts combined, washed with brine (1 x 10 mL), dried over Na₂SO₄, filtered and the solvent removed *in vacuo* to give 56 mg, 59% of the compound of this Example.

**Example 98.** Preparation of 5-{benzyl[(1,3-thiazol-5-ylmethoxy)carbonyl]amino}-2-[(tert-butoxycarbonyl)amino]-1,2,3,5-tetradeoxy-1-phenyl-D-glycero-pentitol

Following the same procedure as in Example 50, using the compound of Example 97 (56 mg, 0.15 mmol), Et₃N (25 µL, 0.18 mmol) and carbonic acid 5-methylthiazole ester 4-nitrophenyl ester hydrochloride (45 mg, 0.16 mmol), gave 15 mg, 19% of the title compound. NMR (CDCI3) δ ppm 8.78 (s, I H) 7.77-7.93 (m, 1H) 6.98-7.42 (m, 10 H) 5.35 (s, 2 H) 4.43 - 4.76 (m, 3 H) 3.65 - 4.40 (m, 3 H) 3.17-3.46 (m, I H) 3.00 - 3.18 (m, 1 H) 2.62 - 2.85 (m, 2 H) 1.39 (s, 9 H) 1.09 - 1.68 (m, 2 H); MS (M + H+) = 526.

**Example 99.** Preparation of 1,3-thiazol-5-ylmethyl (1S,3S,4S)-3-hydroxy-4-{[N-(methoxycarbonyl)-3-methyl-L-valyl]amino}-5-phenyl-1-(4-pyridin-2-ylbenzyl)pentylcarbamate

Following the same procedure as in Example 50, using (1-[4-amino-1-benzyl-2-hydroxy-5-(4-pyridin-2-ylphenyl)pentylcarbamoyl]-2,2-dimethylpropyl) carbamic acid methyl ester (13 mg, 0.024 mmol, WO 2005061487), Et₃N (7 µL, 0.048 mmol) and carbonic acid 5-methylthiazole ester 4-nitrophenyl ester hydrochloride (8 mg, 0.024 mmol), gave the title compound. NMR (CDCI3) δ ppm 8.64 - 8.81 (m, 2 H) 7.66 - 7.97 (m, 5 H) 7.06 - 7.37 (m, 8 H) 6.08 - 6.22 (m, I H) 5.23 (s, 2 H) 4.92 - 5.07 (m, 1H) 3.89-4.12 (m, 1H) 3.64-3.68 (m, 3 H) 3.58-3.83 (m, 1 H) 2.99-3.23 (m, 3 H) 2.72 - 2.93 (m, 3 H) 1.32 - 1.47 (m, 4 H) 0.91 (s, 9 H); MS (M + H+) = 674.

**Example 100.** Preparation of methyl (1S)-1-({[(1R,3S,4S)-3-hydroxy-4-({3-methyl-N-[(1,3-thiazol-5-ylmethoxy)carbonyl]-L-valyl}amino)-5-phenyl-1-(4-pyridin-2-ylbenzyl)pentyl]amino} carbonyl)-2,2-dimethylpropylcarbamate

Following the same procedure as in Example 50, using (1-[4-(2-amino-3,3-dimethylbutyryl amino)-3-hydroxy-5-phenyl-1-(4-pyridin-2-ylbenzyl)pentylcarbamoyl]-2,2-dimethylpropyl) carbamic acid methyl ester (10 mg, 0.015 mmol, WO 2005061487), Et₃N (4.3 µL, 0.031 mmol) and carbonic acid 5-methylthiazole ester 4-nitrophenyl ester hydrochloride (4.8 mg, 0.017 mmol), gave 6.5 mg, 53% of the title compound. NMR (CDCI3) δ ppm 8.80 (s, 1H) 8.67 - 8.68 (d, 1 H) 7.82 - 8.02 (m, 2 H) 7.58-7.82 (m, 2 H) 6.97-7.37 (m, 9 H) 6.30 - 6.52 (m, I H) 5.8- 5.99 (m, 1 H) 5.13-5.49 (m, 4 H) 4.68-4.84 (m, 1 H) 4.22 - 4.44 (m, 1 H) 3.75 - 3.97 (m, I H) 3.64 (s, 3H) 3.58-3.74 (m, 1 H) 3.42 - 3.57 (m, 1 H) 2.69-2.95 (m, 3 H) 1.16-1.36 (m, 1 H) 0.69 - 1.00 (m, 18 H); MS (M + H+) = 787.

The foregoing description of the present invention provides illustration and description, but is not intended to be exhaustive or to limit the invention to the precise one disclosed. Modifications and variations are possible in light of the above teachings or may be acquired from practice of the invention. Thus, it is noted that the scope of the invention is defined by the claims and their equivalents,

The invention further relates to the following items:
1. A compound of formula I, 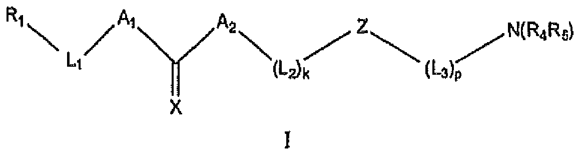 or a pharmaceutically acceptable salt, solvate or prodrug thereof, wherein
   R₁ is a 3-, 4-, 5-, 6-, 7-, 8-, 9-, or 10-membered heterocyclyl comprising at least one nitrogen ring atom;
   L₁ is a bond, C₁-C₁₀alkylene, C₂-C₁₀alkenylene or C₂-C₁₀alkynylene;
   A₁ is a bond or selected from the group consisting of ―O―L_{A1}―, ―S―L_{A1}― and ―N(R_{A1})―L_{A1}―, wherein L_{A1} is a bond, C₁-C₁₀alkylene, C₂-C₁₀alkenylene or C₂-C₁₀alkynylene, and R_{A1} is hydrogen, C₁-C₆ alkyl, C₂-C₆alkenyl or C₂-C₆alkynyl;
   X is O or S;
   A₂ is a bond or selected from the group consisting of ―L_{A2}―O―, ―L_{A2}―S― and ―L_{A2}―N(R_{A2})―, wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, ―L_{D}―O―R_{D}, ―L_{D}―S―R_{D}, ―L_{D}― C(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}―SO₂R_{D}, ―L_{D}―C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, carbocyclyl, carbocyclylC₁-C₆alkyl, heterocyclyl, heterocycloC₁-C₆alkyl, ―L_{E}―carbocyclyl―L₄―Y―L_{4'}―R_{E} and ―L_{E}―heterocyclyl―L₄―Y―L_{4'}―R_{E};
   k is 0 or 1, and at each occurrence L₂ independently represents ―L₉―V―L_{9'}―, wherein L₉ and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, ―S―, ―O―, ―C(O)―, ―N(R_{V})C(O)―, ―C(O)N(R_{V})―, ―C(O)O― and ―OC(O)―, wherein R_{V} is independently selected at each occurrence from hydrogen, C₁-C₆ alkyl, C₂-C₆alkenyl or C₂-C₆alkynyl;
   Z is ―C(R₂R₃)―, =C(R₂)― or ―C(R₂)=;
   or Z is selected from the group consisting of 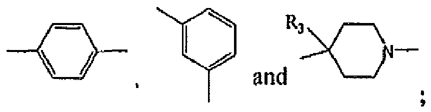 or Z, taken together with (L₃)ₚ and N(R₄R₅), forms 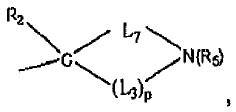 wherein L₇ is C₁-C₁₀alkylene, C₂-C₁₀alkenylene or C₂-C₁₀alkynylene, and 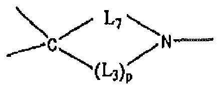 comprises from 3 to 10 ring atoms;
   or Z is a bond;
   p is an integer selected from 0, 1, 2 or 3, and at each occurrence L₃ independently represents ―L₅― W―L_{5'}―, wherein W is independently selected at each occurrence from the group consisting of a bond, C₁-C₁₀alkylene, C₂-C₁₀alkenylene, C₂-C₁₀alkynylene, ―S―, ―O―, ―C(O)―, ―N(R_{W})CO―, ―C(O)N(R_{W})―, ―C(O)O― and ―OC(O)―, and R_{W} is independently selected at each occurrence from hydrogen, C₁-C₆alkyl, C₂-C₆alkenyl or C₂-C₆alkynyl, wherein L₅ and L_{5'} are each independently selected at each occurrence from a bond, C₁-C₁₀alkylene, C₂-C₁₀alkenylene or C₂-C₁₀alkynylene, and are each independently optionally substituted at each occurrence with 1, 2, 3 or more substituents each of which is independently selected at each occurrence from the group consisting of halogen, oxo, thioxo, hydroxy, nitro, cyano, amino, formyl, carbocyclyl, heterocyclyl, ―O―R_{D}, ―S―R_{D}, ―C(O)R_{D}, ―OC(O)R_{D}, ―C(O)OR_{D}, ―NR_{D}R_{D'}, ―S(O)R_{D}, ―SO₂R_{D}, ―C(O)NR_{D}R_{D'}, ―N(R_{D})C(O)R_{D'}, ―N(R_{D})C(O)OR_{D'}, ―N(R_{D})SO₂R_{D'}, ―N(R_{D})SO₂NR_{D'}R_{D''}, ―carbocyclyl―L₄―Y―L_{4'}―R_{E} and ―heterocyclyl―L₄―Y―L_{4'}―R_{E};
   or p is 1, L₃ is ―L₅―C(R₆R₇)―L_{5'}―, A₂ is ―L_{A2}―NR_{A2}―, and R_{A2} and R₇ are bonded together to form ―C(O)O―;
   or p is 1, L₃ is ―L₅―C(R₆R₇)―L_{5'}―, and R₄ and R₇ are bonded together to form ―OC(O)―;
   R₄ and R₅ are each independently selected from the group consisting of N-protecting group, hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L_{E}―carbocyclyl―L₄―Y―L_{4'}―R_{E}, ―L_{E}―heterocyclyl―L₄―Y―L_{4'}―R_{E}, ―L₆―O―R₈, ―L₆― C(O)R₈, ―L₆―C(O)OR₈, ―L₆―OC(O)R₈, ―L₆―C(O)NR₈R₉, ―N(R₉)C(O)OR₈, ―L₆―C(O)―L_{6'}―O―R₈, ―L₆― C(O)―L_{6'}―NR₈R₉, ―L₆―C(O)―L_{6'}―N(R₉)C(O)OR₈, ―L₆―C(O)―L_{6'}―N(R₉)C(O)NR₈R₁₀, ―L₆―S(O)ⱼR₈, ―L₆― N(R₉)S(O)ⱼR₈, ―L₆―S(O)ⱼNR₈R₉ and ―L₆―N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₂-C₁₀alkynylene, wherein R₈, R₉ and R₁₀ are each independently selected at each occurrence from the group consisting of hydrogen, C₁-C₆ alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}― NR_{D}R_{D'}, ―L_{D}―S(O)R_{D}, ―L_{D}SO₂R_{D}, ―L_{D}―C(O)NR_{D}R_{D'}, ―L_{D}―N(R_{D})C(O)R_{D'}, ―L_{D}―N(R_{D})SO₂R_{D'}, ―L_{D}― N(R_{D})SO₂NR_{D'}R_{D''}, ―L_{E}―carbocyclyl―L₄―Y―L_{4'}―R_{E} and ―L_{E}―heterocyclyl―L₄―Y―L_{4'}―R_{E};
   or R₄ and R₅, together with the N attached thereto, form a heterocyclyl;
   wherein R₂ is selected from the group consisting of carbocyclyl, carbocyclylC₁-C₆alkyl, heterocyclyl, heterocycloC₁-C₆alkyl, ―L_{D}―O―R_{E}, ―L_{D}―S―R_{E}, ―L_{D}―C(O)R_{E}, ―L_{D}―OC(O)R_{E}, ―L_{D}― C(O)OR_{E}, ―L_{D}―N_{D}R_{E}R_{D}, ―L_{D}―S(O)R_{E}, ―L_{D}―SO₂R_{E}, ―L_{D}―C(O)NR_{D}R_{E}, ―L_{D}―N(R_{D})C(O)R_{E}, ―L_{D}― N(R_{D})SO₂R_{E}, ―L_{D}―N(R_{D)}SO₂NR_{D'}R_{E}, ―L_{E}―carbocyclyl―L₄―Y―L_{4'}―R_{E} and ―L_{E}―heterocyclyl―L₄―Y―L_{4'}― R_{E};
   wherein R₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{D}-O-R_{D}, ―L_{D}―S― R_{D}, ―L_{D}―C(O)R_{D}, ―L_{D}-OC(O)R_{D}, ―L_{D}―C(O)OR_{D}, ―L_{D}―NR_{D}R_{D'}, ―L_{D}―S(O)R_{D}, ―L_{D}―SO₂R_{D}, ―L_{D}― C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, ―L_{E}―carbocyclyl―L₄―Y― L_{4'}―R_{E} and ―L_{E}―heterocyclyl―L₄―Y―L_{4'}―R_{E};
   wherein R₆ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L_{D}―O―R_{D}, ―L_{D}―S― R_{D}, ―L_{D}―C(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}―SO₂R_{D}, ―L_{D}― C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, ―L_{E}―carbocyclyl―L₄―Y― L_{4'}―R_{E} and ―L_{E}―heterocyclyl―L₄―Y―L_{4'}―R_{E};
   wherein L_{D}, L_{E}, L₄ and L_{4'} are each independently selected at each occurrence from a bond, C₁-C₁₀alkylene, C₂-C₁₀alkenylene or C₂-C₁₀alkynylene;
   wherein R_{D}, R_{D'} and R_{D''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆ thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆ alkylaminoC₁-C₆alkyl, carbocyclyl, carbocyclylC₁-C₆alkyl, heterocyclyl and heterocycloC₁-C₆alkyl;
   wherein Y is independently selected at each occurrence from the group consisting of a bond, C₁-C₁₀alkylene, C₂-C₁₀alkenylene, C₂-C₁₀alkynylene, ―S―, ―O―, ―C(O)―, ―N(R_{Y})C(O)―, ―C(O)N(R_{Y})―, ―C(O)O― and ―OC(O)―, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆alkyl;
   wherein at each occurrence L₁, L_{A1}, R_{A1}, Y, V, W, R_{Y}, R_{V}, Rw, L_{A2}, R_{A2}, L_{D}, L_{E}, L₄, L_{4'}, L₆, L_{6'}, L₇, L₉, L_{9'}, R₂, R₃, R₄, R₅, R₆, R₈, R₉, R₁₀, R_{E}, R_{D}, R_{D'} and R_{D''} are each independently optionally substituted with at least one substituent selected from the group consisting of halogen, oxo, thioxo, hydroxy, nitro, cyano, amino, formyl, carbocyclyl, heterocyclyl, ―O―R_{L}, ―S―R_{L}, ―C(O)R_{L}, ―OC(O)R_{L}, ―C(O)OR_{L}, ―NR_{L}R_{L'}, ―S(O)R_{L}, ―SO₂R_{L}, ―C(O)NR_{L}R_{L'}, ―N(R_{L})C(O)R_{L'}, ―N(R_{L})SO₂R_{L'} and ―N(R_{L})SO₂NR_{L'}R_{L''}, and wherein R_{L}, R_{L'} and R_{L''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆ alkylaminoC₁-C₆alkyl, carbocyclyl, carbocyclylC₁-C₆alkyl, heterocyclyl and heterocycloC₁-C₆alkyl;
   wherein each carbocyclyl moiety in L₁, A₁, A₂, (L₂)ₖ, Z, (L₃)ₚ and N(R₄R₅) is independently selected at each occurrence from 3-, 4-, 5-, 6-, 7-, 8-, 9- or 10-membered carbocyclyls, and each heterocyclyl moiety in L₁, A₁, A₂, (L₂)ₖ, Z, (L₃)ₚ and N(R₄R₅) is independently selected at each occurrence from 3-, 4-, 5-, 6-, 7-, 8-, 9- or 10-membered heterocyclyls; and
   wherein each carbocyclyl and heterocyclyl moiety in said compound is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{S}―O―R_{S}, ―L_{S}―S―R_{S}, ―L_{S}―C(O)R_{S}, ―L_{S}―OC(O)R_{S}, ―L_{S}―C(O)OR_{S}, ―L_{S}―NR_{S}R_{S'}, ―L_{S}―S(O)R_{S}, ―L_{S}―SO₂R_{S}, ―L_{S}― C(O)NR_{S}R_{S'}, ―L_{S}―N(R_{S})C(O)R_{S'}, ―L_{S}―N(R_{S})SO₂R_{S'}, ―L_{S}―N(R_{S})SO₂NR_{S'}R_{S''}, carbocyclyl, carbocyclylC₁-C₆alkyl, heterocyclyl and heterocycloC₁-C₆alkyl, wherein L_{S} is independently selected at each occurrence from the group consisting of a bond, C₁-C₁₀alkylene, C₂-C₁₀alkenylene and C₂-C₁₀alkynylene, and R_{S}, R_{S'} and R_{S''} are each independently selected at each occurrence from the group consisting of hydrogen, C₁-C₆ alkyl, C₂-C₆alkenyl, C₂-C₆alkynyl, C₁-C₆alkoxyC₁-C₆alkyl, C₁-C₆thioalkoxyC₁-C₆alkyl, C₁-C₆ alkyl carbonyl, C₁-C₆alkylcarbonylC₁-C₆alkyl, C₁-C₆alkoxycarbonyl, C₁-C₆alkoxycarbonylC₁-C₆alkyl, C₁-C₆ alkylcarbonyloxy, C₁-C₆alkylcarbonyloxyC₁-C₆alkyl, C₁-C₆alkylaminoC₁-C₆alkyl, C₃-C₁₀carbocyclyl, C₃-C₁₀carbocyclylC₁-C₆alkyl, H₃-H₁₀heterocyclyl and H₃-H₁₀heterocycloC1-C₆alkyl;
   with the proviso that if Z is a bond, then A2 is ―L_{A2}―NR_{A2}, p is an integer selected from 1, 2 or 3, L₃ at each occurrence independently represents ―L₅―W―L_{5'}―, and R_{A2} and R₅ are each independently selected from the group consisting of hydrogen, C₁-C₆alkyl, C₂-C₆alkenyl, C₂-C₆alkynyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'}, ―L_{D}―S(O)R_{D}, ―L_{D}―SO₂R_{D}, ―L_{D}― C(O)NR_{D}R_{D'}, ―L_{D}―N(R_{D})C(O)R_{D'}, ―L_{D}―N(R_{D})SO₂R_{D'}, ―L_{D}―N(R_{D})SO₂NR_{D'}R_{D''}, carbocyclyl, carbocyclylC₁-C₆alkyl, heterocyclyl, heterocycloC₁-C₆alkyl, ―L_{E}―carbocyclyl―L₄―Y―L_{4'}―R_{E} and ―L_{E}― heterocyclyl―L₄―Y―L_{4'}―R_{E}, wherein L_{A2}, L_{D}, L_{E}, L₄, L_{4'}, Y, R_{E}, R_{D}, R_{D'}, R_{D''}, L₅, W and L_{5'} are as defined immediately above;
   with the further proviso that said compound is not ritonavir ((2S,3S,5S)-5-(N-(N-((N-Methyl-N-((2-isopropyl-4-thiazolyl)methyl)amino)carbonyl)-L-valinyl)amino)-2-(N-((5-thiazolyl)methoxycarbonyl) amino)-1,6-diphenyl-3-hydroxyhexane).
2. A compound of item 1, or a pharmaceutically acceptable salt, solvate or prodrug thereof, wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is ―O―L_{A1}―, wherein L_{A1} is a bond;
   X is O;
   A₂ is ―L_{A2}―N(R_{A2})―, wherein L_{A2} is a bond, and R_{A2} is hydrogen, C₁-C₆alkyl, C₂-C₆alkenyl, C₂-C₆ alkynyl, carbocyclylC₁-C₆alkyl or heterocycloC₁-C₆alkyl;
   k is 0 or 1;
   L₂ represents ―L₉―V―L_{9'}―, wherein L₉ is independently selected from a bond, C₁-C₆alkylene, C₂-C₆alkenylene or C₂-C₆alkynylene, L_{9'} is a bond, and V is selected from the group consisting of a bond or ―C(O)N(R_{V})―, and wherein R_{V} is selected from hydrogen, C₁-C₆alkyl, C₂-C₆alkenyl or C₂-C₆alkynyl;
   Z is ―C(R₂R₃)―, wherein R₂ is carbocyclylC₁-C₆alkyl, heterocycloC₁-C₆alkyl, R_{E}―carbocyclylC₁-C₆alkyl― or R_{E}―heterocyclylC₁-C₆alkyl―, and R₃ is hydrogen, C₁-C₆alkyl, C₂-C₆alkenyl or C₂-C₆alkynyl;
   p is 1;
   L₃ represents:
   - L₅―W―L_{5'}―, wherein W is a bond; or
   - L₅C(R₆R₇)―L_{5'}―, and R_{A2} and R₇ are bonded together to form ―C(O)O―; or
   - L₅―C(R₆R₇)―L_{5'}―,wherein R₄ and R₇ are bonded together to form ―OC(O)―;
      wherein L₅ is a bond, C₁-C₆alkylene, C₂-C₆alkenylene or C₂-C₆alkynylene;
      L_{5'} is C₁-C₆alkylene, C₂-C₆alkenylene or C₂-C₆alkynylene and is substituted with at least one moiety selected from the group consisting of carbocyclyl, carbocyclylC₁-C₆alkyl, heterocyclyl, heterocycloC₁-C₆alkyl, ―carbocyclyl―R_{E} and ―heterocyclyl―R_{E};
      L₅ and L_{5'} are each independently optionally substituted with at least one moiety selected from halogen, oxo, thioxo, hydroxy, nitro, cyano, amino, ―O―R_{D}, -S-R_{D}, ―C(O)R_{D}, ―OC(O)R_{D}, ―C(O)OR_{D}, ―NR_{D}R_{D'} and ―C(O)NR_{D}R_{D'}; and
      R₆ is hydrogen, C₁-C₆alkyl, C₂-C₆alkenyl or C₂-C₆alkynyl;
   R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆― O―R₈, ―L₆―C(O)R₈, ―C(O)OR₈, ―OC(O)R₈, ―C(O)NR₈R₉, ―N(R₉)C(O)OR₈, ―C(O)―L_{6'}―O―R₈, ―C(O)― L_{6'}―NR₈R₉, ―C(O)―L_{6'}―N(R₉)C(O)OR₈, ―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}―C(O)R_{D}, ―L_{D}-OC(O)R_{D}, ―L_{D}―C(O)OR_{D}, ―L_{D}―NR_{D}R_{D'} and ―L_{D}―C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene;
   or R₄ and R₅, together with the N attached thereto, form a heterocyclyl;
   wherein R_{E} is independently selected at each occurrence from carbocyclyl, heterocyclyl, carbocyclylC₁-C₆alkyl or heterocycloC₁-C₆alkyl;
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆ alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A₂, Z, (L₃)ₚ and N(R₄R₅) is independently selected at each occurrence from 5-, 6- or 7-membered carbocyclyls, and each heterocyclyl moiety in A₂, Z, (L₃)ₚ and N(R₄R₅) is independently selected at each occurrence from 5-, 6- or 7-membered heterocyclyls;
   wherein at each occurrence L₁, R_{V}, L_{D}, L₆, L_{6'}, L₉, R₂, R₃, R₄, R₅, R₆, R₈, R₉, R₁₀, R_{A2}, R_{E}, R_{D} and R_{D'} are each independently optionally substituted with at least one substituent selected from the group consisting of halogen, oxo, thioxo, hydroxy, nitro, cyano, amino, ―O―R_{L}, ―S―R_{L}, ―C(O)R_{L}, ―OC(O)R_{L}, ―C(O)OR_{L}, ―NR_{L}R_{L'} and ―C(O)NR_{L}R_{L'}, wherein R_{L} and R_{L'} are each independently selected at each occurrence from the group consisting of hydrogen, C₁-C₆alkyl, C₂-C₆alkenyl, C₂-C₆alkynyl, C₁-C₆ alkoxyC₁-C₄alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl; and
   wherein each carbocyclyl and heterocyclyl moiety in A₂, Z, (L₃)ₚ, and N(R₄R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, ―L_{H}―O―R_{K}, ―L_{H}―S―R_{K}, ―L_{H}―C(O)R_{K}, ―L_{H}―OC(O)R_{K}, ―L_{H}―C(O)OR_{K}, ―L_{H}―NR_{K}R_{K'}, ―L_{H}―S(O)R_{K}, ―L_{H}― SO₂R_{K}, ―L_{H}―C(O)NR_{K}R_{K'}, ―L_{H}―N(R_{K})C(O)R_{K'}, ―L_{H}―NR_{K}SO₂R_{K'} and ―L_{H}―NR_{K}SO₂NR_{K'}R_{K''}, wherein L_{H} is independently selected at each occurrence from a bond, C₁-C₆alkylene, C₂-C₆alkenylene or C₂-C₆ alkynylene, and R_{K}, R_{K'} and R_{K''} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl.
3. A compound of item2, or a pharmaceutically acceptable salt, solvate or prodrug thereof,
   wherein R₁ is thiazolyl, L₁ is ―CH₂―, R_{A2} is hydrogen, R₂ is benzyl, L₃ is ―L₅―W―L_{5'}―, L₅ and W are bonds, L_{5'} is ―(CH₂)₂―CH(R₁₃)―, and R₁₃ is pyridylbenzyl, and wherein L_{5'} is optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'}.
4. A compound of item2, or a pharmaceutically acceptable salt, solvate or prodrug thereof,
   wherein R₁ is thiazolyl, L₁ is ―CH₂―, R_{A2} is hydrogen, R₂ is pyridylbenzyl, L₃ is ―L₅―W―L_{5'}-, L₅ and W are bonds, L_{5'} is ―(CH₂)₂―CH(R₁₃)―, and R₁₃ is benzyl, and wherein L_{5'} is optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'}.
5. A compound of item 2, or a pharmaceutically acceptable salt, solvate or prodrug thereof,
   wherein R₁ is thiazolyl, L₁ is ―CH₂―, R_{A2} is hydrogen, R₂ is benzyl, L₃ is ―L₅―W―L_{5'}―, L₅ and W are bonds, L_{5'} is ―(CH₂)₂―CH(R₁₃)―, and R₁₃ is benzyl, and wherein L_{5'} is optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'}.
6. A compound of item 2, or a pharmaceutically acceptable salt, solvate or prodrug thereof,
   wherein R₁ is thiazolyl, L₁ is ―CH₂―, R_{A2} is hydrogen, R₂ is benzyl, L₃ is ―L₅―C(R₆R₇)―L_{5'}―, L₅ is a bond, L_{5'} is ―(CH₂)―CH(R₁₃)―, and R₄ and R₇ are bonded together to form ―OC(O)―, and wherein R₁₃ is benzyl.
7. A compound of item 2, or a pharmaceutically acceptable salt, solvate or prodrug thereof,
   wherein R₁ is thiazolyl, L₁ is ―CH₂―, k is 0, R₂ is benzyl, L₃ is ―L₅―C(R₆R₇)―L_{5'}―, L₅ is a bond, L_{5'} is ―(CH₂)―CH(R₁₃)―, and R_{A2} and R₇ are bonded together to form ―OC(O)―, and wherein R₁₃ is benzyl.
8. A compound of item1, or a pharmaceutically acceptable salt, solvate or prodrug thereof, wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is ―O―L_{A1}―, wherein L_{A1} is a bond;
   X is O;
   A₂ is ―L_{A2}-N(R_{A2})―, wherein L_{A2} is a bond, and R_{A2} is hydrogen, C₁-C₆alkyl, C₂-C₆alkenyl, C₂-C₆ alkynyl, carbocyclylC₁-C₆alkyl or heterocycloC₁-C₆alkyl;
   k is 0 or 1;
   L₂ represents ―L₉―V―L_{9'}―, wherein L₉ is independently selected from a bond, C₁-C₆alkylene, C₂-C₆alkenylene or C₂-C₆alkynylene, L_{9'} is a bond, and V is selected from the group consisting of a bond or ―C(O)N(R_{V})―, and wherein R_{V} is selected from hydrogen, C₁-C₆alkyl, C₂-C₆alkenyl or C₂-C₆alkynyl;
   Z is ―C(R₂R₃)―, wherein R₂ is carbocyclylC₁-C₆alkyl, heterocycloC₁-C₆alkyl, R_{E}―carbocyclylC₁-C₆alkyl― or R_{E}―heterocyclylC₁-C₆alkyl―, and R₃ is hydrogen, C₁-C₆alkyl, C₂-C₆alkenyl or C₂-C₆alkynyl, wherein R_{E} is independently selected at each occurrence from carbocyclyl, heterocyclyl, carbocyclylC₁-C₆alkyl or heterocycloC₁-C₆alkyl;
   p is 0 or 1;
   L₃ represents:
   - L₅―W―L_{5'}―, wherein W is a bond or ―C(O)―; or
   - L₅―C(R₆R₇)―L_{5'}―, and R_{A2} and R₇ are bonded together to form ―C(O)O―; or
   - L₅―C(R₆R₇)―L_{5'}―,wherein R₄ and R₇ are bonded together to form ―OC(O)―;
      wherein L₅ and L_{5'} are each independently selected from a bond, C₁-C₆alkylene, C₂-C₆ alkenylene or C₂-C₆alkynylene, and are each independently optionally substituted with at least one moiety selected from halogen, oxo, thioxo, hydroxy, nitro, cyano, amino, ―O―R_{D}, ―S―R_{D}, ―C(O)R_{D}, ―OC(O)R_{D}, ―C(O)OR_{D}, ―NR_{D}R_{D'} and ―C(O)NR_{D}R_{D'}; and
      wherein R₆ is hydrogen, C₁-C₆alkyl, C₂-C₆alkenyl or C₂-C₆alkynyl;
   R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, ―L₆― O―R₈, ―L₆―C(O)R₈, ―C(O)OR₈, ―OC(O)R₈, ―C(O)NR₈R₉, ―N(R₉)C(O)OR₈, ―C(O)―L_{6'}―O―R₈, ―C(O)― L_{6'}―NR₈R₉, ―C(O)―L_{6'}―N(R₉)C(O)OR₈, ―C(O)―L_{6'}―N(R₉)C(O)NR₈R₁₀, ―L₆―S(O)ⱼR₈, ―L₆―N(R₉)S(O)ⱼR₈, ―L₆―S(O)ⱼNR₈R₉ and ―L₆―N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, ―L_{D}―O―R_{D}, ―L_{D}―S―R_{D}, ―L_{D}―C(O)R_{D}, ―L_{D}―OC(O)R_{D}, ―L_{D}―C(O)OR_{D}, ―L_{D}―NR_{D}R_{D'} and ―L_{D}―C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene;
   or R₄ and R₅, together with the N attached thereto, form a heterocyclyl;
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆a lkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylocarbonyloxy, C₁-C₆alkylcarbonyloxyC₁-C₆alkyl and C₁-C₆alkylaminoC₁-C₆alkyl;
   wherein each carbocyclyl moiety in A₂, Z, and N(R₄R₅) is independently selected at each occurrence from 5-, 6- or 7-membered carbocyclyls, and each heterocyclyl moiety in A₂, Z, and N(R₄R₅) is independently selected at each occurrence from 5-, 6- or 7-membered heterocyclyls;
   wherein at each occurrence L₁, R_{V}, L_{D}, L₆, L_{6'}, L₉, R₂, R₃, R₄, R₅, R₆, R₈, R₉, R₁₀, R_{A2}, R_{E,} R_{D} and R_{D'} are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl. C₁-C₆alkylcarbonyloxy. C₁-C₆alkylcarbonyloxyC₁-C₆ alkyl and C₁-C₆alkylaminoC₁-C₆alkyl;
   wherein each carbocyclyl and heterocyclyl moiety in A₂, Z, N(R₄R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alknyl, -L_{H}-O-R_{K}, -L_{H}-S-R_{K}. -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'},-L_{H}-N(R_{K})C(O)R_{K'},-LH-NR_{K}SO₂R_{K'} and-L_{H}-NR_{K}SO₂NR_{K}'R_{K"}, wherein L_{H} is independently selected at each occurrence from a bond, C₁-C₆alkylene, C₂-C₆alkenylene or C₂-C₆ alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl.
9. A compound of item 8, or a pharmaceutically acceptable salt, solvate or prodrug thereof, wherein R₁ is thiazolyl, L₁ is -CH₂-, R_{A2} is hydrogen, k is 0, R₂ is benzyl, p is 1, and L₃ is -L₅-W-L₅-, wherein W is -C(O)-, and L₅ and L_{5'} are bonds.
10. A compound of item 8, or a pharmaceutically acceptable salt, solvate or prodrug thereof, wherein R₁ is thiazolyl, L, is -CH₂-, R_{A2} is C₁-C₆alkyl or benzyl, k is 1, L₂ is -L₉-V-L₉-, R₂ is benzyl, and p is 0, wherein L₉, and V are bonds, and L₉ is C₁-C₃alkylene optionally substituted with at least one substituent selected from the group consisting of halogen, oxo, thioxo, hydroxy, nitro, cyano and amino.
11. A compound of item 1, or a pharmaceutically acceptable salt, solvate or prodrug thereof,
   wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene,
   A₁ is -O-L_{A1}-, wherein L_{A1} is a bond;
   X is O;
   A₂ is -L_{A2}-N(R_{A2})-, wherein L_{A2} is a bond, and R_{A2} is hydrogen, C₁-C₆alkyl, C₂-C₆alkenyl, C₂-C₆ alkynyl, carbocyclylC₁-C₆alkyl or heterocycloC₁-C₆alkyl;
   k is 0 or 1;
   L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(Rᵥ)-, and wherein Rᵥ is selected from hydrogen, C₁-C₆alkyl, C₂-C₆alkenyl or C₂-C₆alkynyl;
   Z is selected from the group consisting of 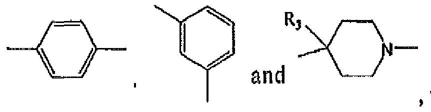 wherein R₃ is hydrogen, C₁-C₆alkyl, C₂-C₆alkenyl, C₂-C₆alkynyl, carboryclylC₁-C₆alkyl or heterocycloC₁-C₆alkyl;
   p is 0 or 1;
   L₃ represents:
   - L₅-W-L₅-, wherein W is a bond or -N(R_{w})CO-, and R_{w} is hydrogen, C₁-C₆alkyl, C₂-C₆alkenyl or C₂-C₆alkynyl; or
   - L₅-C(R₆R₇)-L_{5'}-,wherein R₄ and R₇ are bonded together to form -OC(O)-;
      wherein L₅ and L_{5'} are each independently selected from a bond, C₁-C₆alkylene, C₂-C₆ alkenylene or C₂-C₆alkynylene, and are each independently optionally substituted with at least one moiety selected from halogen, oxo, thioxo, hydroxy, nitro, cyano, amino, carbocyclyl, carbocyclylC₁-C₆alkyl, heterocyclyl, heterocycloC₁-C₆alkyl, -O-R_{D}, -S-R_{D}, -C(O)R_{D}, -OC(O)R_{D}, -C(O)OR_{D}, -NR_{D}R_{D'} and -C(O)NR_{D}R_{D'}; and
      wherein R₆ is hydrogen, C₁-C₆alkyl, C₂-C₆alkenyl or C₂-C₆alkynyl;
   R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆-O-R₈, -L₆-C(O)R₈, -C(O)OR₈, -OC(O)R₈, -C(O)NR₈R₉, -N(R₉)C(O)OR₈, -C(O)-L_{6'}-O-R₈, -C(O)-L₆-NR₈R₉, -C(O)-L_{6'}-N(R₉)C(O)OR₈, -C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆ hydroxyalkyl, carbocyclyl, carbocyclylC₁-C₆alkyl, heterocyclyl, heterocycloC₁-C₆alkyl, carbocyclyl heterocyclylC₁-C₆alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene, or C₂-C₆alkynylene;
   or R₄ and R₅, together with the N attached thereto, form a heterocyclyl;
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆ alkyl, C₁-C₆alkylearbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A₂, Z, (L₃)ₚ and N(R₄R₅) is independently selected at each occurrence from 5-, 6- or 7-membered carbocyclyls, and each heterocyclyl moiety in A₂, Z, (L₃)ₚ and N(R₄R₅) is independently selected at each occurrence from 5-, 6- or 7-membered heterocyclyls;
   wherein at each occurrence L₁, R_{V}, L_{D,} L₆, L_{6'}, L₉, R₃, R₄, R₅, R₆, R₈, R₉, R₁₀, R_{A2}, R_{W}, R_{D} and R_{D'} are each independently optionally substituted with at least one substituent selected from the group consisting of halogen, oxo, thioxo, hydroxy, nitro, cyano, amino, -O-R_{L,} -S-R_{L}, -C(O)R_{L}, -OC(O)R_{L}, -C(O)OR_{L}, -NR_{L}R_{L'} and -C(O)NR_{L}R_{L'}, wherein R_{L} and R_{L}. are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylearbonyloxyC₁-C₆ alkyl and C₁-C₆alkylaminoC₁-C₆alkyl;
   wherein each carbocyclyl and heterocyclyl moiety in A₂, Z, (L₃)ₚ, N(R₄R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K,} -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆ alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl.
12. A compound of item 11, or a pharmaceutically acceptable salt, solvate or prodrug thereof,
   wherein R₁ is thiazolyl, L₁ is -CH₂-, R_{A2} is H, k and p are 0, and Z is 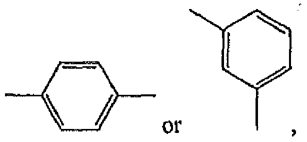 wherein at least one of R₄ or R₅ is selected from the group consisting of carbocyclylC₁-C₆alkyl, heterocycloC₁-C₆alkyl, -C(O)R_{8A}, -C(O)OR_{8A}, -OC(O)R_{8A}, -C(O)NR_{8A}R₉, -C(O)-C₁-C₆alkylene-NR_{8A}R₉, -C(O)-C₁-C₆alkylene-N(R₉)C(O)OR_{8A}, -C(O)-C₁-C₆alkylene-N(R₉)C(O)NR_{8A}R₁₀, -S(O)ⱼR_{SA} and -S(O)ⱼNR_{8A}R₉, and wherein R_{8A} is C₁-C₆alkyl, C₂-C₆alkenyl, C₂-C₆alkynyl, carbocyclyl, carbocyclylC₁-C₆alkyl, heterocyclyl or heterocycloC₁-C₆alkyl and is optionally substituted with at least one moiety selected from halogen, oxo, thioxo, hydroxy, nitro, cyano, amino, C₁-C₆alkyl, C₂-C₆alkenyl or C₂-C₆alkynyl.
13. A compound of item 11, or a pharmaceutically acceptable salt, solvate or prodrug thereof,
   wherein R₁ is thiazolyl, L₁ is -CH₂-, R_{A2} is H, k is 0, Z is 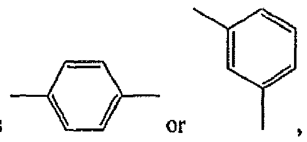 and p is 1, wherein L₃ is -L₅-W-L_{5'}-, L₅ is a bond, W is -N(H)CO-, and L_{5'} is C₁₋C₃alkylene substituted with carbocyclyl, carbocyclylC₁₋C₆alkyl, heterocyclyl or heterocycloC₁-C₆alkyl, wherein at least one of R₄ or R₅ is selected from the group consisting of carbocyclylC₁-C₆alkyl, heterocycloC₁-C₆alkyl, -C(O)R_{BA}, -C(O)OR_{8A,} -OC(O)R_{8A}, -C(O)NR_{8A}R₉, -C(O)-C₁-C₆alkylene-NR_{8A}R₉, -C(O)-C₁-C₆alkylene-N(R₉)C(O)OR_{8A}, -C(O)-C₁-C₆alkylene-N(R₉)C(O)NR_{8A}R₁₀, -S(O)ⱼR_{8A} and -S(O)ⱼNR_{8A}R₉, and wherein R_{8A} is selected from C₁-C₆alkyl, C₂-C₆alkenyl, C₂-C₆alkynyl, carbocyclyl, carbocyclylC₁-C₆alkyl, heterocyclyl or heterocycloC₁-C₆alkyl.
14. A compound of item 11, or a pharmaceutically acceptable salt, solvate or prodrug thereof,
   wherein R₁ is thiazolyl, L₁ is -CH₂-, R_{A2} is H, k is 0, Z is 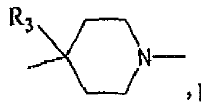 p is 1, and L₃ is -L₅-W- L_{5'}-, wherein L₅ and W are bonds, L_{5'} is C₁-C₃alkylene, and R₃ is benzyl.
15. A compound of item 1, or a pharmaceutically acceptable salt, solvate or prodrug thereof, wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is -O-L_{A1}-, wherein L_{A1} is a bond;
   X is O;
   A₂ is -L_{A2}-N(R_{A2})-, wherein L_{A2} is a bond, and R_{A2} is hydrogen, C₁-C₆alkyl, C₂-C₆alkenyl, C₂-C₆ alkynyl, carbocyclylC₁-C₆alkyl or heterocycloC₁-C₆alkyl;
   k is 0 or 1;
   L₂ represents -L₉-V-L_{9'}—, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(Rᵥ)-, and wherein Rᵥ is selected from hydrogen, C₁-C₆alkyl, C₂-C₆alkenyl or C₂-C₆alkynyl;
   Z is -C(R₂R₃)-, wherein R₂ and R₃ are independently selected from carbocycloC₁-C₆alkyl or heterocyclylC₁-C₆alkyl,
   p is 0 or 1;
   L₃ represents:
   - L₅-W-L_{5'}-, wherein W is a bond; or
   - L₅-C(R₆R₇)-L_{5'}-, and R_{A2} and R₇ are bonded together to form -C(O)O-; or
   - L₅-C(R₆R₇)-L_{5'}-,wherein R₄ and R₇ are bonded together to form -OC(O)-;
      wherein L₅ and L_{5'} are each independently selected from a bond, C₁-C₆alkylene, C₂₋C₆ alkenylene or C₂-C₆alkynylene, and are each independently optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D'} and -C(O)NR_{D}R_{D'}; and
      wherein R₆ is hydrogen, C₁-C₆alkyl, C₂-C₆alkenyl or C₂-C₆alkynyl;
   R₄ and R₅ are each independently selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆- O-R₈, -L₆-C(O)R₈, -C(O)OR₈, -OC(O)R₈, -C(O)NR₈R₉, -N(R₉)C(O)OR₈, -C(O)-L_{6'}-Q-R₈, -C(O)-L_{6'}-NR₈R₉, -C(O)-L_{6'}-N(R₉)C(O)OR₈, -C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R9)S(O)₂NR₈R₁₀;
   or R₄ and R₅, together with the N attached thereto, form a heterocyclyl which is optionally substituted with at least one substituent selected from the group consisting of hydrogen, C₁₋C₆alkyl, C₂-C₆ alkenyl, C₂-C₆alkynyl, carbocyclyl, carbocyclylC₁-C₆alkyl, heterocyclyl, heterorycloC₁-C₆alkyl, -C(O)R₈, -C(O)OR₈, -C(O)NR₈R_{9,} -C(O)-L_{6'}-NR₈R₉, -C(O)-L₆-N(R₉)C(O)OR₈, -C(O)-L_{6'}-N(R₉)C(O)NR₈R₁₀, -S(O)ⱼR₈, and -S(O)ⱼNR₈R₉;
   wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -LD-C(O)OR_{D}, -L_{D}-NR_{D}R_{D'} and -L_{D}-C(O) NR_{D}R_{D'};
   wherein j is independently selected at each occurrence from the group consisting of 0, 1 and 2; wherein L₆, L_{6'} and L_{D} are each independently selected at each occurrence from a bond, C₁-C₆ alkylene, C₂-C₆alkenylene or C₁-C₆alkynylene;
   wherein R_{D} and R_{D}. are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆ alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A_{2,} Z, and N(R₄R₅) is independently selected at each occurrence from 5-, 6- or 7-membered carbocyclyls, and each heterocyclyl moiety in A₂, Z, and N(R₄R₅) is independently selected at each occurrence from 5-, 6- or 7-membered heterocyclyls;
   wherein at each occurrence L₁, R_{V}, L_{D}, L₆, L_{6'}, L₉, R₂, R₃, R₄, R₅, R₆, R₈, R₉, R₁₀, R_{A2}, R_{D} and R_{D'} are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁₋C₆alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl; and
   wherein each carbocyclyl and heterocyclyl moiety in A₂, Z, and N(R₄R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, - L_{H}-O-R_{K,} -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₁-C₆alkenylene or C₂-C₆alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl.
16. A compound of item 15, or a pharmaceutically acceptable salt, solvate or prodrug thereof, wherein R₁ is thiazolyl, L₁ is -CH₂-, R_{A2} is H, k is 0, R₂ and R₃ are benzyl, p is 1, and L₃ is -L₅-W-L_{5'}-, wherein L₃ and W are bonds, and L_{5'} is C₁-C₆alkylene, wherein R₄ and R₅, together with the N attached thereto, form a heterocyclyl selected from 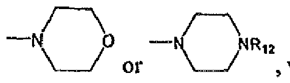 wherein R₁₂ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -C(O)R₈, -C(O)OR₈, -C(O)NR₈R₉, -C(O)-L_{6'}-NR₈R₉, -C(O)-L_{6'}-N(R₉)C(O)OR₈, -C(O)-L₆-N(R₉)C(O)NR₈R₁₀, -S(O)ⱼR₈, and -S(O)ⱼNR₈R₉, wherein each carbocyclyl and heterocyclo moiety in R₁₂ is optionally substituted with at least one moiety selected from the group consisting of halogen, oxo, thioxo, hydroxy, nitro, cyano, amino, formyl, C₁-C₆alkyl, C₂-C₆ alkenyl, C₂₋C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}.
17. A compound of item I, or a pharmaceutically acceptable salt, solvate or prodrug thereof, wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is -O-L_{A1}-, wherein L_{A1} is a bond;
   XisO;
   A₂ is -L_{A2}-N(R_{A2})-, wherein L_{A2} is a bond, and R_{A2} is hydrogen, C₁-C₆alkyl, C₂-C₆alkenyl, C₂-C₆alkynyl, carbocyclylC₁-C₆alkyl or heterocycloC₁-C₆alkyl;
   k is 0 or 1;
   L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(Rᵥ), and wherein Rᵥ is selected from hydrogen, C₁-C₆alkyl, C₂-C₆alkenyl or C₂-C₆alkynyl;
   Z, taken together with (L₃)ₚ and N(R₄R₅), forms 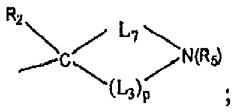 R₂ is selected from carbocycloC₁-C₆alkyl or heterocyclylC₁-C₆alkyl;
   L₇ is C₁-C₄alkylene, C₂-C₄alkenylene or C₂-C₄alkynylene, and is optionally substituted with at least one substituent selected from the group halogen, oxo, thioxo, hydroxy, nitro, cyano, amino, -O-R_{D}. -S-R_{D}, -C(O)R_{D}, -OC(O)R_{D}, -C(O)OR_{D}, -NR_{D}R_{D'} and -C(O)NR_{D}R_{D'};
   p is 0 or 1;
   L₃ is C₁-C₄alkylene, C₂-C₄alkenylene or C₂-C₄alkynylene, and are each independently optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D'} and -C(O)NR_{D}R_{D'};
   R₅ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆-O-R₈, -L₆-C(O)R₈, -C(O)OR₈, -OC(O)R₈, -C(O)NR₈R₉, -N(R₉)C(O)OR₈, -C(O)-L₆-O-R₈, -C(O)-L_{6'}-NR₈R₉, -C(O)-L_{6'}-N(R₉)C(O)OR₈, -C(O)-L_{6'}-N(R₉)C(O)NR₈R₁₀, -L₆-S(O)ⱼR₈, -L₆-N(R₉)S(O)ⱼR_{8,} -L₆-S(O)ⱼNR⁸R⁹ and -L₆-N(R₉)S(O)₂NR₈NR₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆ alkyl, heterocyclocarbocyclyC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0,1 and 2, and L₆, L_{6'} and L_{D} are each independently selected at each occurrence from a bond, C₁-C₆alkylene, C₂-C₆alkenylene or C₂-C₆alkynylene;
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆ alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A₂, R₂ and R₅ is independently selected at each occurrence from 5-, 6- or 7-membered carbocyclyls, and each heterocyclyl moiety in A₂, R₂ and R₅ is independently selected at each occurrence from 5-, 6- or 7-membered heterocyclyls;
   wherein at each occurrence L₁, Rᵥ, L_{D}, L₆, L_{6'}, L₉, R₂, R₅, R₈, R₉, R₁₀, R_{A2}, R_{D} and R_{D'} are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L}', wherein R_{L} and R_{L'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆ thioalkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl; and
   wherein each carbocyclyl and heterocyclyl moiety in A₂ and 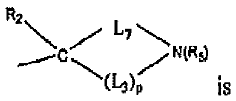 independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K,} -L_{H}-C(O)R_{K}, -L_{H}-OC(O)R_{K}, -L_{H}-C(O)OR_{K}, -L_{H}-NR_{K}R_{K',} -L_{H}-S(O)R_{K}, -L_{H}-SO₂R_{K,} -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆alkynylene, and R_{K}, R_{K'} and R_{K}" are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl.
18. A compound of item 17, or a pharmaceutically acceptable salt, solvate or prodrug thereof, wherein R₁ is thiazolyl, L₁ is -CH₂-, R_{A2} is H, k is 0, R₂ is benzyl, p is 1, L₇ is -(CH₂)₂-, and L₃ is -(CH₂)₂-.
19. A compound of item 1, or a pharmaceutically acceptable salt, solvate or prodrug thereof,
   wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is -O-L_{A1}-, wherein L_{A1} is a bond;
   X is O;
   A₂ is -L_{A2}-N(R_{A2})-, wherein L_{A2} is a bond, and R_{A2} is hydrogen, C₁-C₆alkyl, C₂-C₆alkenyl, C₂₋C₆ alkynyl, carbocyclylC₁-C₆alkyl or heterocycloC₁-C₆alkyl;
   k is 0 or 1;
   L₂ represents -L₉-V-L_{9'}-, wherein L₉ is independently selected from a bond, C₁-C₆alkylene, C₂-C₆alkenylene or C₂-C₆alkynylene, L_{9'} is a bond, and V is selected from the group consisting of a bond or -C(O)N(R_{V})-, and wherein Rᵥ is selected from hydrogen, C₁-C₆alkyl, C₂-C₆alkenyl or C₂-C₆alkynyl;
   Z is -C(R₂R₃)-, wherein R₂ is carbocyclylC₁-C₆alkyl or heterocycloC₁-C₆alkyl, and R₃ is hydrogen, C₁-C₆alkyl, C₂-C₆alkenyl or C₂-C₆alkynyl;
   p is 0 or 1;
   L₃ is C₁-C₆alkylene, C₂-C₆alkenylene or C₂-C₆alkynylene, and is optionally substituted with at least one moiety selected from halogen, oxo, thioxo, hydroxy, nitro, cyano, amino, -O-R_{D}, -S-R_{D}, -C(O)R_{D}, -OC(O)R_{D}, -C(O)OR_{D}, -NR_{D}R_{D}'and -C(O)NR_{D}R_{D'}; and
   R₅ is R_{E}-carbocyclylC₁-C₆alkyl or R_{E}-heterocycloC₁-C₆alkyl, wherein R_{E} is carbocyclyl, heterocyclyl, carbocyclylC₁-C₆alkyl or heterocycloC₁-C₆alkyl;
   R₄ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L₆-O-R₈, -L₆-C(O)R₈, -C(O)OR₈, -OC(O)R₈, -C(O)NR₈R_{9,} -N(R₉)C(O)OR₈, -C(O)-L_{6'}-O-R₈, -C(O)-L_{6'}-NR₈R₉, -C(O)-L_{6'}-N(R₉)C(O)OR₈, -C(O)-L₆-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆alkyl, heterocyclyl, heterocycloC₁-C₆alkyl, carbocyclylheterocyclylC₁-C₆ alkyl, heteroryclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'} and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆alkynylene;
   wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thioalkoxyC₁-C₆ alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A₂, Z, and N(R₄R₅) is independently selected at each occurrence from 5-, 6- or 7-membered carbocyclyls, and each heterocyclyl moiety in A₂, Z, and N(R₄R₅) is independently selected at each occurrence from 5-, 6- or 7-membered heterocyclyls;
   wherein at each occurrence L₁, Rᵥ, L_{D}, L₆, L_{6'}, L₉, R₂, R₃, R₄, R₅, R₈, R₉, R₁₀, R_{A2,} R_{E}, R_{D} and R_{D'} are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L',} wherein R_{L} and R_{L'} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl and C₁-C₆alkylaminoC₁-C₆alkyl; and
   wherein each carbocyclyl and heterocyclyl moiety in A₂, Z, and N(R₄R₅) is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K,} -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K',} -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆ alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl.
20. A compound of item 19, or a pharmaceutically acceptable salt, solvate or prodrug thereof, wherein R₁ is thiazolyl, L₁ is -CH₂-, R_{A2} is H, k is 0, R₂ is benzyl, p is 1, R₅ is pyridylbenzyl, and L₃ is C₁-C₃alkylene optionally substituted with halogen, oxo, thioxo, hydroxy, nitro, cyano, amino or formyl.
21. A compound of item 1, or a pharmaceutically acceptable salt, solvate or prodrug thereof,
   wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is -O-L_{A1}-, wherein L_{A1} is a bond;
   X is O;
   A₂ is -L_{A2}-N(R_{A2)}-, wherein L_{A2} is a bond, and R_{A2} is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{F}-O-R_{F}, -L_{F}-S-R_{F}, -L_{F}-C(O)R_{F}, -L_{F}-C(O)OR_{F}, -L_{F}-OC(O)R_{F}, -L_{F}-NR_{F}R_{F'}, -L_{F}-S(O)R_{F}, -L_{F}-SO₂R_{F}, -L_{F}-C(O)NR_{F}R_{F'}, -L_{F}-N(R_{F})C(O)R_{F'}, -L_{F}-N(R_{F})SO₂R_{F'}, -L_{F}-N(R_{F})SO₂NR_{F'}R_{F"}, -L_{E}-carbocyclyl-L₄-Y-L₄-R_{E} and -L_{E}-heterocyclyl-L₄-Y-L₄-R_{E};
   k is 0 or 1;
   L₂ represents -L₉-V-L_{9'}-, wherein L₉ is independently selected from a bond, C₁-C₆alkylene, C₂-C₆alkenylene or C₂-C₆alkynylene, L₉. is a bond, and V is selected from the group consisting of a bond or -C(O)N(R_{V})-, and wherein R_{V} is selected from hydrogen, C₁-C₆alkyl, C₂-C₆alkenyl or C₂₋C₆alkynyl;
   Z is a bond;
   p is 1, 2 or 3;
   L₃ at each occurrence independently represents -L₅-W-L_{5'}-, wherein at each occurrence W is independently selected from the group consisting of a bond, C₁-C₆alkylene, C₂-C₆alkenylene, C₂-C₆ alkynylene, -S-, -O- and -C(O)-, and wherein L₅ and L_{5'} are each independently selected at each occurrence from a bond, C₁-C₆alkylene, C₂-C₆alkenylene or C₂-C₆alkynylene, and are each independently optionally substituted with at least one substituent selected from the group consisting of halogen, oxo, thioxo, hydroxy, nitro, cyano, amino, -O-R_{M}, -S-R_{M}, -C(O)R_{M}, -OC(O)R_{M}, -C(O)OR_{M}, -NR_{M}R_{M'}, N(R_{M})C(O)R_{M'}, N(R_{M})C(O)OR_{M'}, and -C(O)NR_{M}R_{M'}, wherein R_{M} and R_{M'} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl;
   R₄ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{F}-O-R_{F}, -L_{F}-S-R_{F}, -L_{F}-C(O)R_{F}, -L_{F}-C(O)OR_{F}, -L_{F}-OC(O)R_{F}, -L_{F}-NR_{F}R_{F'}, -L_{F}-S(O)R_{F}, -L_{F}-SO₂R_{F}, -L_{F}-C(O)NR_{F}R_{F'}, -L_{F}-
   N(R_{F})C(O)R_{F'}, -L_{F}-N(R_{F})SO₂R_{F'}, -L_{F}-N(R_{F})SO₂NR_{F}R_{F"}, -L_{E}-carbocyclyl-L₄-Y-L_{4'}-R_{E} and -L_{E}-heterocyclyl-L₄-Y-L_{4'}-R_{E};
   R₅ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{E}-carbocyclyl-L₄-Y-L_{4'}-R_{E}, -L_{E}-heterocyclyl-L₄-Y-L₄-R_{E}, -L₆-O-R₈, -L₆-C(O)R₈, -C(O)OR₈, -OC(O)R₈, -C(O)NR₈R_{9,} -N(R₉)C(O)OR₈, -C(O)-L_{6'}-O-R₈, -C(O)-L_{6'}-NR₈R₉, -C(O)-L_{6'}-N(R₉)C(O)OR₈, -C(O)-L₆-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein R₈, R₉ and R₁₀ are each independently selected at each occurrence from the group consisting of hydrogen, C₁-C₆alkyl, C₂-C₆alkenyl, C₂-C₆alkynyl, C₁-C₆hydroxyalkyl, carbocyclyl, carbocyclylC₁-C₆ alkyl, heterocyclyl, heterocycloC₁-C₆alkyl, carbocyclylheterocyclylC₁-C₆alkyl, heterocyclocarbocyclylC₁-C₆alkyl, heterocycloheterocyclylC₁-C₆alkyl, carbocyclylcarbocyclylC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D}, and -L_{D}-C(O)NR_{D}R_{D'}, wherein j is independently selected at each occurrence from the group consisting of 0, 1 and 2, and L₆, L_{6'} and L_{D} are each independently selected at each occurrence from a bond, C₁-C₆alkylene, C₂-C₆alkenylene or C₂-C₆ alkynylene, and wherein R_{D} and R_{D'} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl, C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl;
   wherein L_{F}, L_{E}, L₄ and L_{4'} are each independently selected at each occurrence from a bond, C₁-C₆alkylene, C₂-C₆alkenylene or C₂-C₄alkynylene, and R_{F}, R_{F'} and R_{F"} are each independently selected at each occurrence from the group consisting of hydrogen, C₁-C₆alkyl, C₂-C₆alkenyl, C₂-C₆alkynyl, C₁-C₆ alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆ alkoxycarbonyl, C₁-C₆alkoxycarbonylC₁-C₆alkyl, C₁-C₆alkylcarbonyloxy, C₁-C₆alkylcarbonyloxyC₁-C₆ alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₆alkylene, C₂-C₆alkenylene, C₂-C₆alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{E} is independently selected at each occurrence from the group consisting of carbocyclyl, heterocyclyl, carbocyclylC₁-C₆alkyl, heterocycloC₁-C₆alkyl, C₁-C₆ alkylene, C₂-C₆alkenylene and C₂-C₆alkynylene;
   wherein each carbocyclyl moiety in R_{A2}, L₃, R₄ and R₃ is independently selected at each occurrence from 5-, 6-, 7-, 8-, 9- or 10-membered carbocyclyls, and each heterocyclyl moiety in R_{A2}, L₃, R₄ and R₅ is independently selected at each occurrence from 5-, 6-, 7-, 8-, 9- or 10-membered heterocyclyls;
   wherein at each occurrence L₁, Rᵥ, L_{D}, L₆, L_{6'}, L₉, L_{F}, L_{E}, L₄, L_{4'}, W, Y, R₄, R₅, R₈, R₉, R₁₀, R_{A2}, R_{E}, R_{F}, R_{F'}, R_{F"}, R_{D} and R_{D'} are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl; and
   wherein each carbocyclyl and heterocyclyl moiety in R_{A2}, L₃, R₄ and R₅ is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{H}-O-R_{K}, -L_{H}-S-R_{K}, -L_{H}-C(O)R_{K}, -L_{H}-OC(O)R_{K}, -L_{H}-C(O)OR_{K}, -L_{H}-NR_{K}R_{K'}, -L_{H}-S(O)R_{K}, -L_{H}-SO₂R_{K}, -L_{H}-C(O)NR_{K}R_{K'}, -L_{H}-N(R_{K})C(O)R_{K'}, -L_{H}-NR_{K}SO₂R_{K'} and -L_{H}-NR_{K}SO₂NR_{K'}R_{K"}, wherein L_{H} is independently selected at each occurrence from a bond, C₁-C₆alkylene, C₂-C₆alkenylene or C₂-C₆ alkynylene, and R_{K}, R_{K'} and R_{K"} are each independently selected at each occurrence from the group consisting of hydrogen, C₁-C₆alkyl, C₂-C₆alkenyl, C₂-C₆alkynyl, C₁-C₆alkoxyC₁-C₆alkyl, C₁-C₆thio alkoxyC₁-C₆alkyl, C₁-C₆alkylcarbonyl, C₁-C₆alkylcarbonylC₁-C₆alkyl. C₁-C₆alkoxycarbonyl, C₁-C₆ alkoxycarbonylC₁-C₆alkyl, C₁-C₆alkylcarbonyloxy, C₁-C₆alkylcarbonyloxyC₁-C₆alkyl and C₁-C₆alkyl aminoC₁-C₆alkyl,
22. A compound of item 21, or a pharmaceutically acceptable salt, solvate or prodrug thereof, wherein R₁ is thiazolyl, L₁ is -CH₂-, k is 0, p is 1, L₅ and W are bonds, and L_{5'} is C₂-C₄alkylene which is optionally substituted with at least one substituent selected from the group consisting of halogen, oxo, thioxo, hydroxy, nitro, cyano and amino, wherein R_{A2} and R₄ are each independently selected from the group consisting of C₅-C₁₀carboryclylC₁-C₆alkyl, H₅-H₁₀heterocycloC₁-C₆alkyl, -L_{G}-C₅-C₇carbocyclyl-L₁₀-U-L_{10'}-R_{H} and -L_{G}-H₅-H₇heterocyclyl-L₁₀-U-L₁₀-R₁₁, wherein at each occurrence L_{G} is independently C₁-C₃alkylene, L₁₀ and L_{10'} are each independently selected at each occurrence from a bond or C₁-C₃alkylene, and U is independently selected at each occurrence from the group consisting of a bond, -S-, -O-, -C(O)-, -C(O)O- and -OC(O)-, and wherein at each occurrence R_{H} is independently C₅-C₇ carbocyclyl, H₅-H₇heterocyclyl, C₁-C₆alkyl, C₂-C₆alkenyl or C₂-C₆alkynyl.
23. A compound of item 21, or a pharmaceutically acceptable salt, solvate or prodrug thereof, wherein R₁ is thiazolyl, L₁ is -CH₂-, k is 0, p is 1, L₅ and W are bonds, and L_{5'} is C₂-C₄alkylene which is optionally substituted with at least one substituent selected from the group consisting of NR_{M}R_{M'}, N(R_{M})C(O)R_{M'} and N(R_{M})C(O)OR_{M'}, wherein at each occurrence R_{M} is independently hydrogen, C₁-C₆ alkyl, C₁-C₆alkenyl or C₂-C₆alkynyl, and at each occurrence R_{M'} is independently C₁-C₆alkyl, C₂-C₆ alkenyl, C₂-C₆alkynyl, C₅-C₁₀carbocyclyl, C₅-C₁₀carbocyclylC₁-C₆alkyl. H₅-H₁₀heterocyclyl or H₅-H₁₀ heterocycloC₁-C₆alkyl, wherein R_{A2} and R₄ are each independently selected from the group consisting of C₅-C₁₀carbocyclylC₁-C₆alkyl, H₅-H₁₀heterocycloC₁-C₆alkyl, -L_{G}-C₅-C₇carbocyclyl-L₁₀-U-L_{10'}-R_{H} and -L_{G}-H₅-H₇heterocyclyl-L₁₀-U-L_{10'}-R_{H,} wherein at each occurrence L_{G} is independently C₁-C₃alkylene, L₁₀ and L_{10'} are each independently selected at each occurrence from a bond or C₁-C₃alkylene, and U is independently selected at each occurrence from the group consisting of a bond, -S-, -O-, -C(O)-, -C(O)O- and -OC(O)-, and wherein at each occurrence R_{H} is independently C₅-C₇carbocyclyl, H₅-H₇ heterocyclyl, C₁-C₆alkyl, C₂-C₆alkenyl or C₂-C₆alkynyl.
24. A compound of formula II, 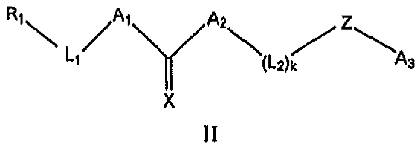 or a pharmaceutically acceptable salt, solvate or prodrug thereof, wherein
   R₁ is a 3-, 4-, 5-, 6-, 7-, 8-, 9-, or 10-membered heterocyclyl comprising at least one nitrogen ring atom;
   L₁ is a bond, C₁-C₁₀alkylene, C₂-C₁₀alkenylene or C₂-C₁₀alkynylene;
   A₁ is a bond or selected from the group consisting of -O-L_{A1}-, -S-L_{A1}-, and -N(R_{A1})-L_{A1}-,
   wherein L_{A1} is a bond, C₁-C₁₀alkylene, C₂-C₁₀alkenylene or C₂-C₁₀alkynylene, and R_{A1} is hydrogen, C₁-C₆ alkyl, C₂-C₆alkenyl or C₂-C₆alkynyl;
   X is O or S;
   A₂ is a bond or selected from the group consisting of -L_{A2}-O-, -L_{A2}-S- and -L_{A2}-N(R_{A2})-,
   wherein L_{A2} is a bond, C₁-C₁₀alkylene, C₂-C₁₀alkenylene or C₂-C₁₀alkynylene, and R_{A2} is selected from the group consisting of hydrogen, C₁-C₆alkyl, C₂-C₆alkenyl, C₂-C₆alkynyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -LD-SO₂R_{D}, -L_{D}-C(O)NR_{D}R_{D',} - L_{D}-N(R_{D})C(O)R_{D'}, -L_{D}-N(R_{D})SO₂R_{D',} -L_{D}-N(R_{D})SO₂NR_{D'}R_{D"}, carbocyclyl, carbocyclylC₁-C₆alkyl, heterocyclyl, heterocycloC₁-C₆alkyl, -L_{E}-carbocyclyl-L₄-Y-L_{4'}-R_{E} and -L_{E}-heterocyclyl-L₄-Y-L₄-R_{E};
   k is 0 or 1, and at each occurrence L₂ independently represents -L₉-V-L_{9'}-, wherein L_{9'} and L_{9'} are each independently selected at each occurrence from a bond, C₁-C₁₀alkylene, C₂-C₁₀alkenylene or C₂-C₁₀alkynylene, and V is independently selected at each occurrence from the group consisting of a bond, C₁-C₁₀alkylene, C₂-C₁₀alkenylene, C₂-C₁₀alkynylene, -S-, -O-, -C(O)-, -N(R_{V})C(O)-, -C(O)N(R_{V})-, -C(O)O- and -OC(O)-, wherein Rᵥ is independently selected at each occurrence from hydrogen, C₁-C₆ alkyl, C₂-C₆alkenyl or C₂-C₆alkynyl;
   Z is -C(R₂R₃)-, =C(R₂)- or -C(R₂)=;
   or Z is selected from the group consisting of 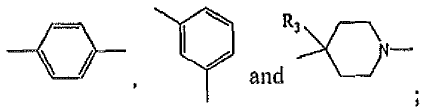 A₃ is selected from the group consisting of hydrogen, C₁-C₆alkyl, C₂-C₆alkenyl, C₂-C₆alkynyl,
   carbocyclyl, carbocyclylC₁-C₆alkyl, heterocyclyl, heterocycloC₁-C₆alkyl, -L_{E}-carbocyclyl-L₄-Y-L₄.-R_{E}, -L_{E}-heterocyclyl-L₄-Y-L₄-R_{E}, -L₆-O-R₈, -L₆-C(O)R₈, -L₆-C(O)OR₈, -L₆-OC(O)R₈, -L₆-C(O)NR₈R₉, -L₆-N(R₈)-C(O)R₉, -L₆-N(R₉)C(O)OR₈, -L₆-NR₈R₉, -L₆-C(O)-L_{6'}-NR₈R₉, -L₆-C(O)-L₆-N(R₉)C(O)OR₈, -L₆-C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀. wherein j is independently selected at each occurrence from the group consisting of 0, 1 and 2, wherein L₆ and L_{6'} are each independently selected at each occurrence from a bond, C₁-C₁₀alkylene, C₂-C₁₀alkenylene or C₂-C₁₀alkynylene, wherein R₈, R₉ and R₁₀ are each independently selected at each occurrence from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D',} -L_{D}-N(R_{D})SO₂R_{D'}, -L_{D}-N(R_{D})SO₂NR_{D'}R_{D"}, -L_{E}-carbocyclyl-L₄-Y-L₄-R_{E} and -L_{E}-heterocyclyl-L₄-Y-L₄-R_{E};
   wherein R₂ is selected from the group consisting of carbocyclyl, carbocyclylC₁-C₆alkyl,
   heterocyclyl, heterocycloC₁-C₆alkyl, -L_{D}-O-R_{E}, -L_{D}-S-R_{E}, -L_{D}-C(O)R_{E}, -L_{D}-OC(O)R_{E}, -L_{D}-C(O)OR_{E}, -L_{D}-N_{D}R_{E}R_{D}, -L_{D}-S(O)R_{E}, -L_{D}-SO₂R_{E}, -L_{D}-C(O)NR_{D}R_{E}, -L_{D}-N(R_{D})C(O)R_{E}, -L_{D}-N(R_{D})SO₂R_{E}, -L_{D}-N(R_{D)}SO₂NR_{D'}R_{E}, -L_{E}-carbocyclyl-L₄-Y-L_{4'}-R_{E}, and -L_{E}-heterocyclyl-L₄-Y-L_{4'}-R_{E,} wherein R₃ is selected from the group consisting of hydrogen, C₁-C₆alkyl, C₂-C₆alkenyl, C₂-C₆ alkynyl, carbocyclyl, carbocyclylC₁-C₆alkyl, heterocyclyl, heterocycloC₁-C₆alkyl, -L_{D}-O-R_{D}, -L_{D}-S-R_{D,} -L_{D}-C(O)R_{D}, -L_{D}-OC(O)R_{D}, -L_{D}-C(O)OR_{D}, -L_{D}-NR_{D}R_{D'}, -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D"}, -L_{E}-carbocyclyl- L₄-Y-L_{4'}-R_{E} and -L_{E}-heterocyclyl-L₄-Y-L_{4'}-R_{E};
   wherein L_{D}, L_{E}, L₄ and L₄, are each independently selected at each occurrence from a bond, C₁-C₁₀alkylene, C₂-C₁₀alkenylene or C₂-C₁₀alkynylene, wherein R_{D}, R_{D'} and R_{D"} are each independently selected at each occurrence from the group consisting of hydrogen, C₁-C₆alkyl, C₂-C₆alkenyl, C₂-C₆ alkynyl, C₁-C₆alkoxyC₁-C₆alkyl, C₁-C₆thioalkoxyC₁-C₆alkyl, C₁-C₆alkylcarbonyl, C₁-C₆alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkyl carbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl, wherein Y is independently selected at each occurrence from the group consisting of a bond, C₁-C₁₀alkylene, C₂-C₁₀alkenylene, C₂-C₁₀alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{E} is independently selected at each occurrence from carbocyclyl, heterocyclyl, carbocyclylC₁-C₆alkyl or heterocycloC₁-C₆ alkyl;
   wherein at each occurrence L₁, L_{A1}, R_{A1}, Y, V, R_{Y}, R_{V}, L_{A2,} R_{A2,} L_{D}, L_{E}, L₄, L_{4'},L₆, L_{6'}, L₉, L_{9'}, R₂, R₃, R₈, R₉, R₁₀, R_{E}, R_{D}, R_{D'}, R_{D"} and A₃ are each independently optionally substituted with at least one substituent selected from the group consisting of halogen, oxo, thioxo, hydroxy, nitro, cyano, amino, formyl, carbocyclyl, heterocyclyl, -O-R_{L}, -S-R_{L}, -C(O)R_{L}, -OC(O)R_{L}, -C(O)OR_{L}, -NR_{L}R_{L'}, -S(O)R_{L}, -SO₂R_{L}, -C(O)NR_{L}R_{L'}, -N(R_{L})C(O)R_{L'}, -N(R_{L})SO₂R_{L'} and -N(R_{L})SO₂NR_{L'}R_{L"}, and wherein R_{L}, R_{L'} and R_{L"} are each independently selected at each occurrence from the group consisting of hydrogen, C₁-C₆ alkyl, C₂-C₆alkenyl, C₂-C₆alkynyl, C₁-C₆alkoxyC₁-C₆alkyl, C₁-C₆thioalkoxyC₁-C₆alkyl, C₁-C₆alkyl carbonyl, C₁-C₆alkylcarbonylC₁-C₆alkyl, C₁-C₆alkoxycarbonyl, C₁-C₆alkoxycarbonylC₁-C₆alkyl, C₁-C₆ alkylcarbonyloxy, C₁-C₆alkylcarbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl;
   wherein each carbocyclyl moiety in L₁, A₁, A₂, (L₂)ₖ, Z and A₃ is independently selected at each occurrence from 3-, 4-, 5-, 6-, 7-, 8-, 9- or 10-membered carbocyclyls, and each heterocyclyl moiety in L₁, A₁, A₂, (L₂)ₖ, Z and A₃ is independently selected at each occurrence from 3-, 4-, 5-, 6-, 7-, 8-, 9- or 10-membered heterocyclyls; and
   wherein each carbocyclyl and heterocyclyl moiety in said compound is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -L_{S}-O-R_{S}, - L_{S}-S-R_{S}, -L_{S}-C(O)R_{S}, -L_{S}-OC(O)R_{S}, -L_{S}-C(O)OR_{S}, -L_{S}-NR_{S}R_{S'}, -L_{S}-S(O)R_{S}, -L_{S}-SO₂R_{S}, -L_{S}-C(O)NR_{S}R_{S'}, -L_{S}-N(R_{S})C(O)R_{S'}, -L_{S}-N(R_{S})SO₂R_{S'}, -L_{S}-N(R_{S})SO₂NR_{S'}R_{S"}, carbocyclyl, carbocyclylC₁-C₆alkyl, heterocyclyl and hetercycloC₁-C₆alkyl, wherein at each occurrence L_{S} is independently a bond, C₁-C₁₀alkylene, C₂-C₁₀alkenylone or C₂-C₁₀alkynylone, and R_{S}, R_{S'} and R_{S"} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆alkylaminoC₁-C₆alkyl, C₃-C₁₀carbocyclyl, C₃-C₁₀carbocyclylC₁-C₆alkyl, H₃-H₁₀heterocyclyl and H₃-H₁₀heterocycloC₁-C₆alkyl;
   with the proviso that said compound is not ritonavir.
25. A compound of item 24, or a pharmaceutically acceptable salt, solvate or prodrug thereof, wherein
   R₁ is a 5- or 6-membered heterocyclyl comprising at least one nitrogen ring atom;
   L₁ is a bond, C₁-C₆alkylene, C₂-C₆alkenylene or C₂-C₆alkynylene;
   A₁ is-O-L_{A1}-, wherein L_{A1} is a bond;
   X is O;
   A₂ is-L_{A2}-N(R_{A2})-, wherein L_{A2} is a bond, and R_{A2} is selected from the group consisting of hydrogen, C₁-C₆alkyl, C₂-C₆alkenyl, C₂-C₆alkynyl, -C(O)R_{F}, -C(O)OR_{F}, carbocyclylC₁-C₆alkyl and heterocycloC₁-C₆alkyl, wherein R_{F} is independently selected at each occurrence from hydrogen, C₁-C₆ alkyl, C₂-C₆alkenyl, C₂-C₆alkynyl, carbocyclyl, heterocyclyl, carbocyclylC₁-C₆alkyl or heterocycloC₁-C₆ alkyl;
   k is 0 or 1;
   L₂ represents -L₉-V-L₉-, wherein L₉ and L_{9'} are each independently selected from a bond, C₁-C₆ alkylene, C₂-C₆alkenylene or C₂-C₆alkynylene, and wherein V is selected from the group consisting of a bond, -S-, -O-, -C(O)- and -C(O)N(R_{V})-, and R_{V} is selected from hydrogen, C₁-C₆alkyl, C₂-C₆alkenyl or C₂-C₆alkynyl;
   Z is -C(R₂R₃)-, wherein R₂ is carbocyclylC₁-C₆alkyl, heterocycloC₁-C₆alkyl, R_{E}-carbocyclylC₁-C₆alkyl- or R_{E}-heterocyclylC₁-C₆alkyl-, and R₃ is hydrogen, C₁-C₆alkyl, C₂-C₆alkenyl, C₂-C₆alkynyl, carbocyclylC₁-C₆alkyl, heterocycloC₁-C₆alkyl, R_{E}-carbocyclylC₁-C₆alkyl- or R_{E}-heterocyclylC₁-C₆ alkyl-;
   A₃ is selected from the group consisting of hydrogen, C₁-C₆alkyl, C₂-C₆alkenyl, C₂-C₆alkynyl, carbocyclyl, carbocyclylC₁-C₆alkyl, heterocyclyl, heterocycloC₁-C₆alkyl, -L_{E}-carbocyclyl-L₄-Y-L₄-R_{E}, -L_{E}-heterocyclyl-L₄-Y-L₄,-R_{E}, -L₆-O-R₈, -L₆-C(O)R₈, -L₆-C(O)OR₈, -L₆-OC(O)R₈, -L₆-C(O)NR₈R₉, -L₆-N(R₈)-C(O)R₉, -L₆-N(R₉)C(O)OR₈, -L₆-NR₈R₉, -L₆-C(O)-L_{6'}-NR₈R_{9,} -L₆-C(O)-L_{6'}-N(R₉)C(O)OR₈, -L₆-C(O)-L_{6'}-N(R₉)C(O)NR₈R₁₀, -L₆-S(O)ⱼR₈, -L₆-N(R₉)S(O)ⱼR₈, -L₆-S(O)ⱼNR₈R₉ and -L₆-N(R₉)S(O)₂NR₈R₁₀, wherein j is independently selected at each occurrence from the group consisting of 0, 1 and 2, wherein L₆ and L_{6'} are each independently selected at each occurrence from a bond, C₁-C₆alkylene, C₂-C₆alkenylene or C₂-C₆alkynylene, wherein R₈, R₉ and R₁₀ are each independently selected at each occurrence from the group consisting of hydrogen, C₁-C₆alkyl, C₂-C₆ alkenyl, C₂-C₆alkynyl, carbocyclyl, carbocyclylC₁-C₆alkyl, heterocyclyl, heterocycloC₁-C₆alkyl, -L_{D}-O-R_{D}, -L_{D}-S-R_{D}, -L_{D}-C(O)R_{D}, -L_{D}-OC(O)R_{D}, -L_{D}-C(O)OR_{D}, -L_{D}-NR_{D}R_{D',} -L_{D}-S(O)R_{D}, -L_{D}-SO₂R_{D}, -L_{D}-C(O)NR_{D}R_{D'}, -L_{D}-N(R_{D})C(O)R_{D'}, -L_{D}-N(R_{D})SO₂R_{D'}, -L_{D}-N(R_{D})SO₂NR_{D'}R_{D"}, -L_{E}-carbocyclyl-L₄-Y-L_{4'}-R_{E} and -L_{E}-heterocyclyl-L₄-Y-L_{4'}-R_{E};
   wherein R_{E} is independently selected at each occurrence from carbocyclyl, heterocyclyl, carbocyclylC₁-C₆alkyl or heterocycloC₁-C₆alkyl;
   wherein L_{D}, L_{E}, L₄ and L_{4'} are each independently selected at each occurrence from a bond, C₁-C₆ alkylene, C₂-C₆alkenylene or C₂-C₆alkynylene, wherein Y is independently selected at each occurrence from the group consisting of a bond, C₁-C₁₀alkylene, C₂-C₁₀alkenylene, C₂-C₁₀alkynylene, -S-, -O-, -C(O)-, -N(R_{Y})C(O)-, -C(O)N(R_{Y})-, -C(O)O- and -OC(O)-, and R_{Y} is independently selected at each occurrence from hydrogen, C₁-C₆alkyl, C₂-C₆alkenyl or C₂-C₆alkynyl, and wherein R_{D}, R_{D'}, and R_{D"} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl, C₁-C₆alkylaminoC₁-C₆alkyl, carbocyclyl, carbocyclylC₁-C₆alkyl, heterocyclyl and heterocycloC₁-C₆alkyl,
   wherein at each occurrence L₁, L₄, L_{4'}, L₆, L_{6'}, L₉, L_{9'}, L_{D}, L_{E}, L_{F}, R₂, R₃, R₈, R₉, R₁₀, R_{A2}, R_{D}, R_{D'}, R_{D"}, R_{E}, R_{F}, R_{V}, R_{Y} and Y are each independently optionally substituted with at least one substituent selected from the group consisting of halogen, oxo, thioxo, hydroxy, nitro, cyano, amino, -O-R_{L}, -S-R_{L}, -C(O)R_{L}, -OC(O)R_{L}, -C(O)OR_{L}, -NR_{L}R_{L'} and -C(O)NR_{L}R_{L'}, wherein R_{L} and R_{L'} are each independently selected at each occurrence from the group consisting of hydrogen, C₁-C₆alkyl, C₂-C₆ alkenyl, C₂-C₆alkynyl, C₁-C₆alkoxyC₁-C₆alkyl, C₁-C₆thioalkoxyC₁-C₆alkyl, C₁-C₆alkylcarbonyl, C₁-C₆ alkylcarbonylC₁-C₆alkyl, C₁-C₆alkoxycarbonyl, C₁-C₆alkoxycarbonylC₁-C₆alkyl, C₁-C₆alkylcarbonyloxy, C₁-C₆alkylcarbonyloxyC₁-C₆alkyl and C₁-C₆alkylaminoC₁-C₆alkyl;
   wherein each carbocyclyl moiety in A_{2,} Z and A₃ is independently selected at each occurrence from 5-, 6- or 7-membered carbocyclyls, and each heterocyclyl moiety in A₂, Z and A₃ is independently selected at each occurrence from 5-, 6- or 7-membered heterocyclyls; and
   wherein each carbocyclyl and heterocyclyl moiety in A₂, Z and A₃ is independently optionally substituted at each occurrence with at least one substituent selected from the group consisting of halogen, oxo, thioxo, hydroxy, nitro, cyano, amino, formyl, C₁-C₆alkyl, C₂-C₆alkenyl, C₂-C₆alkynyl, -Lₛ-O-Rₛ, -Lₛ-S-Rₛ, -Lₛ-C(O)Rₛ, -Lₛ-OC(O)Rₛ, -Lₛ-C(O)ORₛ, -Lₛ-NRₛR_{s'}, -Lₛ-S(O)Rₛ, -Lₛ-SO₂Rₛ, -Lₛ-C(O)NRₛR_{s'}, -Lₛ-N(Rₛ)C(O)R_{s',} -Lₛ-NRₛSO₂R_{s'} and -Lₛ-NRₛSO₂NR_{s'}R_{s"}.
26. A compound of item25, or a pharmaceutically acceptable salt, solvate or prodrug thereof, wherein R₁ is thiazolyl, L₁ is -CH₂-, R_{A2} is hydrogen, k is 0, R₂ is carbocyclylC₁-C₆alkyl, and A₃ is C₁-C₆ alkyl, C₂-C₆alkenyl, C₂-C₆alkynyl, -L₆-O-R₈, -L₆-S-R₈, -L₆-C(O)R₈, -L₆-C(O)OR₈, or -L₆-OC(O)R₈, wherein L₆ is a bond, C₁-C₆alkyl, C₂-C₆alkenyl or C₂-C₆alkynyl,, and R₈ is hydrogen, C₁-C₆alkyl, C₂-C₆ alkenyl or C₁-C₆alkynyl.
27. A compound, or a pharmaceutically acceptable salt, solvate or prodrug thereof, wherein said compound is selected from the group consisting of:
   1,3-thiazol-5-ylmethyl (1*S*,3*S*,4*S*)-3-hydroxy-4-{[*N*-(methoxycarbonyl)-3-methyl-L-valyl]amino} -5-phenyl-1-(4-pyridin-2-ylbenzyl)pentylcarbamate;
   methyl (1*S*)-1-({[(1*R*,3*S*,4*S*)-3-hydroxy-4-({3-methyl-*N*-[(1,3-thiazol-5-ylmethoxy)carbonyl]-L-vatyl}amino)-5-phenyl-1-(4-pyridin-2-ylbenzyl)pentyl]amino}carbonyl)-2,2-dimethylpropylcarbamate;
   methyl (1*S*)-1-({[(1*S*,3*S*,4*S*)-3-hydroxy-4-({3-methyl-*N*-[(1,3-thiazol-5-ylmethoxy)carbonyl]-L-valyl}amino)-5-phenyl-1-(4-pyridin-2-ylbenzyl)pentyl]amino}carbonyl)-2,2-dimethylpropylcarbamate;
   tert-butyl (1*S*,3*S*,4*S*)-3-hydroxy-5-phenyl-1-(4-pyridin-2-ylbenzyl)-4-{[(1,3-thiazol-5-ylmethoxy) carbonyl]amino} pentylcarbamate;
   *tert*-butyl (1*S*,3*S*,4*S*)-3-hydroxy-4-({3-methyl-*N*-[(1,3-thiazol-5-ylmethoxy)carbonyl]-L-valyl} amino)-5-phenyl-1-(4-pyridin-2-ylbenzyl)pentylcarbamate;
   *tert*-butyl (1*S*,3*S*,4*S*)-3-hydroxy-5-phenyl-1-(4-pyridin-2-ylbenzyl)-4-({*N*-[(1,3-thiazol-5-yl methoxy)carbonyl]-L-alanyl}amino)pentylcarbamate;
   methyl (1*S*,3*S*,4*S*)-3-hydroxy-4-({3-methyl-*N*-[(1,3-thiazol-5-ylmethoxy)carbonyl]-L-valyl} amino)-5-phenyl-1-(4-pyridin-2-ylbenzyl)pentylcarbamate;
   methyl (1*S*,3*S*,4*S*)-3-hydroxy-5-phenyl-1-(4-pyridin-2-ylbenzyl)-4-({*N*-[(1-3-thiazol-5-yl methoxy)carbonyl]-L-alanyl}amino)pentylcarbamate;
   *N*¹-((1*S*,3*S*,4*S*)-3-hydroxy-5-phenyl-1-(4-pyridin-2-ylbenzyl)-4-{[(1,3-thiazol-5-ylmethoxy) carbonyl]amino} pentyl)-*N*²-(methoxycarbonyl)-3-methyl-L-valinamide;
   1,3-thiazol-5-ylmethyl (1*S*,3*S*,4*S*)-1-benzyl-3-hydroxy-5-phenyl-4-{[(1,3-thiazol-5-ylmethoxy) carbonyl]amino}pentylcarbamate;
   N¹-{(1S,2S,4S)-1-benzyl-4-[(tert-butoxycarbonyl)amino]-2-hydroxy-5-phenylpentyl}-3-methyl-N²-[(1,3-thiazol-5-ylmethoxy)carbonyl]-L-valinamide;
   N¹-((1S,2S,4S)-1-benzyl-2-hydroxy-5-phenyl-4-{[(1,3-thiazol-5-ylmethoxy)carbonyl]amino} pentyl)-N²-(methoxycarbonyl)-3-methyl-L-valinamide;
   1,3-thiazol-5-ylmethyl (1S,2S,4S)-4-(acetylamino)-1-benzyl-2-hydroxy-5-phenylpentyl carbamate;
   1,3.thiazol-5-ylmethyl (1S,2S,2S,4S)-4-amino-1-benzyl-2-hydroxy-5-phenylpentylcarbamate;
   (1S,3S)-3-(acetylamino)-4-phenyl-1-((1S)-2-phenyl-1-{[(1,3-thiazol-5-ylmethoxy)carbonyl] amino}ethyl)butyl acetate;
   1,3-thiazol-5-ylmethyl (1S,2S,4S)-1-benzyl-2-hydroxy-4-[(methylsulfonyl)amino]-5-phenyl pentylcarbamate;
   1,3-thiazol-5-ylmethyl (1S,2S,4S)-1-benzyl-4-{[(dimethylamino)carbonyl]amino}-2-hydroxy-5-phenylpentylcarbamate;
   methyl (1*S*,3*S*,4*S*)-1-benzyl-3-hydroxy-5-phenyl-4-{[(1,3-thiazol-5-ylmethoxy)carbonyl]amino} pentylcarbamate;
   1,3-thiazol-5-ylmethyl (1S,2S,4S)-1-benzyl-4-{[(dimethylamino)sulfonyl]amino}-2-hydroxy-5-phenylpentylcarbamate;
   *tert*-butyl (1*S*,3*S*,4*S*)-1-benzyl-3-hydroxy-5-phenyl-4-{[(1,3-thiazol-5-ylmethoxy)carbonyl] amino}pentylcarbamate;
   1,3-thiazol-5-ylmethyl (1S,2S,4S)-1-benzyl-2-hydroxy-4-{[(2S)-3-methyl-2-(2-oxotetrahydro pyrimidin-1(2H)-yl)butanoyl]amino}-5-phenylpentylcarbamate;
   isobutyl (1*S*,3*S*,4*S*)-1-benzyl-3-hydroxy-5-phenyl-4-{[(1,3-thiazol-5-ylmethoxy)carbonyl] amino}pentylcarbamate;
   isopropyl (1*S*,3*S*,4*S*)-1-benzyl-3-hydroxy-5-phenyl-4-{[(1,3-thiazol-5-ylmethoxy)carbonyl] amino}pentylcarbamate;
   *tert*-butyl (1*S*,3*R*,4*S*)-1-benzyl-3-hydroxy-5-phenyl-4-{[(1,3-thiazol-5-ylmethoxy)carbonyl] amino}pentylcarbamate;
   1,3-thiazol-5-ylmethyl (48,5S)-4-benzyl-5-{(2S)-2-[(4S)-4-isopropyl-2,5-dioxoimidazolidin-1-yl]-3-phenylpropyl}-2-oxo-1,3-oxazolidine-3-carboxylate;
   N¹-((1S,3S,4S)-1-benzyl-3-hydroxy-5-phenyl-4-{[(1,3-thiazol-5-ylmethoxy)carbonyl]amino} pentyl)-L-valinamide;
   N¹-((1S,3S,4S)-1-benzyl-3-hydroxy-5-phenyl-4-{[(1,3-thiazol-5-ylmethoxy)carbonyl]amino} pentyl)-N²-[(dimethylamino)carbonyl]-L-valinamide;
   N¹-((1S,3S,4S)-1-benzyl-3-hydroxy-5-phenyl-4-{[(1,3-thiazol-5-ylmethoxy)carbonyl]amino} pentyl)-N²-[(methylamino)carbonyl]valinamide;
   N¹-((1S,3S,4S)-1-benzyl-3-hydroxy-5-phenyl-4-{[(1,3-thiazol-5-ylmethoxy)carbonyl]amino} pentyl)-N²-[(2-hydroxypropoxy)carbonyl]-L-valinamide;
   1,3-thiazol-5-ylmethyl (1S)-1-[(4S,6S)-4-benzyl-2-oxo-1,3-oxazinan-6-yl]-2-phenylethyl carbamate;
   N¹-((1S,3S,4S)-1-benzyl-3-hydroxy-5-phenyl-4-{[(1,3-thiazol-5-ylmethoxy)carbonyl]amino} pentyl)-N²-[(2,3-dihydroxypropoxy)carbonyl]-L-valinamide;
   1,3-thiazol-5-ylmethyl (1S,2S,4S)-1-benzyl-2-hydroxy-5-phenyl-4-{[(4-phenylpiperazin-1-yl) carbonyl]amino}pentylcarbamate;
   *N*¹-((1*S*,3*S*,4*S*)-1-benzyl-3-hydroxy-5-phenyl-4-{[(1,3-thiazol-4-ylmethoxy)carbonyl]amino} pentyl)-*N*²-{[[(2-isopropyl-1,3-thiazol-4-yl)methyl](methyl)amino]carbonyl}-L-valinamide;
   1,3-thiazol-4-ylmethyl (1*S*,3*S*,4*S*)-1-benzyl-3-hydroxy-5-phenyl-4-{[(1,3-thiazol-4-ylmethoxy) carbonyl]amino}pentylcarbamate;
   1,3-thiazol-5-ylmethyl (1S,2S,4S)-1-benzyl-2-hydroxy-4-[(4S)-4-isopropyl-2,5-dioxo imidazolidin-1-yl]-5-phenylpentylcarbamate;
   1,3-thiazol-5-ylmethyl (1S,2S,4S)-1-benzyl-2-hydroxy-4-{[(2-isopropyl-1,3-thiazol-4-yl)acetyl] amino}-5-phenylpentylcarbamate;
   1,3-thiazol-5-ylmethyl (1S,2S,4S)-1-benzyl-4-{[(2,6-dimethylphenoxy)acetyl]amino}-2-hydroxy-5-phenylpentylcarbamate;
   1,3-thiazol-5-ylmethyl (1S,2S,4S)-1-benzyl-4-{[(tert-butylamino)carbonyl]amino}-2-hydroxy-5-phenylpentylcarbamate;
   N¹-((1S,3S,4S)-1-benzyl-3-hydroxy-5-phenyl-4-{[(1,3-thiazol-5-ylmethoxy)carbonyl]amino} pentyl)-N²-(methoxycarbonyl)-3-methyl-L-valinamide;
   1,3-thiazol-5-ylmethyl (1S,3S,4S)-1-benzyl-4-{[(2,6-dimethylphenoxy)acetyl]amino}-3-hydroxy-5-phenylpentylcarbamate;
   *tert*-butyl (1*S*,2*R*)-1-benzyl-2-hydroxy-3-{isobutyl[(1,3-thiazol-5-ylmethoxy)carbonyl]amino} propylcarbamate;
   N,N-dimethyl-N-[(1,3-thiazol-5-ylmethoxy)carbonyl]-L-phenylalaninamide;
   *N*-[(1*S*)-1-benzyl-2-morpholin-4-yl-2-oxoethyl]-*N*-[(1,3-thiazol-5-ylmethoxy)carbonyl] amine;
   N,N-diisobutyl-N-[(1,3-thiazol-5-ylmethoxy)carbonyl]-L-phenylalaninamide;
   N-isobutyl-N-[(1,3-thiazol-5-ylmethoxy)carbonyl]-L-phenylalaninamide;
   N,N-dibenzyl-N-[(1,3-thiazol-5-ylmethoxy)carbonyl]-L-phenylalaninamide;
   N-benzyl-N-(2-phenylethyl)-N-[(1,3-thiazol-5-ylmethoxy)carbonyl]-L-phenylalaninamide;
   *tert*-butyl (1*S*,2*S*)-1-benzyl-3-{benzyl[(1,3-thiazol-5-ylmethoxy)carbonyl]amino}-2-hydroxy propylcarbamate;
   *tert*-butyl (1*S*,2*R*)-1-benzyl-3-{benzyl[(1,3-thiazol-5-ylmethoxy)carbonyl]amino}-2-hydroxy propylcarbamate;
   *tert*-butyl benzyl((2*R*,3*S*)-2-hydroxy-4-phenyl-3-{[(1,3-thiazol-5-ylmethoxy)carbonyl]amino} butyl)carbamate;
   1,3-thiazol-5-ylmethyl 4-[benzyl(pyridin-4-ylacetyl)amino]phenylcarbamate;
   benzyl benzyl(4-{[(1,3-thiazol-5-ylmethoxy)carbonyl]amino}phenyl)carbamate;
   *tert*-butyl 3-{[(1,3-thiazol-5-ylmethoxy)carbonyl]amino}phenylcarbamate;
   1,3-thiazol-5-ylmethyl 4-{benzyl[(3-chlorophenyl)acetyl]amino}phenylcarbamate;
   1,3-thiazol-5-ylmethyl 3-{[(3-chlorophenyl)acetyl]amino}phenylcarbamate;
   1,3-thiazol-5-ylmethyl 3-[(pyridin-4-ylacetyl)amino]phenylcarbamate;
   N²-(tert-butoxycarbonyl)-N²-methyl-N¹-(3-{[(1,3-thiazol-5-ylmethoxy)carbonyl amino}phenyl)-L-valinamide;
   1,3-thiazol-5-ylmethyl 3-({[(3-methylphenyl)amino]carbonyl}amino)phenylcarbamate;
   [3-((2S)-2-tert-Butoxycarbonylamino-3-phenyl-propionylamino)-phenyl]-carbamic acid thiazol-5-ylmethyl ester;
   1,3-thiazol-5-ylmethyl 3-({(2S)-2-[(tert-butoxycarbonyl)amino]-2-phenylethanoyl}amino) phenylcarbamate;
   1,3-thiazol-5-ylmethyl 3-[(3,3-dimethylbutanoyl)amino]phenylcarbamate;
   N²-(tert-butoxycarbonyl)-N¹-(3-{[(1,3-thiazol-5-ylmethoxy)carbonyl]amino}phenyl)-L-isoleucinamide;
   N²-(tert-butoxycarbonyl)-N¹-(3-{[(1,3-thiazol-5-ylmethoxy)carbonyl]amino}phenyl)-L-valinamide;
   N²-(tert-butoxycarbonyl)-3-methyl-N¹-(3-{[(1,3-thiazol-5-ylmethoxy)carbonyl]amino} phenyl)-L-valinamide;
   1,3-thiazol-5-ylmethyl 3-({(2S)-2-[(tert-butoxycarbonyl)amino]-2-cyclohexylethanoyl}amino) phenylcarbamate;
   dibenzyl 2-(4-benzyl-4-{[(1,3-thiazol-5-ylmethoxy)carbonyl]amino}piperidin-1-yl)ethylimido dicarbonate;
   1,3-thiazol-5-ylmethyl 4-benzyl-1-(2-{[(4-methylphenyl)sulfonyl]amino}ethyl)piperidin-4-yl carbamate;
   1,3-thiazol-5-ylmethyl 4-benzyl-1-(2-{[(1,3-thiazol-5-ylmethoxy)carbonyl]amino}ethyl) piperidin-4-ylcarbamate;
   1,3-thiazol-5-ylmethyl 4-benzyl-1-[2-(dibenzylamino)ethyl]piperidin-4-ylcarbamate;
   1,3-thiazol-5-ylmethyl 1-(2-aminoethyl)-4-benzylpiperidin-4-ylcarbamate;
   1,3-thiazol-5-ylmethyl 1,1-dibenzyl-3-[4-(4-nitrophenyl)piperazin-1-yl]propylcarbamate;
   1,3-thiazol-5-ylmethyl 1,1-dibenzyl-3-morpholin-4-ylpropylcarbamate;
   1,3-thiazol-5-ylmethyl 4-(3-benzyl-4-phenyl-3-{[(1,3-thiazol-5-ylmethoxy)carbonyl] amino} butyl)piperazine-1-carboxylate;
   1,3-thiazol-5-ylmethyl 1,1-dibenzyl-3-{4-[(4-methylphenyl)sulfonyl]piperazin-1-yl}propyl carbamate;
   ethyl 4-[4-(3-benzyl-4-phenyl-3-{[(1,3-thiazol-5-ylmethoxy)carbonyl]amino}butyl)piperazin-1-yl]benzoate;1,3-thiazol-5-ylmethyl 1,1-dibenzyl-3-piperazin-1-ylpropylcarbamate;
   1,3-thiazol-5-ylmethyl 4-benzyl-1-[(4-methylphenyl)sulfonyl]piperidin-4-ylcarbamate;
   1,3-thiazol-5-ylmethyl 4-benzyl-4-{[(1,3-thiazol-5-ylmethoxy)carbonyl]amino}piperidine-1-carboxylate;
   1,3-thiazol-5-ylmethyl 1,4-dibenzylpiperidin-4-ylcarbamate;
   1,3-thiazol-5-ylmethyl (1S,2R)-1-benzyl-2-hydroxy-3-[isobutyl(4-pyridin-2-ylbenzyl)amino] propylcarbamate;
   tert-butyl 2-((2S,3S)-2-hydroxy-4-phenyl-3-{[(1,3-thiazol-5-ylmethoxy)carbonyl]amino}butyl)-2-(4-pyridin-2-ylbenzyl)hydrazinecarboxylate;
   1,3-thiazol-5-ylmethyl benzyl(3-{benzyl[(1,3-thiazol-5-ylmethoxy)carbonyl]amino}propyl carbamate;
   1,3-thiazol-5-ylmethyl benzyl(3-{benzyl[(1,3-thiazol-5-ylmethoxy)carbonyl]amino}-2-hydroxy propyl)carbamate;
   1,3-thiazol-5-ylmethyl benzyl(3-{benzyl[(1,3-thiazol-5-ylmethoxy)carbonyl]amino}-2-oxo propyl) carbamate;
   1,3-thiazol-5-ylmethyl benzyl[9-benzyl-10-oxo-12-(1,3-thiazol-5-yl)-3,6,11-trioxa-9-azadodec-1-yl]carbamate;
   1,3-thiazol-5-ylmethyl cyclohexylmethyl(3-{(cyclohexylmethyl)[(1,3-thiazol-5-ylmethoxy) carbonyl]amino}propyl)carbamate;
   methyl 4-({(3-{[4-(methoxycarbonyl)benzyl][(1,3-thiazol-5-ylmethoxy)carbonyl]amino}propyl) [(1,3-thiazol-5-ylmethoxy)carbonyl]amino}methyl)benzoate;
   1,3-thiazol-5-ylmethyl 4-benzoylbenzyl(3-{(4-benzoylbenzyl)[(1,3-thiazol-5-ylmethoxy) carbonyl]amino}propyl)carbamate;
   1,3-thiazol-5-ylmethyl quinolin-3-ylmethyl(3-{(quinolin-3-ylmethyl)[(1,3-thiazol-5-ylmethoxy) carbonyl]amino}propyl)carbamate;
   1,3-thiazol-5-ylmethyl 4-pyridin-2-ylbenzyl(3-{(4-pyridin-2-ylbenzyl)[(1,3-thiazol-5-ylmethoxy) carbonyl]amino} propyl)carbamate
   1,3-thiazol-5-ylmethyl 4-(benzyloxy)benzyl(3-{[4-(benzyloxy)benzyl][(1,3-thiazol-5-ylmethoxy) carbonyl]amino}propyl)carbamate;
   1,3-thiazol-5-ylmethyl 3-{[(1,3-thiazol-5-ylmethoxy)carbonyl][4-(1*H*-1,2,4-triazol-1-yl)benzyl] amino}propyl[4-(1*H*-1,2,4-triazol-1-yl)benzyl]carbamate;
   1,3-thiazol-5-ylmethyl 4-methoxybenzyl(3-{(4-methoxybenzyl)[(1,3-thiazol-5-ylmethoxy) carbonyl]amino}propyl)carbamate;
   *tert*-butyl 4-methoxybenzyl(3-{(4-methoxybenzyl)[(1,3-thiazol-5-ylmethoxy)carbonyl]amino} propyl)carbamate;
   1,3-thiazol-5-ylmethyl 4-*tert*-butylbenzyl(3-{(4-*tert*-butylbenzyl)[(1,3-thiazol-5-ylmethoxy) carbonyl]amino}propyl)carbamate;
   *tert*-butyl 4-benzoylbenzyl(3-{(4-benzoylbenzyl)[(1,3-thiazol-5-ylmethoxy)carbonyl]amino} propyl)carbamate;
   1,3-thiazol-5-ylmethyl 1,1'-biphenyl-4-ylmethyl(3-{(1,1'-biphenyl-4-ylmethyl)[(1,3-thiazol-5-yl methoxy)carbonyl]amino}propyl)carbamate;
   1,3-thiazol-5-ylmethyl benzyl{3-[benzyl(pyridin-4-ylmethyl)amino]propyl}carbamate;
   ethyl *N*-(3-{(ethoxycarbonylmethyl)[(1,3-thiazol-5-ylmethoxy)carbonyl]amino}propyl)-*N*-[(1,3-thiazol-5-ylmethoxy)carbonyl]glycinate;
   1,3-thiazol-5-ylmethyl benzyl(3-{benzyl[(5-methylthien-2-yl)methyl]amino} propyl)carbamate;
   1,3-thiazol-5-ylmethyl benzyl{3-[benzyl(2-furylmethyl)amino]propyl}carbamate;
   1,3-thiazol-5-ylmethyl benzyl{3-[benzyl(2-naphthylmethyl)amino]propyl}carbamate;
   methyl 4-({(3-{(tert-butoxycarbonyl)[4-(methoxycarbonyl)benzyl]amino}propyl)[(1,3-thiazol-5-ylmethoxy)carbonyl]amino}methyl)benzoate;
   1,3-thiazol-5-ylmethyl benzyl{3-[benzyl(neopentyl)amino]propyl}carbamate;
   *tert*-butyl benzyl(3-{benzyl[(1,3-thiazol-5-ylmethoxy)carbonyl]amino}propyl)carbamate;
   benzyl benzyl(3-{benzyl[(1,3-thiazol-5-ylmethoxy)carbonyl]amino}propyl)carbamate;
   methyl benzyl(3-{benzyl[(1,3-thiazol-5-ylmethoxy)carbonyl]amino}propyl)carbamate;
   1,3-thiazol-5-ylmethyl benzyl[3-(benzyl{4-[3-(dimethylamino)propoxy]benzyl}amino)propyl] carbamate;
   1,3-thiazol-5-ylmethyl benzyl{3-[benzyl(4-pyridin-2-ylbenzyl)amino]propyl}carbamate;
   *tert*-butyl 3-{[(1,3-thiazol-5-ylmethoxy)carbonyl][4-(1*H*-1,2,4-triazol-1-yl)benzyl]amino}propyl [4-(1*H*-1,2,4-triazol-1-yl)benzyl]carbamate;
   5-{benzyl[(1,3-thiazol-5-ylmethoxy)carbonyl]amino}-2-[(tert-butoxycarbonyl)amino]-1,2,3,5-tetradeoxy-1-phenyl-D-glycero-pentitol;
   *tert*-butyl 1,1'-biphenyl-4-ylmethyl(3-{(1,1'-biphenyl-4-ylmethyl)[(1,3-thiazol-5-ylmethoxy) carbonyl]amino}propyl)carbamate;
   *tert*-butyl 4-pyridin-2-ylbenzyl(3-{(4-pyridin-2-ylbenzyl)[(1,3-thiazol-5-ylmethoxy)carbonyl] amino}propyl)carbamate;
   ethyl N-[(1,3-thiazol-5-ylmethoxy)carbonyl]-N-(3-{[(1,3-thiazol-5-ylmethoxy)carbonyl]amino} propyl)glycinate;
   1,3-thiazol-5-ylmethyl benzyl{3-{benzyl[(1,3-thiazol-5-ylmethoxy) carbonyl]amino}-2-[(phenoxycarbonyl)amino]propyl}carbamate;
   1,3-thiazol-5-ylmethyl benzyl[3-{benzyl[(1,3-thiazol-5-ylmethoxy)carbonyl] amino}-2-(isobutyrylamino)propyl]carbamate;
   1,3-thiazol-5-ylmethyl 3-hydroxy-4-{[*N*-(methoxycarbonyl)-3-methylvalyl]amino}-5-phenyl-1-(4-pyridin-2-ylbenzyl)pentylcarbamate;
   methyl 1-({[3-hydroxy-4-({3-methyl-*N*-[(1,3-thiazol-5-ylmethoxy)carbonyl]valyl}amino)-5-phenyl-1-(4-pyridin-2-ylbenzyl)pentyl]amino}carbonyl)-2,2-dimethylpropylcarbamate;
   methyl 1-({[3-hydroxy-4-({3-methyl-*N*-[(1,3-thiazol-5-ylmethoxy)carbonyl]-valyl}amino)-5-phenyl-1-(4-pyridin-2-ylbenzyl)pentyl]amino}carbonyl)-2,2-dimethylpropylcarbamate;
   *tert*-butyl 3-hydroxy-5-phenyl-1-(4-pyridin-2-ylbenzyl)-4-{[(1,3-thiazol-5-ylmethoxy)carbonyl] amino}pentylcarbamate;
   *tert*-butyl 3-hydroxy-4-({3-methyl-*N*-[(1,3-thiazol-5-ylmethoxy)carbonyl]valyl}amino)-5-phenyl-1-(4-pyridin-2-ylbenzyl)pentylcarbamate;
   *tert*-butyl 3-hydroxy-5-phenyl-1-(4-pyridin-2-ylbenzyl)-4-({*N*-[(1,3-thiazol-5-ylmethoxy) carbonyl]alanyl}amino)pentylcarbamate;
   methyl 3-hydroxy-4-({3-methyl-*N*-[(1,3-thiazol-5-ylmethoxy)carbonyl]-valyl}amino)-5-phenyl-1-(4-pyridin-2-ylbenzyl)pentylcarbamate;
   methyl 3-hydroxy-5-phenyl-1-(4-pyridin-2-ylbenzyl)-4-({*N*-[(1,3-thiazol-5-ylmethoxy)carbonyl]-alanyl}amino)pentylcarbamate;
   *N*¹-(3-hydroxy-5-phenyl-1-(4-pyridin-2-ylbenzyl)-4-{[(1,3-thiazol-5-ylmethoxy)carbonyl] amino}pentyl)-*N*²-(methoxycarbonyl)-3-methylvalinamide;
   1,3-thiazol-5-ylmethyl 1-benzyl-3-hydroxy-5-phenyl-4-{[(1,3-thiazol-5-ylmethoxy)carbonyl]amino}pentylcarbamate;
   N¹-{1-benzyl-4-[(tert-butoxycarbonyl)ammo]-2-hydroxy-5-phenylpentyl}-3-methyl-N²-[(1,3-thiazol-5-ylmethoxy)carbonyl]valinamide;
   N¹-(1-benzyl-2-hydroxy-5-phenyl-4-{[(1,3-thiazol-5-ylmethoxy)carbonyl]amino}pentyl)-N²-(methoxycarbonyl)-3-methylvalinamide;
   1,3-thiazol-5-ylmethyl 4-(acetylamino)-1-benzyl-2-hydroxy-5-phenylpentylcarbamate;
   1,3-thiazol-5-ylmethyl 4-amino-1-benzyl-2-hydroxy-5-phenylpentylcarbamate;
   3-(acetylamino)-4-phenyl-1-((1S)-2-phenyl-1-{[(1,3-thiazol-5-ylmethoxy)carbonyl]amino} ethyl)butyl acetate;
   1,3-thiazol-5-ylmethyl 1-benzyl-2-hydroxy-4-[(methylsulfonyl)amino]-5-phenylpentylcarbamate;
   1,3-thiazol-5-ylmethyl 1-benzyl-4-{[(dimethylamino)carbonyl]amino}-2-hydroxy-5-phenylpentyl carbamate;
   methyl 1-benzyl-3-hydroxy-5-phenyl-4-{[(1,3-thiazol-5-ylmethoxy)carbonyl]amino}pentyl carbamate;
   1,3-thiazol-5-ylmethyl 1-benzyl-4-{[(dimethylamino)sulfonyl]amino}-2-hydroxy-5-phenylpentyl carbamate;
   *tert*-butyl 1-benzyl-3-hydroxy-5-phenyl-4-{[(1,3-thiazol-5-ylmethoxy)carbonyl]amino}pentyl carbamate;
   1,3-thiazol-5-ylmethyl 1-benzyl-2-hydroxy-4-{[(2S)-3-methyl-2-(2-oxotetrahydropyrimidin-1(2H)-yl)butanoyl]amino}-5-phenylpentylcarbamate;
   isobutyl 1-benzyl-3-hydroxy-5-phenyl-4-{[(1,3-thiazol-5-ylmethoxy)carbonyl]amino}pentyl carbamate;
   isopropyl 1-benzyl-3-hydroxy-5-phenyl-4-{[(1,3-thiazol-5-ylmethoxy)carbonyl]amino}pentyl carbamate;
   *tert*-butyl 1-benzyl-3-hydroxy-5-phenyl-4-{[(1,3-thiazol-5-ylmethoxy)carbonyl]amino}pentyl carbamate;
   1,3-thiazol-5-ylmethyl 4-benzyl-5-{2-[4-isopropyl-2,5-dioxoimidazolidin-1-yl]-3-phenylpropyl}-2-oxo-1,3-oxazolidine-3-carboxylate;
   N¹-(1-benzyl-3-hydroxy-5-phenyl-4-{[(1,3-thiazol-5-ylmethoxy)carbonyl]amino}pentyl)valin amide;
   N¹-(1-benzyl-3-hydroxy-5-phenyl-4-{[(1,3-thiazol-5-ylmethoxy)carbonyl] amino}pentyl)-N²-[(dimethylamino)carbonyl]valinamide;
   N¹-(1-benzyl-3-hydroxy-5-phenyl-4-{[(1,3-thiazol-5-ylmethoxy)carbonyl] amino}pentyl)-N²-[(methylamino)cabonyl]valinamide;
   N¹-(1-benzyl-3-hydroxy-5-phenyl-4-{[(1,3-thiazol-5-ylmethoxy)carbonyl]amino}pentyl)-N²-[(2-hydroxypropoxy)carbonyl]valinamide;
   1,3-thiazol-5-ylmethyl 1-[4-benzyl-2-oxo-1,3-oxazinan-6-yl]-2-phenylethylcarbamate;
   N¹-(1-benzyl-3-hydroxy-5-phenyl-4-{[(1,3-thiazol-5-ylmethoxy)carbonyl]amino}pentyl)-N²-[(2,3-dihydroxypropoxy)carbonyl]valinamide;
   1,3-thiazol-5-ylmethyl 1-benzyl-2-hydroxy-5-phenyl-4-{[(4-phenylpiperazin-1-yl)carbonyl] amino}pentylcarbamate;
   N¹-(1-benzyl-3-hydroxy-5-phenyl-4-{[(3H-1lambda∼4∼,3-thiazol-4-ylmethoxy)carbonyl]amino} pentyl)-N²-{[[(2-isopropyl-1,3-thiazol-4-yl)methyl](methyl)amino]carbonyl}valinamide;
   1,3-thiazol-4-ylmethyl 1-benzyl-3-hydroxy-5-phenyl-4-{[(1,3-thiazol-4-ylmethoxy)carbonyl] amino}pentylcarbamate;
   1,3-thiazol-5-ylmethyl 1-benzyl-2-hydroxy-4-[4-isopropyl-2,5-dioxoimidazolidin-1-yl]-5-phenylpentylcarbamate;
   1,3-thiazol-5-ylmethyl 1-benzyl-2-hydroxy-4-{[(2-isopropyl-1,3-thiazol-4-yl)acetyl]amino}-5-phenylpentylcarbamate,
   1,3-thiaxol-5-ylmethyl 1-benzyl-4-{[(2,6-dimethylphenoxy)acetyl]amino}-2-hydroxy-5-phenyl pentylcarbamate;
   1,3-thiazol-5-ylmethyl 1-benzyl-4-{[(tert-butylamino)carbonyl]amino}-2-hydroxy-5-phenyl pentylcarbamate;
   N¹-(1-benzyl-3-hydroxy-5-phenyl-4-{[(1,3-thiazol-5-ylmethoxy)carbonyl]amino} pentyl)-N²-(methoxycarbonyl)-3-methylvalinamide;
   1,3-thiazol-5-ylmethyl 1-benzyl-4-{[(2,6-dimethylphenoxy)acetyl]amino}-3-hydroxy-5-phenyl pentylcarbamate;
   *tert*-butyl 1-benzyl-2-hydroxy-3-{isobutyl[(1,3-thiazol-5-ylmethoxy)carbonyl]amino}propyl carbamate;
   M,N-dimethyl-N-[(1,3-thiazol-5-ylmethoxy)carbonyl]phenylalaninamide;
   *N*-[1-benzyl-2-morpholin-4-yl-2-oxoethyl]-*N*-[(1,3-thiazol-5-ylmethoxy) carbonyl]amine;
   N,N-diisobutyl-N-[(1,3-thiazol-5-ylmethoxy)carbonyl]phenylalaninamide;
   N-isobutyl-N-[(1,3-thiazol-5-ylmethoxy)carbonyl]phenylalaninamide;
   N,N-dibenzyl-N-[(1,3-thiazol-5-ylmethoxy)carbonyl]phenylalaninamide;
   N-benzyl-N-(2-phenylethyl)-N-[(1,3-thiazol-5-ylmethoxy)carbonyl] phenylalaninamide;
   *tert*-butyl 1-benzyl-3-{benzyl[(1,3-thiaxol-5-ylmethoxy)carbonyl]amino}-2-hydroxypropyl carbamate;
   *tert*-butyl (1-benzyl-3-{benzyl[(1,3-thiazol-5-ylmethoxy)carbonyl]amino}-2-hydroxypropyl carbamate;
   *tert*-butyl benzyl(2-hydroxy-4-phenyl-3-{[(1,3-thiazol-5-ylmethoxy)carbonyl] amino} butyl)carbamate;
   [3-(2-tert-Butoxycarbonylamino-3-phenyl-propionylamino)-phenyl]-carbamic acid thiazol-5-ylmethyl ester;
   1,3-thiazol-5-ylmethyl 3-({2-[(tert-butoxycarbonyl)amino]-2-phenylethanoyl}amino)phenyl carbamate;
   N²-(tert-butoxycarbonyl)-N¹-(3-{[(1,3-thiazol-5-ylmethoxy) carbonyl]amino}phenyl)isoleucin amide;
   N²-(tert-butoxycarbonyl)-N¹-(3-{[(1,3-thiazol-5-ylmethoxy) carbonyl]amino}phenyl)valinamide,
   N²-(tert-butoxycarbonyl)-3-methy-N¹-(3-{[(1,3-thiazol-5-ylmethoxy) carbonyl]amino}phenyl) valinamide;
   1,3-thiazol-5-ylmethyl 3-({2-[(tert-butoxycarbonyl)amino]-2-cyclohexylethanoyl}amino)phenyl carbamate;
   1,3-thiazol-5-ylmethyl 1-benzyl-2-hydroxy-3-[isobutyl(4-pyridin-2-ylbenzyl)amino]propyl carbamate;
   tert-butyl 2-(2-hydroxy-4-phenyl-3-{[(1,3-thiazol-5-ylmethoxy)carbonyl] amino}butyl)-2-(4-pyridin-2-ylbenzyl)hydrazinecarboxylate;
   methyl N-[(1,3-thiazol-5-ylmethoxy)carbonyl]phenylalaninate;
   methyl N-[(1,3-thiazol-5-ylmethoxy)carbonyl]-L-phenylalaninate; and
   1,3-thiazol-5-ylmethyl 1,1-dibenzylbut-3-enylcarbamate.
28. A pharmaceutical composition comprising a compound, salt, solvate or prodrug according to one of items 1-27.
29. The pharmaceutical composition of item 28, further comprising a drug which is metabolizable by a CYP enzyme.
30. The pharmaceutical composition of item 29, wherein said CYP enzyme is CYP3A4, CYP2D6 or CYP2C9.
31. The pharmaceutical composition according to item 29 or 30, wherein said drug is an antiviral drug.
32. A method for inhibiting a metabolizing activity of a CYP enzyme, comprising contacting the CYP enzyme with a compound, salt, solvate or prodrug according to one of items 1-27, thereby inhibiting the metabolizing activity of said CYP enzyme.
33. A method for inhibiting a metabolizing activity of a CYP enzyme in a subject of interest, comprise administering to the subject an effective amount of a compound, salt, solvate or prodrug according to one of items 1-27, thereby inhibiting the metabolizing activity of said CYP enzyme in the subject.
34. A method for improving pharmacokinetics of a drug that is metabolizable by a CYP enzyme, comprising administering said drug and an effective amount of a compound, salt, solvate or prodrug according to one of items 1-27 to a subject in need thereof, thereby improving the pharmacokinetics of said drug in the subject.
35. A method for increasing blood or liver level of a drug that is metabolizable by a CYP enzyme, comprising administering said drug and an effective amount of a compound, salt, solvate or prodrug according to one of items 1-27 to a subject in need thereof, thereby increasing the blood or liver level of said drug in the subject.
36. The method as in one of items 32-34, wherein said CYP enzyme is CYP3A4, CYP2D6 or CYP2C9.
37. The method as in one of items 34-35, wherein said drug is an antiviral drug,
38. The use of a compound, salt, solvate or prodrug according to one of items 1-27 for the manufacture of a medicament for the treatment of a disorder, wherein said medication comprises a drug that is effective in treating said disorder and is metabolizable by a CYP enzyme.
39. The use of item 38, wherein said CYP enzyme is CYP3A4, CYP2D6 or CYP2C9.
40. A compound, pharmaceutically acceptable salt, solvate, prodrug, pharmaceutical composition, use, method, or process as described in paragraphs 9-65 and 187-252 and Examples 1-100 of the specification.

## Claims

1. A compound of formula I, 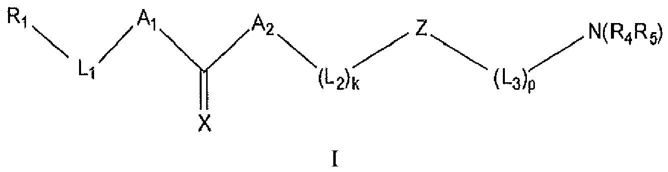 or a pharmaceutically acceptable salt, solvate or prodrug thereof, wherein
R₁ is a 5-membered heterocyclyl comprising at least one nitrogen ring atom;
L₁ is a C₁-C₁₀alkylene;
A₁ is -O-L_{A1}- or -N(R_{A1}) -L_{A1}, wherein L_{A1} is a bond and R_{A1} is C₁-C₆ alkyl;
X is O;
A₂ is -L_{A2}-N(R_{A2})-, wherein L_{A2} is a bond, and R_{A2} is hydrogen;
k is 0 or 1, and at each occurrence L₂ independently represents -L₉-V-L_{9'}-, wherein L₉ is C₁-C₁₀alkylene optionally substituted with heterocyclyl and L_{9'} is a bond, and V is - C(O)N(R_{V})-, wherein R_{V} is hydrogen;;
Z is -C(R₂R₃)-, wherein R₂ is carbocyclylC₁-C₆alkyl, and R₃ is hydrogen;
p is 1, and at each occurrence L₃ independently represents -L₅-W-L_{5'}-, wherein W is a bond, wherein L₅ and L_{5'} are each independently selected at each occurrence from a bond, or C₁-C₁₀alkylene, and are each independently optionally substituted at each occurrence with 1 or 2 substituents each of which is independently selected at each occurrence from the group consisting of hydroxy and carbocyclyl;
R₄ and R₅ are each independently selected from the group consisting of hydrogen, -L₆-C(O)OR₈, -L₆-C(O)NR₈R₉ and -L₆-C(O)-L_{6'}-N(R₉)C(O)NR₈R₁₀, wherein L₆ and L_{6'} are each independently selected at each occurrence from a bond or C₁-C₁₀alkylene, wherein R₈, R₉ and R₁₀ are each independently selected at each occurrence from the group consisting of hydrogen, C₁-C₆ alkyl, and heterocycloC₁-C₆alkyl;
wherein at each occurrence L_{6'} is optionally substituted with at least one substituent selected from the group consisting of heterocyclyl;
wherein each carbocyclyl and heterocyclyl moiety in said compound is independently optionally substituted at each occurrence with C₁-C₆alkyl; and
with the proviso that said compound is not ritonavir ((2S,3S,5S)-5-(N-(N-((N-Methyl-N-((2-isopropyl-4-thiazolyl)methyl)amino)carbonyl)-L-valinyl)amino)-2-(N-((5-thiazolyl)methoxycarbonyl)amino)-1,6-diphenyl-3-hydroxyhexane).

2. A compound of claim 1, or a pharmaceutically acceptable salt, solvate or prodrug thereof, wherein R₁ is thiazolyl.

3. A compound of claim 1, or a pharmaceutically acceptable salt, solvate or prodrug thereof, wherein L₁ is methylene.

4. A compound of claim 1, or a pharmaceutically acceptable salt, solvate or prodrug thereof, wherein R₂ is benzyl.

5. A compound of claim 1, or a pharmaceutically acceptable salt, solvate or prodrug thereof, wherein L₅ is a C₁-C₁₀alkylene optionally substituted with hydroxy.

6. A compound of claim 1, or a pharmaceutically acceptable salt, solvate or prodrug thereof, wherein L_{5'} is a C₁-C₁₀alkylene substituted with carbocyclyl.

7. A compound of claim 6, or a pharmaceutically acceptable salt, solvate or prodrug thereof, wherein the carbocyclyl is phenyl.

8. A compound of claim 1, or a pharmaceutically acceptable salt, solvate or prodrug thereof, wherein R₄ is -L₆-C(O)NR₈R₉, wherein L₆ is a bond, R₈ is C₁-C₆ alkyl, and R₉ is hydrogen or C₁-C₆ alkyl; and wherein R₅ is hydrogen.

9. A compound of claim 1, or a pharmaceutically acceptable salt, solvate or prodrug thereof, wherein R₄ is -L₆-C(O)-L_{6'}-N(R₉)C(O)NR₈R₁₀, wherein L₆ is a bond, L_{6'} is C₁-C₁₀alkylene, R₉ is hydrogen, R₈ is C₁-C₆alkyl, and R₁₀ is heterocycloC₁-C₆alkyl.

10. A compound of claim 9, or a pharmaceutically acceptable salt, solvate or prodrug thereof, wherein L_{6'} is substituted with heterocyclyl.

11. A compound of claim 9, or a pharmaceutically acceptable salt, solvate or prodrug thereof, wherein the heterocyclyl moiety is substituted with C₁-C₆alkyl.

12. A compound of claim 1, or a pharmaceutically acceptable salt, solvate or prodrug thereof, wherein
R₁ is thiazolyl;
L₁ is methylene;
R₂ is benzyl;
L₅ is a C₁-C₁₀alkylene; and
L_{5'} is a C₁-C₁₀alkylene substituted with phenyl.

13. A compound of claim 12, or a pharmaceutically acceptable salt, solvate or prodrug thereof, wherein L₅ is substituted with hydroxyl.

14. A compound of claim 12, or a pharmaceutically acceptable salt, solvate or prodrug thereof, wherein
R₄ is -L₆-C(O)NR₈R₉, wherein L₆ is a bond, R₈ is C₁-C₆ alkyl, and R₉ is hydrogen or C₁-C₆ alkyl; and
R₅ is hydrogen.

15. A compound of claim 12, or a pharmaceutically acceptable salt, solvate or prodrug thereof, wherein R₄ is -L₆-C(O)-L_{6'}-N(R₉)C(O)NR₈R₁₀, wherein
L₆ is a bond,
L_{6'} is C₁-C₁₀alkylene,
R₉ is hydrogen,
R₈ is C₁-C₆ alkyl, and
R₁₀ is hydrogen or C₁-C₆alkyl.

16. A compound of claim 12, or a pharmaceutically acceptable salt, solvate or prodrug thereof, wherein R₄ is -L₆-C(O)-L_{6'}-N(R₉)C(O)NR₈R₁₀, wherein
L₆ is a bond,
L_{6'} is C₁-C₁₀alkylene substituted with heterocyclyl,
R₉ is hydrogen,
R₈ is C₁-C₆ alkyl, and
R₁₀ is heterocycloC₁-C₆alkyl substituted with C₁-C₆alkyl.

17. A compound of claim 1 selected from the group consisting of:
1,3-thiazol-5-ylmethyl (1*S*,2*S*,4*S*)-1-benzyl-4-{[(*tert*-butylamino)carbonyl]amino}-2-hydroxy-5-phenylpentylcarbamate;
1,3-thiazol-5-ylmethyl 1-benzyl-4-{[(*tert*-butylamino)carbonyl]amino}-2-hydroxy-5-phenylpentylcarbamate;
1,3-thiazol-5-ylmethyl (1*S*,2*S*,4*S*)-1-benzyl-4-{[(dimethylamino)carbonyl]amino}-2-hydroxy-5-phenylpentylcarbamate;
1,3-thiazol-5-ylmethyl 1-benzyl-4-{[(dimethylamino)carbonyl]amino}-2-hydroxy-5-phenylpentylcarbamate;
*N*¹-((1*S*,3*S*,4*S*)-1-benzyl-3-hydroxy-5-phenyl-4-{[(1,3-thiazol-5-ylmethoxy)carbonyl] amino}pentyl)-*N*²-[(dimethylamino)carbonyl]-L-valinamide;
*N*¹-((1*S*,3*S*,4*S*)-1-benzyl-3-hydroxy-5-phenyl-4-{[(1,3-thiazol-5-ylmethoxy)carbonyl]amino}pentyl)-*N*²-[(methylamino)carbonyl]valinamide;
*N*¹-(1-benzyl-3-hydroxy-5-phenyl-4-{[(1,3-thiazol-5-ylmethoxy)carbonyl]amino}pentyl)-*N*²-[(methylamino)carbonyl]valinamide;
*N*¹-(1-benzyl-3-hydroxy-5-phenyl-4-{[(1,3-thiazol-5-ylmethoxy)carbonyl]amino}pentyl)-*N*²-[(dimethylamino)carbonyl]valinamide;
*N*¹-((1*S*,3*S*,4*S*)-1-benzyl-3-hydroxy-5-phenyl-4-{[(1,3-thiazol-4-ylmethoxy)carbonyl]amino}pentyl)-*N*²-{[[(2-isopropyl-1,3-thiazol-4-yl)methyl](methyl)amino]carbonyl}-L-valinamide; and
*N*¹-(1-benzyl-3-hydroxy-5-phenyl-4-1{[(3*H*-1lambda⁴,3-thiazol-4-ylmethoxy)carbonyl]amino}pentyl)-*N*²-{[[(2-isopropyl-1,3-thiazol-4-yl)methyl](methyl)amino]carbonyl} valinamide.

18. A pharmaceutical composition comprising a compound of claim 1, or a pharmaceutically acceptable salt, solvate or prodrug thereof.

19. The pharmaceutical composition of claim 18, further comprising a drug which is metabolizable by a CYP enzyme.

20. The pharmaceutical composition of claim 19, wherein said CYP enzyme is CYP3A4, CYP2D6 or CYP2C9.

21. The pharmaceutical composition of claim 19, wherein said drug is an antiviral drug.

22. The pharmaceutical composition of claim 21, wherein said antiviral drug is an HCV protease inhibitor.

23. A compound of claim 1, or salt, solvate or prodrug thereof for use in inhibiting a metabolizing activity of a CYP enzyme.

24. The compound for use of claim 23, wherein said CYP enzyme is CYP3A4, CYP2D6 or CYP2C9.

25. A compound of claim 1, or salt, solvate or prodrug thereof for use in improving pharmacokinetics of a drug that is metabolizable by a CYP enzyme.

26. The compound for use of claim 25, wherein said CYP enzyme is CYP3A4, CYP2D6 or CYP2C9.

27. The compound for use of claim 25, wherein said drug is an antiviral drug.

28. The compound for use of claim 27, wherein said antiviral drug is an HCV protease inhibitor.

29. A compound of claim 1, or salt, solvate or prodrug thereof for use in increasing blood or liver level of a drug that is metabolizable by a CYP enzyme.

30. The compound for use of claim 29, wherein said CYP enzyme is CYP3A4, CYP2D6 or CYP2C9.

31. The compound for use of claim 29, wherein said drug is an antiviral drug.

32. The compound for use of claim 31, wherein said antiviral drug is an HCV protease inhibitor.
